# EUROPÄISCHE PATENTSCHRIFT

(11) **EP 1 751 127 B1**
(45) Veröffentlichungstag und Bekanntmachung des Hinweises auf die Patenterteilung: **11.07.2012**
(21) Anmeldenummer: 05747405.8
(22) Anmeldetag: 14.05.2005
(51) Int. Cl.: C07D 409/10, C07D 405/12, C07D 405/10, C07D 417/12, C07D 307/93

(54) **NEUE CYLOPENTA[B]BENZOFURAN-DERIVATE UND IHRE VERWENDUNG**
NOVEL CYLOPENTA[B]BENZOFURAN DERIVATIVES AND THE UTILIZATION THEREOF
NOUVEAUX DERIVES DE CYLOPENTA[B]BENZOFURANE ET LEUR UTILISATION

(30) Priorität: 18.05.2004 DE 102004024504
(43) Veröffentlichungstag der Anmeldung: 14.02.2007
(73) Patentinhaber: InterMed Discovery GmbH, 44227 Dortmund (DE)
(72) Erfinder: DIEDRICHS, Nicole, 42553 Velbert (DE); FAHRIG, Thomas, 51429 Bergisch Gladbach (DE); GERLACH, Irene, 79540 Lörrach (DE); RAGOT, Jacques, 40625 Düsseldorf (DE); SCHUHMACHER, Joachim, 42113 Wuppertal (DE); THEDE, Kai, 42115 Wuppertal (DE); HORVÁTH, Ervin, 51373 Leverkusen (DE)
(74) Vertreter: Bülle, Jan
(86) Internationale Anmeldenummer: PCT/EP2005/005298
(87) Internationale Veröffentlichungsnummer: WO 2005/113529

(56) Entgegenhaltungen:
- WO-A-00/07579
- WO-A-00/08007
- DIEDRICHS, NICOLE ET AL: "A highly efficient synthesis of rocaglaols by a novel .alpha.-arylation of ketones" EUROPEAN JOURNAL OF ORGANIC CHEMISTRY , (9), 1731-1735 CODEN: EJOCFK; ISSN: 1434-193X, 2005, XP002353478

## Beschreibung

Die vorliegende Anmeldung betrifft Cyclopenta[*b*]benzofuran-Derivate, Verfahren zu ihrer Herstellung sowie ihre Verwendung zur Herstellung von Arzneimitteln, insbesondere zur Prophylaxe und/oder Therapie akuter oder chronischer Erkrankungen, die gekennzeichnet sind durch erhöhten zellulären Stress, durch lokale oder systemische Entzündungsprozesse oder durch Hyperproliferation.

Die erfindungsgemäßen Verbindungen leiten sich ab von einer als Rocaglaole / Rocaglamide bezeichneten Klasse von Naturstoffen, die aus unterschiedlichen Arten der Pflanze *Aglaia* extrahiert werden können. Seit der Erst-Isolation eines als Rocaglamid benannten Dihydrocyclopentabenzofuranol-Derivates (J. Chem. Soc., Chem. Commun. 1982, 1150; US 4,539,414) sind mehrere neue, auch synthetisch hergestellte Derivate und deren biologische Wirkung beschrieben worden (vgl. z.B. J. Chem. Soc., Chem. Commun. 1991, 1137; Phytochemistry 32, 307 (1993); WO 96/04284; Tetrahedron 52, 6931 (1996); Phytochemistry 44, 1455 (1997); Phytochemistry 45, 1579 (1997); Tetrahedron 53, 17625 (1997); JP 11012279; WO 97/08161; WO 00/07579; WO 00/08007; DE 199 34 952-A1).

Die Wirkung von Cyclopentabenzofuran-Derivaten als Inhibitoren der "Nuclear Factor kappa B" (NF-κB)-vermittelten Signalübertragung wurde bereits beschrieben [WO 00/08007; WO 00/07579; J. Biol. Chem. 277, 44791 (2002)]. NF-κR ist ein Transkriptionsfaktor, der eine zentrale Rolle bei Entzündungsprozessen, aber auch bei der Cancerogenese innehat. In seiner aktiven, DNAbindenden Form setzt er sich aus dimeren Kombinationen unterschiedlicher Mitglieder der NF-κB/Rel-Familie von Proteinen zusammen [Ann. Rev. Immunol. 16, 225 (1998)]. Unter basalen, nicht-stimulierten Bedingungen liegt NF-κB durch Bindung an ein inhibitorisches Protein (I-κB) als zytoplasmatische, inaktive Form vor. Nach Stimulation kommt es zur schnellen Phosphorylierung von I-κB durch I-κB-Kinasen und infolgedessen zum proteolytischen Abbau von I-κB. Dadurch wird NF-κB in seiner aktiven Form freigesetzt und dessen Translokation in den Zellkern ermöglicht. In seiner Eigenschaft als Transkriptionsfaktor aktiviert oder moduliert NF-κB die Expression verschiedener Gene, im besonderen solcher, deren Produkte für entzündliche Reaktionen und für Zellwachstum und -differenzierung verantwortlich sind [J. Biol. Chem. 274, 27339 (1999)].

Überraschenderweise wurde nun gefunden, dass die erfindungsgemäßen Verbindungen außerdem die Aktivität eines zweiten Transkriptionsfaktor-Komplexes, des "Activator Protein-1" (AP-1), inhibieren. AP-1 ist ein aus Dimeren der Jun-, Fos-, Maf- und ATF-Familie von Proteinen zusammengesetzter Transkriptionsfaktor, der im Zellkern lokalisiert ist. Die Aktivität von AP-1 wird über eine Reihe unterschiedlichster Stimuli induziert, unter anderem durch Zytokine, bakterielle und virale Infektionen und durch verschiedene Formen von physikalischem oder chemischem Stress. Aktivierende Signale führen einerseits zu einer vermehrten Bildung der Einzelkomponenten des Transkriptionsfaktors, andererseits durch Stimulation bestimmter Kinasen, wie z.B. Jun-Kinasen, zur Phosphorylierung spezifischer Aminosäuren. Beide Prozesse führen zu einer verstärkten Wechselwirkung von AP-1 mit dessen Zielgenen und ermöglichen so deren Expression oder Modulation. Zu diesen gehören neben Genen, deren Produkte in Entzündungsprozessen involviert sind, auch solche, die die Zellteilung steuern oder als Regulatoren von Zelltod oder -überleben wirken [Curr. Opin. Cell Biol. 9, 240 (1997); Nature Cell Biol. 4, E131 (2002)].

Einerseits sind pro-inflammatorische Zytokine, wie z.B. Interleukin-1 (IL-1) oder Tumor Nekrose Faktor (TNF), und oxidativer Stress potente Aktivatoren der NF-κB- und AP-1-vermittelten Signalübertragung. Andererseits bewirkt die Aktivierung von NF-κB und/oder AP-1 die Neubildung verschiedener Zytokine (wie z.B. IL-1 und TNF), unterschiedlicher Chemokine (wie z.B. Interleukin-8 (IL-8) und "Monocyte Chemoattractant Protein-1" (MCP-1)) und verschiedener Enzyme (wie z.B. Cyclooxygenase-2 oder "Nitric Oxide Synthase-2" (NOS-2, iNOS)). Die Hauptfunktion der neugebildeten Peptide/Proteine oder der durch die Aktivität der neugebildeten Enzyme entstehenden Endprodukte ist die Rekrutierung und Aktivierung von Entzündungszellen. NF-κB und AP-1 sind damit zentrale Faktoren bei der Induktion und Aufrechterhaltung von Entzündungsprozessen.

Die Pathogenese oder Pathophysiologie einer Vielzahl von Erkrankungen ist charakterisiert durch akute, überschießende oder chronische Entzündungsreaktionen, die lokal auf ein Gewebe beschränkt oder systemischer Natur sein können. Diese Krankheiten zeichnen sich durch lokal oder systemisch erhöhte Zytokin- und/oder Chemokinspiegel sowie durch eine erhöhte Präsenz von verschiedenartigen Entzündungszellen aus, wie z.B. Makrophagen, polymorphkernige Leukozyten, T-Lymphozyten oder B-Zellen. Zu diesen Erkrankungen gehören chronische Entzündungs-und Autoimmunkrankheiten (wie z.B. die Crohn'sche Krankheit, ulzerative Colitis, rheumatoide Arthritis, Psoriasis, Multiple Sklerose, Lupus, Asthma, Diabetes), kardiovaskuläre Erkrankungen (wie z.B. koronare Herzerkrankungen, Myokard-Infarkt, Artherosklerose, Restenose, Thrombosen), fibrotische Erkrankungen der Leber und anderer Organe, cerebrovaskuläre Erkrankungen (wie z.B. Schlaganfall, Schädel-Hirn-Trauma, Rückenmarksverletzungen) und chronische neurodegenerative Erkrankungen (wie z.B. die Alzheimer'sche Krankheit, die Parkinson'sche Krankheit, Chorea Huntington, Amyotrophe Lateralsklerose, periphere Neuropathien und chronischer Schmerz). Eine fehlregulierte oder überschießende Zytokin/Chemokin-Bildung ist ebenfalls ursächlich verknüpft mit der Entstehung oder den Folgen von Strahlenschäden, Transplantatabstoßung, Sepsis und septischem Schock, sowie bakterieller Meningitis. Die Inhibition oder Modulation der transkriptionellen Aktivität von NF-κB und/oder AP-1, wie für die erfmdungsgemäßen Verbindungen beschrieben, könnte somit ein erfolgsversprechendes neues Therapieprinzip für die zuvor aufgeführten Erkrankungen darstellen.

Neben ihrer zentralen Funktion in Entzündungsprozessen haben NF-κB und AP-1 wesentliche Bedeutung bei der Regulation von Zellteilung, Zellwachstum und Zelldifferenzierung. Bei der Bildung und beim Wachstum von Tumoren werden zelluläre Signalwege aktiviert, die unter normalen Bedingungen Zellwachstum, Differenzierung und andere biologische Vorgänge steuern. Eine Vielzahl Tumor-induzierender Substanzen und Faktoren (wie z.B. Epidermaler Wachstumsfaktor (EGF), Phorbolester, UV-Strahlung) führen zur Aktivierung von NF-κB und/oder AP-1, und eine Reihe der durch NF-κB und/oder AP-1 gesteuerten Gene gehören zu den Oncogenen (wie z.B. c-myc, c-rel, Melanoma Growth Stimulating Activity (MGSA)). Durch ihre inhibitorische/modulatorische Aktivität auf die NF-κB- und/oder AP-1-vermittelte Signalübertragung könnte die Verwendung der erfindungsgemäßen Verbindungen damit ein neues Therapieprinzip zur Behandlung hyperproliferativer Erkrankungen wie solider Tumoren (wie z.B. Brustkrebs, Lungenkrebs, Tumore des Gehirns und des Nervensystems, Hautkrebs, Leberkrebs, Tumore der Reproduktionsorgane, Tumore des Verdauungstraktes, Blasenkrebs, Tumore der Harnwegssysteme, Tumore verschiedener Hormondrüsen, Tumore des Auges), Lymphomen (wie z.B. Hodgkin'sche Krankheit, Lymphome des zentralen Nervensystems), Sarkomen (wie z.B. Osteaosarkome, Lymphosarkome) und Leukämien (wie z.B. akute myeloide Leukämie, lymphoblastische Leukämien, myelogene Leukämien) darstellen.

NF-κB und AP-1 spielen außerdem bei der Replikation lymphotropher Viren wie HIV, HTLV und Epstein-Barr-Virus eine wesentliche Rolle. Die Aktivierung viraler, für die Replikation notwendiger Gene kann über die Virus-mediierte Aktivierung von NF-κR und/oder AP-1 in der Wirtszelle bewirkt werden. Neben der Bedeutung für die Vermehrung lymphotropher Viren wird auch eine positive Beeinflussung der Genexpression beim Zytomegalievirus (CMV) wie auch bei Adenoviren durch NF-κB/AP-1 vermutet. Inhibitoren/Modulatoren der NF-κB- und/oder AP-1-Aktivität könnten somit auch anti-virale Effekte ausüben.

Gegenstand der vorliegenden Erfindung sind Verbindungen der allgemeinen Formel (I) 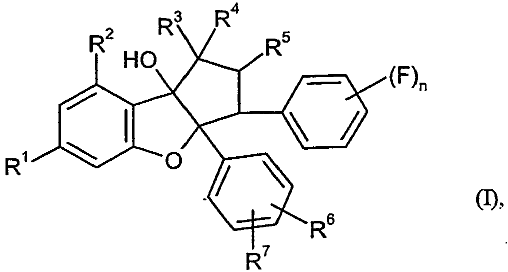 in welcher
- R¹: Wasserstoff, Benzyloxy, Ethoxy, n-Propoxy oder eine Gruppe der Formel R⁸-C(=O)-NH-bedeutet, wobei Ethoxy in 2-Position und n-Propoxy in 2- oder 3-Position durch (C₁-C₆)-Alkoxy, Amino, Mono- oder Di-(C₁-C₆)-alkylamino, (C₃-C₈)-Cycloalkylamino, *N*-(C₃-C₈)-Cycloalkyl-*N*-(C₁-C₆)-alkylamino oder durch einen über ein N-Atom gebundenen 4- bis 7-gliedrigen Heterocyclus substituiert sein können,
wobei Mono- und Di-(C₁-C₆)-alkylamino ihrerseits durch Hydroxy, (C₁-C₄)-Alkoxy, Amino, Mono- oder Di-(C₁-C₄)-alkylamino substituiert sein können,
und worin
R⁸ für 5- oder 6-gliedriges Heteroaryl, das durch (C₁-C₄)-Alkyl oder Halogen substituiert sein kann, steht,
- R²: Wasserstoff, Ethoxy oder n-Propoxy bedeutet, wobei Ethoxy in 2-Position und n-Propoxy in 2- oder 3-Position durch (C₁-C₆)-Alkoxy, Amino, Mono- oder Di-(C₁-C₆)-alkylamino, (C₃-C₈)-Cycloalkylamino, *N*-(C₃-C₈)-Cycloalkyl-*N*-(C₁-C₆)-alkylamino oder durch einen über ein N-Atom gebundenen 4- bis 7-gliedrigen Heterocyclus substituiert sein können,
wobei Mono- und Di-(C₁-C₆)-alkylamino ihrerseits durch Hydroxy, (C₁-C₄)-Alkoxy, Amino, Mono- oder Di-(C₁-C₄)-alkylamino substituiert sein können,
wobei R¹ und R² jedoch nicht gleichzeitig für Wasserstoff stehen,
- R³: Hydroxy oder Amino
und
- R⁴: Wasserstoff bedeutet
oder
- R³ und R⁴: gemeinsam mit dem Kohlenstoffatom, an das sie gebunden sind, eine Gruppe der Formel >C=O oder >C=N-OH bilden,
- R⁵: Mono- oder Di-(C₁-C₆)-alkylaminocarbonyl bedeutet,
- n: für die Zahl 0, 1, 2 oder 3 steht,
- R⁶: sich in meta- oder para-Position, relativ zur Verknüpfungsstelle des Phenylrings mit dem Tricyclus, befindet und Halogen, (C₁-C₆)-Alkyl, (C₁-C₆)-Alkoxy, (C₆-C₁₀)-Aryl, 5- bis 10-gliedriges Heteroaryl oder eine Gruppe der Formel -NR⁹R¹⁰ bedeutet,
wobei Aryl und Heteroaryl ihrerseits jeweils ein- bis zweifach, gleich oder verschieden, durch Halogen, Cyano, (C₁-C₄)-Alkylsulfonyl oder eine Gruppe der Formel -NR⁹R¹⁰ substituiert sein können,
und worin
R⁹ und R¹⁰ unabhängig voneinander für Wasserstoff, (C₁-C₆)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden, und
- R⁷: sich in meta- oder para-Position, relativ zur Verknüpfungsstelle des Phenylrings mit dem Tricyclus, und in ortho-Stellung zu R⁶ befindet und Wasserstoff, Halogen, (C₁-C₆)-Alkyl, (C₁-C₆)-Alkoxy oder eine Gruppe der Formel -NR¹¹R¹² bedeutet, worin
R¹¹ und R¹² unabhängig voneinander für Wasserstoff, (C₁-C₆)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden, oder
- R⁶ und R⁷: gemeinsam mit dem Phenylring, an den sie gebunden sind, eine Gruppe der Formel 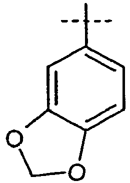
bilden,
sowie ihre Salze, Solvate und Solvate der Salze.

Weiterer Gegenstand der vorliegenden Erfindung sind Verbindungen der allgemeinen Formel (I), in welcher
- R¹: Wasserstoff, Benzyloxy, Ethoxy, n-Propoxy oder eine Gruppe der Formel R₈-C(=O)-NH-bedeutet, wobei Ethoxy in 2-Position und n-Propoxy in 2- oder 3-Position durch (C₁-C₆)-Alkoxy, Amino, Mono- oder Di-(C₁-C₆)-alkylamino, (C₃-C₈)-Cycloalkylamino, *N*-(C₃-C₈)-Cyloalkyl-*N*-(C₁-C₆)-alkylamino oder durch einen über ein N-Atom gebundenen 4- bis 7-gliedrigen Heterocyclus substituiert sein können,
wobei Mono- und Di-(C₁-C₆)-alkylamino ihrerseits durch Hydroxy, (C₁-C₄)-Alkoxy, Amino, Mono- oder Di-(C₁-C₄)-alkylamino substituiert sein können,
und worin
R⁸ für 5- oder 6-gliedriges Heteroaryl, das durch (C₁-C₄)-Alkyl oder Halogen substituiert sein kann, steht,
- R²: Wasserstoff, Ethoxy oder n-Propoxy bedeutet, wobei Ethoxy in 2-Position und n-Propoxy in 2- oder 3-Position durch (C₁-C₆)-Alkoxy, Amino, Mono- oder Di-(C₁-C₆)-alkylamino, (C₃-C₈)-Cycloalkylamino, *N*-(C₃-C₈)-Cycloalkyl-*N*-(C₁-C₆)-alkylamino oder durch einen über ein N-Atom gebundenen 4- bis 7-gliedrigen Heterocyclus substituiert sein können,
wobei Mono- und Di-(C₁-C₆)-alkylamino ihrerseits durch Hydroxy, (C₁-C₄)-Alkoxy, Amino, Mono- oder Di-(C₁-C₄)-alkylamino substituiert sein können,
wobei R¹ und R² jedoch nicht gleichzeitig für Wasserstoff stehen,
- R³: Hydroxy oder Amino
und
- R⁴: Wasserstoff bedeutet
oder
- R³ und R⁴: gemeinsam mit dem Kohlenstoffatom, an das sie gebunden sind, eine Gruppe der Formel >C=O oder >C=N-OH bilden,
- R⁵: Wasserstoff bedeutet,
- n: für die Zahl 0, 1, 2 oder 3 steht,
- R⁶: sich in meta- oder para-Position, relativ zur Verknüpfungsstelle des Phenylrings mit dem Tricyclus, befindet und (C₆-C₁₀)-Aryl, 5- bis 10-gliedriges Heteroaryl oder eine Gruppe der Formel -NR⁹R¹⁰ bedeutet, wobei Aryl und Heteroaryl ihrerseits jeweils ein- bis zweifach, gleich oder verschieden, durch Halogen, Cyano, (C₁-C₄)-Alkylsulfonyl oder eine Gruppe der Formel -NR⁹R¹⁰ substituiert sein können,
und worin
R⁹ und R¹⁰ unabhängig voneinander für Wasserstoff, (C₁-C₆)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden, und
- R⁷: sich in meta- oder para-Position, relativ zur Verknüpfungsstelle des Phenylrings mit dem Tricyclus, und in ortho-Stellung zu R⁶ befindet und Wasserstoff, Halogen, (C₁-C₆)-Alkyl, (C₁-C₆)-Alkoxy oder eine Gruppe der Formel -NR¹¹R¹² bedeutet, worin R¹¹ und R¹² unabhängig voneinander für Wasserstoff, (C₁-C₆)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden,
oder
- R⁶ und R⁷: gemeinsam mit dem Phenylring, an den sie gebunden sind, eine Gruppe der Formel 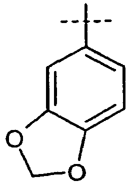
bilden,
sowie ihre Salze, Solvate und Solvate der Salze.

Weiterer Gegenstand der vorliegenden Erfindung sind Verbindungen der allgemeinen Formel (I), in welcher
- R¹: Wasserstoff, Benzyloxy, Ethoxy, n-Propoxy oder eine Gruppe der Formel R⁸-C(=O)-NH-bedeutet, wobei Ethoxy in 2-Position und n-Propoxy in 2- oder 3-Position durch (C₁-C₆)-Alkoxy, Amino, Mono- oder Di-(C₁-C₆)-alkylamino, (C₃-C₈)-Cycloalkylamino, *N*-(C₃-C₈)-Cycloalkyl-*N*-(C₁-C₆)-alkylamino oder durch einen über ein N-Atom gebundenen 4- bis 7-gliedrigen Heterocyclus substituiert sein können,
wobei Mono- und Di-(C₁-C₆)-alkylamino ihrerseits durch Hydroxy, (C₁-C₄)-Alkoxy, Amino, Mono- oder Di-(C₁-C₄)-alkylamino substituiert sein können,
und worin
R⁸ für 5- oder 6-gliedriges Heteroaryl, das durch (C₁-C₄)-Alkyl oder Halogen substituiert sein kann, steht,
- R²: Wasserstoff, Ethoxy oder n-Propoxy bedeutet, wobei Ethoxy in 2-Position und n-Propoxy in 2- oder 3-Position durch (C₁-C₆)-Alkoxy, Amino, Mono- oder Di-(C₁-C₆)-alkylamino, (C₃-C₈)-Cycloalkylamino, *N*-(C₃-C₈)-Cyloalkyl-*N*-(C₁-C₆)-alkylamino oder durch einen über ein N-Atom gebundenen 4- bis 7-gliedrigen Heterocyclus substituiert sein können,
wobei Mono- und Di-(C₁-C₆)-alkylamino ihrerseits durch Hydroxy, (C₁-C₄)-Akoxy, Amino, Mono- oder Di-(C₁-C₄)-alkylamino substituiert sein können,
wobei R¹ oder R² jedoch nicht beide gleichzeitig für Wasserstoff stehen,
- R³: Amino
und
- R⁴: Wasserstoff bedeutet
oder
- R³ und R⁴: gemeinsam mit dem Kohlenstoffatom, an das sie gebunden sind, eine Gruppe der Formel >C=N-OH bilden,
- R⁵: Wasserstoff bedeutet,
- n: für die Zahl 0, 1, 2 oder 3 steht,
- R⁶: sich in meta- oder para-Position, relativ zur Verknüpfungsstelle des Phenylrings mit dem Tricyclus, befindet und Halogen, (C₁-C₆)-Alkyl oder (C₁-C₆)-Alkoxy bedeutet,
und
- R¹: sich in meta- oder para-Position, relativ zur Verknüpfungsstelle des Phenylrings mit dem Tricyclus, und in ortho-Stellung zu R⁶ befmdet und Wasserstoff, Halogen, (C₁-C₆)-Alkyl, (C₁-C₆)-Alkoxy oder eine Gruppe der Formel NR¹¹R¹² bedeutet, worin
R¹¹ und R¹² unabhängig voneinander für Wasserstoff, (C₁-C₆)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden, oder
- R⁶ und R⁷: gemeinsam mit dem Phenylring, an den sie gebunden sind, eine Gruppe der Formel 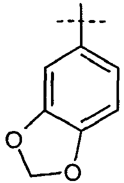
bilden,
sowie ihre Salze, Solvate und Solvate der Salze.

Weiterer Gegenstand der vorliegenden Erfindung sind Verbindungen der allgemeinen Formel (I), in welcher
- R¹: Wasserstoff, Benzylöxy, Ethoxy, n-Propoxy oder eine Gruppe der Formel R⁸-C(=O) NHbedeutet, wobei Ethoxy in 2-Position und n-Propoxy in 2- oder 3-Position durch (C₁-C₆)-Alkoxy, Amino, Mono- oder Di-(C₁-C₆)-alkylamino, (C₃-C₈)-Cycloalkylamino, *N*-(C₃-C₈)-Cycloalkyl-*N*-(C₁-C₆)-alkylamino oder durch einen über ein N-Atom gebundenen 4- bis 7-gliedrigen Heterocyclus substituiert sein können,
wobei Mono- und Di-(C₁-C₆)-alkylamino ihrerseits durch Hydroxy, (C₁-C₄)-Alkoxy, Amino, Mono- oder Di-(C₁-C₄)-alkylamino substituiert sein können,
und worin
R⁸ für 5- oder 6-gliedriges Heteroaryl, das durch (C₁-C₄)-Alkyl oder Halogen substituiert sein kann, steht,
- R²: Wasserstoff, Ethoxy oder n-Propoxy bedeutet, wobei Ethoxy in 2-Position und n-Propoxy in 2- oder 3-Position durch (C₁-C₆)-Alkoxy, Amino, Mono- oder Di-C₁-C₆)-alkylamino, (C₃-C₈)-Cycloalkylamino, *N*-(C₃-C₈)-Cycloalkyl-*N*-(C₁-C₆)-alkylamino oder durch einen über ein N-Atom gebundenen 4- bis 7-gliedrigen Heterocyclus substituiert sein können,
wobei Mono- und Di-(C₁-C₆)-alkylamino ihrerseits durch Hydroxy, (C₁-C₄)-Alkoxy, Amino, Mono- oder Di-(C₁-C₄)-alkylamino substituiert sein können,
wobei entweder R¹ oder R² für Wasserstoff steht, jedoch nicht beide gleichzeitig Wasserstoff bedeuten,
- R³: Hydroxy
und
- R⁴: Wasserstoff bedeutet
oder
- R³ und R⁴: gemeinsam mit dem Kohlenstoffatom, an das sie gebunden sind, eine Gruppe der Formel >C=O bilden,
- R⁵: Wasserstoff bedeutet,
- n: für die Zahl 0, 1, 2 oder 3 steht,
- R⁶: sich in meta- oder para-Position, relativ zur Verknüpfungsstelle des Phenylrings mit dem Tricyclus, befindet und Halogen, (C₁-C₆)-Alkyl oder (C₁-C₆)-Alkoxy bedeutet,
und
- R⁷: sich in meta- oder para-Position, relativ zur Verknüpfungsstelle des Phenylrings mit dem Tricyclus, und in ortho-Stellung zu R⁶ befindet und Wasserstoff, Halogen, (C₁-C₆)-Alkyl, (C₁-C₆)-Alkoxy oder eine Gruppe der Formel NR¹¹R¹² bedeutet, worin R¹¹ und R¹² unabhängig voneinander für Wasserstoff, (C₁-C₆)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden,
oder
- R⁶ und R⁷: gemeinsam mit dem Phenylring, an den sie gebunden sind, eine Gruppe der Formel 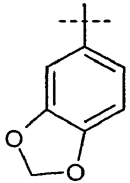
bilden,
sowie ihre Salze, Solvate und Solvate der Salze.

Weiterer Gegenstand der vorliegenden Erfindung sind Verbindungen der allgemeinen Formel (I), in welcher
- R¹: Ethoxy, n-Propoxy oder eine Gruppe der Formel R⁸-C(=O)-NH- bedeutet, wobei Ethoxy in 2-Position und n-Propoxy in 2- oder 3-Position durch (C₁-C₆)-Alkoxy, Amino, Mono- oder Di-(C₁-C₆)-alkylamino, (C₃-C₈)-Cycloalkylamino, *N*-(C₃-C₈)-Cycloalkyl-*N*-(C₁-C₆)-alkyl-amino oder durch einen über ein N-Atom gebundenen 4- bis 7-gliedrigen Heterocyclus substituiert sind,
wobei Mono- und Di-(C₁-C₆)-alkylamino ihrerseits durch Hydroxy, (C₁-C₄)-Alkoxy, Amino, Mono- oder Di-(C₁-C₄)-alkylamino substituiert sein können,
und worin
R⁸ für 5- oder 6-gliedriges Heteroaryl, das durch (C₁-C₄)-Alkyl oder Halogen substituiert sein kann, steht,
- R²: Ethoxy oder n-Propoxy bedeutet, wobei Ethoxy in 2-Position und n-Propoxy in 2- oder 3-Position durch (C₁-C₆)-Alkoxy, Amino, Mono- oder Di-(C₁-C₆)-alkylamino, (C₃-C₈)-Cycloalkylamino, *N*-(C₃-C₈)-Cycloalkyl-*N*-(C₁-C₆)-alkylamino oder durch einen über ein N-Atom gebundenen 4- bis 7-gliedrigen Heterocyclus substituiert sind,
wobei Mono- und Di-(C₁-C₆)-alkylamino ihrerseits durch Hydroxy, (C₁-C₄)-Alkoxy, Amino, Mono- oder Di-(C₁-C₄)-alkylamino substituiert sein können,
- R³: Hydroxy
und
- R⁴: Wasserstoff bedeutet
oder
- R³ und R⁴: gemeinsam mit dem Kohlenstoffatom, an das sie gebunden sind, eine Gruppe der Formel >C=O bilden,
- R⁵: Wasserstoff bedeutet,
- n: für die Zahl 0, 1, 2 oder 3 steht,
- R⁶: sich in meta- oder para-Position, relativ zur Verknüpfungsstelle des Phenylrings mit dem Tricyclus, befindet und Halogen, (C₁-C₆)-Alkyl oder (C₁-C₆)-Alkoxy bedeutet,
und
- R⁷: sich in meta- oder para-Position, relativ zur Verknüpfungsstelle des Phenylrings mit dem Tricyclus, und in ortho-Stellung zu R⁶ befindet und Wasserstoff, Halogen, (C₁-C₆)-Alkyl, (C₁-C₆)-Alkoxy oder eine Gruppe der Formel -NR¹¹R¹² bedeutet, worin
R¹¹ und R¹² unabhängig voneinander für Wasserstoff, (C₁-C₆)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden, oder
- R⁶ und R⁷: gemeinsam mit dem Phenylring, an den sie gebunden sind, eine Gruppe der Formel 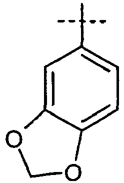
bilden,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Im Rahmen der vorliegenden Erfindung haben die Substituenten, soweit nicht anders spezifiziert, die folgende Bedeutung:

(C₁-C₆)-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, n-Propyl, Isopropyl, n-Butyl, Isobutyl, sec.-Butyl, tert.-Butyl, 1-Ethylpropyl, n-Pentyl und n-Hexyl.

(C₃-C₈)-Cycloalkyl und (C₃-C₆)-Cycloalkyl stehen im Rahmen der Erfindung für eine mono- oder gegebenenfalls bicyclische Cycloalkylgruppe mit 3 bis 8 bzw. 3 bis 6 Kohlenstoffatomen. Bevorzugt ist ein monocyclischer Cycloalkylrest mit 3 bis 6 Kohlenstoffatomen. Beispielhaft und vorzugsweise seien genannt: Cyclopropyl, Cyclobutyl, Cyclopentyl, Cyclohexyl und Cycloheptyl.

(C₆-C₁₀)-Aryl steht im Rahmen der Erfindung für einen aromatischen Rest mit vorzugsweise 6 bis 10 Kohlenstoffatomen. Bevorzugte Arylreste sind Phenyl und Naphthyl.

(C₁-C₂)-Alkoxy und (C₁-C₄)-Alkoxy stehen im Rahmen der Erfindung für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4 Kohlenstoffatomen. Beispielhaft und vorzugsweise seien genannt: Methoxy, Ethoxy, n-Propoxy, Isopropoxy und tert.-Butoxy.

Mono-(C₁-C₆)-alkylamino und Mono-(C₁-C₄)-alkylamino stehen im Rahmen der Erfindung für eine Amino-Gruppe mit einem geradkettigen oder verzweigten Alkylsubstituenten, der 1 bis 6 bzw. 1 bis 4 Kohlenstoffatome aufweist. Bevorzugt ist ein geradkettiger oder verzweigter Monoalkylamino-Rest mit 1 bis 4 Kohlenstoffatomen. Beispielhaft und vorzugsweise seien genannt: Methylamino, Ethylamino, n-Propylamino, Isopropylamino und tert.-Butylamino.

Di-(C₁-C₆)-alkylamino und Di-(C₁-C₄)-alkylamino stehen im Rahmen der Erfindung für eine Amino-Gruppe mit zwei gleichen oder verschiedenen geradkettigen oder verzweigten Alkylsubstituenten, die jeweils 1 bis 6 bzw. 1 bis 4 Kohlenstoffatome aufweisen. Bevorzugt sind geradkettige oder verzweigte Dialkylamino-Reste mit jeweils 1 bis 4 Kohlenstoffatomen. Beispielhaft und vorzugsweise seien genannt: *N,N-*Dimethylamino, *N,N-*Diethylamino, *N*-Ethyl-*N*-methylamino, *N-*Methyl-*N*-n-propylamino, *N-*Isopropyl-N-n-propylamino, *N-*tert.-Butyl-*N*-methylamino, *N-*Ethyl-*N-*n-pentylamino und *N-*n-Hexyl-*N-*methylamino.

Mono- oder Di-(C₁-C₆)-alkylaminocarbonyl bzw. Mono- oder Di-(C₁-C₄)-alkylaminocarbonyl stehen im Rahmen der Erfindung für eine Amino-Gruppe, die über eine Carbonylgruppe verknüpft ist und die einen geradkettigen oder verzweigten bzw. zwei gleiche oder verschiedene geradkettige oder verzweigte Alkylsubstituenten mit jeweils 1 bis 6 bzw. 1 bis 4 Kohlenstoffatomen aufweist. Beispielhaft und vorzugsweise seien genannt: Methylaminocarbonyl, Ethylaminocarbonyl, Isopropylaminocarbonyl, tert.-Butylaminocarbonyl, *N,N*-Dimethylaminocarbonyl, *N,N*-Diethylaminocarbonyl, *N*-Ethyl-*N*-methylaminocarbonyl und *N*-tert.-Butyl-*N*-methylaminocarbonyl.

(C₃-C₈)-Cycloalkylamino und (C₃-C₆)-Cycloalkylamino stehen im Rahmen der Erfindung für eine Amino-Gruppe mit einem mono- oder gegebenenfalls bicyclischen Cycloalkyl-Substituenten, der 3 bis 8 bzw. 3 bis 6 Ring-Kohlenstoffatome aufweist. Bevorzugt ist ein monocyclischer Cycloalkyl-Substituent mit 3 bis 6 Ring-Kohlenstoffatomen. Beispielhaft und vorzugsweise seien genannt: Cyclopropylamino, Cyclobutylamino, Cyclopentylamino, Cyclohexylamino, Cycloheptylamino und Cyclooctylamino.

(C₁-C₄)-Alkylsulfonyl steht im Rahmen der Erfindung für einen geradkettigen oder verzweigten Alkylsulfonyl-Rest mit 1 bis 4 Kohlenstoffatomen. Bevorzugt ist ein geradkettiger oder verzweigter Alkylsulfonyl-Rest mit 1 bis 3 Kohlenstoffatomen. Beispielhaft und vorzugsweise seien genannt: Methylsulfonyl, Ethylsulfonyl, n-Propylsulfonyl, Isopropylsulfonyl, n-Butylsulfonyl und tert.-Butylsulfonyl.

Ein 4- bis 7-gliedriger Heterocyclus steht im Rahmen der Erfindung für einen gesättigten oder partiell ungesättigten Heterocyclus mit 4 bis 7 Ringatomen, der ein Ring-Stickstoffatom enthält, über dieses verknüpft ist und ein weiteres Heteroatom aus der Reihe N, O, S, SO oder SO₂ enthalten kann. Bevorzugt ist ein 4- bis 7-gliedriger gesättigter, N-verknüpfter Heterocyclus, der ein weiteres Heteroatom aus der Reihe N, O oder S enthalten kann. Beispielhaft und vorzugsweise seien genannt: Azetidinyl, Pyrrolidinyl, Pyrrolinyl, Piperidinyl, Piperazinyl, Morpholinyl, Thiomorpholinyl, Azepinyl und 1,4-Diazepinyl.

5- bis 10-gliedriges Heteroaryl steht im Rahmen der Erfindung für einen mono- oder gegebenenfalls bicyclischen aromatischen Heterocyclus (Heteroaromaten) mit bis zu vier gleichen oder verschiedenen Heteroatomen aus der Reihe N, O und/oder S, der über ein Ringkohlenstoffatom oder gegebenenfalls über ein Ringstickstoffatom des Heteroaromaten verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Tetrazolyl, Pyridyl, Pyrimidinyl, Pyridazinyl, Pyrazinyl, Triazinyl, Benzofuranyl, Benzothienyl, Benzimidazolyl, Benzoxazolyl, Benzothiazolyl, Benzotriazolyl, Indolyl, Indazolyl, Chinolinyl, Isochinolinyl, Naphthyridinyl, Chinazolinyl, Chinoxalinyl. Bevorzugt sind monocyclische 5- oder gliedrige Heteroarvl-Reste mit bis zu drei Heteroatomen aus der Reihe N, O und/oder S wie beispielsweise Furyl, Thienyl, Thiazolyl, Oxazolyl, Isothiazolyl, Isoxazolyl, Pyrazolyl, Imidazolyl, Triazolyl, Oxadiazolyl, Thiadiazolyl, Pyridyl, Pyrimidinyl, Pyridazinyl, Pyrazinyl, Triazinyl.

Halogen schließt im Rahmen der Erfindung Fluor, Chlor, Brom und Iod ein. Bevorzugt sind Fluor, Chlor oder Brom.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Bevorzugt im Rahmen der vorliegenden Erfindung sind Verbindungen der Formel (I), in welcher
- R¹: Wasserstoff, Benzyloxy, Ethoxy, n-Propoxy oder eine Gruppe der Formel R⁸-C(=O)-NH-bedeutet, wobei Ethoxy in 2-Position und n-Propoxy in 2- oder 3-Position durch (C₁-C₄)-Alkoxy, Amino, Mono- oder Di-(C₁-C₄)-alkylamino, (C₃-C₆)-Cycloalkylamino, *N*-(C₃-C₆)-Cyloalkyl-*N*-(C₁-C₄)-alkylamino oder durch einen über ein N-Atom gebundenen 4- bis 7-gliedrigen Heterocyclus substituiert sein können,
wobei Mono- und Di-(C₁-C₄)-alkylamino ihrerseits durch Hydroxy, (C₁-C₄)-Alkoxy, Amino, Mono- oder Di-(C₁-C₄)-alkylamino substituiert sein können,
und worin
R⁸ für 5- oder 6-gliedriges Heteroaryl, das durch (C₁-C₄)-Alkyl oder Halogen substituiert sein kann, steht,
- R²: Wasserstoff, Ethoxy oder n-Propoxy bedeutet, wobei Ethoxy in 2-Position und n-Propoxy in 2- oder 3-Position durch (C₁-C₄)-Alkoxy, Amino, Mono- oder Di-(C₁-C₄)-alkylamino, (C₃-C₆)-Cycloalkylamino, *N*-(C₃-C₆)-Cycloalkyl-N-(C₁-C₄)-alkylamino oder durch einen über ein *N*-Atom gebundenen 4- bis 6-gliedrigen Heterocyclus substituiert sein können,
wobei Mono- und Di-(C₁-C₄)-alkylamino ihrerseits durch Hydroxy, (C₁-C₄)-Alkoxy, Amino, Mono- oder Di-(C₁-C₄)-alkylamino substituiert sein können,
wobei R¹ und R² jedoch nicht gleichzeitig für Wasserstoff stehen,
- R³: Hydroxy oder Amino
und
- R⁴: Wasserstoff bedeutet
oder
- R³ und R⁴: gemeinsam mit dem Kohlenstoffatom, an das sie gebunden sind, eine Gruppe der Formel >C=O oder >C=N-OH bilden,
- R⁵: Mono- oder Di-(C₁-C₄)-alkylaminocarbonyl bedeutet,
- n: für die Zahl 0, 1, 2 oder 3 steht,
- R⁶: sich in meta- oder para-Position, relativ zur Verknüpfungsstelle des Phenylrings mit dem Tricyclus, befindet und Halogen, (C₁-C₄)-Alkyl, (C₁-C₄)-Alkoxy, (C₆-C₁₀)-Aryl, 5- bis 6-gliedriges Heteroaryl oder eine Gruppe der Formel -NR⁹R¹⁰ bedeutet,
wobei Aryl und Heteroaryl ihrerseits jeweils ein- bis zweifach, gleich oder verschieden, durch Halogen, Cyano, (C₁-C₄)-Alkylsulfonyl oder eine Gruppe der Formel -NR⁹R¹⁰ substituiert sein können,
und worin
R⁹ und R¹⁰ unabhängig voneinander für Wasserstoff, (C₁-C₄)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden, und
- R⁷: sich in meta- oder para-Position, relativ zur Verknüpfungsstelle des Phenylrings mit dem Tricyclus, und in ortho-Stellung zu R⁶ befindet und Wasserstoff, Halogen, (C₁-C₄)-Alkyl, (C₁-C₄)-Alkoxy oder eine Gruppe der Formel NR¹¹R¹² bedeutet, worin
R¹¹ und R¹² unabhängig voneinander für Wasserstoff, (C₁-C₄)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden,
oder
- R⁶ und R⁷: gemeinsam mit dem Phenylring, an den sie gebunden sind, eine Gruppe der Formel 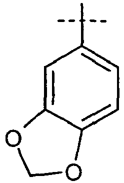
bilden,
sowie ihre Salze, Solvate und Solvate der Salze.

Ebenfalls bevorzugt im Rahmen der vorliegenden Erfmdung sind Verbindungen der Formel (I), in welcher
- R¹: Wasserstoff, Benzyloxy, Ethoxy, n-Propoxy oder eine Gruppe der Formel R⁸-C(=O)-NH-bedeutet, wobei Ethoxy in 2-Position und n-Propoxy in 2- oder 3-Position durch (C₁-C₄)-Alkoxy, Amino, Mono- oder Di-(C₁-C₄)-alkylamino, (C₃-C₆)-Cycloalkylamino, *N*-(C₃-C₆)-Cycloalkyl-*N*-(C₁-C₄)-alkylamino oder durch einen über ein N-Atom gebundenen 4- bis 7-gliedrigen Heterocyclus substituiert sein können,
wobei Mono- und Di-(C₁-C₄)-alkylamino ihrerseits durch Hydroxy, (C₁-C₄)-Alkoxy, Amino, Mono- oder Di-(C₁-C₄)-alkylamino substituiert sein können,
und worin
R⁸ für 5- oder 6-gliedriges Heteroaryl, das durch (C₁-C₄)-Alkyl oder Halogen substituiert sein kann, steht,
- R²: Wasserstoff, Ethoxy oder n-Propoxy bedeutet, wobei Ethoxy in 2-Position und n-Propoxy in 2- oder 3-Position durch (C₁-C₄)-Alkoxy, Amino, Mono- oder Di-(C₁-C₄)-alkylamino, (C₃-C₆)-Cycloalkylamino, *N*-(C₃-C₆)-Cyloalkyl-*N*-(C₁-C₄)-alkylamino oder durch einen über ein N-Atom gebundenen 4- bis 7-gliedrigen Heterocyclus substituiert sein können,
wobei Mono- und Di-(C₁-C₄)-alkylamino ihrerseits durch Hydroxy, (C₁-C₄)-Alkoxy, Amino, Mono- oder Di-(C₁-C₄)-alkylamino substituiert sein können,
wobei R¹ und R² jedoch nicht gleichzeitig für Wasserstoff stehen,
- R³: Hydroxy oder Amino
und
- R⁴: Wasserstoff bedeutet
oder
- R³ und R⁴: gemeinsam mit dem Kohlenstoffatom, an das sie gebunden sind, eine Gruppe der Formel >C=O oder >C=N-OH bilden,
- R⁵: Wasserstoff bedeutet,
- n: für die Zahl 0, 1, 2 oder 3 steht,
- R⁶: sich in meta- oder para-Position, relativ zur Verknüpfungsstelle des Phenylrings mit dem Tricyclus, befindet und (C₆-C₁₀)-Aryl, 5- bis 6-gliedriges Heteroaryl oder eine Gruppe der Formel -NR⁹R¹⁰ bedeutet,
wobei Aryl und Heteroaryl ihrerseits jeweils ein- bis zweifach, gleich oder verschieden, durch Halogen, Cyano, (C₁-C₄)-Alkylsulfonyl oder eine Gruppe der Formel -NR⁹R¹⁰ substituiert sein können,
und worin
R⁹ und R¹⁰ unabhängig voneinander für Wasserstoff, (C₁-C₄)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden, und
- R⁷: sich in meta- oder para-Position, relativ zur Verknüpfungsstelle des Phenylrings mit dem Tricyclus, und in ortho-Stellung zu R⁶ befmdet und Wasserstoff, Halogen, (C₁-C₄)-Alkyl, (C₁-C₄)-Alkoxy oder eine Gruppe der Formel -NR¹¹R¹² bedeutet, worin
- R¹¹ und R¹²: unabhängig voneinander für Wasserstoff, (C₁-C₄)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden,
oder
- R⁶ und R⁷: gemeinsam mit dem Phenylring, an den sie gebunden sind, eine Gruppe der Formel 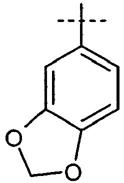
bilden,
sowie ihre Salze, Solvate und Solvate der Salze.

Ebenfalls bevorzugt im Rahmen der vorliegenden Erfindung sind Verbindungen der Formel (I), in welcher
- R¹: Wasserstoff, Benzyloxy, Ethoxy, n-Propoxy oder eine Gruppe der Formel R⁸-C(=O)-NH-bedeutet, wobei Ethoxy in 2-Position und n-Propoxy in 2- oder 3-Position durch (C₁-C₄)-Alkoxy, Amino, Mono- oder Di-(C₁-C₄)-alkylamino, (C₃-C₆)-Cycloalkylamino, *N*-(C₃-C₆)-Cyloalkyl-*N*-(C₁-C₄)-alkylamino oder durch einen über ein N-Atom gebundenen 4- bis 7-gliedrigen Heterocyclus substituiert sein können,
wobei Mono- und Di-(C₁-C₄)-alkylamino ihrerseits durch Hydroxy, (C₁-C₄)-Alkoxy, Amino, Mono- oder Di-(C₁-C₄)-alkylamino substituiert sein können,
und worin
R⁸ für 5- oder 6-gliedriges Heteroaryl, das durch (C₁-C₄)-Alkyl oder Halogen substituiert sein kann, steht,
- R²: Wasserstoff, Ethoxy oder n-Propoxy bedeutet, wobei Ethoxy in 2-Position und n-Propoxy in 2- oder 3-Position durch (C₁-C₄)-Alkoxy, Amino, Mono- oder Di-(C₁-C₄)-alkylamino, (C₃-C₆)-Cycloalkylamino, *N*-(C₃-C₆)-Cycloalkyl-*N*-(C₁-C₄)-alkylamino oder durch einen über ein N-Atom gebundenen 4- bis 7-gliedrigen Heterocyclus substituiert sein können,
wobei Mono- und Di-(C₁-C₄)-alkylamino ihrerseits durch Hydroxy, (C₁-C₄)-Alkoxy, Amino, Mono- oder Di-(C₁-C₄)-alkylamino substituiert sein können,
wobei R¹ oder R² jedoch nicht beide gleichzeitig für Wasserstoff stehen,
- R³: Amino
und
- R⁴: Wasserstoff bedeutet
oder
- R³ und R⁴: gemeinsam mit dem Kohlenstoffatom, an das sie gebunden sind, eine Gruppe der Formel >C=N-OH bilden,
- R⁵: Wasserstoff bedeutet,
- n: für die Zahl 0, 1, 2 oder 3 steht,
- R⁶: sich in meta- oder para-Position, relativ zur Verknüpfungsstelle des Phenylrings mit dem Tricyclus, befindet und Halogen, (C₁-C₄)-Alkyl oder (C₁-C₄)-Alkoxy bedeutet,
und
- R⁷: sich in meta- oder para-Position, relativ zur Verknüpfungsstelle des Phenylrings mit dem Tricyclus, und in ortho-Stellung zu R⁶ befmdet und Wasserstoff, Halogen, (C₁-C₄)-Alkyl, (C₁-C₄)-Alkoxy oder eine Gruppe der Formel NR¹¹R¹² bedeutet, worin
R¹¹ und R¹² unabhängig voneinander für Wasserstoff, (C₁-C₄)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden,
oder
- R⁶ und R⁷: gemeinsam mit dem Phenylring, an den sie gebunden sind, eine Gruppe der Formel 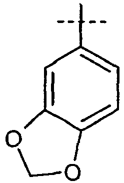
bilden,
sowie ihre Salze, Solvate und Solvate der Salze.

Ebenfalls bevorzugt im Rahmen der vorliegenden Erfindung sind Verbindungen der Formel (I), in welcher
- R¹: Wasserstoff, Benzyloxy, Ethoxy, n-Propoxy oder eine Gruppe der Formel R⁸-C(=O) NHbedeutet, wobei Ethoxy in 2-Position und n-Propoxy in 2- oder 3-Position durch (C₁-C₄)-Alkoxy, Amino, Mono- oder Di-(C₁-C₄)-alkylamino, (C₃-C₆)-Cycloalkylamino, *N*-(C₃-C₆)-Cycloalkyl-*N*-(C₁-C₄)-alkylamino oder durch einen über ein N-Atom gebundenen 4- bis 7-gliedrigen Heterocyclus substituiert sein können,
wobei Mono- und Di-(C₁-C₄)-alkylamino ihrerseits durch Hydroxy, (C₁-C₄)-Alkoxy, Amino, Mono- oder Di-(C₁-C₄)-alkylamino substituiert sein können,
und worin
R⁸ für 5- oder 6-gliedriges Heteroaryl, das durch (C₁-C₄)-Alkyl oder Halogen substituiert sein kann, steht,
- R²: Wasserstoff, Ethoxy oder n-Propoxy bedeutet, wobei Ethoxy in 2-Position und n-Propoxy in 2- oder 3-Position durch (C₁-C₄)-Alkoxy, Amino, Mono- oder Di-(C₁-C₄)-alkylamino, (C₃-C₆)-Cycloalkylamino, *N*-(C₃-C₆)-Cycloalkyl-*N*-(C₁-C₄)-alkylamino oder durch einen über ein N-Atom gebundenen 4- bis 7-gliedrigen Heterocyclus substituiert sein können,
wobei Mono- und Di-(C₁-C₄)-alkylamino ihrerseits durch Hydroxy, (C₁-C₄)-Alkoxy, Amino, Mono- oder Di-(C₁-C₄)-alkylamino substituiert sein können,
wobei entweder R¹ oder R² für Wasserstoff steht, jedoch nicht beide gleichzeitig Wasserstoff bedeuten,
- R³: Hydroxy
und
- R⁴: Wasserstoff bedeutet
oder
- R³ und R⁴: gemeinsam mit dem Kohlenstoffatom, an das sie gebunden sind, eine Gruppe der Formel >C=O bilden,
- R⁵: Wasserstoff bedeutet,
- n: für die Zahl 0, 1, 2 oder 3 steht,
- R⁶: sich in meta- oder para-Position, relativ zur Verknüpfungsstelle des Phenylrings mit dem Tricyclus, befindet und Halogen, (C₁-C₄)-Alkyl oder (C₁-C₄)-Alkoxy bedeutet,
und
- R⁷: sich in meta- oder para-Position, relativ zur Verknüpfungsstelle des Phenylrings mit dem Tricyclus, und in ortho-Stellung zu R⁶ befindet und Wasserstoff, Halogen, (C₁-C₄)-Alkyl, (C₁-C₄)-Alkoxy oder eine Gruppe der Formel -NR¹¹R¹² bedeutet, worin
R¹¹ und R¹² unabhängig voneinander für Wasserstoff, (C₁-C₄)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden, oder
- R⁶ und R⁷: gemeinsam mit dem Phenylring, an den sie gebunden sind, eine Gruppe der Formel 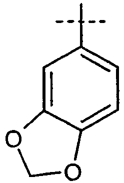
bilden,
sowie ihre Salze, Solvate und Solvate der Salze.

Ebenfalls bevorzugt im Rahmen der vorliegenden Erfindung sind Verbindungen der Formel (I), in welcher
- R¹: Ethoxy, n-Propoxy oder eine Gruppe der Formel R⁸-C(=O)-NH- bedeutet, wobei Ethoxy in 2-Position und n-Propoxy in 2- oder 3-Position durch (C₁-C₄)-Alkoxy, Amino, Mono- oder Di-(C₁-C₄)-alkylamino, (C₃-C₆)-Cycloalkylamino, *N*-(C₃-C₆)-Cyloalkyl-*N*-(C₁-C₄)-alkyl-amino oder durch einen über ein N-Atom gebundenen 4- bis 7-gliedrigen Heterocyclus substituiert sind,
wobei Mono- und Di-(C₁-C₄)-alkylamino ihrerseits durch Hydroxy, (C₁-C₄)-Alkoxy, Amino, Mono- oder Di-(C₁-C₄)-alkylamino substituiert sein können,
und worin
R⁸ für 5- oder 6-gliedriges Heteroaryl, das durch (C₁-C₄)-Alkyl oder Halogen substituiert sein kann, steht,
- R²: Ethoxy oder n-Propoxy bedeutet, wobei Ethoxy in 2-Position und n-Propoxy in 2- oder 3-Position durch (C₁-C₄)-Alkoxy, Amino, Mono- oder Di-(C₁-C₄)-alkylamino, (C₃-C₆)-Cycloalkylamino, *N*-(C₃-C₆)-Cycloalkyl-*N*-(C₁-C₄)-alkylamino oder durch einen über ein N-Atom gebundenen 4- bis 7-gliedrigen Heterocyclus substituiert sind,
wobei Mono- und Di-(C₁-C₄)-alkylamino ihrerseits durch Hydroxy, (C₁-C₄)-Alkoxy, Amino, Mono- oder Di-(C₁-C₄)-alkylamino substituiert sein können,
- R³: Hydroxy
und
- R⁴: Wasserstoff bedeutet
oder
- R³ und R⁴: gemeinsam mit dem Kohlenstoffatom, an das sie gebunden sind, eine Gruppe der Formel >C=O bilden,
- R⁵: Wasserstoff bedeutet,
- n: für die Zahl 0, 1, 2 oder 3 steht,
- R⁶: sich in meta- oder para-Position, relativ zur Verknüpfungsstelle des Phenylrings mit dem Tricyclus, befindet und Halogen, (C₁-C₄)-Alkyl oder (C₁-C₄)-Akoxy bedeutet,
und
- R⁷: sich in meta- oder para-Position, relativ zur Verknüpfungsstelle des Phenylrings mit dem Tricyclus, und in ortho-Stellung zu R⁶ befindet und Wasserstoff, Halogen, (C₁-C₄)-Alkyl, (C₁-C₄)-Alkoxy oder eine Gruppe der Formel NR¹¹R¹² bedeutet, worin
R¹¹ und R¹² unabhängig voneinander für Wasserstoff, (C₁-C₄)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden, oder
- R⁶ und R⁷: gemeinsam mit dem Phenylring, an den sie gebunden sind, eine Gruppe der Formel 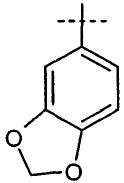
bilden,
sowie ihre Salze, Solvate und Solvate der Salze.

Besonders bevorzugt im Rahmen der vorliegenden Erfindung sind Verbindungen der Formel (I), in welcher
- R¹ und R²: unabhängig voneinander Wasserstoff, Ethoxy oder n-Propoxy bedeuten, wobei Ethoxy in 2-Position und n-Propoxy in 3-Position durch Methoxy, Ethoxy, Amino, Methylamino, Ethylamino, Isopropylamino, Dimethylamino, Diethylamino, Cyclopropylamino, N-Cyclopropyl-*N*-methylamino, Azetidino oder Pyrrolidino substituiert sind,
- wobei R¹ und R²: jedoch nicht gleichzeitig für Wasserstoff stehen,
- R³: Hydroxy oder Amino bedeutet,
- R⁴: Wasserstoff bedeutet,
- R⁵: Methylaminocarbonyl oder Dimethylaminocarbonyl bedeutet,
- n: für die Zahl 0 oder 1 steht,
- R⁶: sich in para-Position, relativ zur Verknüpfungsstelle des Phenylrings mit dem Tricyclus, befindet und Fluor, Chlor, Brom, Methyl, Ethyl, Methoxy oder Ethoxy bedeutet,
und
- R⁷: Wasserstoff bedeutet,
sowie ihre Salze, Solvate und Solvate der Salze.

Gleichfalls besonders bevorzugt im Rahmen der vorliegenden Erfindung sind Verbindungen der Formel (I), in welcher
- R¹: Wasserstoff, Ethoxy, n-Propoxy oder eine Gruppe der Formel R⁸-C(=O)-NH- bedeutet, wobei Ethoxy in 2-Position und n-Propoxy in 3-Position durch Methoxy, Ethoxy, Amino, Methylamino, Ethylamino, Isopropylamino, Dimethylamino, Diethylamino, Cyclopropylamino, *N*-Cyclopropyl-*N*-methylamino, Azetidino oder Pyrrolidino substituiert sein können, worin
R⁸ für Pyridyl, Pyrrolyl, Imidazolyl, Pyrazolyl, Triazolyl oder Thiadiazolyl, die jeweils durch Methyl, Ethyl, Fluor oder Chlor substituiert sein können, steht,
- R²: Wasserstoff, Ethoxy oder n-Propoxy bedeutet, wobei Ethoxy in 2-Position und n-Propoxy in 3-Position durch Methoxy, Ethoxy, Amino, Methylamino, Ethylamino, Isopropylamino, Dimethylamino, Diethylamino, Cyclopropylamino, *N*-Cyclopropyl-*N*-methylamino, Azetidino oder Pyrrolidino substituiert sein können,
- wobei R¹ und R²: jedoch nicht gleichzeitig für Wasserstoff stehen,
- R³: Hydroxy oder Amino bedeutet,
- R⁴: Wasserstoff bedeutet,
- R⁵: Wasserstoff bedeutet,
- n: für die Zahl 0 oder 1 steht,
- R⁶: sich in meta- oder para-Position, relativ zur Verknüpfungsstelle des Phenylrings mit dem Tricyclus, befindet und Phenyl, Thienyl, Indolyl, Chinoxalinyl oder eine Gruppe der Formel -NR⁹R¹⁰ bedeutet,
wobei Phenyl, Thienyl und Indolyl ihrerseits jeweils ein- bis zweifach, gleich oder verschieden, durch Fluor, Chlor, Brom, Cyano oder Amino substituiert sein können,
und worin
R⁹ und R¹⁰ unabhängig voneinander für Wasserstoff, Methyl, Ethyl, Benzyl oder Pyridylmethyl stehen oder gemeinsam mit dem Stickstoffatom, an das sie gebunden sind, einen Pyrrolidino-Ring bilden, und
- R⁷: Wasserstoff bedeutet,
sowie ihre Salze, Solvate und Solvate der Salze.

Gleichfalls besonders bevorzugt im Rahmen der vorliegenden Erfindung sind Verbindungen der Formel (I), in welcher
- R¹: Wasserstoff, Ethoxy, n-Propoxy oder eine Gruppe der Formel R⁸-C(=O)-NH- bedeutet, wobei Ethoxy in 2-Position und n-Propoxy in 3-Position durch Methoxy, Ethoxy, Amino, Methylamino, Ethylamino, Isopropylamino, Dimethylamino, Diethylamino, Cyclopropylamino, *N*-Cyclopropyl-*N*-methylamino, Azetidino oder Pyrrolidino substituiert sein können, worin
R⁸ für Pyridyl, Pyrrolyl, Imidazolyl, Pyrazolyl, Triazolyl oder Thiadiazolyl, die jeweils durch Methyl, Ethyl, Fluor oder Chlor substituiert sein können, steht,
- R²: Wasserstoff, Ethoxy oder n-Propoxy bedeutet, wobei Ethoxy in 2-Position und n-Propoxy in 3-Position durch Methoxy, Ethoxy, Amino, Methylamino, Ethylamino, Isopropylamino, Dimethylamino, Diethylamino, Cyclopropylamino, *N*-Cyclopropyl-*N*-methylamino, Azetidino oder Pyrrolidino substituiert sein können,
- wobei R¹ und R²: jedoch nicht gleichzeitig für Wasserstoff stehen,
- R³: Amino bedeutet,
- R⁴: Wasserstoff bedeutet,
- R⁵: Wasserstoff bedeutet,
- n: für die Zahl 0 oder 1 steht,
- R⁶: sich in meta- oder para-Position, relativ zur Verknüpfungsstelle des Phenylrings mit dem Tricyclus, befindet und Fluor, Chlor, Brom, Methyl, Ethyl, Methoxy oder Ethoxy bedeutet,
und
- R⁷: sich in meta- oder para-Position, relativ zur Verknüpfungsstelle des Phenylrings mit dem Tricyclus, und in ortho-Stellung zu R⁶ befindet und Wasserstoff, Fluor, Chlor, Brom, Methyl, Ethyl, Methoxy, Ethoxy oder eine Gruppe der Formel -NR¹¹R¹² bedeutet, worin
R¹¹ und R¹² unabhängig voneinander für Wasserstoff, Methyl, Ethyl, Benzyl oder Pyridylmethyl stehen oder gemeinsam mit dem Stickstoffatom, an das sie gebunden sind, einen Pyrrolidino- oder Piperidino-Ring bilden,
oder
- R⁶ und R⁷: gemeinsam mit dem Phenylring, an den sie gebunden sind, eine Gruppe der Formel 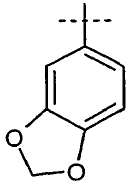
bilden,
sowie ihre Salze, Solvate und Solvate der Salze.

Gleichfalls besonders bevorzugt im Rahmen der vorliegenden Erfindung sind Verbindungen der Formel (I), in welcher
- R¹: Wasserstoff, Ethoxy, n-Propoxy oder eine Gruppe der Formel R⁸-C(=O)-NH- bedeutet, wobei Ethoxy in 2-Position und n-Propoxy in 3-Position durch Methoxy, Ethoxy, Amino, Methylamino, Ethylamino, Isopropylamino, Dimethylamino, Diethylamino, Cyclopropylamino, *N*-Cyclopropyl-*N*-methylamino, Azetidino oder Pyrrolidino substituiert sein können, worin
R⁸ für Pyridyl, Pyrrolyl, Imidazolyl, Pyrazolyl, Triazolyl oder Thiadiazolyl, die jeweils durch Methyl, Ethyl, Fluor oder Chlor substituiert sein können, steht,
- R²: Wasserstoff, Ethoxy oder n-Propoxy bedeutet, wobei Ethoxy in 2-Position und n-Propoxy in 3-Position durch Methoxy, Ethoxy, Amino, Methylamino, Ethylamino, Isopropylamino, Dimethylamino, Diethylamino, Cyclopropylamino, *N*-Cyclopropyl-*N*-methylamino, Azetidino oder Pyrrolidino substituiert sein können,
- wobei entweder R¹ oder R²: für Wasserstoff steht, jedoch nicht beide gleichzeitig Wasserstoff
bedeuten,
- R³: Hydroxy
und
- R⁴: Wasserstoff bedeutet
oder
- R³ und R⁴: gemeinsam mit dem Kohlenstoffatom, an das sie gebunden sind, eine Gruppe der Formel >C=O bilden,
- R⁵: Wasserstoff bedeutet,
- n: für die Zahl 0 oder 1 steht,
- R⁶: sich in meta- oder para-Position, relativ zur Verknüpfungsstelle des Phenylrings mit dem Tricyclus, befindet und Fluor, Chlor, Brom, Methyl, Ethyl, Methoxy oder Ethoxy bedeutet,
und
- R⁷: sich in meta- oder para-Position, relativ zur Verlaiüpfungsstelle des Phenylrings mit dem Tricyclus, und in ortho-Stellung zu R⁶ befindet und Wasserstoff, Fluor, Chlor, Brom, Methyl, Ethyl, Methoxy, Ethoxy oder eine Gruppe der Formel -NR¹¹R¹² bedeutet, worin R¹¹ und R¹² unabhängig voneinander für Wasserstoff, Methyl, Ethyl, Benzyl oder Pyridylmethyl stehen oder gemeinsam mit dem Stickstoffatom, an das sie gebunden sind, einen Pyrrolidino- oder Piperidino-Ring bilden,
oder
- R⁶ und R⁷: gemeinsam mit dem Phenylring, an den sie gebunden sind, eine Gruppe der Formel 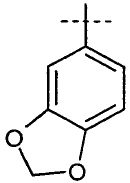
bilden,
sowie ihre Salze, Solvate und Solvate der Salze.

Gleichfalls besonders bevorzugt im Rahmen der vorliegenden Erfindung sind Verbindungen der Formel (I), in welcher
- R¹: Ethoxy, n-Propoxy oder eine Gruppe der Formel R⁸-C(=O)-NH- bedeutet, wobei Ethoxy in 2-Position und n-Propoxy in 3-Position durch Methoxy, Ethoxy, Amino, Methylamino, Ethylamino, Isopropylamino, Dimethylamino, Diethylamino, Cyclopropylamino, *N*-Cyclopropyl-*N*-methylamino, Azetidino oder Pyrrolidino substituiert sind, worin
R⁸ für Pyridyl, Pyrrolyl, Imidazolyl, Pyrazolyl, Triazolyl oder Thiadiazolyl, die jeweils durch Methyl, Ethyl, Fluor oder Chlor substituiert sein können, steht,
- R²: Ethoxy oder n-Propoxy bedeutet, wobei Ethoxy in 2-Position und n-Propoxy in 3-Position durch Methoxy, Ethoxy, Amino, Methylamino, Ethylamino, Isopropylamino, Dimethylamino, Diethylamino, Cyclopropylamino, *N*-Cyclopropyl-*N*-methylamino, Azetidino oder Pyrrolidino substituiert sind,
- R³: Hydroxy
und
- R⁴: Wasserstoff bedeutet
oder
- R³: und R⁴ gemeinsam mit dem Kohlenstoffatom, an das sie gebunden sind, eine Gruppe der Formel >C=O bilden,
- R⁵: Wasserstoff bedeutet,
- n: für die Zahl 0 oder 1 steht,
- R⁶: sich in meta- oder para-Position, relativ zur Verknüpfungsstelle des Phenylrings mit dem Tricyclus, befindet und Fluor, Chlor, Brom, Methyl, Ethyl, Methoxy oder Ethoxy bedeutet,
und
- R⁷: sich in meta- oder para-Position, relativ zur Verknüpfungsstelle des Phenylrings mit dem Tricyclus, und in ortho-Stellung zu R⁶ befindet und Wasserstoff, Fluor, Chlor, Brom, Methyl, Ethyl, Methoxy, Ethoxy oder eine Gruppe der Formel -NR¹¹R¹² bedeutet, worin
R¹¹ und R¹² unabhängig voneinander für Wasserstoff, Methyl, Ethyl, Benzyl oder Pyridylmethyl stehen oder gemeinsam mit dem Stickstoffatom, an das sie gebunden sind, einen Pyrrolidino- oder Piperidino-Ring bilden, oder
- R⁶ und R⁷: gemeinsam mit dem Phenylring, an den sie gebunden sind, eine Gruppe der Formel 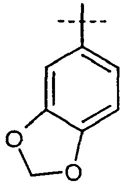
bilden,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Die erfindungsgemäßen Verbindungen der Formel (I), in welcher R⁵ für Wasserstoff steht, können prinzipiell nach den in der WO 00/08007 beschriebenen Verfahren hergestellt werden. Der Inhalt der WO 00/08007, insbesondere die Seiten 14-26, wird hiermit ausdrücklich als Bestandteil der Offenbarung einbezogen. In Abhängigkeit von der spezifischen Bedeutung der Substituenten in (I), insbesondere von R¹ und R², sind jedoch einzelne in der WO 00/08007 beschriebene Verfahrensstufen in manchen Fällen nur mit sehr geringen Ausbeuten verbunden. Weiterer Gegenstand der vorliegenden Erfindung ist daher ein neues Verfahren zur Herstellung der erfmdungsgemäßen Verbindungen der Formel (I), in welcher R⁵ für Wasserstoff steht, dadurch gekennzeichnet, dass man entweder

### [A] Verbindungen der Formel (II)

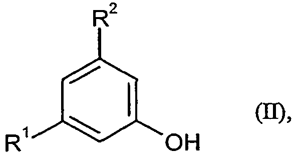

in welcher R¹ und R² jeweils die oben angegebene Bedeutung haben,
in einem inerten Lösungsmittel in Gegenwart einer Base mit einer Verbindung der Formel (III) 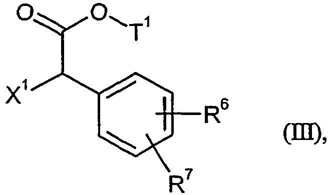 in welcher R⁶ und R⁷ jeweils die oben angegebene Bedeutung haben,
- X¹: für eine geeignete Fluchtgruppe wie beispielsweise Halogen, Mesylat, Tosylat oder Triflat
und
- T¹: für (C₁-C₄)-Alkyl steht,

zu Verbindungen der Formel (IV) 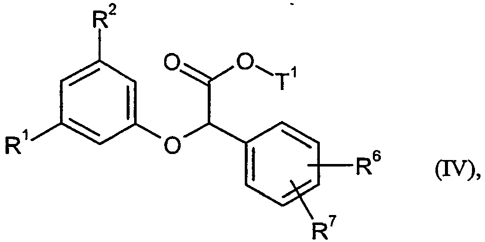 in welcher R¹, R², R⁶, R⁷ und T¹ jeweils die oben angegebene Bedeutung haben,
umsetzt, anschließend durch basische oder saure Hydrolyse in Carbonsäuren der Formel (V) 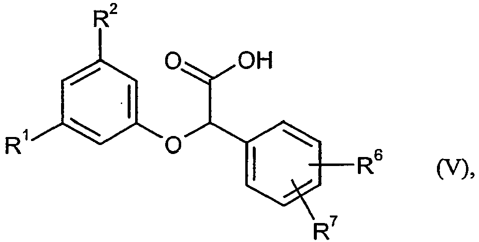 in welcher R¹, R², R⁶ und R⁷ jeweils die oben angegebene Bedeutung haben,
überführt, diese dann nach Aktivierung mit Phosphorylchlorid in Gegenwart einer LewisSäure zu Verbindungen der Formel (VI) 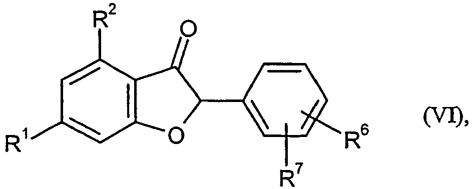 in welcher R¹, R², R⁶ und R⁷ jeweils die oben angegebene Bedeutung haben,
cyclisiert,
oder

### [B] Verbindungen der Formel (VII)

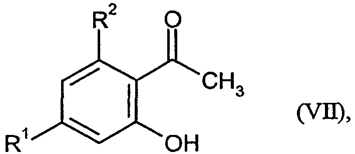

in welcher R¹ und R² jeweils die oben angegebene Bedeutung haben,
zunächst nach üblichen Methoden in Phenacylbromide der Formel (VIII) 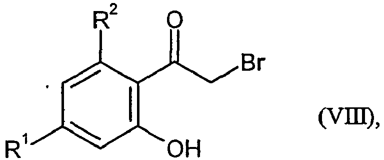 in welcher R¹ und R² jeweils die oben angegebene Bedeutung haben,
überführt und diese dann in Gegenwart einer Base zu Verbindungen der Formel (IX) 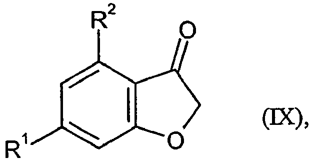 in welcher R¹ und R² jeweils die oben angegebene Bedeutung haben,
cyclisiert, anschließend in einem inerten Lösungsmittel zu Verbindungen der Formel (X) 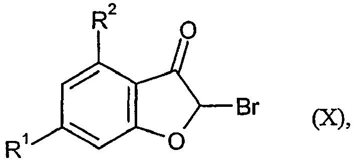 in welcher R¹ und R² jeweils die oben angegebene Bedeutung haben,
bromiert und nach üblichen Methoden in Silylenolether der Formel (XI) 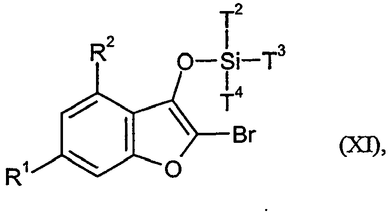 in welcher R¹ und R² jeweils die oben angegebene Bedeutung haben
und
- T², T³ und T⁴: gleich oder verschieden sind und jeweils für (C₁-C₄)-Akyl oder Phenyl stehen,
überführt, nachfolgend in einem inerten Lösungsmittel in Gegenwart eines geeigneten Palladium-Katalysators und einer Base mit einer Verbindung der Formel (XII) 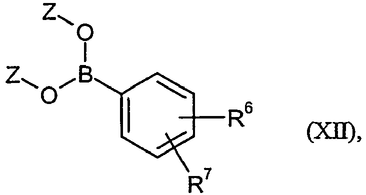 in welcher R⁶ und R⁷ jeweils die oben angegebene Bedeutung haben
und
- Z: für Wasserstoff oder Methyl steht oder beide Z-Gruppen zusammen eine CH₂CH₂-oder C(CH₃)₂-C(CH₃)₂-Brücke bilden,
zu Verbindungen der Formel (XIII) 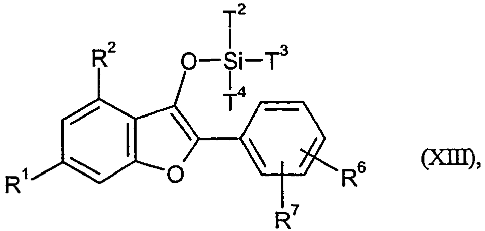 in welcher R¹, R², R⁶, R⁷, T², T³ und T⁴ jeweils die oben angegebene Bedeutung haben, umsetzt, die Silylgruppe anschließend nach üblichen Methoden wieder zu Verbindungen der Formel (VI) abspaltet,
und die jeweils resultierenden Verbindungen der Formel (VI) sodann in einem inerten Lösungsmittel in Gegenwart einer Base gemäß dem in der WO 00/08007 beschriebenen Verfahren mit einem Zimtaldehyd der Formel (XIV) 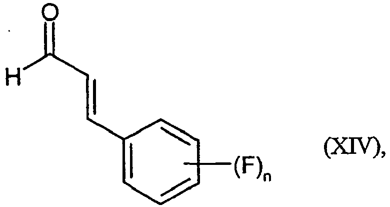 in welcher n die oben angegebene Bedeutung hat,
in Verbindungen der Formel (XV) 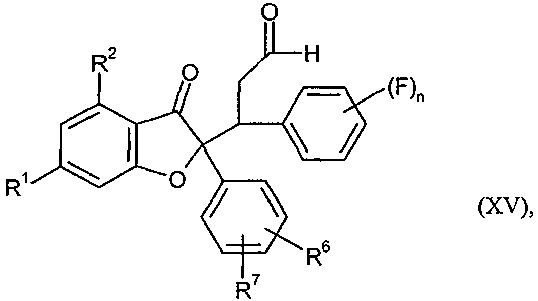 in welcher R¹, R², R⁶, R⁷ und n jeweils die oben angegebene Bedeutung haben,
überführt und diese dann nach der in der WO 00/08007 beschriebenen Reaktionssequenz weiter umsetzt,
und die Verbindungen der Formel (I) gegebenenfalls mit den entsprechenden (i) Lösungsmitteln und/oder (ii) Basen oder Säuren zu ihren Solvaten, Salzen und/oder Solvaten der Salze umsetzt.

Der Verfahrensschritt (VII) → (IX) kann auch über ein dreistufiges Eintopfverfahren über den aus (VII) nach üblichen Methoden erhältlichen Silylenolether der Formel (XVI) 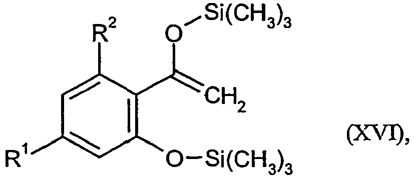 in welcher R¹ und R² jeweils die oben angegebene Bedeutung haben,
dessen Bromierung mit N-Bromsuccinimid sowie anschließende Cyclisierung zu (IX) in Gegenwart von Natronlauge durchgeführt werden.

Die Verbindungen der Formeln (II), (III), (VII), (XII) und (XIV) sind kommerziell erhältlich, literaturbekannt oder können in Analogie zu literaturbekannten Verfahren hergestellt werden.

Inerte Lösungsmittel für den Verfahrensschritt (II) + (III) → (IV) sind beispielsweise Halogenkohlenwasserstoffe wie Dichlormethan, Trichlormethan, Tetrachlormethan, Trichlorethan, Tetrachlorethan, 1,2-Dichlorethan oder Trichlorethylen, Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder andere Lösungsmittel wie Ethylacetat, Aceton, 2-Butanon, Dimethylformamid, Dimethylsulfoxid, Pyridin oder Acetonitril. Ebenso ist es möglich, Gemische der genannten Lösungsmittel einzusetzen. Bevorzugt sind 2-Butanon, Diethylether, Dioxan, Tetrahydrofuran, Dichlormethan, Toluol oder Benzol.

Als Basen für den Verfahrensschritt (II) + (III) → (IV) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate oder -hydrogencarbonate wie Lithium-, Natrium-, Kalium-, Calcium- oder Cäsiumcarbonat, oder Natrium- oder Kaliumhydrogencarbonat, Alkalihydride wie Natriumhydrid, Amide wie Natriumamid, Lithiumbis(trimethylsilyl)amid oder Lithiumdiisopropylamid, oder organische Amine wie Pyridin, Triethylamin, Ethyldiisopropylamin, *N*-Methylmorpholin oder *N*-Methylpiperidin. Besonders bevorzugt sind Natrium- oder Kaliumhydroxid, Natrium- oder Kaliumcarbonat oder Natriumhydrid.

Der Verfahrensschritt (II) + (III) → (IV) wird im Allgemeinen in einem Temperaturbereich von +20°C bis +160°C, bevorzugt von +60°C bis +100°C durchgeführt. Die Umsetzung kann bei normalem, erhöhtem oder erniedrigtem Druck erfolgen (z.B. von 0.5 bis 5 bar). Im Allgemeinen arbeitet man bei Normaldruck.

Inerte Lösungsmittel für den Verfahrensschritt (IV) → (V) sind beispielsweise Wasser, Alkohole wie Methanol, Ethanol, n-Propanol, iso-Propanol oder n-Butanol, Kohlenwasserstoffe wie Benzol oder andere Lösungsmittel wie Aceton, Dimethylformamid, Dimethylsulfoxid oder Acetonitril. Ebenso ist es möglich, Gemische der genannten Lösungsmittel einzusetzen. Bevorzugt sind Methanol, Ethanol, n-Propanol und/oder Wasser.

Als Basen für den Verfahrensschritt (IV) → (V) eignen sich die üblichen anorganischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium- oder Calciumcarbonat, oder Alkali-Alkoholate wie Natrium- oder Kaliummethanolat, Natrium- oder Kaliumethanolat oder Kalium-*tert*.-butylat. Besonders bevorzugt sind Natrium- oder Kaliumhydroxid oder Natrium- oder Kaliumcarbonat.

Als Säuren für den Verfahrensschritt (IV) → (V) eignen sich im Allgemeinen Schwefelsäure, Chlorwasserstoff/Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der *tert.*-Butylester und Salzsäure oder Schwefelsäure im Falle der Methylester.

Der Verfahrensschritt (IV) → (V) wird im Allgemeinen in einem Temperaturbereich von 0°C bis +100°C, bevorzugt von +40°C bis +80°C durchgeführt. Die Umsetzung kann bei normalem, erhöhtem oder erniedrigtem Druck erfolgen (z.B. von 0.5 bis 5 bar). Im Allgemeinen arbeitet man bei Normaldruck.

Der Verfahrensschritt (V) → (VI) wird bevorzugt ohne weiteres Lösungsmittel durchgeführt. Als Lewis-Säuren eignen sich für diesen Verfahrensschritt die üblichen anorganischen Lewis-Säuren wie beispielsweise Aluminiumtrichlorid, Eisentrichlorid, Bortrifluorid, Bortrichlorid, Bortribromid, Titantetrachlorid, Titantrichlorid, Zinndichlorid, Zinntetrachlorid oder Zinkdichlorid. Bevorzugt ist Zinkdichlorid.

Der Verfahrensschritt (V) → (VI) wird im Allgemeinen in einem Temperaturbereich von 0°C bis +100°C, bevorzugt von 0°C bis +40°C durchgeführt. Die Umsetzung kann bei normalem, erhöhtem oder erniedrigtem Druck erfolgen (z.B. von 0.5 bis 5 bar). Im Allgemeinen arbeitet man bei Normaldruck.

Inerte Lösungsmittel für die Verfahrensschritte (VII) → (VIII) und (IX) → (X) sind beispielsweise Halogenkohlenwasserstoffe wie Dichlormethan, Trichlormethan, Tetrachlormethan, Trichlorethan, Tetrachlorethan, 1,2-Dichlorethan oder Trichlorethylen, Ether wie Diethylether, Dioxan, Tetrahydrofuran, Glykoldimethylether oder Diethylenglykoldimethylether, Kohlenwasserstoffe wie Hexan oder Cyclohexan, oder andere Lösungsmittel wie Ethylacetat, Dimethylformamid oder Dimethylsulfoxid. Ebenso ist es möglich, Gemische der genannten Lösungsmittel einzusetzen. Bevorzugt sind Diethylether, Dioxan, Tetrahydrofuran, Ethylacetat, Trichlormethan und/oder Tetrachlormethan.

Als Bromierungsmittel für die Verfahrensschritte (VII) → (VIII) und (IX) → (X) eignen sich die üblichen anorganischen oder organischen Reagenzien. Hierzu gehören bevorzugt Brom, N-Bromsuccinimid, Kupferdibromid, Pyridinhydrotribromid, Dimethylbenzylammoniumtribromid oder Phenyltrimethylammoniumtribromid. Besonders bevorzugt sind Brom und Kupferdibromid.

Die Verfahrensschritte (VII) → (VIII) und (IX) → (X) werden im Allgemeinen in einem Temperaturbereich von -20°C bis +150°C, bevorzugt von 0°C bis +80°C durchgeführt. Die Umsetzung kann bei normalem, erhöhtem oder erniedrigtem Druck erfolgen (z.B. von 0.5 bis 5 bar). Im Allgemeinen arbeitet man bei Normaldruck.

Inerte Lösungsmittel für den Verfahrensschritt (VIII) → (IX) sind beispielsweise Wasser, Alkohole wie Methanol, Ethanol, n-Propanol, iso-Propanol, n-Butanol oder *tert*.-Butanol, Halogenkohlenwasserstoffe wie Dichlormethan, Trichlormethan, Tetrachlormethan, Trichlorethan, Tetrachlorethan, 1,2-Dichlorethan oder Trichlorethylen, Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder andere Lösungsmittel wie Dimethylformamid, Dimethylsulfoxid, Pyridin oder Acetonitril. Ebenso ist es möglich, Gemische der genannten Lösungsmittel einzusetzen. Bevorzugt sind Methanol, Ethanol, Wasser und/oder Tetrahydrofuran.

Als Basen für den Verfahrensschritt (VIII) → (IX) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate oder -hydrogencarbonate wie Lithium-, Natrium-, Kalium-, Calcium- oder Cäsiumcarbonat, oder Natrium- oder Kaliumhydrogencarbonat, Alkali-Alkoholate wie Natrium- oder Kaliummethanolat, Natrium- oder Kaliumethanolat oder Kalium-*tert*.-butylat, Alkali-Acetate wie Natrium- oder Kaliumacetat, Alkalihydride wie Natriumhydrid, Amide wie Natriumamid, Lithiumbis(trimethylsilyl)amid oder Lithiumdiisopropylamid, oder organische Amine wie Pyridin, Triethylamin, Ethyldiisopropylamin, *N*-Methylmorpholin oder *N*-Methylpiperidin. Besonders bevorzugt sind Natrium- oder Kaliumhydroxid oder Natriumacetat.

Der Verfahrensschritt (VIII) → (IX) wird im Allgemeinen in einem Temperaturbereich von 0°C bis +100°C, bevorzugt von +20°C bis +80°C durchgeführt. Die Umsetzung kann bei normalem, erhöhtem oder erniedrigtem Druck erfolgen (z.B. von 0.5 bis 5 bar). Im Allgemeinen arbeitet man bei Normaldruck.

Inerte Lösungsmittel für den Verfahrensschritt (X) → (XI) sind beispielsweise Halogenkohlenwasserstoffe wie Dichlormethan, Trichlormethan, Tetrachlormethan, Trichlorethan, Tetrachlorethan, 1,2-Dichlorethan oder Trichlorethylen, Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder andere Lösungsmittel wie Dimethylformamid, Dimethylsulfoxid, Pyridin oder Acetonitril. Ebenso ist es möglich, Gemische der genannten Lösungsmittel einzusetzen. Bevorzugt sind Toluol, Hexan, Diethylether oder Tetrahydrofuran.

Als Basen für den Verfahrensschritt (X) → (XI) eignen sich die üblichen anorganischen oder organischen Basen. Hierzu gehören bevorzugt Alkalihydride wie Natriumhydrid, Amide wie Natriumamid, Lithiumbis(trimethylsilyl)amid oder Lithiumdiisopropylamid, oder organische Amine wie Pyridin, Triethylamin, Ethyldiisopropylamin, *N*-Methylmorpholin oder *N*-Methylpiperidin. Besonders bevorzugt sind Lithiumdiisopropylamid, Triethylamin oder Ethyldiisopropylamin.

Der Verfahrensschritt (X) → (XI) wird im Allgemeinen in einem Temperaturbereich von -20°C bis +50°C, bevorzugt von 0°C bis +30°C durchgeführt. Die Umsetzung kann bei normalem, erhöhtem oder erniedrigtem Druck erfolgen (z.B. von 0.5 bis 5 bar). Im Allgemeinen arbeitet man bei Normaldruck.

Inerte Lösungsmittel für den Verfahrensschritt (XI) + (XII) → (XIII) sind beispielsweise Halogenkohlenwasserstoffe wie Dichlormethan, Trichlormethan, Tetrachlormethan, Trichlorethan, Tetrachlorethan, 1,2-Dichlorethan oder Trichlorethylen, Ether wie Diethylether, Dioxan, Tetrahydrofuran, Glykoldimethylether oder Diethylenglykoldimethylether, Alkohole wie Methanol, Ethanol, n-Propanol, iso-Propanol, n-Butanol oder *tert*.-Butanol, Kohlenwasserstoffe wie Benzol, Toluol, Xylol, Hexan, Cyclohexan oder Erdölfraktionen, oder andere Lösungsmittel wie Ethylacetat, Aceton, Wasser, Dimethylformamid, Dimethylsulfoxid, Pyridin oder Acetonitril. Ebenso ist es möglich, Gemische der genannten Lösungsmittel einzusetzen. Bevorzugt sind Toluol, Tetrahydrofuran, Dioxan oder Dimethylformamid.

Als Basen für den Verfahrensschritt (XI) + (XII) → (XIII) eignen sich die üblichen anorganischen oder organischen Basen. Hierzu gehören bevorzugt Alkalihydroxide wie beispielsweise Lithium-, Natrium- oder Kaliumhydroxid, Alkali-, Erdalkali- oder Schwermetallcarbonate wie Silber-, Thallium-, Lithium-, Natrium-, Kalium-, Cäsium- oder Calciumcarbonat, Alkali- oder Erdalkalihydrogencarbonate wie Natrium- oder Kaliumhydrogencarbonat, Alkali-Alkoholate wie Natrium-oder Kaliummethanolat, Natrium- oder Kaliumethanolat oder Lithium-, Natrium- oder Kalium-*tert*.-butylat, Alkalihydride wie Natriumhydrid, Amide wie Natriumamid, Lithium- oder Natriumbis(trimethylsilyl)amid oder Lithiumdiisopropylamid, oder organische Amine wie Pyridin, Triethylamin, Ethyldiisopropylamin, 1,5-Diazabicyclo[5.4.0]undec-5-en (DBU), *N*-Methylmorpholin oder *N*-Methylpiperidin. Besonders bevorzugt sind Cäsium- oder Natriumcarbonat, Natriumhydrid, Kalium-*tert*.-butylat, Lithiumdiisopropylamid, DBU, Triethylamin oder Ethyldiisopropylamin.

Als Katalysatoren für den Verfahrensschritt (XI) + (XII) → (XIII) eignen sich die für Suzuki-Reaktionsbedingungen üblichen Palladium-Katalysatoren. Bevorzugt sind Katalysatoren wie beispielsweise Dichlorbis(triphenylphosphin)palladium, Tetrakis(triphenylphosphin)palladium(0), Palladium(II)acetat oder Bis(diphenylphosphanferrocenyl)palladium(II)chlorid. Als Katalysatorliganden eignen sich bevorzugt die für Suzuki-Reaktionen üblichen Liganden wie beispielsweise Triphenylphospin, Tri(o-tolyl)phosphin, Tributylphosphin, 2,2'-Bis(diphenylphosphino)-1,1'-bi-naphthyl (BINAP), 1,1'-Bis(diphenylphosphino)ferrocen (dppf) oder 1,3-Bis(diphenylphosphino)-propan (dppp).

Der Verfahrensschritt (XI) + (XII) → (XIII) wird im Allgemeinen in einem Temperaturbereich von +20°C bis +200°C, bevorzugt von +50°C bis +150°C durchgeführt. Die Umsetzung kann bei normalem, erhöhtem oder erniedrigtem Druck erfolgen (z.B. von 0.5 bis 5 bar). Im Allgemeinen arbeitet man bei Normaldruck.

Inerte Lösungsmittel für den Verfahrensschritt (XIII) → (VI) sind beispielsweise Halogenkohlenwasserstoffe wie Dichlormethan, Trichlormethan, Tetrachlormethan, Trichlorethan, Tetrachlorethan, 1,2-Dichlorethan oder Trichlorethylen, Ether wie Diethylether, Dioxan, Tetrahydrofuran, Glykoldimethylether oder Diethylenglykoldimethylether, Alkohole wie Methanol, Ethanol, n-Propanol, iso-Propanol, n-Butanol oder *tert*.-Butanol, Kohlenwasserstoffe wie Benzol, Toluol, Xylol, Hexan oder Cyclohexan, oder andere Lösungsmittel wie Dimethylformamid, Dimethylsulfoxid, Acetonitril oder Wasser. Ebenso ist es möglich, Gemische der genannten Lösungsmittel einzusetzen. Bevorzugt sind Methanol, Ethanol, Wasser, Tetrahydrofuran oder Dioxan.

Die Abspaltung der Silylgruppe im Verfahrensschritt (XIII) → (VI) kann nach den üblichen Methoden alternativ mit Hilfe einer Base oder mit Hilfe einer Säure durchgeführt werden. Als Basen eignen sich bevorzugt Tetrabutylammoniumfluorid, Pyridin oder Triethylamin, als Säuren bevorzugt Fluorwasserstoff, Chlorwasserstoff/Salzsäure, Ameisensäure, Essigsäure, Trifluoressigsäure oder Toluolsulfonsäure.

Der Verfahrensschritt (XIII) → (VI) wird im Allgemeinen in einem Temperaturbereich von -80°C bis +100°C, bevorzugt von 0°C bis +80°C durchgeführt. Die Umsetzung kann bei normalem, erhöhtem oder erniedrigtem Druck erfolgen (z.B. von 0.5 bis 5 bar). Im Allgemeinen arbeitet man bei Normaldruck.

Die erfindungsgemäßen Verbindungen der Formel (I), in welcher R⁵ für Mono- oder Di-(C₁-C₆)-alkylaminocarbonyl steht, können hergestellt werden, indem man Verbindungen der Formel (XVII) 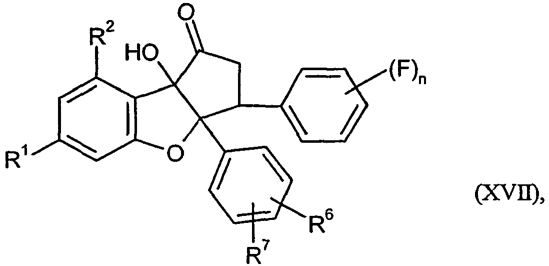 in welcher R¹, R², R⁶, R⁷ und n jeweils die oben angegebene Bedeutung haben,
zunächst in einem inerten Lösungsmittel mit Methoxymagnesiummethylcarbonat [M. Stiles, J. Amer. Chem. Soc. 81, 2598 (1959)] in Carbonsäuren der Formel (XVIII) 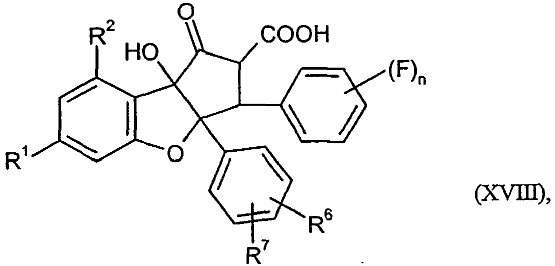 in welcher R¹, R², R⁶, R⁷ und n jeweils die oben angegebene Bedeutung haben,
überführt, anschließend in Gegenwart eines Kondensationsmittels und einer Base mit einer Verbindung der Formel (XIX)

HNR¹³R¹⁴ (XIX),

in welcher
- R¹³: für Wasserstoff oder (C₁-C₆)-Alkyl
und
- R¹⁴: für (C₁-C₆)-Alkyl steht,
zu Verbindungen der Formel (XX) 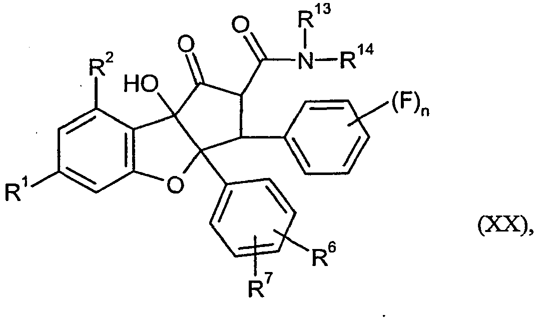 in welcher R¹, R², R⁶, R⁷, R¹³, R¹⁴ und n jeweils die oben angegebene Bedeutung haben,
umsetzt und diese dann gegebenenfalls nach den in der WO 00/08007 beschriebenen Reaktionssequenzen weiter umwandelt.

Die Verbindungen der Formel (XVII) sind nach den oben bzw. in WO 00/08007 beschriebenen Verfahren erhältlich. Die Verbindungen der Formel (XIX) sind kommerziell erhältlich oder literaturbekannt.

Inerte Lösungsmittel für den Verfahrensschritt (XVII) → (XVIII) sind beispielsweise Halogenkohlenwasserstoffe wie Dichlormethan, Trichlormethan, Tetrachlormethan, Trichlorethan, Tetrachlorethan, 1,2-Dichlorethan oder Trichlorethylen, Ether wie Diethylether, Dioxan, Tetrahydrofuran, Glykoldimethylether oder Diethylenglykoldimethylether, oder andere Lösungsmittel wie Dimethylformamid, Dimethylsulfoxid oder Acetonitril. Ebenso ist es möglich, Gemische der genannten Lösungsmittel einzusetzen. Bevorzugt ist Dimethylformamid.

Der Verfahrensschritt (XVII) → (XVIII) wird im Allgemeinen in einem Temperaturbereich von 0°C bis +200°C, bevorzugt von +50°C bis +150°C durchgeführt. Die Umsetzung kann bei normalem, erhöhtem oder erniedrigtem Druck erfolgen (z.B. von 0.5 bis 5 bar). Im Allgemeinen arbeitet man bei Normaldruck.

Inerte Lösungsmittel für den Verfahrensschritt (XVIII) + (XIX) → (XX) sind beispielsweise Halogenkohlenwasserstoffe wie Dichlormethan, Trichlormethan, Tetrachlormethan, Trichlorethan, Tetrachlorethan, 1,2-Dichlorethan oder Trichlorethylen, Ether wie Diethylether, Methyl-*tert*.-butylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, oder andere Lösungsmittel wie Ethylacetat, Aceton, 2-Butanon, Dimethylformamid, Dimethylacetamid, Dimethylsulfoxid, Acetonitril, *N*-Methylpyrrolidon oder Pyridin. Ebenso ist es möglich, Gemische der genannten Lösungsmittel einzusetzen. Bevorzugt sind Dimethylformamid und Tetrahydrofuran.

Als Kondensationsmittel für die Amidbildung im Verfahrensschritt (XVIII) + (XIX) → (XX) eignen sich beispielsweise Carbodiimide wie *N,N*'-Diethyl-, *N,N*'-Dipropyl-, *N,N*'-Diisopropyl-, *N,N*'-Dicyclohexylcarbodiimid (DCC), *N*-(3-Dimethylaminoisopropyl)-N'-ethylcarbodiimid-Hydrochlorid (EDC), N-Cyclohexylcarbodiimid-*N*-propyloxymethyl-Polystyrol (PS-Carbodiimid) oder Phosgen-Derivate wie *N,N'*-Carbonyldiimidazol, oder 1,2-Oxazoliumverbindungen wie 2-Ethyl-5-phenyl-1,2-oxazolium-3-sulfat oder 2-*tert*.-Butyl-5-methyl-isoxazolium-perchlorat, oder Acylaminoverbindungen wie 2-Ethoxy-1-ethoxycarbonyl-1,2-dihydrochinolin, oder Isobutylchlorformiat, Propanphosphonsäureanhydrid, Cyanophosphonsäurediethylester, Bis-(2-oxo-3-oxazolidinyl)-phosphorylchlorid, Benzotriazol-1-yloxy-tris(dimethylamino)phosphonium-hexafluorophosphat (BOP), Benzotriazol-1-yloxy-tris(pyrrolidino)phosphonium-hexafluorophosphat (PyBOP), *O-*(Benzotriazol-1-yl)-*N,N,N',N*'-tetramethyluronium-hexafluorophosphat (HBTU), 2-(2-Oxo-1-(2*H*)-pyridyl)-1,1,3,3-tetramethyluronium-tetrafluoroborat (TPTU) oder *O*-(7-Azabenzotriazol-1-yl)-*N,N,N',N'*-tetramethyluronium-hexafluorophosphat (HATU), gegebenenfalls in Kombination mit weiteren Hilfsstoffen wie 1-Hydroxybenzotriazol (HOBt) oder N-Hydroxysuccinimid (HOSu), sowie als Basen Alkalicarbonate, z.B. Natrium- oder Kaliumcarbonat oder -hydrogencarbonat, oder organische Amin-Basen wie z.B. Triethylamin, *N*-Methylmorpholin, *N*-Methylpiperidin, *N,N-*Diisopropylethylamin, Pyridin, 4-*N,N-*Dimethylaminopyridin, 1,5-Diazabicyclo[4.3.0]non-5-en (DBN) oder 1,8-Diazabicyclo[5.4.0]undec-7-en (DBU). Bevorzugt sind BOP, PyBOP oder HATU jeweils in Kombination mit Triethylamin oder *N,N*-Diisopropylethylamin.

Der Verfahrensschritt (XVIII) + (XIX) → (XX) wird im Allgemeinen in einem Temperaturbereich von -20°C bis +100°C, bevorzugt von 0°C bis +50°C durchgeführt. Die Umsetzung kann bei normalem, erhöhtem oder erniedrigtem Druck erfolgen (z.B. von 0.5 bis 5 bar). Im Allgemeinen arbeitet man bei Normaldruck.

Die erfindungsgemäßen Verbindungen der Formel (I), in welcher R¹ und R² jeweils für Ethoxy oder n-Propoxy stehen, die in 2- bzw. 3-Position durch Amino, Mono- oder Di-(C₁-C₆)-alkylamino, (C₃-C₈)-Cycloalkylamino, *N*-(C₃-C₈)-Cycloalkyl-*N*-(C₁-C₆)-alkylamino oder durch einen über ein N-Atom gebundenen 4- bis 7-gliedrigen Heterocyclus substituiert sind, können auch hergestellt werden, indem man Verbindungen der Formel (XXI) 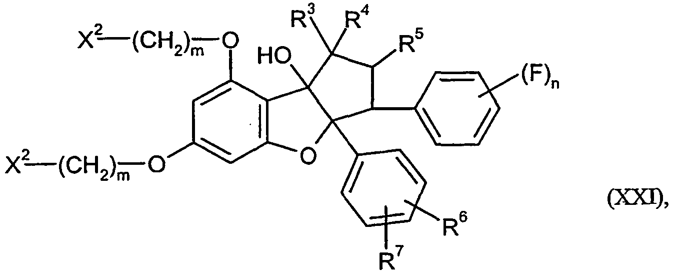 in welcher R³, R⁴, R⁵, R⁶, R⁷ und n jeweils die oben angegebene Bedeutung haben,
- X²: für eine geeignete Fluchtgruppe wie beispielsweise Halogen, Mesylat oder Tosylat
und
- m: für die Zahl 2 oder 3 steht,
in einem inerten Lösungsmittel gegebenenfalls in Gegenwart einer Hilfsbase mit einer Verbindung der Formel (XXII)

HNR¹⁵R¹⁶ (XXII),

in welcher
- R¹⁵: für Wasserstoff, (C₁-C₆)-Alkyl oder (C₃-C₈)-Cycloalkyl
und
- R¹⁶: für Wasserstoff oder (C₁-C₆)-Alkyl steht
oder
- R¹⁵ und R¹⁶: gemeinsam mit dem Stickstoffatom, an das sie gebunden sind, einen 4- bis 7-gliedrigen Heterocyclus bilden,
zu Verbindungen der Formel (XXIII) 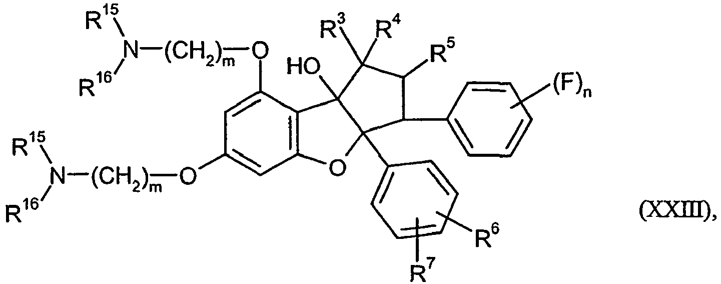 in welcher R³, R⁴, R⁵, R⁶, R⁷, R¹⁵, R¹⁶ m und n jeweils die oben angegebene Bedeutung haben,
umsetzt und diese dann gegebenenfalls nach den in der WO 00/08007 beschriebenen Reaktionssequenzen weiter modifiziert.

Die Verbindungen der Formel (XXI) sind nach den oben bzw. in WO 00/08007 beschriebenen Verfahren erhältlich. Die Verbindungen der Formel (XXII) sind kommerziell erhältlich oder literaturbekannt.

Inerte Lösungsmittel für den Verfahrensschritt (XXI) + (XXII) → (XXIII) sind beispielsweise Ether wie Tetrahydrofuran, Dioxan, Glykoldimethylether oder Diethylenglykoldimethylether, Alkohole wie Methanol, Ethanol, n-Propanol, iso-Propanol, n-Butanol oder *tert*.-Butanol, Kohlenwasserstoffe wie Toluol oder Xylol, oder andere Lösungsmittel wie Aceton, Dimethylformamid oder Dimethylsulfoxid. Ebenso ist es möglich, Gemische der genannten Lösungsmittel einzusetzen. Bevorzugt sind Ethanol, Dimethylformamid, Dimethylsulfoxid oder Xylol.

Als Hilfsbasen für den Verfahrensschritt (XXI) + (XXII) → (XXIII) eignen sich die üblichen anorganischen oder organischen Basen. Hierzu gehören bevorzugt Alkalihydroxide wie beispielsweise Natrium- oder Kaliumhydroxid, Alkali- oder Erdalkalicarbonate wie Natrium- oder Kaliumcarbonat, Alkalihydride wie Natriumhydrid, oder organische Amine wie Triethylamin oder Ethyldiisopropylamin.

Der Verfahrensschritt (XXI) + (XXII) → (XXIII) wird im Allgemeinen in einem Temperaturbereich von +20°C bis +200°C, bevorzugt von +70°C bis +150°C durchgeführt. Die Umsetzung kann bei normalem, erhöhtem oder erniedrigtem Druck erfolgen (z.B. von 0.5 bis 5 bar). Im Allgemeinen arbeitet man bei Normaldruck.

Die erfindungsgemäßen Verbindungen können, falls zweckmäßig, auch hergestellt werden durch synthetische Umwandlungen von funktionellen Gruppen einzelner Substituenten in Verbindungen der Formel (I), die nach den zuvor beschriebenen Verfahren erhalten werden. Solche Umwandlungen funktioneller Gruppen werden nach literaturüblichen Methoden durchgeführt und umfassen beispielsweise Verfahren zur Alkylierung, Acylierung, Aminierung, Veresterung, Esterspaltung, Hydrierung, Oxidation und Reduktion.

Die Herstellung der erfindungsgemäßen Verbindungen kann durch die folgenden Syntheseschemata veranschaulicht werden:

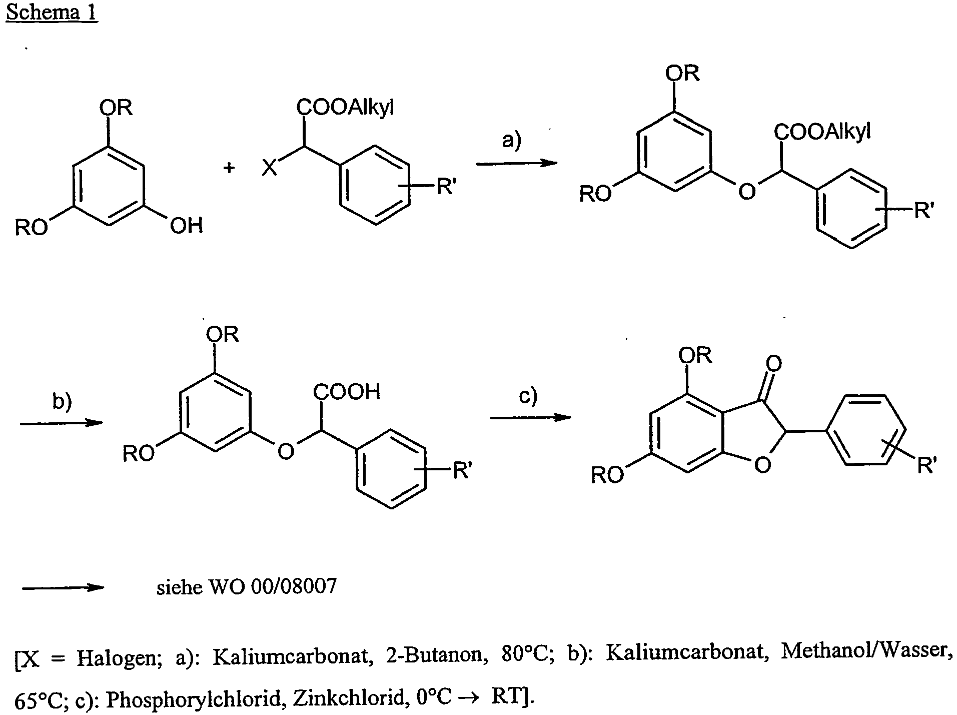

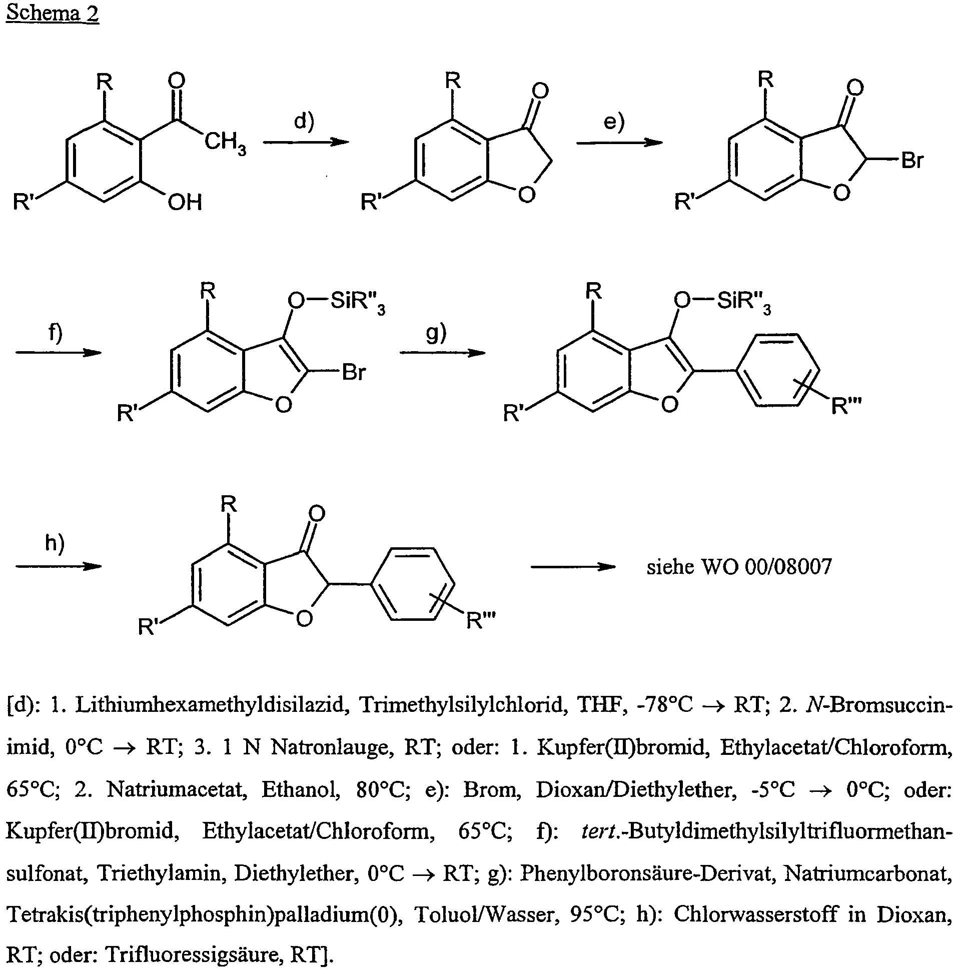

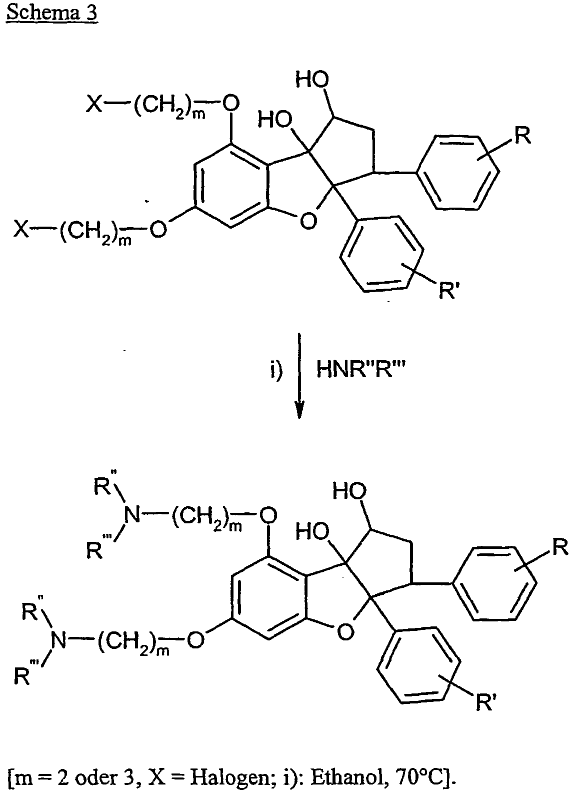

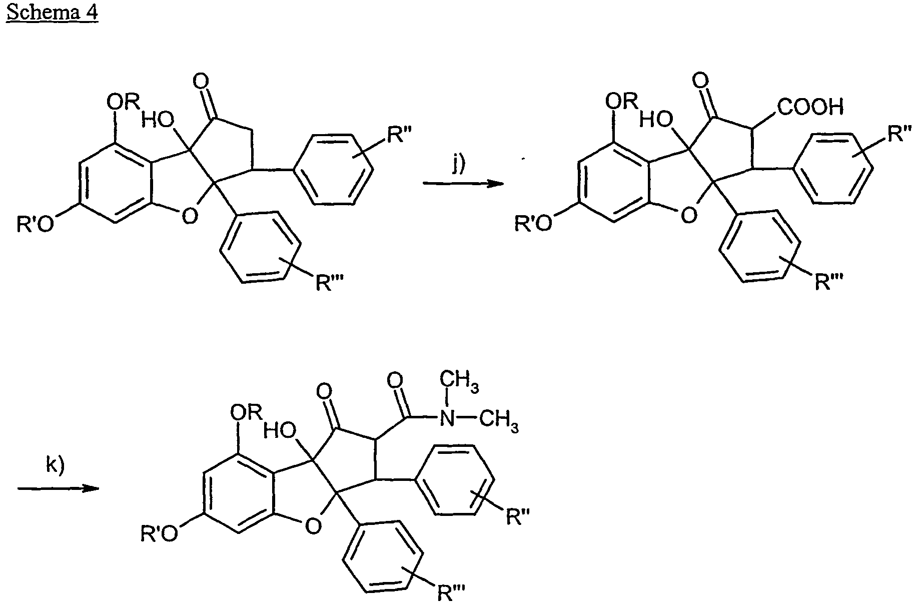

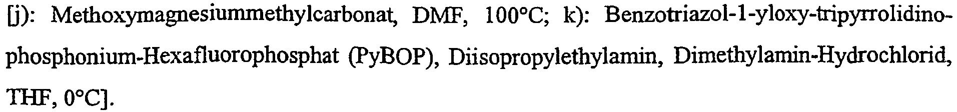

Die erfindungsgemäßen Verbindungen besitzen wertvolle pharmakologische Eigenschaften und können zur Vorbeugung und Behandlung von Erkrankungen bei Menschen und Tieren verwendet werden. Sie zeichnen sich zudem durch eine erhöhte metabolische Stabilität im Vergleich zu den in der WO 00/08007 beschriebenen Verbindungen aus.

Die erfindungsgemäßen Verbindungen sind potente Inhibitoren/Modulatoren der NF-κB- und/oder AP-1-Aktivität und eignen sich als solche insbesondere zur Behandlung chronischer Entzündungs-und Autoimmunkrankheiten (wie z.B. die Crohn'sche Krankheit, ulzerative Colitis, rheumatoide Arthritis, Psoriasis, Multiple Sklerose, Lupus, Asthma, Diabetes), kardiovaskulärer Erkrankungen (wie z.B. koronare Herzerkrankungen, Myokard-Infarkt, Artherosklerose, Restenose, Thrombosen), fibrotischer Erkrankungen der Leber und anderer Organe, cerebrovaskulärer Erkrankungen (wie z.B. Schlaganfall, Schädel-Hirn-Trauma, Rückenmarksverletzungen) und chronischer neurodegenerativer Erkrankungen (wie z.B. die Alzheimer'sche Krankheit, die Parkinson'sche Krankheit, Chorea Huntington, Amyotrophe Lateralsklerose, periphere Neuropathien und chronischer Schmerz). Darüber hinaus können sie zur Prophylaxe und/oder Therapie von Strahlenschäden, Transplantatabstoßung, Sepsis und septischem Schock sowie der bakteriellen Meningitis verwendet werden.

Weiterhin eignen sich die erfindungsgemäßen Verbindungen aufgrund ihrer inhibitorischen/modulatorischen Aktivität auf die NF-κB- und/oder AP-1-vermittelte Signalübertragung zur Behandlung hyperproliferativer Erkrankungen wie solider Tumoren (wie z.B. Brustkrebs, Lungenkrebs, Tumore des Gehirns und des Nervensystems, Hautkrebs, Leberkrebs, Tumore der Reproduktionsorgane, Tumore des Verdauungstraktes, Blasenkrebs, Tumore der Harnwegssysteme, Tumore verschiedener Hormondrüsen, Tumore des Auges), Lymphomen (wie z.B. Hodgkin'sche Krankheit, Lymphome des zentralen Nervensystems), Sarkomen (wie z.B. Osteaosarkome, Lymphosarkome) und Leukämien (wie z.B. akute myeloide Leukämie, lymphoblastische Leukämien, myelogene Leukämien). Ferner können die erfindungsgemäßen Verbindungen zur Prophylaxe und/oder Therapie viraler Erkrankungen, insbesondere durch HIV, HTLV, Epstein-Barr-Virus, Zytomegalievirus (CMV) und/oder Adenoviren ausgelöster Erkrankungen, eingesetzt werden.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfmdung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer wirksamen Menge von mindestens einer der erfmdungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfmdung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise cytostatisch oder cytotoxisch wirkende Substanzen, entzündungshemmende Substanzen (z.B. Kortikosteroide, NSAIDs) und neuroprotektive wirkende Substanzen genannt.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Weiterer Gegenstand der vorliegenden Erfindung sind Arzneimittel, die mindestens eine erfmdungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgabe über den Tag zu verteilen.

Die nachfolgenden Ausführungsbeispiele erläutern die Erfindung.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- DCI: direkte chemische Ionisation (bei MS)
- DMF: *N,N-*Dimethylformamid
- DMSO: Dimethylsulfoxid
- d. Th.: der Theorie (bei Ausbeute)
- ESI: Elektrospray-Ionisation (bei MS)
- H: Stunde(n)
- HPLC: Hochdruck- / Hochleistungsflüssigchromatographie
- Kp.: Siedepunkt
- LC-MS: Flüssigchromatographie mit gekoppelter Massenspektroskopie
- min.: Minute(n)
- MS: Massenspektroskopie
- NMR: Kernresonanzspektroskopie
- RF: Rückfluss
- RT: Raumtemperatur (20°C)
- Rₜ: Retentionszeit (bei HPLC)
- TEA: Triethylamin
- tert.: tertiär
- THF: Tetrahydrofuran
- UV: Ultraviolett
- v/v: Volumen zu Volumen (bei flüssig/flüssig-Gemischen)

### HPLC- und LC/MS-Methoden:

### Methode 1:

Instrument: Micromass Platform LCZ mit HPLC Agilent Serie 1100; Säule: Grom-Sil 120 ODS-4 HE, 50 mm x 2.0 mm, 3 µm; Eluent A: 1 1 Wasser + 1 ml 50%-ige Ameisensäure, Eluent B: 1 1 Acetonitril + 1 ml 50%-ige Ameisensäure; Gradient: 0.0 min 100% A → 0.2 min 100% A → 2.9 min 30% A → 3.1 min 10% A → 4.5 min 10% A; Ofen: 55°C; Fluss: 0.8 ml/min; UV-Detektion: 208-400 nm.

### Methode 2:

Instrument: Micromass Quattro LCZ mit HPLC Agilent Serie 1100; Säule: Grom-Sil 120 ODS-4 HE, 50 mm x 2.0 mm, 3 µm; Eluent A: 1 1 Wasser + 1 ml 50%-ige Ameisensäure, Eluent B: 1 1 Acetonitril + 1 ml 50%-ige Ameisensäure; Gradient: 0.0 min 100% A → 0.2 min 100% A → 2.9 min 30% A → 3.1 min 10% A → 4.5 min 10% A; Ofen: 55°C; Fluss: 0.8 ml/min; UV-Detektion: 208-400 nm.

### Methode 3:

Gerätetyp MS: Micromass ZQ; Gerätetyp HPLC: Waters Alliance 2790; Säule: Grom-Sil 120 ODS-4 HE, 50 mm x 2 mm, 3.0 µm; Eluent B: Acetonitril + 0.05% Ameisensäure, Eluent A: Wasser + 0.05% Ameisensäure; Gradient: 0.0 min 5% B → 2.0 min 40% B → 4.5 min 90% B → 5.5 min 90% B; Ofen: 45°C; Fluss: 0.0 min 0.75 ml/min → 4.5 min 0.75 ml/min → 5.5 min 1.25 ml/min; UV-Detektion: 210 nm.

### Methode 4:

Gerätetyp MS: Micromass ZQ; Gerätetyp HPLC: TSP P4000, TSP AS300, TSP UV3000; Säule: Grom-Sil 120 ODS-4 HE, 50 mm x 2 mm, 3.0 µm; Eluent A: Wasser + 250 µl 50%-ige Ameisensäure / I, Eluent B: Acetonitril + 250 µl 50%-ige Ameisensäure / 1; Gradient: 0.0 min 0% B → 0.2 min 0% B → 2.9 min 70% B → 3.1 min 90% B → 4.5 min 90% B; Ofen: 50°C; Fluss: 0.8 ml/min; UV-Detektion: 210 nm.

### Methode 5:

Gerätetyp MS: Micromass ZQ; Gerätetyp HPLC: Waters Alliance 2790; Säule: Grom-Sil 120 ODS-4 HE, 50 mm x 2 mm, 3.0 µm; Eluent B: Acetonitril + 500 µl 50%-ige Ameisensäure /1; Eluent A: Wasser + 500 µl 50%-ige Ameisensäure / 1; Gradient: 0.0 min 0% B -+ 0.2 min 0% B → 2.9 min 70% B → 3.1 min 90% B → 4.5 min 90% B; Ofen: 50°C; Fluss: 0.8 ml/min; UV-Detektion: 210 nm.

### Methode 6:

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A, Fluss 1 ml/min → 2.5 min 30% A, Fluss 2 ml/min → 3.0 min 5% A, Fluss 2 ml/min → 4.5 min 5% A, Fluss 2 ml/min; Ofen: 50°C; UV-Detektion: 210 nm.

### Methode 7:

Instrument: Micromass Platform LCZ mit HPLC Agilent Serie 1100; Säule: Phenomenex Synergi 2µ Hydro-RP Mercury 20 mm x 4 mm; Eluent A: 11 Wasser + 0.5 ml 50%-ige Ameisensäure, Eluent B: 11Acetonitril + 0.5 ml 50%-ige Ameisensäure; Gradient: 0.0 min 90% A, Fluss 1ml/min → 2.5 min 30% A, Fluss 2 ml/min → 3.0 min 5% A, Fluss 2 ml/min → 4.5 min 5% A, Fluss 2 ml/min; Ofen: 50°C; UV-Detektion: 210 nm.

### Methode 8:

Instrument: Micromass Platform LCZ mit HPLC Agilent Serie 1100; Säule: Grom-Sil 120 ODS-4 HE, 50 mm x 2.0 mm, 3 µm; Eluent A: 1 1 Wasser + 1 ml 50%-ige Ameisensäure, Eluent B: 1 1 Acetonitril + 1 ml 50%-ige Ameisensäure; Gradient: 0.0 min 100% A → 0.2 min 100% A → 2.9 min 30% A → 3.1 min 10% A → 4.5 min 10% A; Ofen: 55°C; Fluss: 0.8 ml/min; UV-Detektion: 210 nm.

### Methode 9:

Gerätetyp MS: Micromass ZQ; Gerätetyp HPLC: Waters Alliance 2795; Säule: Merck Chromolith SpeedROD RP-18e 50 mm x 4.6 mm; Eluent A: Wasser + 500 µl 50%-ige Ameisensäure / 1; Eluent B: Acetonitril + 500 µl 50%-ige Ameisensäure / 1; Gradient: 0.0 min 10% B → 3.0 min 95% B → 4.0 min 95% B; Ofen: 35°C; Fluss: 0.0 min 1.0 ml/min → 3.0 min 3.0 ml/min → 4.0 min 3.0 ml/min; UV-Detektion: 210 nm.

### Methode 10:

Gerätetyp MS: Micromass TOF (LCT); Gerätetyp HPLC: 2-Säulen-Schaltung, Waters 2690; Säule: YMC-ODS-AQ, 50 mm x 4.6 mm, 3.0 µm; Eluent A: Wasser + 0.1% Ameisensäure, Eluent B: Acetonitril + 0.1% Ameisensäure; Gradient: 0.0 min 100% A → 0.2 min 95% A → 1.8 min 25% A → 1.9 min 10% A → 3.2 min 10% A; Ofen: 40°C; Fluss: 3.0 ml/min; UV-Detektion: 210 nm.

### Methode 11:

Instrument: Micromass Quattro LCZ mit HPLC Agilent Serie 1100; Säule: Uptisphere HDO, 50 mm x 2.0 mm, 3 µm; Eluent A: 11 Wasser + 1 ml 50%-ige Ameisensäure, Eluent B: 11 Acetonitril. + 1 ml 50%-ige Ameisensäure; Gradient: 0.0 min 100% A → 0.2 min 100% A → 2.9 min 30% A → 3.1 min 10% A → 4.5 min 10% A; Ofen: 55°C; Fluss: 0.8 ml/min; UV-Detektion: 208-400 nm.

### Methode 12:

Gerätetyp MS: Micromass ZQ; Gerätetyp HPLC: Waters Alliance 2790; Säule: Uptisphere C 18, 50 mm x 2.0 mm, 3.0 µm; Eluent B: Acetonitril + 0.05% Ameisensäure, Eluent A: Wasser + 0.05% Ameisensäure; Gradient: 0.0 min 5% B → 2.0 min 40% B → 4.5 min 90% B → 5.5 min 90% B; Ofen: 45°C; Fluss: 0.0 min 0.75 ml/min → 4.5 min 0.75 ml/min → 5.5 min 1.25 ml/min; UV-Detektion: 210 nm.

### Methode 13:

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 11 Wasser + 0.5 ml 50%-ige Ameisensäure, Eluent B: 11 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 14:

Gerätetyp MS: Micromass ZQ; Gerätetyp HPLC: Waters Alliance 2795; Säule: Merck Chromolith SpeedROD RP-18e 50 mm x 4.6 mm; Eluent A: Wasser + 500 µl 50%-ige Ameisensäure / 1, Eluent B: Acetonitril + 500 µl 50%-ige Ameisensäure / 1; Gradient: 0.0 min 10% B → 2.0 min 95% B → 4.0 min 95% B; Ofen: 35°C; Fluss: 0.0 min 1.0 ml/min → 2.0 min 3.0 ml/min → 4.0 min 3.0 ml/min; UV-Detektion: 210 nm.

### Chirale HPLC-Methoden:

### Methode 15A (präparativ):

Säule: Chiraler Kieselgelselektor Daicel Chiralpak AD; 10 µm, 250 x 20 mm; Elutionsmittel: i-Hexan/Ethanol + 0.2% Diethylamin 40:60 (v/v); Fluss: 20 ml/min; UV-Detektion: 220 nm; Temperatur: 30°C; Probenaufgabe in i-Hexan/Ethanol 1:1.

### Methode 15B (analytisch):

Säule: Chiraler Kieselgelselektor Daicel Chiralpak AD; 10 µm, 250 x 4.6 mm; Elutionsmittel: i-Hexan/Ethanol + 0.2% Diethylamin 40:60 (v/v); Fluss: 0.7 ml/min; UV-Detektion: 220 nm; Temperatur: 30°C; Probenaufgabe im Eluenten.

### Methode 16A (präparativ):

Säule: Chiraler Kieselgelselektor Daicel Chiralpak AD-H; 5 µm, 250 x 20 mm; Elutionsmittel: Acetonitril/Methanol + 0.2% Diethylamin 90:10 (v/v); Fluss: 20 ml/min; UV-Detektion: 220 nm; Temperatur: 25°C; Probenaufgabe in Acetonitril/Methanol 54:46 (v/v).

### Methode 16B (analytisch):

Säule: Daicel Chiralpak AD-H; 5 µm, 250 x 20 mm; Elutionsmittel: Acetonitril/ Methanol + 0.2% Diethylamin 90:10 (v/v); Fluss: 1.0 ml/min; UV-Detektion: 220 nm; Temperatur: 25°C; Probenaufgabe im Eluenten.

### Methode 17A (präparative):

Säule: Chiraler Kieselgelselektor Daicel Chiralpak AD-H; 5 µm, 250 x 20 mm; Elutionsmittel: Acetonitril/Methanol + 0.2% Diethylamin 85:15 (v/v); Fluss: 20 ml/min; UV-Detektion: 230 nm; Temperatur: 25°C; Probenaufgabe in Acetonitril.

### Methode 17B (analytisch):

Säule: Chiraler Kieselgelselektor Daicel Chiralpak AD-H; 5 µm, 250 x 4.6 mm; Elutionsmittel: Methanol/Acetonitril + 0.5% Diethylamin 20:80 (v/v); Fluss: 0.5 ml/min; UV-Detektion: 230 nm; Temperatur: 25°C; Probenaufgabe im Eluenten.

### Methode 18A (präparativ):

Säule: Chiraler Kieselgelselektor Daicel Chiralpak AD-H; 5 µm, 250 x 20 mm; Elutionsmittel: Acetonitril/Methanol + 0.2% Diethylamin 60:40 (v/v); Fluss: 20 ml/min; UV-Detektion: 230 nm; Temperatur: 25°C; Probenaufgabe in Acetonitril/Methanol 58:42 (v/v).

### Methode 18B (analytisch):

Säule: Chiraler Kieselgelselektor Daicel Chiralpak AD-H; 5 µm, 250 x 4.6 mm; Elutionsmittel: Methanol/Acetonitril + 0.5% Diethylamin 20:80 (v/v); Fluss: 1.0 ml/min; UV-Detektion: 230 nm; Temperatur: 30°C; Probenaufgabe im Eluenten.

### Methode 19A (präparativ):

Säule: Chiraler Kieselgelselektor Daicel Chiralpak AD; 10 µm, 250 x 10 mm; Elutionsmittel: Ethanol + 0.2% Diethylamin; Fluss: 10 ml/min; UV-Detektion: 220 nm; Temperatur: 40°C; Probenaufgabe in Ethanol.

### Methode 19B (analytisch):

Säule: Chiraler Kieselgelselektor Daicel Chiralpak AD; 10 µm, 250 x 4.6 mm; Elutionsmittel: Ethanol + 0.2% Diethylamin; Fluss: 0.7 ml/min; UV-Detektion: 220 nm; Temperatur: 25°C; Probenaufgabe im Eluenten.

### Methode 20A (ρräparativ):

Säule: Chiraler Kieselgelselektor KBD 5326A, basierend auf dem Selektor Poly-(N-methacryloyl-L-leucin-dicyclopropylamid); 10 µm, 250 x 20 mm; Elutionsmittel: iso-Hexan/Methyl-tert.-butylether 1:1 (v/v); Fluss: 25 ml/min; UV-Detektion: 254 nm; Temperatur: 24°C; Probenaufgabe in iso-Hexan/Methyl-tert.-butylether 1:1.

### Methode 20B (analytisch):

Säule: Chiraler Kieselgelselektor KBD 5326A; 10 µm, 250 x 4.6 mm; Elutionsmittel: iso-Hexan/Methyl-tert.-butylether 2:3 (v/v); Fluss: 1 ml/min; UV-Detektion: 254 nm; Temperatur: 25°C; Probenaufgabe im Eluenten.

### Methode 21A (präparativ):

Säule: Chiraler Kieselgelselektor KBD 5326A, basierend auf dem Selektor Poly-(N-methacryloyl-L-leucin-dicyclopropylamid); 10 µm, 250 x 20 mm; Elutionsmittel: iso-Hexan/Methyl-tert.-butylether 2:3 (v/v); Fluss: 25 ml/min; UV-Detektion: 254 nm; Temperatur: 24°C; Probenaufgabe in iso-Hexan/Methyl-tert.butylether 1:1.

### Methode 21B (analytisch):

Säule: Chiraler Kieselgelselektor KBD 5326A; 10 µm, 250 x 4.6 mm; Elutionsmittel: iso-Hexan/Methyl-tert.-butylether 2:3 (v/v); Fluss: 1 ml/min; UV-Detektion: 254 nm; Temperatur: 25°C; Probenaufgabe im Eluenten.

### Methode 22A (präparativ):

Säule: Chiraler Kieselgelselektor KBD 8361, basierend auf dem Selektor Poly-(N-methacryloyl-L-leucin-1-menthylamid); 10 µm, 250 x 20 mm; Elutionsmittel: iso-Hexan/Methyl-tert.-butylether 3:2 (v/v); Fluss: 25 ml/min; UV-Detektion: 254 nm; Temperatur: 24°C; Probenaufgabe in iso-Hexan/Methyl-tert.-butylether 3:2.

### Methode 22B (analytisch):

Säule: Chiraler Kieselgelselektor KBD 5326A; 10 µm, 250 x 4.6 mm; Elutionsmittel: iso-Hexan/Methyl-tert.-butylether 2:3 (v/v); Fluss: 1 ml/min; UV-Detektion: 254 nm; Temperatur: 25°C; Probenaufgabe im Eluenten.

### Methode 23A (präparativ):

Säule: Kromasil 100 C18; 5 µm, 250 x 20 mm; Fluss: 25 ml/min; Laufzeit: 20 min; Eluent A: Wasser + 0.2% Trifluoressigsäure, Eluent B: Acetonitril; Gradient: 5% B (0 min) → 95% B (15 min) → 5% B (15.1 min) → 5% B (20 min); UV-Detektion: 210 nm; Temperatur: 40°C; Probenaufgabe in Acetonitril/Wasser 2:1 (v/v).

### Methode 23B (analytisch):

Säule: Kromasil 100 C18; 5 µm, 250 x 4 mm; Fluss: 1.0 ml/min; Laufzeit: 20 min; Eluent A: Wasser + 0.2% Trifluoressigsäure, Eluent B: Acetonitril; Gradient: 5% B (0 min) → 95% B (10 min) → 95% B (15.0 min) → 5% B (15.1 min) → 5% B (20 min); UV-Detektion: 210 nm; Temperatur: 40°C; Probenaufgabe in Acetonitril/Wasser 2:1 (v/v).

### Methode 24A (präparativ):

Säule: Chiraler Kieselgelselektor Daicel Chiralpak AD; 10 µm, 250 x 20 mm; Elutionsmittel: i-Hexan/Ethanol + 0.2% Diethylamin 90:10 (v/v); Fluss: 25 ml/min; UV-Detektion: 220 nm; Temperatur: 25°C; Probenaufgabe in i-Hexan/Ethanol 5:1 (v/v).

### Methode 25A (präparativ):

Säule: Chiraler Kieselgelselektor Daicel Chiralpak AD; 20 µm, 500 x 40 mm; Elutionsmittel: Isopropanol/Methanol + 0.1% Diethylamin 85:15 (v/v); Fluss: 100 ml/min; UV-Detektion: 220 nm; Temperatur: 30°C; Probenaufgabe in Isopropanol.

### Methode 25B (analytisch):

Säule: Chiraler Kieselgelselektor Daicel Chiralpak AD; 10 µm, 250 x 4.6 mm; Elutionsmittel: Isopropanol/Methanol + 0.1% Diethylamin 85:15 (v/v); Fluss: 1.0 ml/min; UV-Detektion: 250 nm; Temperatur: 25°C; Probenaufgabe im Eluenten.

### Methode 26A (präparativ):

Säule: Chiraler Kieselgelselektor Daicel Chiralpak AD; 20 µm, 350 x 30 mm; Elutionsmittel: Isopropanol/Methanol + 0.1% Diethylamin 75:25 (v/v); Fluss: 50 ml/min; UV-Detektion: 220 nm; Temperatur: 25°C; Probenaufgabe in Isopropanol/Methanol 75:25 (v/v).

### Methode 26B (analytisch):

Säule: Chiraler Kieselgelselektor Daicel Chiralpak AD; 10 µm, 250 x 4.6 mm; Elutionsmittel: Isopropanol/Methanol + 0.2% Diethylamin 85:15 (v/v); Fluss: 1.0 ml/min; UV-Detektion: 250 nm; Temperatur: 25°C; Probenaufgabe im Eluenten.

### Methode 27A (präparativ):

Säule: Chiraler Kieselgelselektor Daicel Chiralpak AD; 20 µm, 500 x 40 mm; Elutionsmittel: Isopropanol/Methanol + 0.1% Diethylamin 75:25 (v/v); Fluss: 50 ml/min; UV-Detektion: 220 nm; Temperatur: 25°C; Probenaufgabe in Isopropanol/Methanol 75:25 (v/v).

### Methode 27B (analytisch):

Säule: Chiraler Kieselgelselektor Daicel Chiralpak AD; 10 µm, 250 x 4.6 mm; Elutionsmittel: Isopropanol/Methanol + 0.2% Diethylamin 85:15 (v/v); Fluss: 1.0 ml/min; UV-Detektion: 250 nm; Temperatur: 25°C; Probenaufgabe im Eluenten.

### Methode 28A (präparativ):

Säule: Chiraler Kieselgelselektor Daicel Chiralpak AD; 20 µm, 500 x 40 mm; Elutionsmittel: Isopropanol/Methanol + 0.1% Diethylamin 85:15 (v/v); Fluss: 50 ml/min; UV-Detektion: 220 nm; Temperatur: 25°C; Probenaufgabe in Isopropanol/Methanol 85:15 (v/v).

### Methode 28B (analytisch):

Säule: Chiraler Kieselgelselektor Daicel Chiralpak AD; 10 µm, 250 x 4.6 mm; Elutionsmittel: Isopropanol/Methanol + 0.1% Diethylamin 5:1 (v/v); Fluss: 1 ml/min; UV-Detektion: 250 nm; Temperatur: 25°C; Probenaufgabe im Eluenten.

### Methode 29A (präparativ):

Säule: Chiraler Kieselgelselektor Daicel Chiralpak AD; 10 µm, 250 x 20 mm; Elutionsmittel: i-Hexan/Ethanol + 0.2% Diethylamin 85:15 (v/v); Fluss: 25 ml/min; UV-Detektion: 220 nm; Temperatur: 25°C; Probenaufgabe in Ethanol.

### Methode 29B (analytisch):

Säule: Chiraler Kieselgelselektor Daicel Chiralpak AD; 10 µm, 250 x 4.6 mm; Elutionsmittel: Isopropanol/Methanol + 0.1% Diethylamin 5:1 (v/v); Fluss: 1 ml/min; UV-Detektion: 250 nm; Temperatur: 25°C; Probenaufgabe im Eluenten.

### Methode 30A (präparativ):

Säule: Chiraler Kieselgelselektor ZWE 803AB, basierend auf dem Selektor Poly-(N-methacryloyl-L-phenylalanin-d-neomenthylamid); 10 µm, 250 x 20 mm; Elutionsmittel: iso-Hexan/Methyl-tert.-butylether 1:4 (v/v); Fluss: 25 ml/min; UV-Detektion: 254 nm; Temperatur: 24°C; Probenaufgabe in Methyl-tert. butylether.

### Methode 30B (analytisch):

Säule: Chiraler Kieselgelselektor ZWE 803AB; 10 µm, 250 x 4.6 mm; Elutionsmittel: Methyl-tert.-butylether; Fluss: 1 ml/min; UV-Detektion: 254 nm; Temperatur: 25°C; Probenaufgabe im Eluenten.

### Ausgangsverbindungen und Intermediate:

### Beispiel 1A

### 3,5-Diethoxyphenol

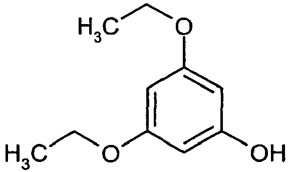

In eine Lösung von 188 g (1.49 mol) Phloroglucinol in 600 ml Ethanol wird unter RF für 5 h Chlorwasserstoff-Gas eingeleitet. Nach dem Abkühlen wird über Nacht bei RT gerührt. Anschließend wird erneut unter RF für 5 h Chlorwasserstoff-Gas eingeleitet. Nach dem Abkühlen wird die Reaktionsmischung eingeengt und der Rückstand in Dichlormethan und Wasser aufgenommen. Die organische Phase wird abgetrennt, die wässrige Phase zweimal mit Dichlormethan extrahiert, und die vereinigten organischen Phasen werden über Natriumsulfat getrocknet, filtriert und eingeengt. Der Rückstand wird im Vakuum destilliert (Kp.: 145-150°C / 0.5 mbar). Man löst das Destillat in Dichlormethan, extrahiert zur Entfernung von 5-Ethoxyresorcin fünfmal mit 5%-iger wässriger Kaliumcarbonat-Lösung, trocknet die organische Phase über Natriumsulfat, filtriert und engt ein. Man erhält 156 g (57% d. Th.) des Produkts.
LC-MS (Methode 1): Rₜ = 3.32 min.
MS (ESIpos): m/z = 183 (M+H)⁺
¹H-NMR (400 MHz, DMSO-d₆): δ = 9.35 (s, 1 H), 5.90 (s, 3 H), 3.91 (q, 4 H), 1.27 (t, 6 H).

### Beispiel 2A

### (4-Bromphenyl)-(3,5-diethoxyphenoxy)-essigsäuremethylester

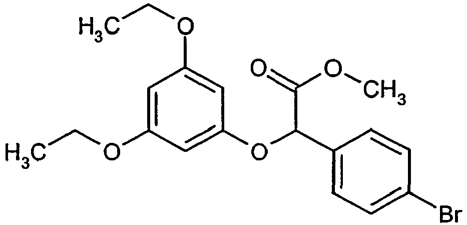

20.0 g (110 mmol) 3,5-Diethoxyphenol und 30.7 g (99.8 mmol) Brom-(4-bromphenyl)-essigsäure-methylester werden unter Argon bei RT in 39 ml 2-Butanon gelöst und mit 31.0 g (225 mmol) Kaliumcarbonat versetzt. Man erhitzt die Reaktionsmischung 4 h unter RF. Nach dem Abkühlen wird der Niederschlag abgesaugt, mit 2-Butanon nachgewaschen und das Filtrat eingeengt. Nach Chromatographie an Kieselgel 60 (Laufmittel: Toluol) erhält man 33.1 g (81% d. Th.) des Produkts.
LC-MS (Methode 2): Rₜ = 4.64 min.
MS (ESIpos): m/z = 409 (M+H)⁺
¹H-NMR (400 MHz, DMSO-d₆): δ = 7.66-7.60 (m, 2 H), 7.53-7.46 (m, 2 H), 6.11 (d, 2 H), 6.09 (t, 1 H), 6.05 (s, 1 H), 3.95 (q, 4 H), 3.66 (s, 3 H), 1.28 (t, 6 H).

### Beispiel 3A

### (4-Bromphenyl)-(3,5-diethoxyphenoxy)-essigsäure

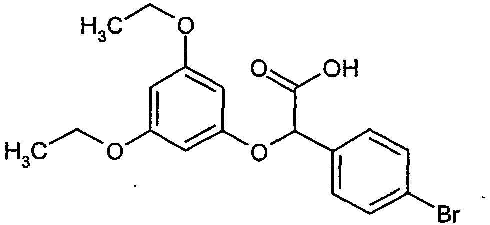

16.9 g (41.3 mmol) (4-Bromphenyl)-(3,5-diethoxyphenoxy)-essigsäuremethylester werden unter Argon bei RT in 80 ml Methanol und 8 ml Wasser gelöst und mit 7.99 g (57.8 mmol) Kaliumcarbonat versetzt. Man erhitzt 4 h unter RF. Nach dem Abkühlen wird der Niederschlag abfiltriert, das Filtrat eingeengt, der Rückstand in Wasser aufgenommen und die Lösung viermal mit Diethylether extrahiert. Man stellt in der wässrigen Phase durch Zusatz von 10%-iger Salzsäure einen pH-Wert von 2 ein, extrahiert dreimal mit Essigsäureethylester, wäscht die vereinigten Essigsäureethylester-Phasen mit gesättigter Natriumchlorid-Lösung, trocknet über Natriumsulfat, filtriert und engt ein. Man erhält 16.1 g (99% d. Th.) des Produkts.
LC-MS (Methode 4): Rₜ = 3.85 min.
MS (ESIpos): m/z = 395 (M+H)⁺
¹H-NMR (400 MHz, DMSO-d₆): δ = 13.37 (s, 1 H), 7.66-7.58 (m, 2 H), 7.53-7.45 (m, 2 H), 6.11-6.06 (m, 3 H), 5.83 (s, 1 H), 3.95 (q, 4 H), 1.28 (t, 6 H).

### Beispiel 4A

### 2-(4-Bromphenyl)-4,6-diethoxybenzofuran-3(2H)-on

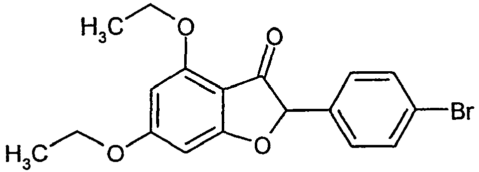

Zu 16.1 g (40.7 mmol) (4-Bromphenyl)-(3,5-diethoxyphenoxy)-essigsäure gibt man unter Argon bei RT 38 ml (407 mmol) Phosphorylchlorid. Man kühlt auf 0°C und setzt 8.32 g (61.0 mmol) Zinkchlorid hinzu. Nach 10 min entfernt man das Eisbad und rührt über Nacht bei RT. Die Reaktionsmischung wird in viel Eiswasser gegossen und 15 min gerührt. Man setzt Dichlormethan hinzu, trennt die Phasen, wäscht die organische Phase mit Wasser, trocknet über Natriumsulfat, filtriert und engt ein. Nach Gradienten-Chromatographie an Kieselgel 60 (Laufmittel: Toluol /Essigsäureethylester) erhält man 10.2 g (67% d. Th.) des Produkts.
LC-MS (Methode 4): Rₜ = 4.04 min.
MS (ESIpos): m/z = 377 (M+H)⁺
¹H-NMR (400 MHz, DMSO-d₆): δ = 7.64-7.58 (m, 2 H), 7.28-7.23 (m, 2 H), 6.46 (d, 1 H), 6.19 (d, 1 H), 5.72 (s, 1 H), 4.22-4.05 (m, 4 H), 1.39-1.26 (m, 6 H).

### Beispiel 5A

### (S*,R*)-3-[2-(4-Bromphenyl)-4,6-diethoxy-3-oxo-2,3-dihydrobenzofuran-2-yl]-3-phenylpropanal

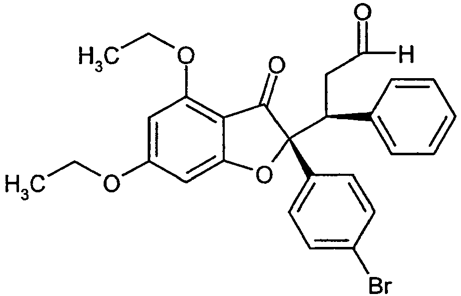

Unter Argon werden 20.9 g (55.4 mmol) 2-(4-Bromphenyl)-4,6-diethoxybenzofuran-3-on in 422 ml entgastem Methanol und 100 ml entgastem Toluol gelöst. Bei RT setzt man 2.00 g (11.1 mmol) einer 30%-igen methanolischen Natriummethylat-Lösung hinzu. Eine Minute später gibt man in 111 ml entgastem Toluol gelösten Zimtaldehyd (9.52 g, 72.0 mmol) hinzu. Es wird über Nacht bei RT gerührt und dann 48 h stehen gelassen. Anschließend wird gesättigte Ammoniumchlorid-Lösung hinzugesetzt und dreimal mit Dichlormethan extrahiert. Man trocknet die vereinigten organischen Phasen über Natriumsulfat, filtriert und engt ein. Nach Säulen-Chromatographie an Kieselgel 60 (Laufmittel: Toluol / Essigsäureethylester 95:5) erhält man 10.7 g (38% d. Th.) der Titelverbindung als racemisches Gemisch und 6.00 g (21% d. Th.) des Diastereomers (R*,R*)-3-[2-(4-Bromphenyl)-4,6-diethoxy-3-oxo-2,3-dihydrobenzofuran-2-yl]-3-phenylpropanal als racemisches Gemisch.
LC-MS (Methode 4): Rₜ = 4.19 min.
MS (ESIpos): m/z = 509 (M+H)⁺
¹H-NMR (400 MHz, DMSO-d₆): δ = 9.32 (d, 1 H), 7.69-7.62 (m, 2 H), 7.61-7.54 (m, 2 H), 7.31-7.10 (m, 5 H), 6.48 (d, 1 H), 5.97 (d, 1H), 4.24 (dd, 1 H), 4.12 (q, 2 H), 4.01-3.81 (m, 2 H), 3.02 (ddd, 1 H), 2.57-2.44 (m, 1 H), 1.33 (t, 3 H), 1.18 (t, 3 H).

### Beispiel 6A

### (1S*,3S*,3aR*,8bS*)-3a-(4-Bromphenyl)-6,8-diethoxy-3-phenyl-2,3,3a,8b-tetrahydrocyclopenta-[b]benzofuran-1,8b-(1H)-diol

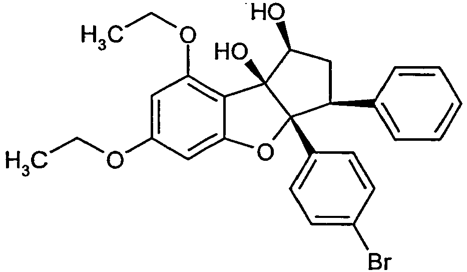

Man legt unter Argon 178 ml (17.9 mmol) einer 0.1 M Lösung von Samariumdiiodid in THF bei 0°C vor und tropft eine Lösung von 2.60 g (5.10 mmol) (S*,R*)-3-[2-(4-Bromphenyl)-4,6-diethoxy-3-oxo-2,3-dihydrobenzofuran-2-yl]-3-phenylpropanal in 30 ml entgastem THF hinzu. Man rührt 1h bei 0°C und 1 h bei RT. Man gibt eine eisgekühlte gesättigte Natrium-Kalium-Tartrat-Lösung mit 10% Kaliumcarbonat hinzu, trennt die Phasen, extrahiert die wässrige Phase dreimal mit Dichlormethan, wäscht die vereinigten organischen Phasen mit gesättigter Natriumchlorid-Lösung, trocknet über Natriumsulfat, filtriert und engt ein. Nach Säulenchromatographie an Kieselgel 60 (Laufmittel: Toluol / Essigsäureethylester 95:5) erhält man 1.55 g (58% d. Th.) des Produkts als racemisches Gemisch.
LC-MS (Methode 3): Rₜ = 4.62 min.
MS (ESIpos): m/z = 511 (M+H)⁺
¹H-NMR (400 MHz, DMSO-d₆): δ = 7.35-7.28 (m, 2 H), 7.24-7.02 (m, 5 H), 6.91-6.84 (m, 2 H), 6.28 (d, 1 H), 6.14 (d, 1 H), 5.81 (d, 1H), 4.84 (s, 1H), 4.71 (ddd, 1 H), 4.13-3.98 (m, 4 H), 3.32-3.18 (m, 1 H), 2.58-2.41 (m, 1 H), 2.15 (dt, 1 H), 1.35 (t, 3 H), 1.33 (t, 3 H).

### Beispiel 7A

### (3S*,3aR*8bR*)-3a-(4-Bromphenyl)-6,8-diethoxy-8b-hydroxy-3-phenyl-2,3,3a,8b-tetrahydro-cyclopenta[b]benzofuran-1-on

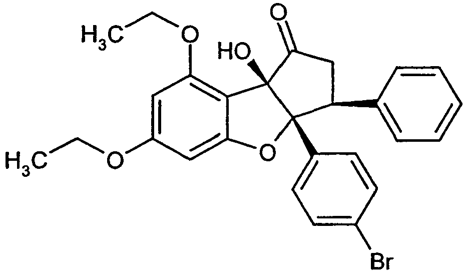

2.60 g (5.08 mmol) (1S*,3S*,3aR*,8bS*)-3a-(4-Bromphenyl)-6,8-diethoxy-3-phenyl-2,3,3a,8b-tetrahydrocyclopenta[b]benzofuran-1,8b-(1*H*)-diol werden unter Argon in 19 ml DMSO gelöst und auf 0°C gekühlt. Man tropft 7.80 ml (55.9 mmol) TEA und eine Lösung von 2.43 g (15.3 mmol) Schwefeltrioxid-Pyridin-Komplex in 9.60 ml DMSO hinzu, lässt auf RT erwärmen und rührt über Nacht. Anschließend wird mit eisgekühlter gesättigter Ammoniumchlorid-Lösung versetzt und 30 min gerührt. Der Niederschlag wird abgesaugt und mit wenig Wasser gewaschen. Nach Gradienten-Chromatographie an Kieselgel 60 (Laufmittel: Toluol / Essigsäureethylester) erhält man 2.00 g (77% d. Th.) des Produkts als racemisches Gemisch.
LC-MS (Methode 5): Rₜ = 4.02 min.
MS (ESIpos): m/z = 509 (M+H)⁺
¹H-NMR (400 MHz, DMSO-d₆): δ = 7.39-6.90 (m, 9 H), 6.43 (d, 1 H), 6.17 (d, 1 H), 5.82 (s, 1 H), 4.20-3.93 (m, 4 H), 3.70 (dd, 1 H), 3.12 (dd, 1 H), 2.93 (dd, 1 H), 1.34 (t, 3 H), 1.30 (t, 3 H).

### Beispiel 8A

### (1R*,3S*,3aR*,8bS*)-3a-(4-Bromphenyl)-6,8-diethoxy-3-phenyl-2,3,3a,8b-tetrahydrocyclopenta-[b]benzofuran-1,8b-(1H)-diol

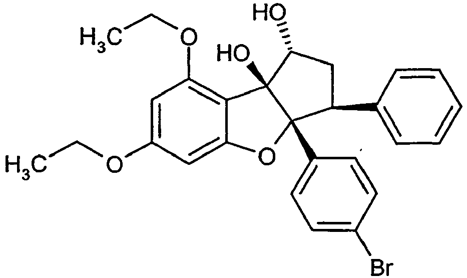

Man legt unter Argon 814 mg (3.09 mmol) Tetramethylammonium-triacetoxyborhydrid in 1.2 ml Acetonitril vor, gibt 1.2 ml Eisessig hinzu und rührt 0.5 h bei RT. Dann werden 105 mg (0.21 mmol) (3S*,3aR*,8bR*)-3a-(4-Bromphenyl)-6,8-diethoxy-8b-hydroxy-3-phenyl-2,3,3a,8b-tetrahydrocyclopenta[*b*]benzofuran-1-on als Lösung in 12 ml Acetonitril hinzugetropft. Man rührt bei RT über Nacht. Bei 0°C gibt man zu der Reaktionsmischung gesättigte Natriumhydrogencarbonat-Lösung, extrahiert die wässrige Phase zweimal mit Dichlormethan, trocknet die vereinigten organischen Phasen über Natriumsulfat, filtriert und engt ein. Nach Gradienten-Chromatographie an Kieselgel 60 (Laufmittel: Toluol / Essigsäureethylester) erhält man 77 mg (71% d. Th.) des Produkts als racemisches Gemisch.
LC-MS (Methode 1): Rₜ = 4.20 min.
MS (ESIpos): m/z = 512 (M+H)⁺
¹H-NMR (200 MHz, DMSO-d₆): δ = 7.27-7.18 (m, 2 H), 7.15-6.96 (m, 7 H), 6.24 (d, 1 H), 6.10 (d, 1 H), 4.95 (s, 1 H), 4.54-4.46 (m, 1 H), 4.42 (d, 1 H), 4.13-3.89 (m, 5 H), 2.70 (dt, 1 H), 2.01 (dd, 1 H), 1.33 (t, 3 H), 1.31 (t, 3 H).

Die präparative Trennung des Racemats in die Enantiomeren wird über HPLC an chiraler Phase nach Methode 22A durchgeführt.
Analytische Daten (Methode 22B):
Enantiomer A: Rₜ = 3.66 min., Enantiomer B: Rₜ = 4.30 min.

### Beispiel 9A

### (2R*,3S*,3aR*,8bR*)-3a-(4-Bromphenyl)-6,8-diethoxy-8b-hydroxy-1-oxo-3-phenyl-2,3,3a,8b-tetrahydrocyclopenta[b]benzofuran-2-carbonsäure

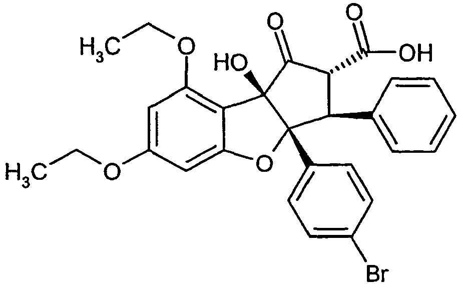

Die Titelverbindung wird in Analogie zur Synthese von Beispiel 17A ausgehend von Beispiel 7A hergestellt. Die Verbindung wird direkt ohne weitere Charakterisierung in der nächsten Stufe eingesetzt.

### Beispiel 10A

### (4-Chlorphenyl)-(3,5-diethoxyphenoxy)-essigsäuremethylester

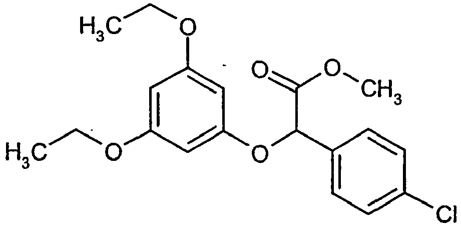

Die Titelverbindung wird in Analogie zur Synthese von Beispiel 2A ausgehend von Brom-(4-chlorphenyl)-essigsäuremethylester hergestellt.
Ausbeute: 96% d. Th.
LC-MS (Methode 2): Rₜ = 3.33 min.
MS (ESIpos): m/z = 365 (M+H)⁺
¹H-NMR (200 MHz, DMSO-d₆): δ = 7.58-7.47 (m, 4 H), 6.12-6.08 (m, 4 H), 3.95 (q, 4 H), 3.66 (s, 3 H), 1.28 (t, 3 H).

### Beispiel 11A

### (4-Chlorphenyl)-(3,5-diethoxyphenoxy)-essigsäure

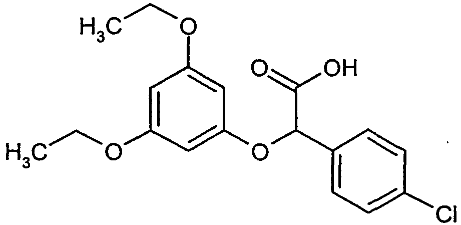

Die Titelverbindung wird in Analogie zur Synthese von Beispiel 3A ausgehend von Beispiel 10A hergestellt.
Ausbeute: 91% d. Th.
LC-MS (Methode 1): Rₜ = 3.80 min.
MS (ESIpos): m/z = 351 (M+H)⁺
¹H-NMR (300 MHz, DMSO-d₆): δ = 13.26 (br. s, 1H), 7.57 (d, 2 H), 7.48 (d, 2 H), 6.11-6.09 (m, 3 H), 5.85 (s, 1H), 3.95 (q, 2 H), 1.28 (t, 3 H).

### Beispiel 12A

### 2-(4-Chlorphenyl)-4,6-diethoxybenzofuran-3(2H)-on

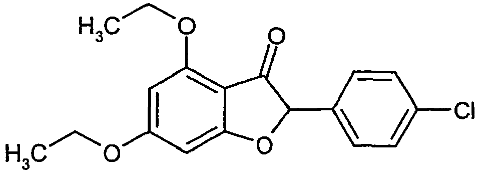

Die Titelverbindung wird in Analogie zur Synthese von Beispiel 4A ausgehend von Beispiel 11A hergestellt.
Ausbeute: 85% d. Th.
LC-MS (Methode 1): Rₜ = 4.00 min.
MS (ESIpos): m/z = 333 (M+H)⁺
¹H-NMR (200 MHz, DMSO-d₆): δ = 7.48 (d, 2 H), 7.31 (d, 2 H), 6.47 (d, 1 H), 6.20 (d, 1H), 5.75 (s, 1H), 4.22-4.04 (m, 4 H), 1.39-1.37 (m, 6 H).

### Beispiel 13A

### (S*,R*)-3-[2-(4-Chlorphenyl)-4,6-diethoxy-3-oxo-2,3-dihydrobenzofuran-2-yl]-3-phenylpropanal

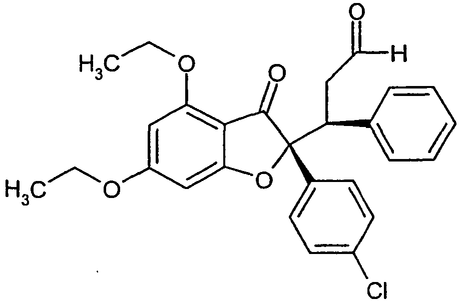

Die Titelverbindung wird in Analogie zur Synthese von Beispiel 5A ausgehend von Beispiel 12A hergestellt.
Ausbeute: 35% d. Th. (reines Diastereomer)
LC-MS (Methode 4): Rₜ = 4.09 min.
MS (ESIpos): m/z = 465 (M+H)⁺
¹H-NMR (300 MHz, DMSO-d₆): δ = 9.32 (d, 1H), 7.64 (d, 2 H), 7.51 (d, 2 H), 7.28-7.25 (m, 2 H), 7.19-7.08 (m, 3 H), 6.46 (d, 1H), 5.97 (d, 1H), 4.23 (dd, 1H), 4.12 (q, 2 H), 3.98-3.85 (m, 2 H), 3.01 (ddd, 1H), 2.54-2.47 (m, 1H), 1.33 (t, 3 H), 1.17 (t, 3 H).

### Beispiel 14A

### (1S*,3S*,3aR*,8bS*)-3a-(4-Chlorphenyl)-6,8-diethoxy-3-phenyl-2,3,3a,8b-tetrahydrocyclopenta-[b]benzofuran-1,8b-(1H)-diol

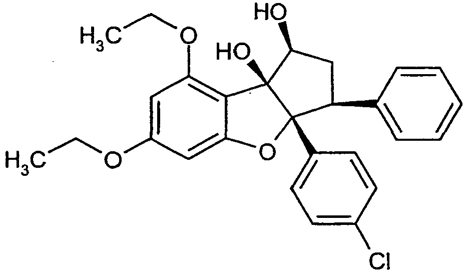

Die Titelverbindung wird in Analogie zur Synthese von Beispiel 6A ausgehend von Beispiel 13A hergestellt.
Ausbeute: 55% d. Th.
LC-MS (Methode 3): Rₜ = 3.79 min.
MS (ESIpos): m/z = 467 (M+H)⁺
¹H-NMR (200 MHz, DMSO-d₆): δ = 7.26-7.05 (m, 7 H), 6.89-6.85 (m, 2 H), 6.28 (d, 1H), 6.14 (d, 1 H), 5.81 (d, 1H), 4.84 (s, 1H), 4.76-4.67 (m, 1H), 4.12-3.99 (m, 4 H), 3.31-3.19 (m, 1H), 2.47-2.42 (m, 1 H), 2.23-2.04 (m, 1 H), 1.39-1.30 (m, 6 H).

### Beispiel 15A

### (3S*,3aR*,8bR*)-3a-(4-Chlorphenyl)-6,8-diethoxy-8b-hydroxy-3-phenyl-2,3,3a,8b-tetrahydro-cyclopenta[b]benzofuran-1-on

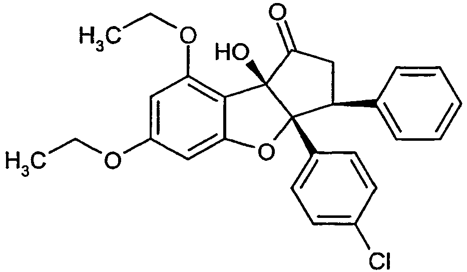

Die Titelverbindung wird in Analogie zur Synthese von Beispiel 7A ausgehend von Beispiel 14A hergestellt.
Ausbeute: 93% d. Th.
LC-MS (Methode 5): Rₜ = 1.27 min.
MS (ESIpos): m/z = 465 (M+H)⁺
¹H-NMR (300 MHz, DMSO-d₆): δ = 7.19-6.97 (m, 9 H), 6.43 (d, 1 H), 6.16 (d, 1 H), 5.78 (s, 1 H), 4.12-3.95 (m, 4 H), 3.71 (dd, 1 H), 3.15-2.89 (m, 2 H), 1.34 (t, 3 H), 1.30 (t, 3 H).

### Beispiel 16A

### (1R*,3S*,3aR*,8bS*)-3a-(4-Chlorphenyl)-6,8-diethoxy-3-phenyl-2,3,3a,8b-tetrahydrocyclopenta-[b]benzofuran-1,8b-(1H)-diol

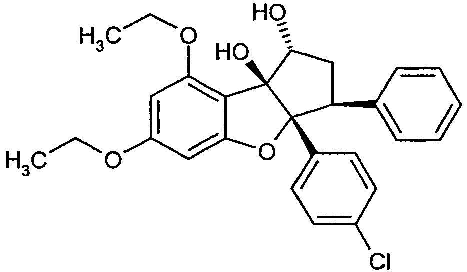

Die Titelverbindung wird in Analogie zur Synthese von Beispiel 8A ausgehend von Beispiel 15A hergestellt.
Ausbeute: 83% d. Th.
LC-MS (Methode 2): Rₜ = 4.59 min.
MS (ESIneg): m/z = 465 (M-H)⁻
¹H-NMR (200 MHz, DMSO-d₆): δ = 7.20-6.96 (m, 9 H), 6.24 (d, 1 H), 6.10 (d, 1 H), 4.95 (s, 1 H), 4.54-4.47 (m, 1 H), 4.42 (d, 1 H), 4.13-3.89 (m, 5 H), 2.70 (dt, 1 H), 2.01 (dd, 1 H), 1.33 (t, 3 H), 1.31 (t, 3 H).

Die präparative Trennung des Racemats in die Enantiomeren wird über HPLC an chiraler Phase nach Methode 21 A durchgeführt.
Analytische Daten (Methode 21B):
Enantiomer A: Rₜ = 4.52 min., Enantiomer B: Rₜ = 7.56 min.

### Beispiel 17A

### (2R*,3S*,3aR*,8bR*)-3a-(4-Chlorphenyl)-6,8-diethoxy-8-hydroxy-3-phenyl-2,3,3a,8b-tetrahydro-cyclopenta[b]benzofuran-2-carbonsäure

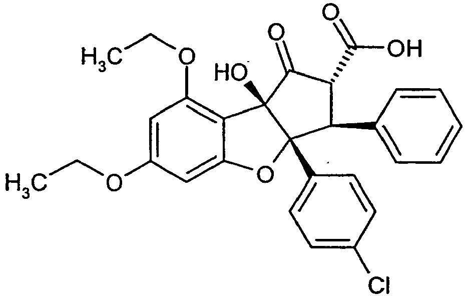

In einem geschlossenen Gefäß wird eine Mischung aus 500 mg (1.08 mmol) (3S*,3aR*,8bR*)-3a-(4-Chlorphenyl)-6,8-diethoxy-8b-hydroxy-3-phenyl-2,3,3a,8b-tetrahydrocyclopenta[*b*]benzofuran-1-on (Beispiel 15A) und 1.08 ml (2.15 mmol) einer 2 M Lösung von Metioxymagnesiummethylcarbonat in DMF für 16 h bei 100°C unter Rühren erhitzt. Die entstandene Suspension wird dann auf eine Mischung aus eisgekühlter 5 N Salzsäure und Ethylacetat gegossen. Die organische Phase wird abgetrennt, mit gesättigter Natriumchlorid-Lösung gewaschen, über Magnesiumsulfat getrocknet, abgesaugt und eingeengt. 531 mg Rohprodukt werden direkt ohne weitere Charakterisierung in der nächsten Stufe eingesetzt.

### Beispiel 18A

### 3,5-Bis-(2-methoxyethoxy)-phenol

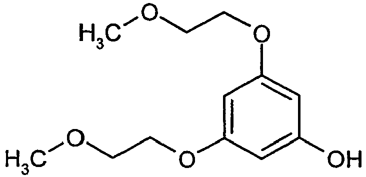

In eine Lösung von 50.0 g (397 mmol) Phloroglucinol in 500 ml Ethylenglykolmonomethylether wird bei 80°C über 4 h Chlorwasserstoff-Gas eingeleitet. Nach Zugabe von weiteren 300 ml Ethylenglykolmonomethylether wird für 2 h bei weiterer Einleitung von Chlorwasserstoff-Gas unter RF erhitzt. Man kühlt ab, engt ein, nimmt den Rückstand in 200 ml DMF auf, gibt 10.9 g (79.2 mmol) Kaliumcarbonat hinzu und erwärmt auf 50°C. Dann werden 10.9 g (78.2 mmol) 2-Bromethylmethylether als Lösung in 100 ml DMF zugetropft. Man rührt 2 h bei 50°C. Anschließend engt man ein, nimmt den Rückstand in einem Gemisch aus Diethylether und Wasser auf, trennt die Phasen und extrahiert die wässrige Phase zweimal mit Diethylether. Die wässrige Phase wird mit konzentrierter Salzsäure auf einen pH-Wert von 5 eingestellt und dreimal mit Diethylether extrahiert. Man wäscht die vereinigten organischen Phasen mit gesättigter Natriumchlorid-Lösung, trocknet über Natriumsulfat, filtriert und engt ein. Nach Gradienten-Chromatographie an Kieselgel 60 (Laufmittel: Toluol / Essigsäureethylester) erhält man 9.20 g (10% d. Th.) des Produkts.
LC-MS (Methode 9): Rₜ = 1.60 min.
MS (ESIpos): m/z = 243 (M+H)⁺
¹H-NMR (300 MHz, DMSO-d₆): δ = 9.35 (s, 1 H), 5.97-5.91 (m, 3 H), 4.01-3.96 (m, 4 H), 3.63-3.58 (m, 4 H), 3.29 (s, 6 H).

### Beispiel 19A

### [3,5-Bis-(2-methoxyethoxy)-phenoxy]-(4-bromphenyl)-essigsäuremethylester

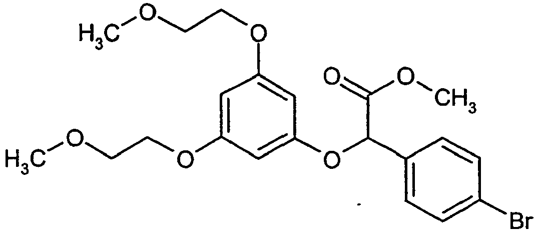

Die Titelverbindung wird in Analogie zur Synthese von Beispiel 2A ausgehend von Beispiel 18A hergestellt.
Ausbeute: 73% d. Th.
LC-MS (Methode 3): Rₜ = 4.17 min.
MS (ESIpos): m/z = 470 (M+H)⁺
¹H-NMR (300 MHz, DMSO-d₆): δ = 7.66-7.60 (m, 2 H), 7.52-7.46 (m, 2 H), 6.17-6.13 (m, 3 H), 6.07 (s, 1 H), 4.05-4.00 (m, 4 H), 3.66 (s, 3 H), 3.63-3.59 (m, 4 H), 3.29 (s, 6 H).

### Beispiel 20A

### [3,5-Bis-(2-methoxyethoxy)-phenoxy]-(4-bromphenyl)-essigsäure

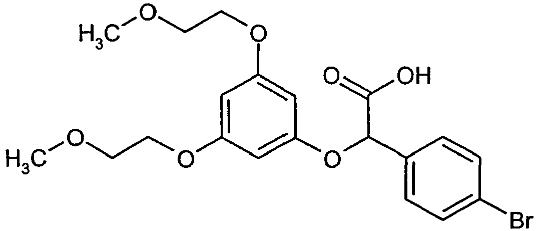

Die Titelverbindung wird in Analogie zur Synthese von Beispiel 3A ausgehend von Beispiel 19A hergestellt.
Ausbeute: 86% d. Th.
LC-MS (Methode 3): Rₜ = 3.68 min.
MS (ESIpos): m/z = 456 (M+H)⁺
¹H-NMR (300 MHz, DMSO-d₆): δ = 13.3 (s, 1 H), 7.65-7.59 (m, 2 H), 7.53-7.47 (m, 2 H), 6.14 (s, 3 H), 5.86 (s, 1 H), 4.05-4.00 (m, 4 H), 3.64-3.59 (m, 4 H), 3.29 (s, 6 H).

### Beispiel 21A

### 4,6-Bis-(2-methoxyethoxy)-2-(4-bromphenyl)-benzofuran-3(2H)-on

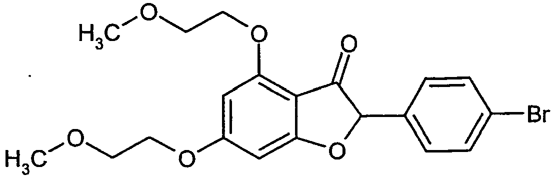

Die Titelverbindung wird in Analogie zur Synthese von Beispiel 4A ausgehend von Beispiel 20A hergestellt.
Ausbeute: 19% d. Th.
LC-MS (Methode 2): Rₜ = 4.29 min.
MS (ESIpos): m/z = 438 (M+H)⁺
¹H-NMR (300 MHz, DMSO-d₆): δ = 7.64-7.58 (m, 2 H), 7.29-7.23 (m, 2 H), 6.50 (d, 1 H), 6.26 (d, 1 H), 5.74 (s, 1H), 4.27-4.15 (m, 4 H), 3.71-3.62 (m, 4 H), 3.32 (s, 3 H), 3.31 (s, 3 H).

### Beispiel 22A

### (S*,R*)-3-[4,6-Bis-(2-methoxyethoxy)-2-(4-bromphenyl)-3-oxo-2,3-dihydrobenzofuran-2-yl]-3-phenylpropanal

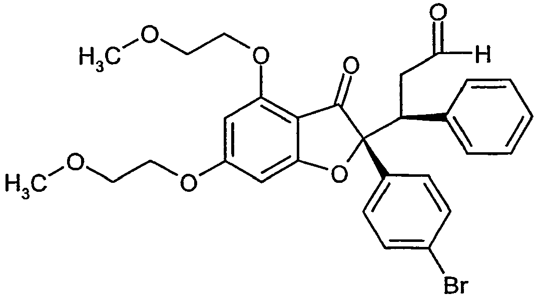

Die Titelverbindung wird in Analogie zur Synthese von Beispiel 5A ausgehend von Beispiel 21A hergestellt.
Ausbeute: 30% d. Th.
LC-MS (Methode 2): Rₜ = 4.48 min.
MS (ESIpos): m/z = 570 (M+H)⁺
¹H-NMR (300 MHz, DMSO-d₆): δ = 9.32 (d, 1 H), 7.67-7.62 (m, 2 H), 7.61-7.55 (m, 2 H), 7.29-7.23 (m, 2 H), 7.20-7.09 (m, 3 H), 6.51 (d, 1 H), 6.04 (d, 1 H), 4.24 (dd, 1 H), 4.22-4.17 (m, 2 H), 4.08-3.93 (m, 2 H), 3.68-3.63 (m, 2 H), 3.54-3.49 (m, 2 H), 3.30 (s, 3 H), 3.22 (s, 3 H), 3.01 (ddd, 1 H), 2.56-2.46 (m, 1 H).

### Beispiel 23A

### 3,5-Bis-(2-chlorethoxy)-phenol

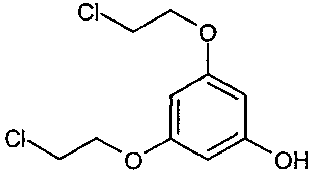

In eine Lösung von 150 g (1.19 mol) Phloroglucinol in 1000 ml 2-Chlorethanol wird bei 80°C über 5 h Chlorwasserstoff-Gas eingeleitet. Nach dem Abkühlen wird über Nacht bei RT gerührt. Anschließend erhitzt man unter weiterer Einleitung von Chlorwasserstoff-Gas für 2 h auf 80°C. Man kühlt ab, engt ein, nimmt den Rückstand in einem Gemisch aus Dichlormethan und Wasser auf, trennt die Phasen und extrahiert die wässrige Phase zweimal mit Dichlormethan. Man trocknet die vereinigten organischen Phasen über Natriumsulfat, filtriert und engt ein. Nach Säulen-chromatographie an Kieselgel 60 (Laufmittel: Toluol / Essigsäureethylester 95:5) erhält man 81.2 g (27% d. Th.) des Produkts.
LC-MS (Methode 4): Rₜ = 3.27 min.
MS (ESIpos): m/z = 252 (M+H)⁺
¹H-NMR (200 MHz, DMSO-d₆): δ = 9.51 (s, 1 H), 6.03-5.96 (m, 3 H), 4.20-4.13 (m, 4 H), 3.94-3.86 (m, 4 H).

### Beispiel 24A

### [3,5-Bis-(2-chlorethoxy)-phenoxy]-(4-chlorphenyl)-essigsäuremethylester

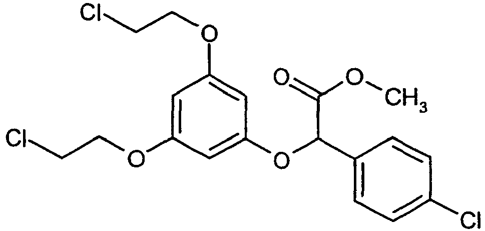

Die Titelverbindung wird in Analogie zur Synthese von Beispiel 2A ausgehend von Beispiel 23A hergestellt.
Ausbeute: 76% d. Th.
LC-MS (Methode 4): Rₜ = 4.05 min.
MS (ESIpos): m/z = 433 (M+H)⁺
¹H-NMR (400 MHz, DMSO-d₆): δ = 7.59-7.54 (m, 2 H), 7.52-7.46 (m, 2 H), 6.23-6.1 (m, 3 H), 6.13 (s, 1 H), 4.24-4.19 (m, 4 H), 3.93-3.88 (m, 4 H), 3.67 (s, 3 H).

### Beispiel 25A

### [3,5-Bis-(2-chlorethoxy)-phenoxy]-(4-chlorphenyl)-essigsäure

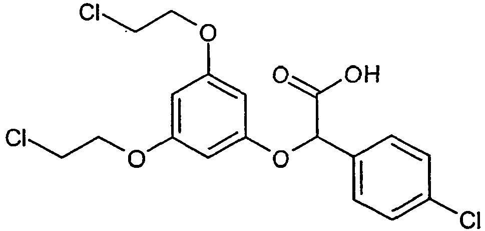

Die Titelverbindung wird in Analogie zur Synthese von Beispiel 3A ausgehend von Beispiel 24A hergestellt.
Ausbeute: 92% d. Th.
LC-MS (Methode 4): Rₜ = 3.91 min.
MS (ESIpos): m/z = 419 (M+H)⁺
¹H-NMR (200 MHz, DMSO-d₆): δ = 13.4 (s, 1 H), 7.61-7.53 (m, 2 H), 7.52-7.45 (m, 2 H), 6.19 (s, 3 H), 5.92 (s, 1 H), 4.25-4.16 (m, 4 H), 3.96-3.88 (m, 4 H).

### Beispiel 26A

### 4,6-Bis-(2-chlorethoxy)-2-(4-chlorphenyl)-benzofuran-3(2H)-on

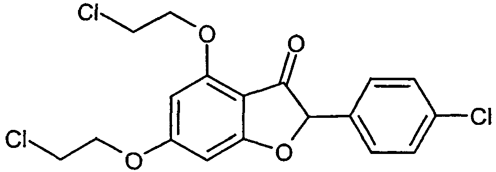

Die Titelverbindung wird in Analogie zur Synthese von Beispiel 4A ausgehend von Beispiel 25A hergestellt.
Ausbeute: 55% d. Th.
LC-MS (Methode 3): Rₜ = 3.82 min.
MS (ESIpos): m/z = 401 (M+H)⁺
¹H-NMR (300 MHz, DMSO-d₆): δ = 7.51-7.45 (m, 2 H), 7.36-7.30 (m, 2 H), 6.57 (d, 1 H), 6.32 (d, 1 H), 5.79 (s, 1 H), 4.45-4.35 (m, 4 H), 4.02-3.89 (m, 4 H).

### Beispiel 27A

### (S*,R*)-3-[4,6-Bis-(2-chlorethoxy)-2-(4-chlorphenyl)-3-oxo-2,3-dihydrobenzofuran-2-yl]-3-phenylpropanal

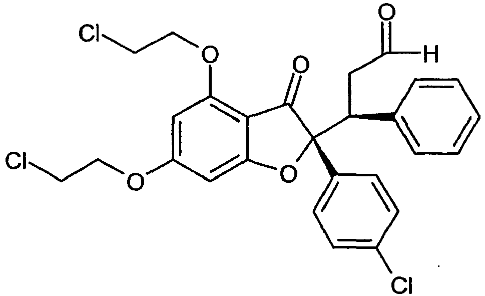

Die Titelverbindung wird in Analogie zur Synthese von Beispiel 5A ausgehend von Beispiel 26A hergestellt.
Ausbeute: 48% d. Th.
LC-MS (Methode 4): Rₜ = 3.72 min.
MS (ESIpos): m/z = 533 (M+H)⁺
¹H-NMR (200 MHz, DMSO-d₆): δ = 9.33 (d, 1 H), 7.68-7.62 (m, 2 H), 7.54-7.48 (m, 2 H), 7.29-7.24 (m, 2 H), 7.19-7.09 (m, 3 H), 6.57 (d, 1 H), 6.09 (d, 1 H), 4.40-4.35 (m, 2 H), 4.30-4.09 (m, 3 H), 3.99-3.94 (m, 2 H), 3.81-3.76 (m, 2 H), 3.02 (ddd, 1 H), 2.56-2.46 (m, 1 H).

### Beispiel 28A

### (1S*,3S*,3aR*,8bS*)-6,8-Bis-(2-chlorethoxy)-3a-(4-chlorphenyl)-3-phenyl-2,3,3a,8b-tetrahydro-cyclopenta[b]benzofuran-1,8b-(1H)-diol

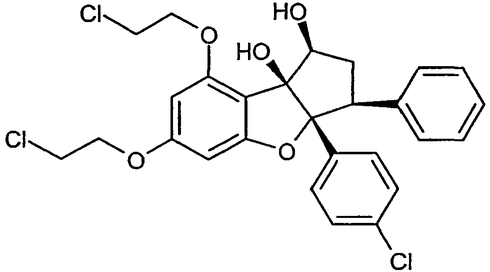

Die Titelverbindung wird in Analogie zur Synthese von Beispiel 6A ausgehend von Beispiel 27A hergestellt.
Ausbeute: 31% d. Th.
LC-MS (Methode 5): Rₜ = 4.01 min.
MS (ESIpos): m/z = 535 (M+H)⁺
¹H-NMR (300 MHz, DMSO-d₆): δ = 7.26-7.15 (m, 4 H), 7.10-7.03 (m, 3 H), 6.92-6.86 (m, 2 H), 6.38 (d, 1 H), 6.25 (d, 1 H), 5.68 (d, 1 H), 4.83 (s, 1 H), 4.82-4.73 (m, 1 H), 4.36-4.28 (m, 4 H), 4.02-3.93 (m, 4 H), 3.29-3.23 (m, 1 H), 2.51-2.43 (m, 1 H), 2.17 (ddd, 1 H).

### Beispiel 29A

### (3S*,3aR*,8bR*)-6,8-Bis-(2-chlorethoxy)-3a-(4-chlorphenyl)-8b-hydroxy-3-phenyl-2,3,3a,8b-tetrahydrocyclopenta[b]benzofuran-1-on

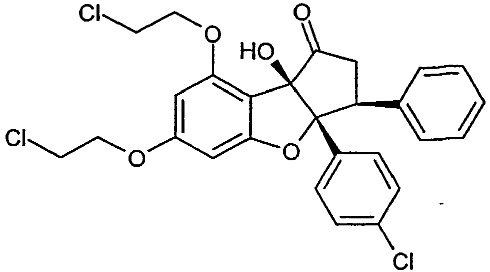

Die Titelverbindung wird in Analogie zur Synthese von Beispiel 7A ausgehend von Beispiel 28A hergestellt.
Ausbeute: 71% d. Th.
LC-MS (Methode 2): Rₜ = 3.40 min.
MS (ESIpos): m/z = 533 (M+H)⁺
¹H-NMR (300 MHz, DMSO-d₆): δ = 7.21-7.15 (m, 2 H), 7.12-7.05 (m, 3 H), 7.02-6.96 (m, 4 H), 6.54 (d, 1 H), 6.30 (d, 1 H), 5.87 (s, 1 H), 4.36-4.26 (m, 4 H), 3.99-3.94 (m, 2 H), 3.90-3.85 (m, 2 H), 3.73 (dd, 1 H), 3.11 (dd, 1 H), 2.93 (dd, 1 H).

### Beispiel 30A

### (1R*,3S*,3aR*,8bS*)-6,8-Bis-(2-chlorethoxy)-3a-(4-chlorphenyl)-3-phenyl-2,3,3a,8b-tetrahydro-cyclopenta[b]benzofuran-1,8b-(1H)-diol

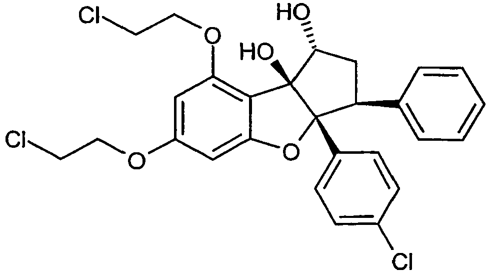

Die Titelverbindung wird in Analogie zur Synthese von Beispiel 8A ausgehend von Beispiel 29A hergestellt.
Ausbeute: 85% d. Th.
LC-MS (Methode 1): Rₜ = 4.09 min.
MS (ESIpos): m/z = 535 (M+H)⁺
¹H-NMR (300 MHz, DMSO-d₆): δ = 7.11-6.98 (m, 9 H), 6.33 (d, 1 H), 6.21 (d, 1 H), 4.97 (s, 1 H), 4.55-4.50 (m, 1 H), 4.36 (d, 1 H), 4.32-4.26 (m, 4 H), 4.02-3.90 (m, 5 H), 2.72 (ddd, 1 H), 2.03 (ddd, 1 H).

### Beispiel 31A

### [3,5-Bis-(2-chlorethoxy)-phenoxy]-(4-bromphenyl)-essigsäuremethylester

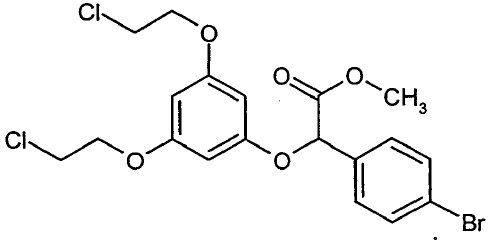

Die Titelverbindung wird in Analogie zur Synthese von Beispiel 2A ausgehend von Beispiel 23A hergestellt.
Ausbeute: 48% d. Th.
LC-MS (Methode 4): Rₜ = 4.11 min.
MS (ESIpos): m/z = 477 (M+H)⁺
¹H-NMR (400 MHz, DMSO-d₆): δ = 7.68-7.60 (m, 2 H), 7.54-7.45 (m, 2 H), 6.23-6.18 (m, 3 H), 6.13 (s, 1 H), 4.25-4.17 (m, 4 H), 3.95-3.87 (m, 4 H), 3.66 (s, 3 H).

### Beispiel 32A

### [3,5-Bis-(2-chlorethoxy)-phenoxy]-(4-bromphenyl)-essigsäure

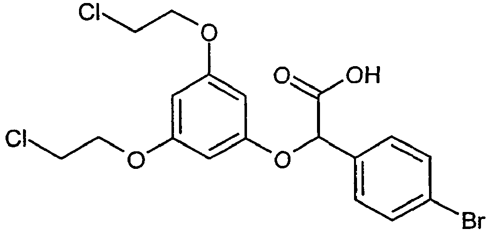

Die Titelverbindung wird in Analogie zur Synthese von Beispiel 3A ausgehend von Beispiel 31A hergestellt.
Ausbeute: 99% d. Th.
LC-MS (Methode 2): Rₜ = 4.33 min.
MS (ESIpos): m/z = 463 (M+H)⁺
¹H-NMR (300 MHz, DMSO-d₆): δ = 13.4 (s, 1 H), 7.64-7.58 (m, 2 H), 7.52-7.46 (m, 2 H), 6.21-6.16 (m, 3 H), 5.85 (s, 1 H), 4.24-4.18 (m, 4 H), 3.93-3.88 (m, 4 H).

### Beispiel 33A

### 4,6-Bis-(2-chlorethoxy)-2-(4-bromphenyl)-benzofuran-3(2H)-on

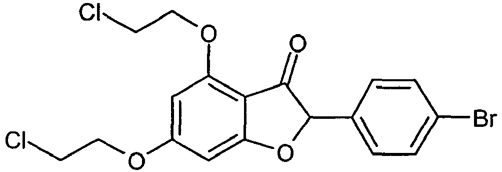

Die Titelverbindung wird in Analogie zur Synthese von Beispiel 4A ausgehend von Beispiel 32A hergestellt.
Ausbeute: 16% d. Th.
LC-MS (Methode 4): Rₜ = 3.96 min.
MS (ESIpos): m/z = 446 (M+H)⁺
¹H-NMR (200 MHz, DMSO-d₆): δ = 7.66-7.57 (m, 2 H), 7.30-7.22 (m, 2 H), 6.58 (d, 1 H), 6.32 (d, 1 H), 5.79 (s, 1 H), 4.46-4.34 (m, 4 H), 4.04-3.88 (m, 4 H).

### Beispiel 34A

### (S*,R*)-3-[4,6-Bis-(2-chlorethoxy)-2-(4-bromphenyl)-3-oxo-2,3-dihydrobenzofuran-2-yl]-3-phenylpropanal

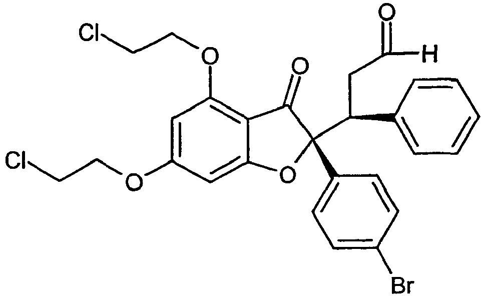

Die Titelverbindung wird in Analogie zur Synthese von Beispiel 5A ausgehend von Beispiel 33A hergestellt.
Ausbeute: 35% d. Th.
LC-MS (Methode 4): Rₜ = 4.05 min.
MS (ESIpos): m/z = 578 (M+H)⁺
¹H-NMR (200 MHz, DMSO-d₆): δ = 9.33 (d, 1 H), 7.70-7.63 (m, 2 H), 7.62-7.54 (m, 2 H), 7.31-7.09 (m, 5 H), 6.58 (d, 1 H), 6.10 (d, 1 H), 4.42-4.33 (m, 2 H), 4.31-4.07 (m, 3 H), 4.02-3.93 (m, 2 H), 3.83-3.76 (m, 2 H), 3.03 (ddd, 1 H), 2.58-2.44 (m, 1 H).

### Beispiel 35A

### (1 S*,3S*,3aR*,8bS*)-6,8-Bis-(2-chlorethoxy)-3a-(4-bromphenyl)-3-phenyl-2,3,3a,8b-tetrahydro-cyclopenta[b]benzofuran-1,8b-(1H)-diol

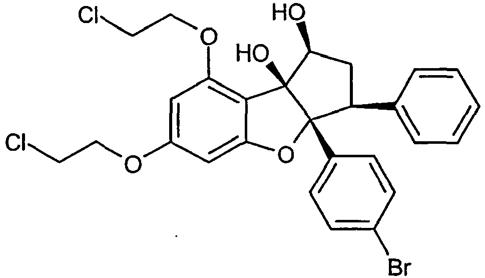

Die Titelverbindung wird in Analogie zur Synthese von Beispiel 6A ausgehend von Beispiel 34A hergestellt.
Ausbeute: 30% d. Th.
LC-MS (Methode 2): Rₜ = 4.60 min.
MS (ESIpos): m/z = 579 (M+H)⁺
¹H-NMR (400 MHz, DMSO-d₆): δ = 7.34-7.28 (m, 2 H), 7.18-7.12 (m, 2 H), 7.09-7.03 (m, 3 H), 6.92-6.87 (m, 2 H), 6.37 (d, 1 H), 6.25 (d, 1 H), 5.68 (d, 1 H), 4.83 (s, 1 H), 4.81-4.73 (m, 1 H), 4.35-4.27 (m, 4 H), 4.01-3.93 (m, 4 H), 3.28-3.23 (m, 1 H), 2.54-2.42 (m, 1 H), 2.17 (ddd, 1 H).

### Beispiel 36A

### (4-Chlorphenyl)-(3-ethoxyphenoxy)-essigsäuremethylester

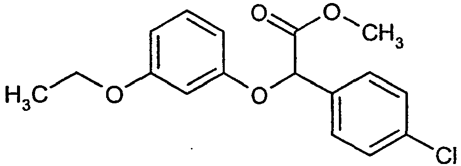

Die Titelverbindung wird in Analogie zur Synthese von Beispiel 2A ausgehend von 3-Ethoxyphenol hergestellt.
Ausbeute: 97% d. Th.
LC-MS (Methode 5): Rₜ = 3.86 min.
MS (ESIpos): m/z = 321 (M+H)⁺
¹H-NMR (300 MHz, DMSO-d₆): δ = 7.57 (d, 2 H), 7.49 (d, 2 H), 7.19-7.13 (m, 1 H), 6.54-6.52 (m, 3 H), 6.07 (s, 1 H), 3.98 (q, 2 H), 3.66 (s, 3 H), 1.29 (t, 3 H).

### Beispiel 37A

### (4-Chlorphenyl)-(3-ethoxyphenoxy)-essigsäure

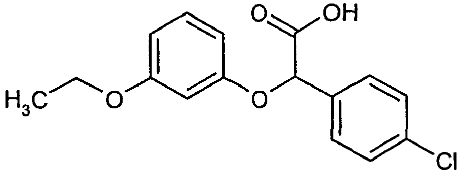

Die Titelverbindung wird in Analogie zur Synthese von Beispiel 3A ausgehend von Beispiel 36A hergestellt.
Ausbeute: 96% d. Th.
LC-MS (Methode 4): Rₜ = 3.18 min.
MS (ESIpos): m/z = 307 (M+H)⁺
¹H-NMR (200 MHz, DMSO-d₆): δ = 7.58 (d, 2 H), 7.48 (d, 2 H), 7.20-7.12 (m, 1 H), 6.55-6.50 (m, 3 H), 5.88 (s, 1 H), 3.98 (q, 2 H), 1.30 (t, 3 H).

### Beispiel 38A

### 2-(4-Chlorphenyl)-6-ethoxybenzofuran-3 (2H)-on

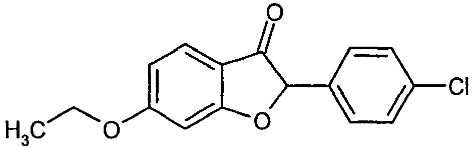

Die Titelverbindung wird in Analogie zur Synthese von Beispiel 4A ausgehend von Beispiel 37A hergestellt.
Ausbeute: 26% d. Th.
LC-MS (Methode 2): Rₜ = 3.37 min.
MS (ESIpos): m/z = 289 (M+H)⁺.

### Beispiel 39A

### (S*,R*)-3-[2-(4-Chlorphenyl)-6-ethoxy-3-oxo-2,3-dihydrobenzofuran-2-yl]-3-phenylpropanal

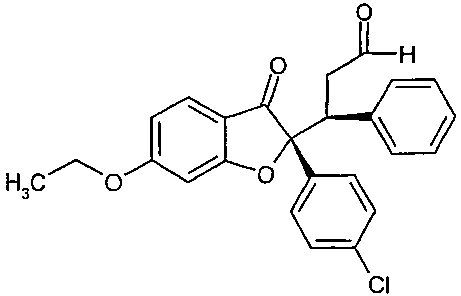

Die Titelverbindung wird in Analogie zur Synthese von Beispiel 5A ausgehend von Beispiel 38A hergestellt.
Ausbeute: 19% d. Th. (reines Diastereomer)
LC-MS (Methode 12): Rₜ = 4.53 min.
MS (ESIpos): m/z = 421 (M+H)⁺
¹H-NMR (400 MHz, DMSO-d₆): δ = 9.34 (s, 1 H), 7.69 (d, 2 H), 7.52 (d, 2 H), 7.24 (d, 2 H), 7.18-7.06 (m, 4 H), 6.90 (s, 1 H), 6.50 (dd, 1 H), 4.28 (dd, 1 H), 4.14 (q, 2 H), 3.12-3.04 (m, 1 H), 2.57-2.52 (m, 1 H), 1.34 (t, 3 H).

### Beispiel 40A

### (2R*,3S*,3aR*,8bR*)-3a-(4-Chlorphenyl)-6-ethoxy-8b-hydroxy-1-oxo-3-phenyl-2,3,3a,8b-tetra-hydro-1H-cyclopenta[b]benzofuran-2-carbonsäure

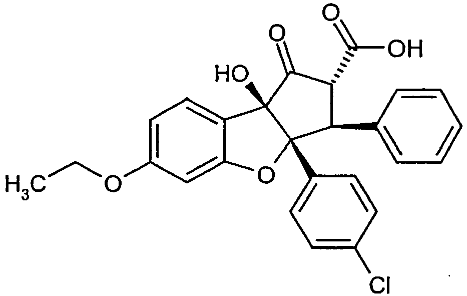

Die Titelverbindung wird in Analogie zur Synthese von Beispiel 17A ausgehend von Beispiel 24 hergestellt. Die Verbindung wird direkt ohne weitere Charakterisierung in der nächsten Stufe eingesetzt.

### Beispiel 41A

### 4-(2-Chlorethoxy)-2-hydroxyacetophenon

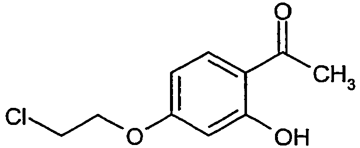

300 g (1.97 mol) 2,4-Dihydroxyacetophenon, 238 g (2.96 mol) 2-Chlorethanol und 776 g (2.96 mol) Triphenylphosphin werden in 4800 ml THF vorgelegt. Bei RT werden 380 g (2.96 mol) Azodicarbonsäurediisopropylester als Lösung in 1200 ml THF hinzugetropft. Man erhitzt 16 h unter RF, kühlt dann ab und engt ein. Der Rückstand wird mit 2 N Natronlauge verrührt und abgesaugt. Man verrührt den Feststoff mit Essigsäureethylester, saugt ab, wäscht mit Essigsäureethylester und trocknet den Feststoff. Man nimmt den Feststoff in einem Gemisch aus 4 N Salzsäure und Essigsäureethylester auf, trennt die Phasen und extrahiert die wässrige Phase noch einmal mit Essigsäureethylester. Man wäscht die vereinigten organischen Phasen mit gesättigter Natriumchlorid-Lösung, trocknet über Natriumsulfat, filtriert und engt ein. Nach zweimaliger Säulenchromatographie an Kieselgel 60 (1. Laufmittel: Toluol, 2. Laufmittel: Cyclohexan) erhält man 232 g (55% d. Th.) des Produkts.
LC-MS (Methode 13): Rₜ = 2.41 min.
MS (ESIpos): m/z = 215 (M+H)⁺
¹H-NMR (200 MHz, DMSO-d₆): δ = 12.6 (s, 1 H), 7.86 (d, 1 H), 6.57 (dd, 1 H), 6.50 (d, 1 H), 4.38-4.30 (m, 2 H), 4.00-3.92 (m, 2 H), 2.57 (s, 3 H).

### Beispiel 42A

### 6-(2-Chlorethoxy)-benzofuran-3-on

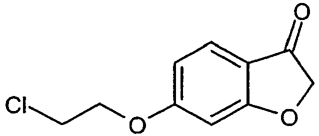

Man legt 76.0 g (354 mmol) 4-(2-Chlorethoxy)-2-hydroxyacetophenon bei -78°C in 1050 ml THF unter Argon vor und tropft 148 g (885 mmol) Lithiumhexamethyldisilazid als Lösung in 750 ml THF hinzu. Nach Erwärmen auf RT und Zusatz von 96.2 g (885 mmol) Chlortrimethylsilan wird 2 h bei RT gerührt. Anschließend gibt man bei 0°C portionsweise 65.6 g (369 mmol) N-Bromsuccinimid hinzu, rührt 0.5 h bei 0°C und 1 h bei RT. Danach gibt man 369 ml 1 N Natronlauge hinzu und rührt 0.5 h bei RT. Nach Zugabe von gesättigter Ammoniumchlorid-Lösung extrahiert man die wässrige Phase mehrfach mit Diethylether, trocknet die vereinigten organischen Phasen über Natriumsulfat, filtriert und engt ein. Nach Gradienten-Chromatographie an Kieselgel 60 (Laufmittel: Petrolether / Essigsäureethylester) erhält man 59.0 g (78% d. Th.) des Produkts.
LC-MS (Methode 6): Rₜ = 1.80 min.
MS (ESIpos): m/z = 213 (M+H)⁺
¹H-NMR (300 MHz, DMSO-d₆): δ = 7.54 (d, 1 H), 6.86 (d, 1 H), 6.73 (dd, 1 H), 4.77 (s, 2 H), 4.41-4.36 (m, 2 H), 4.00-3.95 (m, 2 H).

### Beispiel 43A

### 2-Brom-6-(2-chlorethoxy)-benzofuran-3-on

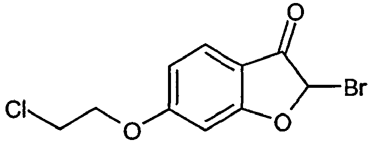

### Methode a):

127 g (597 mmol) 6-(2-Chlorethoxy)-benzofuran-3-on werden bei RT unter Argon in 1850 ml Dioxan und 1850 ml Diethylether gelöst und auf -5°C gekühlt. Unter starkem Rühren tropft man langsam 95.6 g (597 mmol) Brom hinzu und rührt 1 h bei 0°C. Anschließend gibt man auf Eiswasser, trennt die Phasen, extrahiert die wässrige Phase zweimal mit Dichlormethan, trocknet die vereinigten organischen Phasen über Natriumsulfat, filtriert und engt ein. Nach Säulen-chromatographie an Kieselgel 60 (Laufmittel: Toluol) erhält man 150 g (86% d. Th.) des Produkts als Hydrat.

### Methode b):

Man gibt zu einer siedenden Suspension von 10.5 g (47.0 mmol) Kupfer(II)bromid in 60 ml Essigsäureethylester 5.00 g (23.5 mmol) 6-(2-Chlorethoxy)-benzofuran-3-on in 30 ml Chloroform. Es wird über Nacht unter RF erhitzt. Nach dem Abkühlen wird der Feststoff abfiltriert und mit Essigsäureethylester gewaschen. Das Filtrat wird mit Wasser und gesättigter wässriger Natriumchlorid-Lösung gewaschen, über Natriumsulfat getrocknet, filtriert und eingeengt. Nach Gradienten-Chromatographie an Kieselgel 60 (Laufmittel: Toluol / Cyclohexan) erhält man 1.50 g (20% d. Th.) des Produkts als Hydrat.
LC-MS (Methode 2): Rₜ = 4.13 min.
MS (ESIpos): m/z = 291 (M+H)⁺
¹H-NMR (400 MHz, DMSO-d₆): δ = 7.52 (d, 1 H), 7.30 (s, 2 H), 6.75 (d, 1 H), 6.70 (dd, 1 H), 5.57 (s, 1 H), 4.42-4.37 (m, 2 H), 4.00-3.95 (m, 2 H).

### Beispiel 44A

### 2-Brom-3-tert.-butyldimethylsilyloxy-6-(2-chlorethoxy)-benzofuran

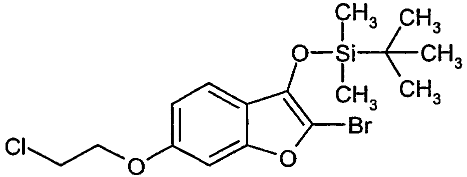

1.50 g (5.15 mmol) des Hydrats von 2-Brom-6-(2-chlorethoxy)-benzofuran-3-on (Beispiel 43A) werden zur azeotropen Entfernung von Wasser dreimal mit Toluol unter Argon eingeengt. Anschließend wird der Rückstand unter Argon in 25 ml Diethylether gelöst und auf 0°C gekühlt. Man tropft nacheinander langsam 0.79 ml (5.66 mmol) TEA und 1.30 ml (5.66 mmol) tert.-Butyl-dimethylsilyltrifluormethansulfonat zu und rührt 10 min bei 0°C und 1 h bei RT. Danach trennt man die Diethylether-Phase ab, extrahiert den Rückstand zweimal mit Diethylether und engt die vereinigten Diethylether-Phasen ein. Nach Säulenchromatographie an Kieselgel 60 (Laufmittel: Cyclohexan) erhält man 2.00 g (96% d. Th.) des Produkts.
LC-MS (Methode 2): Rₜ = 4.92 min.
MS (ESIpos): m/z = 405 (M+H)⁺
¹H-NMR (400 MHz, DMSO-d₆): δ = 7.36 (d, 1 H), 7.21 (d, 1 H), 6.96 (dd, 1 H), 4.32-4.27 (m, 2 H), 3.98-3.93 (m, 2 H), 1.03 (s, 9 H), 0.23 (s, 6 H).

### Beispiel 45A

### 3-tert.-Butyldimethylsilyloxy-6-(2-chlorethoxy)-2-(4-chlorphenyl)-benzofuran

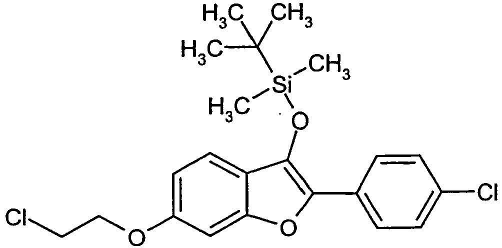

### Methode a):

Unter Argon löst man 2.00 g (4.93 mmol) 2-Brom-3-tert.-butyldimethylsilyloxy-6-(2-chlorethoxy)-benzofuran in 41 ml Toluol und gibt 0.92 g (5.91 mmol) 4-Chlorphenylboronsäure und eine Lösung aus 1.15 g (10.8 mmol) Natriumcarbonat in 5.4 ml Wasser hinzu. Man entgast und belüftet zweimal mit Argon, setzt 0.28 g (0.25 mol) Tetrakis(triphenylphosphin)palladium(0) hinzu und erwärmt für 2 h auf 95°C. Nach dem Abkühlen gießt man die Reaktionsmischung in eine Mischung aus eiskalter, gesättigter Ammoniumchlorid-Lösung und Diethylether, trennt die Phasen, extrahiert die wässrige Phase zweimal mit Diethylether, wäscht die vereinigten organischen Phasen mit Wasser und gesättigter Kochsalz-Lösung, trocknet über Natriumsulfat, filtriert und engt ein. Nach Säulenchromatographie an Kieselgel 60 (Laufmittel: Toluol / Cyclohexan 1:1) erhält man 1.69 g (78% d. Th.) des Produkts.

### Methode b):

39.4 g (135 mmol) des Hydrats von 2-Brom-6-(2-chlorethoxy)-benzofuran-3-on (Beispiel 43A) werden zur azeotropen Entfernung von Wasser dreimal mit Toluol unter Argon eingeengt. Anschließend wird der Rückstand unter Argon in 1200 ml Toluol gelöst und auf -10°C gekühlt. Man tropft nacheinander langsam 22.7 ml (162 mmol) TEA und 34.2 ml (149 mmol) tert.-Butyl-dimethylsilyltrifluormethansulfonat zu und rührt 0.5 h bei 0°C und 0.5 h bei RT. Danach trennt man die untere Triethylamin-Trifluormethansulfonsäuresalz-Phase ab und gibt die überstehende Lösung ohne weitere Aufreinigung unter Argon zu 25.4 g (162 mmol) 4-Chlorphenylboronsäure. Nach Zusatz von 31.6 g (298 mmol) Natriumcarbonat als Lösung in 148 ml Wasser wird durch Anlegen eines Vakuums entgast und mit Argon belüftet. Man gibt 7.82 g (6.76 mmol) Tetrakis-(triphenylphosphin)palladium(0) hinzu und entgast und belüftet mit Argon. Unter starkem Rühren wird für 2 h auf 95°C erhitzt. Nach dem Abkühlen trennt man die Phasen, wäscht die organische Phase dreimal mit gesättigter Ammoniumchlorid-Lösung, einmal mit Wasser und einmal mit gesättigter Natriumchlorid-Lösung, trocknet über Magnesiumsulfat, filtriert und engt ein. Nach Säulenchromatographie an Kieselgel 60 (Laufmittel: Toluol / Cyclohexan 1:1) erhält man 53.1 g (90% d. Th.) des Produkts, das noch zu 9% mit 4,4'-Dichlorbiphenyl verunreinigt ist.
MS (DCI): m/z = 437 (M+H)⁺
¹H-NMR (400 MHz, DMSO-d₆): δ = 7.83-7.77 (m, 2 H), 7.56-7.51 (m, 2 H), 7.43 (d, 1 H), 7.21 (d, 1 H), 6.95 (dd, 1 H), 4.35-4.30 (m, 2 H), 3.99-3.94 (m, 2 H), 1.02 (s, 9 H), 0.12 (s, 6 H).

### Beispiel 46A

### 6-(2-Chlorethoxy)-2-(4-chlorphenyl)-benzofuran-3(2H)-on

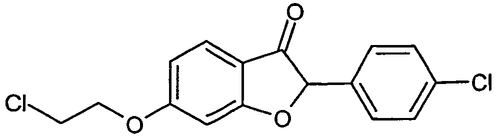

66.5 g (152 mmol) 3-*tert*.-Butyldimethylsilyloxy-6-(2-chlorethoxy)-2-(4-chlorphenyl)-benzofuran werden unter Argon bei RT in 500 ml einer 4 M Chlorwasserstoff-Lösung in Dioxan gelöst und 1.5 h gerührt. Man engt ein und erhält 49.2 g eines Rückstands, den man unter Argon lagert und ohne Aufreinigung weiter umsetzt.
LC-MS (Methode 5): Rₜ = 3.77 min.
MS (ESIpos): m/z = 323 (M+H)⁺.

### Beispiel 47A

### (S*,R*)-3-[6-(2-Chlorethoxy)-2-(4-chlorphenyl)-3-oxo-2,3-dihydrobenzofuran-2-yl]-3-phenyl-propanal

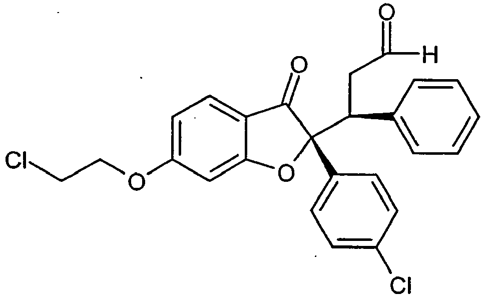

Die Titelverbindung wird in Analogie zur Synthese von Beispiel 5A ausgehend von Beispiel 46A hergestellt.
Ausbeute: 31 % d. Th.
LC-MS (Methode 4): Rₜ = 3.73 min.
MS (ESIpos): m/z = 455 (M+H)⁺
¹H-NMR (200 MHz, DMSO-d₆): δ = 9.34 (d, 1 H), 7.75-7.66 (m, 2 H), 7.58-7.49 (m, 2 H), 7.30-7.05 (m, 6 H), 6.98 (d, 1 H), 6.56 (dd, 1 H), 4.43-4.34 (m, 2 H), 4.30 (dd, 1 H), 4.02-3.94 (m, 2 H), 3.09 (ddd, 1 H), 2.55 (dd, 1 H).

### Beispiel 48A

### (1S*,3S*,3aR*,8bS*)-6-(2-Chlorethoxy)-3a-(4-chlorphenyl)-3-phenyl-2,3,3a,8b-tetrahydrocyclo-penta[b]benzofuran-1,8b-(1H)-diol

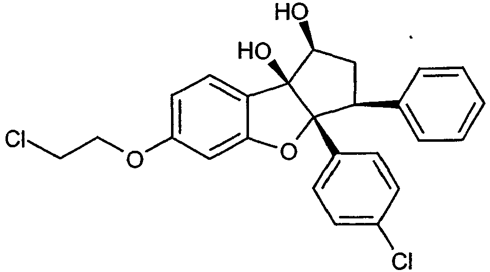

Die Titelverbindung wird in Analogie zur Synthese von Beispiel 6A ausgehend von Beispiel 47A hergestellt.
Ausbeute: 94% d. Th.
LC-MS (Methode 6): Rₜ = 2.73 min.
MS (ESIpos): m/z = 457 (M+H)⁺
¹H-NMR (200 MHz, DMSO-d₆): δ = 7.32-7.02 (m, 8 H), 6.96-6.88 (m, 2 H), 6.72 (d, 1 H), 6.62 (dd, 1 H), 5.86 (d, 1 H), 5.07 (s, 1 H), 4.45 (q, 1 H), 4.34-4.25 (m, 2 H), 4.00-3.93 (m, 2 H), 3.40-3.25 (m, 1 H), 2.49-2.36 (m, 1 H), 2.20 (ddd, 1 H).

### Beispiel 49A

### (1S*,3S*,3aR*,8bS*)-6-(2-Azidoethoxy)-3a-(4-chlorphenyl)-3-phenyl-2,3,3a,8b-tetrahydrocyclopenta[b]benzofuran-1,8b-(1H)-diol

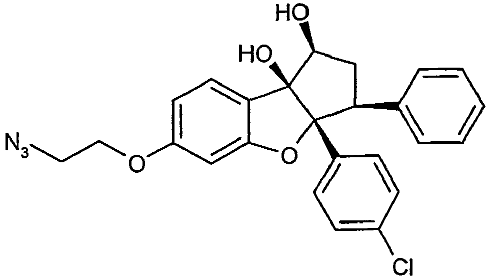

Zu 200 mg (0.44 mmol) (1S*,3S*,3aR*,8bS*)-6-(2-Chlorethoxy)-3a-(4-chlorphenyl)-3-phenyl-2,3,3a,8b-tetrahydrocylopenta[*b*]benzofuran-1,8b-(1*H*)-diol (Beispiel 48A) in 4 ml DMF unter Argon gibt man 56.9 mg (0.87 mmol) Natriumazid und erhitzt über Nacht auf 100°C. Nach dem Abkühlen engt man ein, nimmt den Rückstand mit Wasser und Dichlormethan auf, trennt die Phasen, extrahiert die wässrige Phase zweimal mit Dichlormethan, wäscht die vereinigten organischen Phasen mit gesättigter Natriumchlorid-Lösung, trocknet über Magnesiumsulfat, filtriert und engt ein. Nach Gradienten-Chromatographie an Kieselgel 60 (Laufmittel: Toluol /Essigsäureethylester) erhält man 190 mg (94% d. Th.) des Produkts.
LC-MS (Methode 9): Rₜ = 2.66 min.
MS (ESIpos): m/z = 464 (M+H)⁺
¹H-NMR (200 MHz, DMSO-d₆): δ = 7.29 (d, 1 H), 7.26-7.02 (m, 7 H), 6.96-6.90 (m, 2 H), 6.71 (d, 1 H), 6.61 (dd, 1 H), 5.83 (d, 1 H), 5.02 (s, 1 H), 4.49-4.41 (m, 1 H), 4.24-4.19 (m, 2 H), 3.69-3.63 (m, 2 H), 3.34-3.25 (m, 1 H), 2.50-2.40 (m, 1 H), 2.21 (ddd, 1 H).

Die präparative Trennung des Racemats in die Enantiomeren wird über HPLC an chiraler Phase nach Methode 25A durchgeführt.
Analytische Daten (Methode 25B):
Enantiomer A: Rₜ = 5.52 min., Enantiomer B: Rₜ = 8.56 min.

### Beispiel 50A

### (3S*,3aR*,8bR*)-6-(2-Chlorethoxy)-3a-(4-chlorphenyl)-8b-hydroxy-3-phenyl-2,3,3a,8b-tetra-hydrocyclopenta[b]benzofuran-1-on

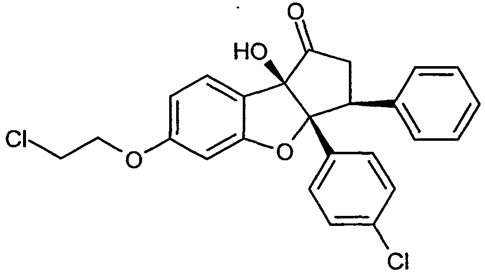

Die Titelverbindung wird in Analogie zur Synthese von Beispiel 7A ausgehend von Beispiel 48A hergestellt.
Ausbeute: 92% d. Th.
LC-MS (Methode 4): Rₜ = 3.54 min.
MS (ESIpos): m/z = 455 (M+H)⁺
¹H-NMR (200 MHz, DMSO-d₆): δ = 7.29-7.00 (m, 10 H), 6.89 (d, 1 H), 6.66 (dd, 1 H), 6.31 (s, 1 H), 4.36-4.28 (m, 2 H), 4.02-3.94 (m, 2 H), 3.65 (dd, 1 H), 3.42-3.20 (m, 1 H), 2.84 (dd, 1 H).

### Beispiel 51A

### (1R*,3S*,3aR*,8bS*)-6-(2-Chlorethoxy)-3a-(4-chlorphenyl)-3-phenyl-2,3,3a,8b-tetrahydrocyclo-penta[b]benzofuran-1,8b-(1H)-diol

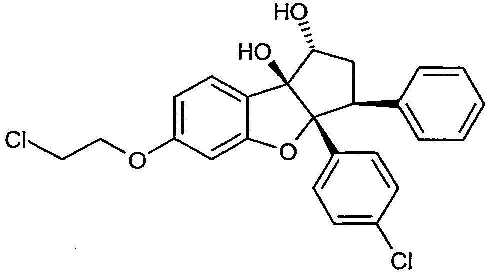

Die Titelverbindung wird in Analogie zur Synthese von Beispiel 8A ausgehend von Beispiel 50A hergestellt.
Ausbeute: 99% d. Th.
LC-MS (Methode 2): Rₜ = 4.24 min.
MS (ESIneg): m/z = 501 (M-H)⁻
¹H-NMR (200 MHz, DMSO-d₆): δ = 7.32 (d, 1 H), 7.14-6.91 (m, 9 H), 6.66 (d, 1 H), 6.55 (dd, 1 H), 5.26 (s, 1 H), 5.17 (d, 1 H), 4.62-4.51 (m, 1 H), 4.32-4.24 (m, 2 H), 4.00-3.92 (m, 2 H), 3.90-3.77 (m, 1 H), 2.66 (dt, 1 H), 1.98-1.83 (m, 1 H).

### Beispiel 52A

### (2R*,3S*,3aR*,8bR*)-6-(2-Chlorethoxy)-3a-(4-chlorphenyl)-8b-hydroxy-1-oxo-3-phenyl-2,3,3a,8b-tetrahydrocyclopenta[b]benzofuran-2-carbonsäure

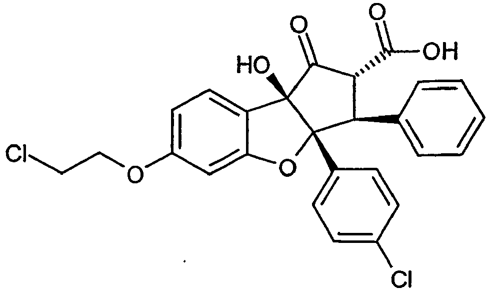

Die Titelverbindung wird in Analogie zur Synthese von Beispiel 17A ausgehend von Beispiel 50A hergestellt. Die Verbindung wird ohne weitere Aufreinigung in der nächsten Stufe eingesetzt.

### Beispiel 53A

### (2R*,3S*,3aR*,8bR*)-6-(2-Chlorethoxy)-3a-(4-chlorphenyl)-2-dimethylcarbamid-8b-hydroxy-3-phenyl-2,3,3a,8b-tetrahydrocyclopenta[b]benzofuran-1-on

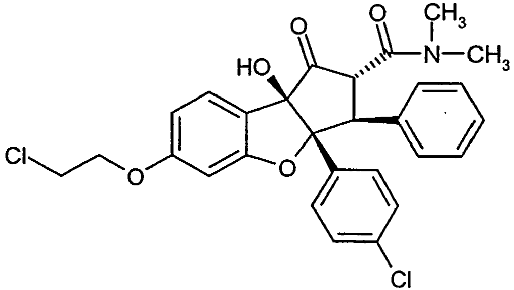

Die Titelverbindung wird in Analogie zur Synthese von Beispiel 16 ausgehend von Beispiel 52A hergestellt.
Ausbeute: 14% d. Th. (ausgehend von Beispiel 50A)
LC-MS (Methode 13): Rₜ = 2.71 min.
MS (ESIpos): m/z = 526 (M+H)⁺
¹H-NMR (200 MHz, DMSO-d₆): δ = 7.23-7.16 (m, 5 H), 7.14-7.05 (m, 3 H), 7.02-6.94 (m, 2 H), 6.89 (d, 1 H), 6.64 (dd, 1 H), 6.47 (s, 1 H), 4.79 (d, 1 H), 4.38-4.29 (m, 2 H), 4.19 (d, 1 H), 4.02-3.94 (m, 2 H), 3.28 (s, 3 H), 2.77 (s, 3 H).

### Beispiel 54A

### (1R*,2R*,3S*,3aR*,8bS*)-6-(2-Chlorethoxy)-3a-(4-chlorphenyl)-2-dimethylcarbamid-3-phenyl-2,3,3a,8b-tetrahydrocyclopenta[b]benzofuran-1,8b-(1H)-diol

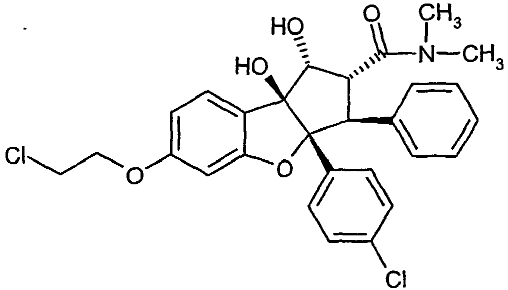

Die Titelverbindung wird in Analogie zur Synthese von Beispiel 17 ausgehend von Beispiel 53A hergestellt.
Ausbeute: 81% d. Th.
LC-MS (Methode 6): Rₜ = 2.31 min.
MS (ESIpos): m/z = 528 (M+H)⁺
¹H-NMR (200 MHz, DMSO-d₆): δ = 7.36 (d, 1 H), 7.23-7.10 (m, 4 H), 7.06-6.96 (m, 3 H), 6.86-6.79 (m, 2 H), 6.70 (d, 1 H), 6.55 (dd, 1 H), 5.50 (d, 1 H), 5.32 (s, 1 H), 4.92-4.82 (m, 1 H), 4.34-4.23 (m, 2 H), 4.20-3.93 (m, 4 H), 3.20 (s, 3 H), 2.72 (s, 3 H).

### Beispiel 55A

### (3S*,3aR*,8bR*)-6-(2-Chlorethoxy)-3a-(4-chlorphenyl)-8b-hydroxy-3-phenyl-2,3,3a,8b-tetra-hydrocyclopenta[b]benzofuran-1-oxim

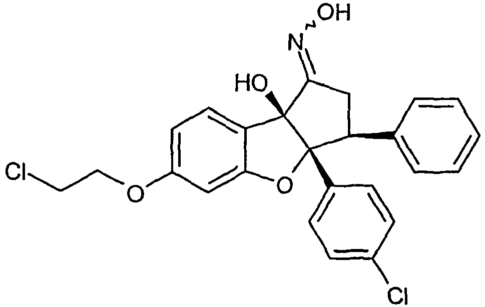

Die Titelverbindung wird in Analogie zur Synthese von Beispiel 14 ausgehend von Beispiel 50A hergestellt.
Ausbeute: 82% d. Th.
LC-MS (Methode 6): Rₜ = 2.55 min.
MS (ESIneg): m/z = 468 (M-H)⁻
¹H-NMR (300 MHz, DMSO-d₆): δ = 11.1 (s, 1 H), 7.35 (d, 1 H), 7.23-7.06 (m, 7 H), 7.02-6.97 (m, 2 H), 6.79 (d, 1 H), 6.65 (dd, 1 H), 5.78 (s, 1 H), 4.34-4.28 (m, 2 H), 3.99-3.93 (m, 2 H), 3.45 (t, 1 H), 3.03 (d, 2 H).

### Beispiel 56A

### (1R*,3S*,3aR*,8bS*)-6-(2-Chlorethoxy)-3a-(4-chlorphenyl)-8b-hydroxy-3-phenyl-2,3,3a,8b-tetrahydrocyclopenta[b]benzofuran-1-amin

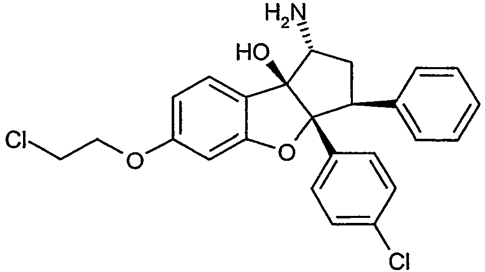

Die Titelverbindung wird in Analogie zur Synthese von Beispiel 15 ausgehend von Beispiel 55A durchgeführt.
Ausbeute: 89% d. Th.
LC-MS (Methode 6): Rₜ = 1.89 min.
MS (ESIpos): m/z = 456 (M+H)⁺
¹H-NMR (200 MHz, DMSO-d₆): δ = 7.27-6.90 (m, 10 H), 6.69 (d, 1 H), 6.58 (dd, 1 H), 4.32-4.24 (m, 2 H), 3.99-3.92 (m, 2 H), 3.62 (dd, 1 H), 3.43 (dd, 1 H), 2.44-2.31 (m, 1 H), 2.25-2.02 (m, 1 H).

### Beispiel 57A

### 4-Benzyloxy-6-hydroxyacetophenon

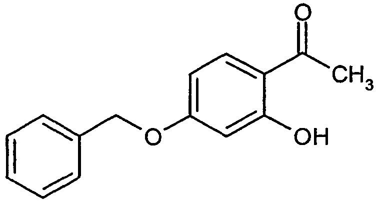

Zu einer Lösung von 50.00 g (329 mmol) 2,4-Dihydroxyacetophenon in 500 ml DMF werden 41 ml (345 mmol) Benzylbromid und 47.69 g (345 mmol) Kaliumcarbonat gegeben und die resultierende Suspension über Nacht bei Raumtemperatur gerührt. Über eine Glasfritte wird dann vom Feststoff abgesaugt und das Filtrat auf 200 ml Wasser und 200 ml Ethylacetat gegossen. Die Phasen werden getrennt und die organische Phase mit gesättigter Ammoniumchlorid-Lösung, Wasser, gesättigter Natriumhydrogencarbonat-Lösung und gesättigter Natriumchlorid-Lösung gewaschen, über Magnesiumsulfat getrocknet, filtriert und eingeengt. Nach Kristallisation aus einem Diethylether/Petrolether-Gemisch resultieren 56.40 g (71% d. Th.) der Titelsubstanz als rosafarbene Kristalle.
LC-MS (Methode 1): Rₜ = 3.70 min.
MS (ESIpos): m/z = 243 (M+H)⁺
¹H-NMR (300 MHz, DMSO-d₆): δ = 12.60 (s, 1 H), 7.85 (d, 1 H), 7.48-7.31 (m, 5 H), 6.60 (dd, 1 H), 6.55 (d, 1 H), 5.19 (s, 2 H), 2.56 (s, 3 H).

### Beispiel 58A

### 6-Benzyloxy-benzofuran-3-on

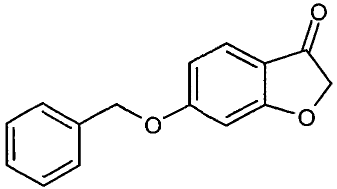

Zu einer siedenden Suspension von 18.44 g (82.55 mmol) Kupfer(II)bromid in 70 ml Ethylacetat werden 10 g (41.28 mmol) in 30 ml Chloroform suspendiertes 4-Benzyloxy-6-hydroxyacetophenon gegeben und die Suspension über Nacht unter Rückfluß erhitzt. Die noch warme Lösung wird dann filtriert und der Filterkuchen mit Ethylacetat nachgewaschen. Die organische Phase wird mit Wasser und gesättigter Natriumchlorid-Lösung gewaschen, über Magnesiumsulfat getrocknet, filtriert und eingeengt. Der Rückstand wird in 150 ml Ethanol gelöst, 7.72 g (94.06 mmol) Natriumacetat-Trihydrat werden zugegeben und die resultierende Lösung eine Stunde unter Rückfluß erhitzt. Die Reaktionsmischung wird dann auf Eis gegeben und das Ethanol abgezogen. Der wässrige Rückstand wird dreimal mit Ethylacetat extrahiert und die vereinigten organischen Phasen mit 1 N Natronlauge, 1 N Salzsäure und anschließend mit gesättigter Natriumchlorid-Lösung gewaschen, über Magnesiumsulfat getrocknet, filtriert und eingeengt. Nach Säulen-chromatographie an Kieselgel 60 (Laufmittel: Toluol → Toluol / Ethylacetat 1:1) erhält man 5.52 g (56% d. Th.) des Produkts als beigefarbene Kristalle.
LC-MS (Methode 4): Rₜ = 3.53 min.
MS (ESIpos): m/z = 241 (M+H)⁺
¹H-NMR (200 MHz, DMSO-d₆): δ = 7.56-7.35 (m, 6 H), 6.91 (d, 1 H), 6.77 (dd, 1 H), 5.23 (s, 2 H), 4.77 (s, 2 H).

### Beispiel 59A

### 2-Brom-6-benzyloxy-benzofuran-3(2H)-on

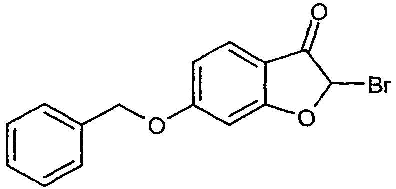

Die Titelverbindung wird in Analogie zur Synthese von Beispiel 43A ausgehend von Beispiel 58A hergestellt.
Ausbeute: 40% d. Th.
LC-MS (Methode 12): Rₜ = 4.12 min.
MS (ESIpos): m/z = 319 (M+H)⁺
¹H-NMR (200 MHz, DMSO-d₆): δ = 7.54-7.37 (m, 5 H), 6.81-6.71 (m, 2 H), 5.56 (s, 1 H), 5.24 (s, 2 H).

### Beispiel 60A

### 3-tert.-Butyldimethylsilyloxy-6-benzyloxy-2-(4-chlorphenyl)-benzofuran

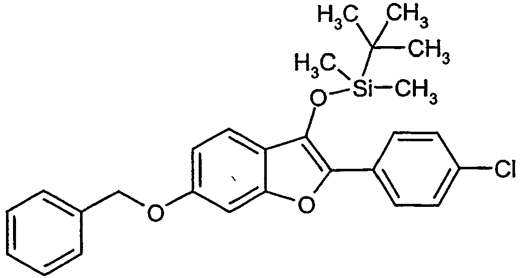

Die Titelverbindung wird in Analogie zur Synthese von Beispiel 44A ausgehend von Beispiel 59A hergestellt.
Ausbeute: 77% d. Th.
LC-MS (Methode 4): Rₜ = 5.20 min.
MS (ESIpos): m/z = 465 (M+H)⁺
¹H-NMR (300 MHz, DMSO-d₆): δ = 7.85 (d, 2 H), 7.59-7.20 (m, 9 H), 7.04 (dd, 1 H), 5.22 (s, 2 H), 1.07 (s, 9 H), 0.17 (s, 3 H), 0.04 (s, 3 H).

### Beispiel 61A

### (S*,R*)-3-[6-Benzyloxy-2-(4-chlorphenyl)-3-oxo-2,3-dihydrobenzofuran-2-yl]-3-phenylpropanal

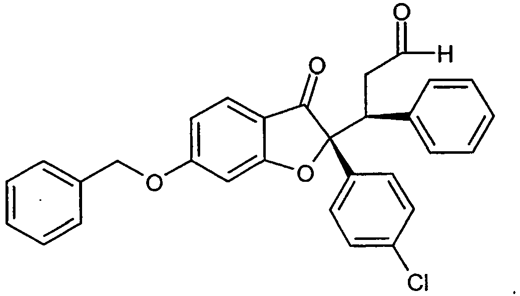

Die Titelverbindung (als Diastereomerengemisch) wird in Analogie zur Synthese von Beispiel 5A ausgehend von Beispiel 60A hergestellt.

### Beispiel 62A

### 2-(2-Chlorethoxy)-6-hydroxyacetophenon

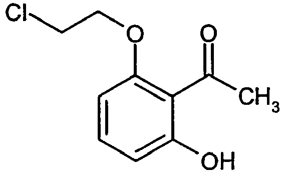

Die Titelverbindung wird in Analogie zur Synthese von Beispiel 41A ausgehend von 2,6-Dihydroxyacetophenon hergestellt.
Ausbeute: 90% d. Th.
LC-MS (Methode 2): Rₜ = 2.86 min.
MS (ESIpos): m/z = 215 (M+H)⁺
¹H-NMR (200 MHz, DMSO-d₆): δ = 12.1 (s, 1 H), 7.34 (t, 1 H), 6.56 (dd, 1 H), 6.52 (dd, 1 H), 4.36-4.29 (m, 2 H), 4.05-3.97 (m, 2 H), 2.61 (s, 3 H).

### Beispiel 63A

### 4-(2-Chlorethoxy)-benzofuran-3-on

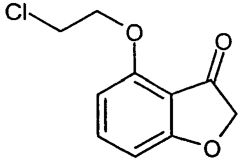

Die Titelverbindung wird in Analogie zur Synthese von Beispiel 42A ausgehend von Beispiel 62A hergestellt.
Ausbeute: 69% d. Th.
LC-MS (Methode 2): Rₜ = 2.42 min.
MS (ESIpos): m/z = 213 (M+H)⁺
¹H-NMR (200 MHz, DMSO-d₆): δ = 7.61 (t, 1 H), 6.80 (d, 1 H), 6.67 (d, 1 H), 4.70 (s, 2 H), 4.43-4.35 (m, 2 H), 4.00-3.93 (m, 2 H).

### Beispiel 64A

### 2-Brom-4-(2-chlorethoxy)-benzofuran-3(2H)-on

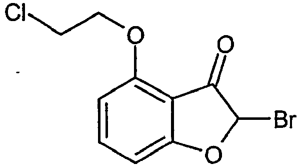

Die Titelverbindung wird in Analogie zur Synthese von Beispiel 43A ausgehend von Beispiel 63A hergestellt.
Ausbeute: 86% d. Th.
LC-MS (Methode 4): Rₜ = 2.90 min.
MS (ESIpos): m/z = 291 (M+H)⁺
¹H-NMR (300 MHz, DMSO-d₆): δ = 7.62 (t, 1 H), 6.69 (d, 1 H), 6.65 (d, 1 H), 5.47 (s, 1 H), 4.42-4.37 (m, 2 H), 3.98-3.93 (m, 2 H).

### Beispiel 65A

### 2-Brom-3-tert.-butyldimethylsilyloxy-4-(2-chlorethoxy)-benzofuran

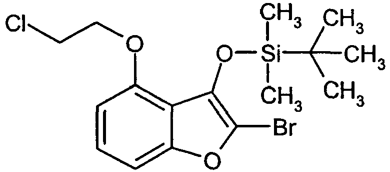

Die Titelverbindung wird in Analogie zur Synthese von Beispiel 44A ausgehend von Beispiel 64A hergestellt.
Ausbeute: 46% d. Th.
LC-MS (Methode 9): Rₜ = 2.14 min.
MS (ESIpos): m/z = 292 [M+H-Si(CH₃)₂C(CH₃)₃]⁺
¹H-NMR (300 MHz, DMSO-d₆): δ = 7.22 (t, 1 H), 7.11 (dd, 1 H), 6.84 (dd, 1 H), 4.43-4.38 (m, 2 H), 3.99-3.94 (m, 2 H), 1.03 (s, 9 H), 0.26 (s, 6 H).

### Beispiel 66A

### 3-tert.-Butyldimethylsilyloxy-4-(2-chlorethoxy)-2-(4-chlorphenyl)-benzofuran

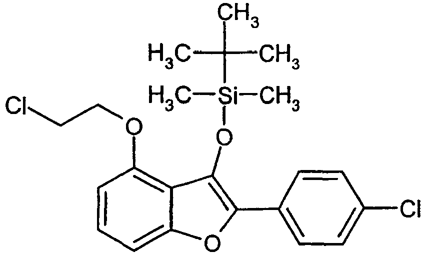

Die Titelverbindung wird in Analogie zur Synthese von Beispiel 45A ausgehend von Beispiel 65A hergestellt.
Ausbeute: 75% d. Th.
LC-MS (Methode 4): Rₜ = 3.29 min.
MS (ESIpos): m/z = 437 (M+H)⁺
¹H-NMR (400 MHz, DMSO-d₆): δ = 7.82-7.77 (m, 2 H), 7.57-7.52 (m, 2 H), 7.26 (t, 1 H), 7.16 (d, 1 H), 6.83 (d, 1 H), 4.45-4.41 (m, 2 H), 4.02-3.98 (m, 2 H), 1.03 (s, 9 H), -0.02 (s, 6 H).

### Beispiel 67A

### 4-(2-Chlorethoxy)-2-(4-chlorphenyl)-benzofuran-3-on

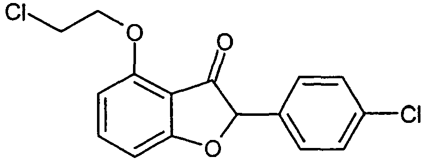

Die Titelverbindung wird in Analogie zur Synthese von Beispiel 46A ausgehend von Beispiel 66A hergestellt. Die Verbindung wird ohne weitere Aufreinigung in der nächsten Stufe eingesetzt.

### Beispiel 68A

### (S*,R*)-3-[4-(2-Chlorethoxy)-2-(4-chlorphenyl)-3-oxo-2,3-dihydrobenzofuran-2-yl]-3-phenyl-propanal

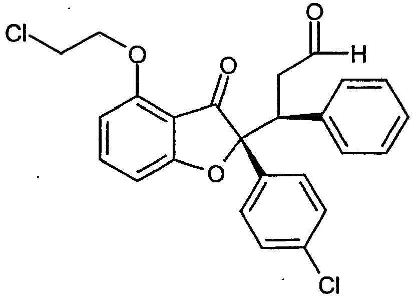

Die Titelverbindung wird in Analogie zur Synthese von Beispiel 5A ausgehend von Beispiel 67A hergestellt.
Ausbeute: 28% d. Th.
LC-MS (Methode 9): Rₜ = 2.68 min.
MS (ESIpos): m/z = 455 (M+H)⁺
¹H-NMR (300 MHz, DMSO-d₆): δ = 9.33 (dd, 1 H), 7.70-7.65 (m, 2 H), 7.57 (t, 1 H), 7.55-7.49 (m, 2 H), 7.30-7.25 (m, 2 H), 7.17-7.06 (m, 3 H), 6.93 (d, 1 H), 6.51 (d, 1 H), 4.29 (dd, 1 H), 4.28-4.10 (m, 2 H), 3.83-3.77 (m, 2 H), 3.07 (ddd, 1 H), 2.53 (ddd, 1 H).

### Beispiele 69A

### (1S*,3S*,3aR*,8bS*)-8-(2-Chlorethoxy)-3a-(4-chlorphenyl)-3-phenyl-2,3,3a,8b-tetrahydrocyclo-penta[b]benzofuran-1,8b-(1H)-diol

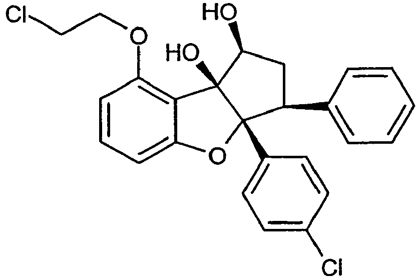

Die Titelverbindung wird in Analogie zur Synthese von Beispiel 6A ausgehend von Beispiel 68A hergestellt.
Ausbeute: 55% d. Th.
LC-MS (Methode 2): Rₜ = 3.47 min.
MS (ESIpos): m/z = 457 (M+H)⁺
¹H-NMR (300 MHz, DMSO-d₆): δ = 7.28 (t, 1 H), 7.25-7.20 (m, 2 H), 7.20-7.15 (m, 2 H), 7.12-7.03 (m, 3 H), 6.91-6.86 (m, 2 H), 6.72 (d, 1 H), 6.64 (d, 1 H), 5.72 (d, 1 H), 4.91 (s, 1 H), 4.80 (ddd, 1 H), 4.36-4.31 (m, 2 H), 4.03-3.97 (m, 2 H), 3.30-3.24 (m, 1 H), 2.54-2.42 (m, 1 H), 2.19 (ddd, 1 H).

### Beispiel 70A

### (3S*,3aR*,8bR*)-8-(2-Chlorethoxy)-3a-(4-chlorphenyl)-8b-hydroxy-3-phenyl-2,3,3a,8b-tetra-hydrocyclopenta[b]benzofuran-1-on

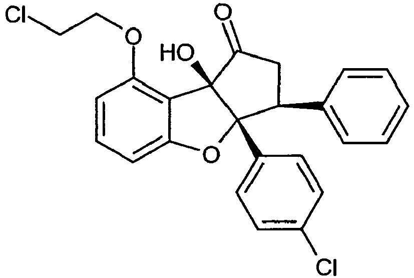

Die Titelverbindung wird in Analogie zur Synthese von Beispiel 7A ausgehend von Beispiel 69A hergestellt.
Ausbeute: 63% d. Th.
LC-MS (Methode 9): Rₜ = 2.70 min.
MS (ESIpos): m/z = 455 (M+H)⁺
¹H-NMR (400 MHz, DMSO-d₆): δ = 7.40 (t, 1 H), 7.22-7.17 (m, 2 H), 7.14-7.05 (m, 3 H), 7.04-6.96 (m, 4 H), 6.87 (d, 1 H), 6.71 (d, 1 H), 6.03 (s, 1 H), 4.40-4.24 (m, 2 H), 3.93-3.86 (m, 2 H), 3.70 (dd, 1 H), 3.14 (dd, 1 H), 2.95 (dd, 1 H).

### Beispiel 71A

### (1R*,3S*,3aR*,8bS*)-8-(2-Chlorethoxy)-3a-(4-chlorphenyl)-3-phenyl-2,3,3a,8b-tetrahydrocyclo-penta[b]benzofuran-1,8b-(1H)-diol

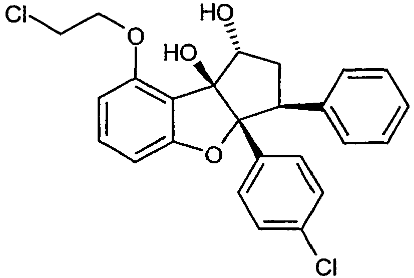

Die Titelverbindung wird in Analogie zur Synthese von Beispiel 8A ausgehend von Beispiel 70A hergestellt.
Ausbeute: 70% d. Th.
LC-MS (Methode 8): Rₜ = 3.67 min.
MS (ESIpos): m/z = 457 (M+H)⁺
¹H-NMR (200 MHz, DMSO-d₆): δ = 7.24 (t, 1 H), 7.16-6.98 (m, 9 H), 6.68 (d, 1 H), 6.59 (d, 1 H), 5.11 (s, 1 H), 4.60-4.53 (m, 1 H), 4.50 (d, 1 H), 4.33-4.25 (m, 2 H), 4.03-3.90 (m, 3 H), 2.75 (dt, 1 H), 2.05 (dd, 1 H).

### Beispiel 72A

### 4-Hydroxybenzofuran-3-on

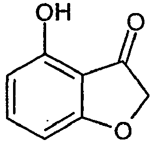

Die Titelverbindung wird in Analogie zur Synthese von Beispiel 42A ausgehend von 2,6-Dihydroxyacetophenon hergestellt.
Ausbeute: 54% d. Th.
LC-MS (Methode 4): Rₜ = 2.04 min.
MS (ESIpos): m/z = 151 (M+H)⁺
¹H-NMR (300 MHz, DMSO-d₆): δ = 10.8 (s, 1 H), 7.43 (t, 1 H), 6.56 (dd, 1 H), 6.44 (dd, 1 H), 4.64 (s, 2 H).

### Beispiel 73A

### 4-(2-Methoxyethoxy)-benzofuran-3-on

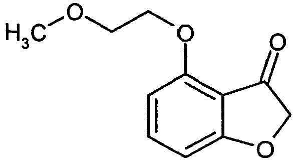

Die Titelverbindung wird in Analogie zur Synthese von Beispiel 18A ausgehend von Beispiel 72A hergestellt.
Ausbeute: 68% d. Th.
LC-MS (Methode 4): Rₜ = 2.23 min.
MS (ESIpos): m/z = 209 (M+H)⁺
¹H-NMR (300 MHz, DMSO-d₆): δ = 7.59 (t, 1 H), 6.75 (d, 1 H), 6.65 (d, 1 H), 4.68 (s, 2 H), 4.25-4.20 (m, 2 H), 3.71-3.66 (m, 2 H), 3.34 (s, 3 H).

### Beispiel 74A

### 2-Brom-4-(2-methoxyethoxy)-benzofuran-3-on

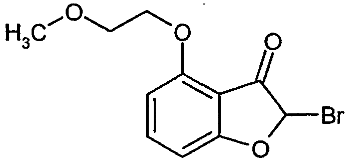

Die Titelverbindung wird in Analogie zur Synthese von Beispiel 43A ausgehend von Beispiel 73A hergestellt.
Ausbeute: 84% d. Th.
LC-MS (Methode 4): Rₜ = 2.63 min.
MS (ESIpos): m/z = 287 (M+H)⁺
¹H-NMR (300 MHz, DMSO-d₆): δ = 8.24 (s, 2 H), 7.61 (t, 1 H), 6.65 (d, 1 H), 6.63 (d, 1 H), 5.45 (s, 1 H), 4.25-4.20 (m, 2 H), 3.71-3.65 (m, 2 H), 3.34 (s, 3 H).

### Beispiel 75A

### 2-Brom-3-tert.-butyldimethylsilyloxy-4-(2-methoxyethoxy)-benzofuran

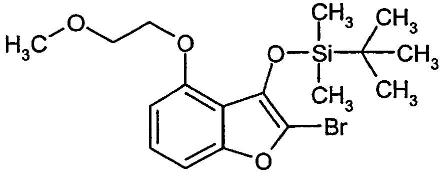

Die Titelverbindung wird in Analogie zur Synthese von Beispiel 44A ausgehend von Beispiel 74A hergestellt.
Ausbeute: 99% d. Th.
LC-MS (Methode 14): Rₜ = 2.43 min.
MS (ESIpos): m/z = 401 (M+H)⁺
¹H-NMR (300 MHz, DMSO-d₆): δ = 7.21 (t, 1 H), 7.07 (dd, 1 H), 6.81 (dd, 1 H), 4.25-4.20 (m, 2 H), 3.72-3.66 (m, 2 H), 3.29 (s, 3 H), 1.02 (s, 9 H), 0.24 (s, 6 H).

### Beispiele 76A

### 3-tert.-Butyldimethylsilyloxy-2-(4-chlorphenyl)-4-(2-methoxyethoxy)-benzofuran

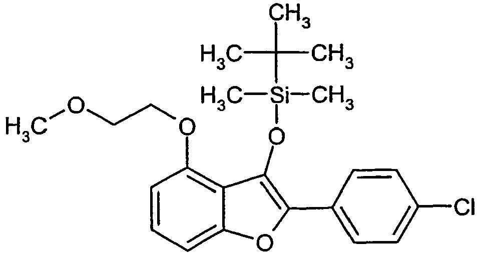

Die Titelverbindung wird in Analogie zur Synthese von Beispiel 45A ausgehend von Beispiel 75A hergestellt.
Ausbeute: 68% d. Th.
LC-MS (Methode 9): Rₜ = 3.58 min.
MS (ESIpos): m/z = 433 (M+H)⁺
¹H-NMR (300 MHz, DMSO-d₆): δ = 7.85-7.79 (m, 2 H), 7.58-7.53 (m, 2 H), 7.25 (t, 1 H), 7.14 (dd, 1 H), 6.82 (dd, 1 H), 4.27-4.22 (m, 2 H), 3.75-3.70 (m, 2 H), 3.29 (s, 3 H), 1.02 (s, 9 H), -0.02 (s, 6 H).

### Beispiel 77A

### 2-(4-Chlorphenyl)-4-(2-methoxyethoxy)-benzofuran-3-on

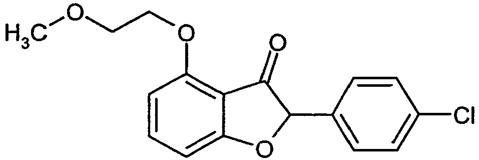

Die Titelverbindung wird in Analogie zur Synthese von Beispiel 46A ausgehend von Beispiel 76A hergestellt. Die Verbindung wird ohne weitere Aufreinigung in der nächsten Stufe eingesetzt.

### Beispiel 78A

### (S*,R*)-3-[2-(4-Chlorphenyl)-4-(2-methoxyethoxy)-3-oxo-2,3-dihydrobenzofuran-2-yl]-3-phenyl-propanal

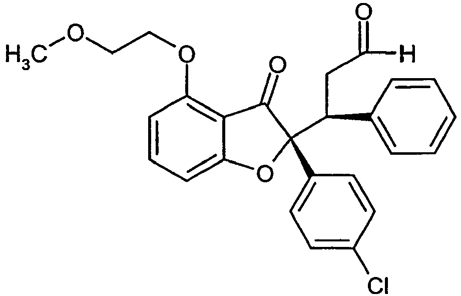

Die Titelverbindung wird in Analogie zur Synthese von Beispiel 5A ausgehend von Beispiel 77A hergestellt.
Ausbeute: 8% d. Th.
LC-MS (Methode 9): Rₜ = 2.58 min.
MS (ESIpos): m/z = 451 (M+H)⁺.

### Beispiel 79A

### (1S*,3S*,3aR*,8bS*)-6-(3-Chlorpropoxy)-3a-(4-chlorphenyl)-3-phenyl-2,3,3a,8b-tetrahydrocyclo-penta[b]benzofuran-1,8b-(1H)-diol

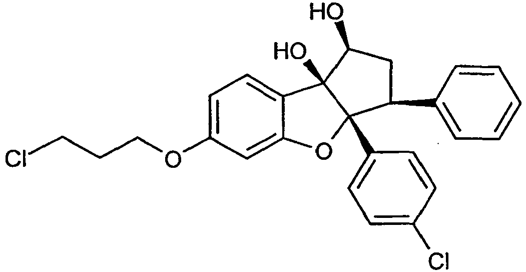

Die Titelverbindung wird in Analogie zur Synthese der Beispiele 41A - 48A ausgehend von 2,4-Dihydroxyacetophenon und 3-Chlorpropanol hergestellt.
LC-MS (Methode 13): Rₜ = 3.02 min.
MS (ESIneg): m/z = 469 (M-H)⁻
¹H-NMR (300 MHz, DMSO-d₆): δ = 7.32-7.01 (m, 8 H), 6.94-6.92 (m, 2 H), 6.69 (d, 1 H), 6.60 (dd, 1 H), 5.81 (d, 1 H), 5.01 (s, 1 H), 4.48-4.42 (m, 1 H), 4.13 (t, 2 H), 3.80 (t, 2 H), 3.35-3.27 (m, 1 H), 2.47-2.41 (m, 1 H), 2.30-2.14 (m, 3 H).

### Beispiel 80A

### N-(3-Oxo-2,3-dihydro-1-benzofuran-6-yl)-acetamid

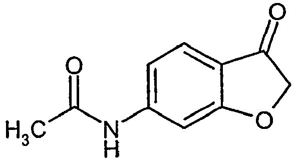

Zu einer Lösung von 64.6 g (0.484 mol) Aluminiumchlorid in 100 ml 1,2-Dichlorethan werden bei 0°C unter Argon 32.6 g (0.181 mol) Chloracetylchlorid und anschließend innerhalb von 15 Minuten 20 g (0.121 mol) N-(3-Methoxyphenyl)-acetamid zugegeben. Die Temperatur steigt dabei auf 10°C an. Der Ansatz wird dann langsam auf Raumtemperatur erwärmt und über Nacht gerührt. Die braune Mischung wird auf Eiswasser gegeben und Ethylacetat hinzugefügt. Nach kräftigem Rühren fällt *N*-(3-Methoxy-4-chloracetylphenyl)-acetamid aus, welches über eine Nutsche abgesaugt und im Hochvakuum getrocknet wird. Der erhaltene Feststoff wird in 140 ml Ethanol vorgelegt, und nach Zugabe von 13.2 g (0.154 mol) Natriumacetat wird über Nacht unter Rückfluß erhitzt. Nach dem Abkühlen wird mit Wasser versetzt und das Ethanol am Rotationsverdampfer abgezogen. Der ausfallende Feststoff wird über eine Fritte abgesaugt und getrocknet. Es resultieren 9.35 g (40% d. Th.) des Produkts als Feststoff mit leicht rötlicher Färbung.
¹H-NMR (200 MHz, DMSO-d₆): δ = 10.5 (s, 1 H), 7.7 (s, 1 H), 7.6 (s, 1 H), 7.1 (s, 1 H), 4.8 (s, 2 H), 3.3 (s, 3 H).

### Beispiel 81A

### 6-Amino-1-benzofuran-3(2H)-on

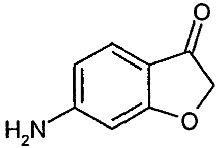

Zu einer Lösung von 500 mg (2.62 mmol) *N*-(3-Oxo-2,3-dihydro-1-benzofuran-6-yl)-acetamid in 5 ml Methanol werden 5 ml 1 N Salzsäure gegeben und eine Stunde unter Rückfluß erhitzt. Nach dem Abkühlen wird der Ansatz auf eine Mischung aus Eiswasser, gesättigter Natriumhydrogencarbonat-Lösung und Ethylacetat gegeben. Die organische Phase wird abgetrennt, mit Wasser und gesättigter Natriumchlorid-Lösung gewaschen, über Magnesiumsulfat getrocknet, filtriert und eingeengt. Es resultieren 318 mg (61% d. Th.) der Zielverbindung als braunes Pulver mit einer Reinheit von 75%.
LC-MS (Methode 4): Rₜ = 1.71 min.
MS (ESIpos): m/z = 150 (M+H)⁺
¹H-NMR (300 MHz, DMSO-d₆): δ = 7.24 (s, 1 H), 6.51 (br. s, 2 H), 6.32 (dd, 1 H), 6.12 (d, 1 H), 4.75 (s, 2 H).

### Beispiel 82A

### Benzyl-(3-oxo-2,3-dihydro-1-benzofuran-6-yl)carbamat

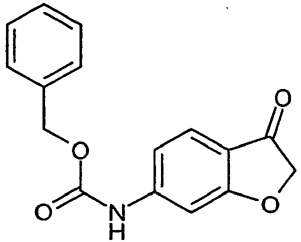

Zu einer Lösung von 23.42 g (157 mmol) 6-Amino-1-benzofuran-3(2H)-on in 400 ml THF werden bei 0°C 54.7 ml (314 mmol) Diisopropylethylamin und 28.3 ml (188 mmol) Chlorameisensäurebenzylester gegeben und dann für 3 Stunden bei Raumtemperatur gerührt. Dann werden erneut 4.7 ml (31 mmol) Chlorameisensäurebenzylester zugegeben und über Nacht gerührt. Der Ansatz wird auf Eiswasser gegeben und zweimal mit Ethylacetat extrahiert. Die vereinigten organischen Phasen werden mit Wasser und gesättigter Natriumchlorid-Lösung gewaschen, über Magnesiumsulfat getrocknet, filtriert und eingeengt. Chromatographie an Kieselgel 60 (Laufmittel: Dichlormethan → Dichlormethan / Ethylacetat 5:1) liefert 30.80 g (64% d. Th.) des Produkts als farblose Kristalle.
LC-MS (Methode 4): Rₜ = 3.43 min.
MS (ESIpos): m/z = 284 (M+H)⁺
¹H-NMR (300 MHz, DMSO-d₆): δ = 10.38 (s, 1 H), 7.56-7.31 (m, 7 H), 7.12 (dd, 1 H), 5.20 (s, 2 H), 4.74 (s, 1 H).

### Beispiel 83A

### Benzyl-[(1S*,3S*,3aR*,8bS*)-3a-(4-chlorphenyl)-1,8b-dihydroxy-3-phenyl-2,3,3a,8b-tetrahydro-1H-benzo[b]cyclopenta[d]furan-6-yl]-carbamat

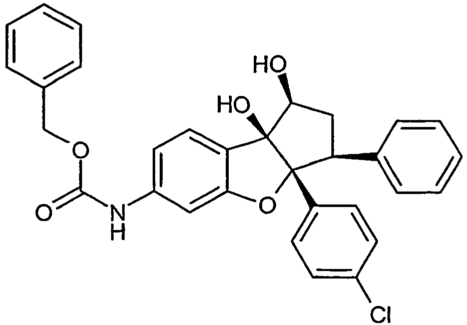

Die Titelverbindung wird in Analogie zur Synthese der Beispiele 43A (Methode a), 44A, 45A, 46A, 5A und 6A ausgehend von Beispiel 82A hergestellt.
LC-MS (Methode 7): Rₜ = 2.90 min.
MS (ESIneg): m/z = 526 (M-H)⁻
¹H-NMR (400 MHz, DMSO-d₆): δ = 9.88 (s, 1 H), 7.46-6.91 (m, 17 H), 5.87 (d, 1 H), 5.18 (m, 2 H), 5.07 (s, 1 H), 4.45-4.44 (m, 1 H), 3.35-3.28 (m, 1 H), 2.48-2.43 (m, 1 H), 2.25-2.20 (m, 1 H).

### Beispiel 84A

### (1S*,3S*,3aR*,8bS*)-6-Amino-3a-(4-chlorphenyl)-3-phenyl-1,2,3,3a-tetrahydrocyclopenta[b]-benzofuran-1,8b-(1H)-diol

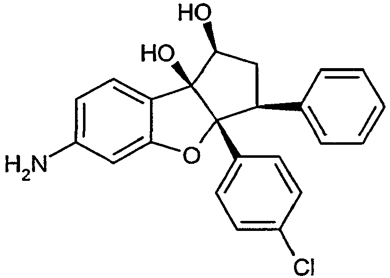

Zu einer Lösung von 8.18 g (15.5 mmol) Benzyl-[(1S*,3S*,3aR*,8bS*)-3a-(4-chlorphenyl)-1,8b-dihydroxy-3-phenyl-2,3,3a,8b-tetrahydro-1H-benzo[b]cyclopenta[d]furan-6-yl]-carbamat in 100 ml Methanol wird 1 g 10%-iges Palladium auf Aktivkohle gegeben und für 2 Stunden bei 2 bar hydriert. Nach Abtrennen des Katalysators wird der Rückstand eingeengt, und man erhält 6.06 g (92% d. Th.) der Titelverbindung, welche ca. 7% der entsprechenden dehalogenierten Verbindung als Verunreinigung enthält.
LC-MS (Methode 13): Rₜ = 2.34 min.
MS (ESIpos): m/z = 394 (M+H)⁺
¹H-NMR (300 MHz, DMSO-d₆): δ = 7.22-6.87 (m, 10 H), 6.24-6.21 (m, 2 H), 5.73 (d, 1 H), 5.19 (s, 2 H), 4.78 (s, 1 H), 4.44-4.37 (m, 1 H), 3.35-3.24 (m, 1 H), 2.48-2.40 (m, 1 H), 2.27-2.11 (m, 1 H).

### Beispiel 85A

### Brom-(4-chlorphenyl)-essigsäure

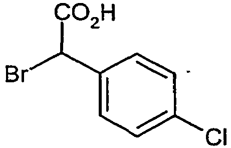

80.0 g (469 mmol) 4-Chlorphenylessigsäure werden unter Argon in 200 ml Tetrachlorkohlenstoff gelöst und unter RF erhitzt. In der Siedehitze gibt man 100 g (563 mmol) *N*-Bromsuccinimid und 7.70 g (46.9 mmol) 2,2'-Azo-bis-2-methylpropannitril hinzu und erhitzt über Nacht unter RF. Anschließend kühlt man auf 0°C, saugt ab, wäscht den Niederschlag mit kaltem Tetrachlorkohlenstoff und engt das Filtrat ein. Der Rückstand wird in Diethylether gelöst und dreimal mit gesättigter wässriger Natriumhydrogencarbonat-Lösung extrahiert. Die vereinigten wässrigen Phasen werden mit konzentrierter Salzsäure auf einen pH-Wert von 1 eingestellt und viermal mit Diethylether extrahiert. Man trocknet die vereinigten organischen Phasen über Magnesiumsulfat, filtriert, engt ein und erhält 83.0 g (71% d. Th.) des Produkts, das ohne weitere Aufreinigung weiter umgesetzt wird.
LC-MS (Methode 11): Rₜ = 3.42 min.
MS (ESIneg): m/z = 249 (M-H)⁻
¹H-NMR (300 MHz, DMSO-d₆): δ = 13.5 (s, 1 H), 7.61-7.55 (m, 2 H), 7.49-7.43 (m, 2 H), 5.80 (s, 1 H).

### Beispiel 86A

### (4-Chlorphenyl)-(3,5-dipropoxyphenoxy)-essigsäure

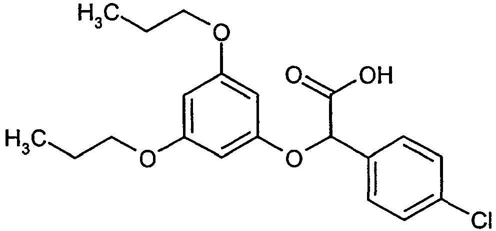

Zu einer Lösung von 10.0 g (47.6 mmol) 3,5-Dipropoxyphenol und 11.9 g (47.6 mmol) Brom-(4-chlorphenyl)-essigsäure in 150 ml THF werden unter Argon portionsweise 4.37 g (109 mmol) Natriumhydrid als 60%-ige Dispersion in Mineralöl gegeben. Man rührt 0.5 h bei RT und erhitzt über Nacht unter RF. Anschließend gibt man unter Eiskühlung Wasser hinzu, extrahiert dreimal mit Chloroform, wäscht die vereinigten organischen Phasen mit 1 N Natronlauge und gesättigter wässriger Natriumchlorid-Lösung, trocknet über Magnesiumsulfat, filtriert und engt ein (Rückstand 1). Die vereinigten wässrigen Phasen werden mit konzentrierter Salzsäure auf einen pH-Wert von 1 eingestellt und dreimal mit Diethylether extrahiert. Man wäscht die vereinigten organischen Phasen mit gesättigter wässriger Natriumchlorid-Lösung, trocknet über Magnesiumsulfat, filtriert und engt ein (Rückstand 2). Nach Chromatographie der vereinigten Rückstände 1 und 2 an Kieselgel 60 (1. Laufmittel: Toluol / Essigsäureethylester 9: 1, zur Abtrennung von nicht umgesetztem 3,5-Dipropoxy-phenol, 2. Laufmittel: Dichlormethan / Methanol 9:1) erhält man 12.1 g (67% d. Th.) des Produkts.
LC-MS (Methode 1): Rₜ = 4.21 min.
MS (ESIpos): m/z = 379 (M+H)⁺
¹H-NMR (400 MHz, DMSO-d₆): δ = 7.54 (d, 2 H), 7.38 (d, 2 H), 6.09-6.01 (m, 3 H), 5.41 (s, 1 H), 3.84 (t, 4 H), 1.67 (sext, 4 H), 0.94 (t, 6 H).

### Beispiel 87A

### 2-(4-Chlorphenyl)-4,6-dipropoxybenzofuran-3(2H)-on

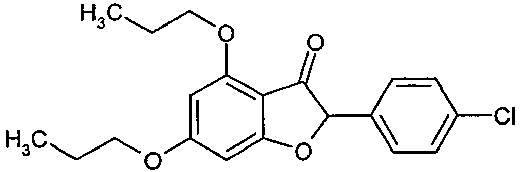

Die Titelverbindung wird in Analogie zur Synthese von Beispiel 4A ausgehend von Beispiel 86A hergestellt.
Ausbeute: 44% d. Th.
LC-MS (Methode 12): Rₜ = 4.75 min.
MS (ESIpos): m/z = 361 (M+H)⁺
¹H-NMR (300 MHz, DMSO-d₆): δ = 7.50-7.44 (m, 2 H), 7.35-7.30 (m, 2 H), 6.46 (d, 1 H), 6.21 (d, 1 H), 5.74 (s, 1 H), 4.11-3.98 (m, 4 H), 1.82-1.64 (m, 4 H), 0.99 (t, 3 H), 0.96 (t, 3 H).

### Beispiel 88A

### (S*,R*)-3-[2-(4-Chlorphenyl)-4,6-dipropoxy-3-oxo-2,3-dihydrobenzofuran-2-yl]-3-phenylpropanal

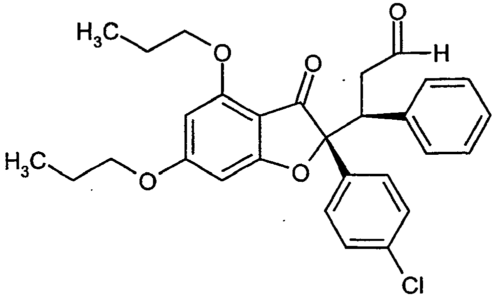

Die Titelverbindung wird in Analogie zur Synthese von Beispiel 5A ausgehend von Beispiel 87A hergestellt.
Ausbeute: 49% d. Th.
LC-MS (Methode 12): Rₜ = 4.93 min.
MS (ESIpos): m/z = 493 (M+H)⁺
¹H-NMR (200 MHz, DMSO-d₆): δ = 9.32 (d, 1 H), 7.69-7.61 (m, 2 H), 7.55-7.47 (m, 2 H), 7.31-7.09 (m, 5 H), 6.47 (d, 1 H), 5.98 (d, 1 H), 4.24 (dd, 1 H), 4.02 (t, 2 H), 3.90-3.74 (m, 2 H), 3.02 (ddd, 1 H), 2.57-2.43 (m, 1 H), 1.73 (sext, 2 H), 1.57 (sext, 2 H), 0.97 (t, 3 H), 0.84 (t, 3 H).

### Ausführungsbeispiele:

### Beispiel 1

### (3S*,3aR*,8bR*)-3a-(4-Bromphenyl)-6,8-diethoxy-8b-hydroxy-3-phenyl-2,3,3a,8b-tetrahydro-cyclopenta[b]benzofuran-1-oxim

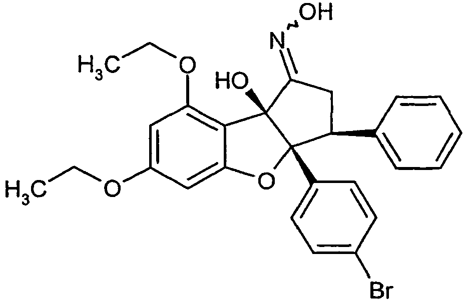

Die Titelverbindung wird in Analogie zur Synthese von Beispiel 14 ausgehend von Beispiel 7A hergestellt.
Ausbeute: 51 % d. Th.
LC-MS (Methode 4): Rₜ = 3.66 min.
MS (ESIneg): m/z = 522 (M-H)⁻
¹H-NMR (300 MHz, DMSO-d₆): δ = 11.0 (s, 1 H), 7.36-7.30 (m, 2 H), 7.14-7.05 (m, 3 H), 7.00-6.94 (m, 4 H), 6.35 (d, 1 H), 6.14 (d, 1 H), 5.25 (s, 1 H), 4.11-3.95 (m, 4 H), 3.52 (t, 1 H), 3.02-2.93 (m, 2 H).

### Beispiel 2

### (1R*,3S*,3aR*,8bS*)-3a-(3'-Aminobiphenyl-4-yl)-6,8-diethoxy-3-phenyl-2,3,3a,8b-tetrahydro-cyclopenta[b]benzofuran-1,8b-(1H)-diol

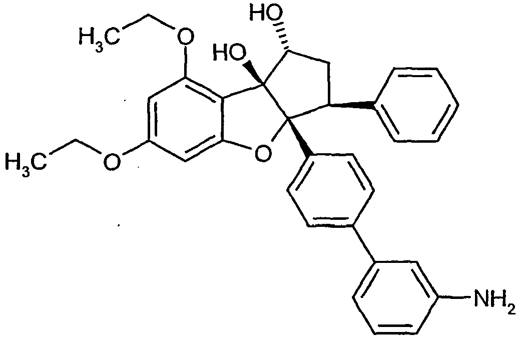

51.1 mg (0.10 mmol) (1R*,3S*,3aR*,8bS*)-3a-(4-Bromphenyl)-6,8-diethoxy-3-phenyl-2,3,3a,8b-tetrahydrocyclopenta[b]benzofuran-1,8b-(1*H*)-diol (Beispiel 8A), 15.5 mg (0.10 mmol) 3-Aminophenylboronsäure, 10.6 mg (0.10 mmol) Natriumcarbonat und 5.8 mg (0.005 mmol) Tetrakis-(triphenylphosphin)palladium(0) werden unter Argon in einem Gemisch aus 0.1 ml Wasser und 0.5 ml Dioxan über Nacht auf 80°C erhitzt. Anschließend wird mit DMSO verdünnt, filtriert und das Filtrat über präparative HPLC gereinigt. Man erhält 17.4 mg (25% d. Th.) des Produkts.
LC-MS (Methode 10): Rₜ = 2.07 min.
MS (ESIpos): m/z = 524 (M+H)⁺.

### Beispiel 3

### (1R*,3S*,3aR*,8bS*)-6,8-Diethoxy-3-phenyl-2,3,3a,8b-tetrahydro-3a-(4-thiophen-3ylphenyl)-cyclopenta[b]benzofuran-1,8b-(1H)-diol

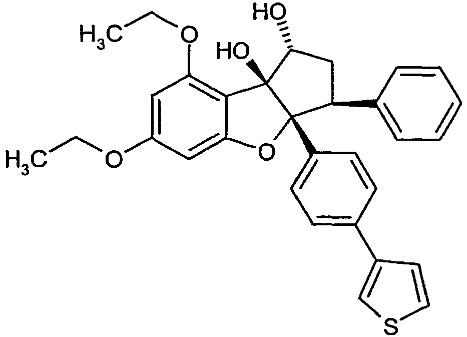

Die Titelverbindung wird in Analogie zur Synthese von Beispiel 2 hergestellt.
LC-MS (Methode 10): Rₜ = 2.41 min.
MS (ESIpos): m/z = 515 (M+H)⁺.

### Beispiel 4

### (1R*,3S*,3aR*,8bS*)-3a-(3'-Cyanobiphenyl-4-yl)-6,8-diethoxy-3-phenyl-2,3,3a,8b-tetrahydro-cyclopenta[b]benzofuran-1,8b-(1H)-diol

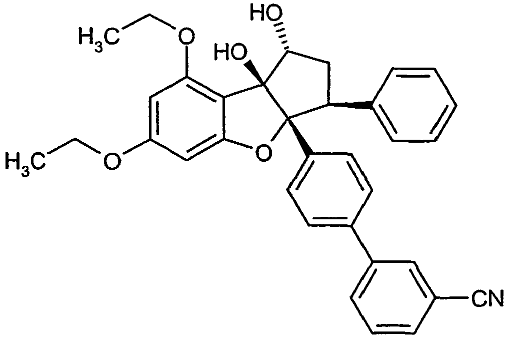

Die Titelverbindung wird in Analogie zur Synthese von Beispiel 2 hergestellt.
LC-MS (Methode 10): Rₜ = 2.38 min.
MS (ESIpos): m/z = 534 (M+H)⁺.

### Beispiel 5

### (1R*,3S*,3aR*,8bS*)-6,8-Diethoxy-3a-[4-(1H-indol-5-yl)-phenyl]-3-phenyl-2,3,3a,8b-tetrahydro-cyclopenta[b]benzofuran-1,8b-(1H)-diol

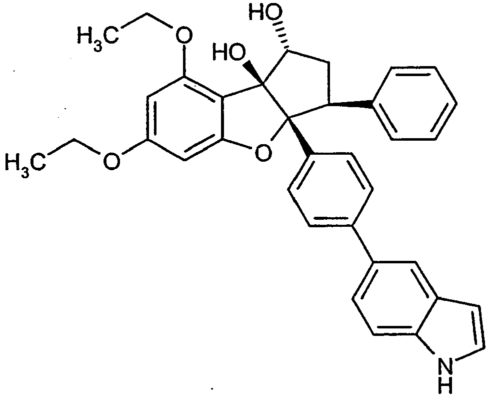

Die Titelverbindung wird in Analogie zur Synthese von Beispiel 2 hergestellt.
LC-MS (Methode 10): Rₜ = 2.31 min.
MS (ESIpos): m/z = 548 (M+H)⁺.

### Beispiel 6

### (1R*,3S*,3aR*,8bS*)-6,8-Diethoxy-3a-(3'-ethylsulfonyl-biphenyl-4-yl)-3-phenyl-2,3,3a,8b-tetrahydrocyclopenta[b]benzofuran-1,8b-(1H)-diol

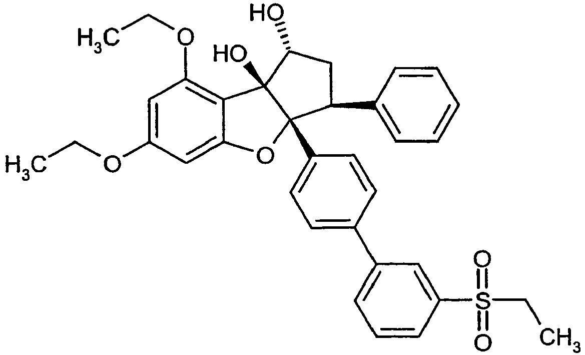

Die Titelverbindung wird in Analogie zur Synthese von Beispiel 2 hergestellt.
LC-MS (Methode 10): Rₜ = 2.26 min.
MS (ESIpos): m/z = 601 (M+H)⁺.

### Beispiel 7

### (1R*,3S*,3aR*,8bS*)-3a-(5'-Amino-2'-fluorbiphenyl-4-yl)-6,8-diethoxy-3-phenyl-2,3,3a, 8b-tetrahydrocyclopenta[b]benzofuran-1,8b-(1H)-diol

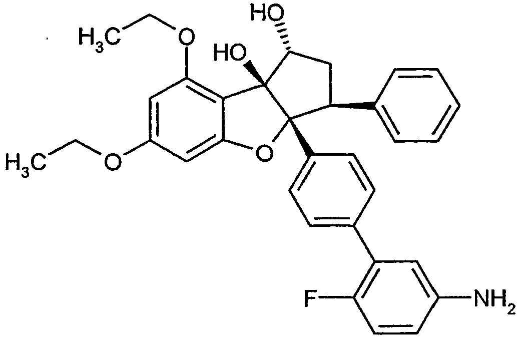

Die Titelverbindung wird in Analogie zur Synthese von Beispiel 2 hergestellt.
LC-MS (Methode 10): Rₜ = 2.05 min.
MS (ESIpos): m/z = 542 (M+H)⁺.

### Beispiel 8

### (1R*,3S*,3aR*,8bS*)-3a-(4-Chinoxalin-6-ylphenyl)-6,8-diethoxy-3-phenyl-2,3,3a,8b-tetrahydrocyclopenta[b]benzofuran-1,8b-(1H)-diol

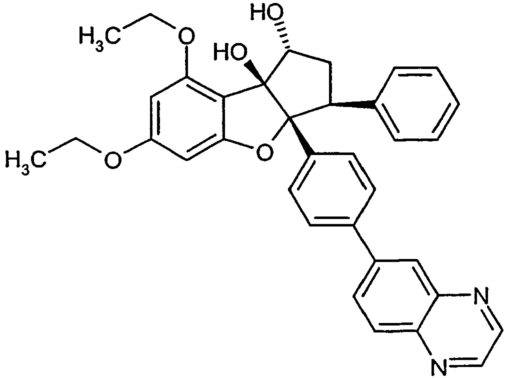

Die Titelverbindung wird in Analogie zur Synthese von Beispiel 2 hergestellt.
LC-MS (Methode 10): Rₜ = 2.29 min.
MS (ESIpos): m/z = 561 (M+H)⁺.

### Beispiel 9

### (1R*,3S*,3aR*,8bS*)-6,8-Diethoxy-3-phenyl-3a-(4-pyrrolidin-1-ylphenyl)-2,3,3a,8b-tetrahydrocyclopenta[b]benzofuran-1,8b-(1H)-diol

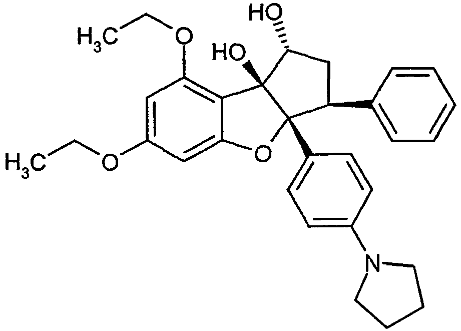

51.1 mg (0.10 mmol) (1R*,3S*,3aR*,8bS*)-3a-(4-Bromphenyl)-6,8-diethoxy-3-phenyl-2,3,3a,8b-tetrahydrocyclopenta[*b*]benzofuran-1,8b-(1*H*)-diol (Beispiel 8A), 4.7 mg (0.07 mmol) Pyrrolidin, 7.1 mg (0.07 mmol) Natrium-*tert*.-butylat, 1.2 mg (0.001 mmol) Tris(dibenzylidenaceton)-dipalladium(0) und 1.7 mg (0.003 mmol) rac-2,2'-Bis-(diphenylphosphino)-l,l'-binaphthyl werden unter Argon in 0.6 ml Toluol über Nacht auf 80°C erhitzt. Anschließend engt man ein, nimmt mit DMSO auf, filtriert und reinigt das Filtrat über präparative HPLC. Man erhält 14.5 mg (43% d. Th.) des Produkts.
LC-MS (Methode 10): Rₜ = 2.38 min.
MS (ESIpos): m/z = 502 (M+H)⁺.

### Beispiel 10

### (1R*,3S*,3aR*,8bS*)-3a-[4-(Benzyl-methyl-amino)-phenyl]-6,8-diethoxy-3-phenyl-2,3,3a,8b-tetrahydrocyclopenta[b]benzofuran-1,8b-(1H)-diol

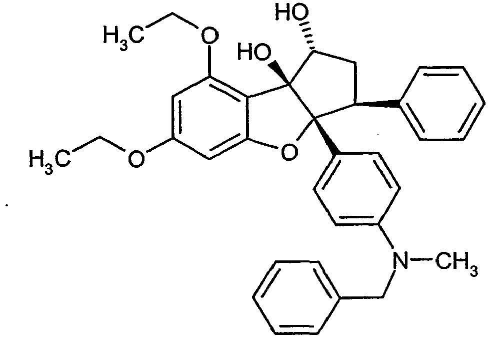

Die Titelverbindung wird in Analogie zur Synthese von Beispiel 9 hergestellt.
LC-MS (Methode 10): Rₜ = 2.44 min.
MS (ESIpos): m/z = 552 (M+H)⁺.

### Beispiel 11

### (1R*,3S*,3aR*,8bS*)-6,8-Diethoxy-3a-[4-(methyl-pyridin-4-ylmethyl-amino)-phenyl]-3-phenyl-2,3,3a,8b-tetrahydrocyclopenta[b]benzofuran-1,8b-(1H)-diol

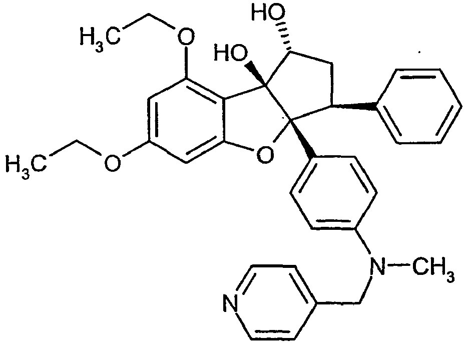

Die Titelverbindung wird in Analogie zur Synthese von Beispiel 9 hergestellt.
LC-MS (Methode 10): Rₜ = 1.73 min.
MS (ESIpos): m/z = 553 (M+H)⁺.

### Beispiel 12

### (2R*,3S*,3aR*,8bR*)-3a-(4-Bromphenyl)-6,8-diethoxy-2-dimethylcarbamid-8b-hydroxy-3-phenyl-2,3,3a,8b-tetrahydrocyclopenta[b]benzofuran-1-on

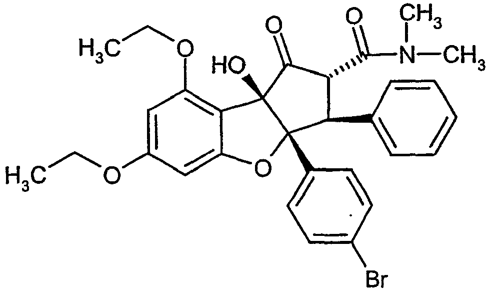

Die Titelverbindung wird in Analogie zur Synthese von Beispiel 16 ausgehend von Beispiel 9A hergestellt.
Ausbeute: 29% d. Th.
LC-MS (Methode 4): Rₜ = 3.53 min.
MS (ESIpos): m/z = 581 (M+H)⁺
¹H-NMR (200 MHz, DMSO-d₆): δ = 7.37-7.29 (m, 2 H), 7.15-6.90 (m, 7 H), 6.43 (d, 1H), 6.16 (d, 1H), 6.00 (s, 1 H), 4.64 (d, 1 H), 4.28 (d, 1 H), 4.15-3.91 (m, 4 H), 3.30 (s, 3 H), 2.77 (s, 3 H), 1.35 (t, 3 H), 1.22 (t, 3 H).

### Beispiel 13

### (1R*,2R*,3S*,3aR*,8bS*)-3a-(4-Bromphenyl)-6,8-diethoxy-2-dimethylcarbamid-3-phenyl-2,3,3a,8b-tetrahydrocyclopenta[b]benzofuran-1,8b-(1H)-diol

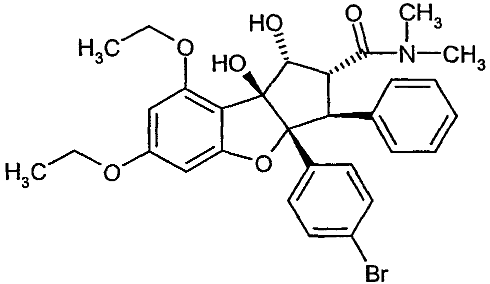

Die Titelverbindung wird in Analogie zur Synthese von Beispiel 17 ausgehend von Beispiel 12 hergestellt.
Ausbeute: 80% d. Th.
LC-MS (Methode 5): Rₜ = 3.61 min.
MS (ESIpos): m/z = 583 (M+H)⁺
¹H-NMR (200 MHz, DMSO-d₆): δ = 7.27-7.19 (m, 2 H), 7.12-6.95 (m, 5 H), 6.88-6.80 (m, 2 H), 6.29 (d, 1 H), 6.12 (d, 1 H), 5.20 (s, 1 H), 4.84-4.76 (m, 1 H), 4.60 (d, 1 H), 4.29 (d, 1 H), 4.13-3.97 (m, 5 H), 3.26 (s, 3 H), 2.75 (s, 3 H), 1.34 (t, 3 H), 1.31 (t, 3H).

### Beispiel 14

### (3S*,3aR*,8bS*)-3a-(4-Chlorphenyl)-6,8-diethoxy-8b-hydroxy-3-phenyl-2,3,3a,8b-tetrahydrocyclopenta[b]benzofuran-1-oxim

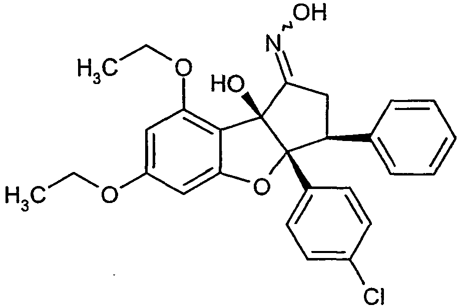

Zu 500 mg (1.08 mmol) in 20 ml Ethanol gelöstem (3S*,3aR*,8bR*)-3a-(4-Chlorphenyl)-6,8-diethoxy-8b-hydroxy-3-phenyl-2,3,3a,8b-tetrahydrocyclopenta[*b*]benzofuran-1-on (Beispiel 15A) werden bei Raumtemperatur zunächst 20 ml Pyridin und anschließend 90 mg (1.30 mmol) Hydroxylammoniumchlorid gegeben und die Lösung für einen Tag gerührt. Nach Abzug der flüchtigen Bestandteile am Rotationsverdampfer wird der Rückstand in Ethylacetat gelöst. Die organische Phase wird dann mit 1 N Salzsäure, gesättigter Natriumhydrogencarbonat-Lösung und gesättigter Natriumchlorid-Lösung gewaschen, über Magnesiumsulfat getrocknet, filtriert und eingeengt. Es resultieren 515 mg (100% d. Th.) des racemischen Produkts als farblose Kristalle.
LC-MS (Methode 9): Rₜ = 2.66 min.
MS (ESIneg): m/z = 478 (M-H)⁻
¹H-NMR (300 MHz, DMSO-d₆): δ = 11.01 (s, 1 H), 7.21-6.94 (m, 9 H), 6.35 (d, 1 H), 6.14 (d, 1 H), 5.25 (s, 1 H), 4.10-3.99 (m, 4 H), 3.55-3.49 (m, 1 H), 3.00-2.94 (m, 2 H), 1.36-1.30 (m, 6 H).

### Beispiel 15

### (1R*,3S*,3aR*,8bS*)-1-Amino-3a-(4-chlorphenyl)-6,8-diethoxy-8b-hydroxy-3 phenyl-2,3,3a,8b-tetrahydrocyclopenta[b]benzofuran

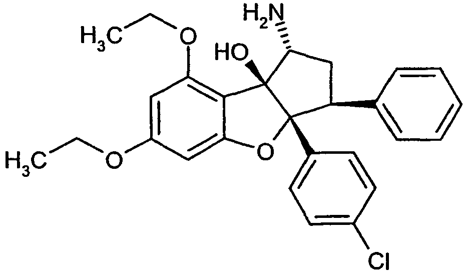

Bei 0°C wird zu einer Lösung von 3.22 ml (3.22 mmol) einer 1 N Lösung von Lithiumaluminiumhydrid in Diethylether in zusätzlichen 6 ml Diethylether portionsweise 115 mg (1.07 mmol) (3S*,3aR*,8bR*)-3a-(4-Chlorphenyl)-6,8-diethoxy-8b-hydroxy-3-phenyl-2,3,3a,8b-tetrahydrocyclopenta[b]benzofuran-1-oxim (Beispiel 14) unter Rühren zugegeben. Nachdem die Gasentwicklung beendet ist, wird für 30 Minuten auf Rückfluß erhitzt. Bei 0°C wird dann mit Ethylacetat verdünnt und 1 N Natronlauge zugetropft. Es wird fünf Minuten gerührt und anschließend die Phasen getrennt. Die wäßrige Phase wird zweimal mit Ethylacetat extrahiert. Die vereinigten organischen Phasen werden mit gesättigter Natriumchlorid-Lösung gewaschen, über Magnesiumsulfat getrocknet, filtriert und eingeengt. Der Rückstand kristallisiert aus einer Mischung aus Dichlormethan, Diethylether und Petrolether. Die erhaltenen Kristalle werden über eine Glasfritte abgesaugt und getrocknet. Es resultieren 155 mg (31% d. Th.) des racemischen Produkts als farblose Kristalle.
LC-MS (Methode 8): Rₜ = 2.62 min.
MS (ESIneg): m/z = 464 (M-H)⁻
¹H-NMR (400 MHz, DMSO-d₆): δ = 7.13-7.03 (m, 7 H), 6.91 (d, 2 H), 6.24 (d, 1 H), 6.11 (d, 1 H), 4.06-4.00 (m, 4 H), 3.75 (dd, 1 H), 3.34 (dd, 1 H), 2.41-2.35 (m, 1 H), 2.15-2.05 (m, 1 H), 1.35-1.31 (m, 1 H).

Die präparative Trennung des Racemats in die Enantiomeren wird über HPLC an chiraler Phase nach Methode 29A durchgeführt.

### Analytische Daten (Methode 29B):

Enantiomer A: Rₜ = 3.89 min., Enantiomer B: Rₜ = 6.09 min.

### Beispiel 16

### (2R*,3S*,3aR*,8bR*)-3a-(4-Chlorphenyl)-6,8-diethoxy-2-dimethylcarbamid-8-hydroxy-3-phenyl-2,3,3a,8b-tetrahydrocyclopenta[b]benzofuran-1-on

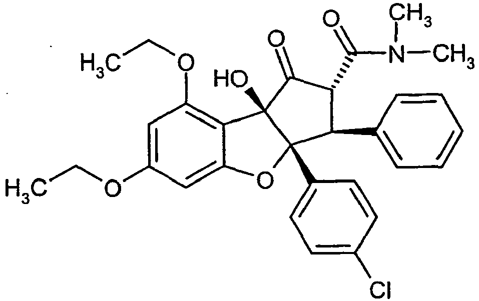

Zu einer Lösung von 550 mg (1.08 mmol) (2R*,3S*,3aR*,8bR*)-3a-(4-Chlorphenyl)-6,8-diethoxy-8-hydroxy-3-phenyl-2,3,3a,8b-tetrahydrocyclopenta[*b*]benzofuran-2-carbonsäure (Beispiel 17A) in THF werden bei 0°C 674 mg (1.30 mmol) Benzotriazol-1-yloxy-tripyrrolidinophosphonium-Hexafluorophosphat, 106 mg (1.30 mmol) Dimethylamin-Hydrochlorid und 0.47 ml (2.70 mmol) *N,N-*Diisopropylethylamin zugegeben und die Mischung dann für 4 h bei 0°C gerührt. Die Reaktionsmischung wird dann auf eine Mischung aus 50 ml gesättigter Ammoniumchlorid-Lösung, Eiswasser und Ethylacetat gegossen. Nach Abtrennen der organischen Phase wird die wäßrige Phase zweimal mit Ethylacetat extrahiert. Die vereinigten organischen Phasen werden mit Wasser, gesättigter Natriumhydrogencarbonat-Lösung und gesättigter Natriumchlorid-Lösung gewaschen, über Magnesiumsulfat getrocknet, filtriert und eingeengt. Der Rückstand wird über Kieselgel 60 (Laufmittel: Toluol / Ethylacetat 10:1, 6:1, 4:1) gereinigt. Es resultieren 159 mg (27% d. Th.) racemisches Produkt als farbloser Schaum.
LC-MS (Methode 2): Rₜ = 3.41 min.
MS (ESIpos): m/z = 536 (M+H)⁺
¹H-NMR (400 MHz, DMSO-d₆): δ = 7.25-6.94 (m, 9 H), 6.43 (d, 1 H), 6.16 (d, 1 H), 6.00 (s, 1 H), 4.63 (d, 1 H), 4.29 (d, 1 H), 4.13-3.94 (m, 4 H), 3.30 (s, 3 H), 2.77 (s, 3 H), 1.35 (t, 3 H), 1.22 (t, 3 H).

### Beispiel 17

### (1R*,2R*,3S*,3aR*,8bS*)-3a-(4-Chlorphenyl)-6,8-diethoxy-2-dimethylcarbamid-3-phenyl-2,3,3a,8b-tetrahydrocyclopenta[b]benzofuran-1,8b-(1H)-diol

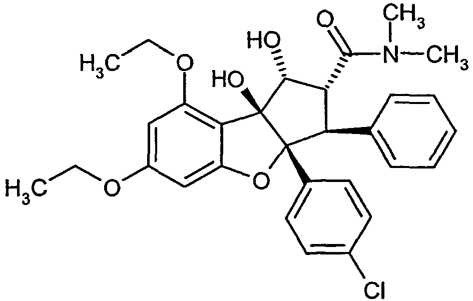

Eine Lösung von 1104 mg (4.20 mmol) Tetramethylammoniumtriacetoxyborhydrid in 1.5 ml Acetonitril und 1.5 ml Eisessig wird für 30 Minuten bei Raumtemperatur gerührt. 150 mg (0.28 mmol) (2R*,3S*,3aR*,8bR*)-3a-(4-Chlorphenyl)-6,8-diethoxy-2-dimethylcarbamid-8-hydroxy-3-phenyl-2,3,3a,8b-tetrahydrocyclopenta[*b*]benzofuran-1-on werden als Lösung in 13.5 ml Acetonitril zugegeben und für zwei Stunden bei Raumtemperatur gerührt. Bei 0°C wird gesättigte Natriumhydrogencarbonat-Lösung zugegeben und dreimal mit Ethylacetat extrahiert. Die vereinigten organischen Phasen werden über Magnesiumsulfat getrocknet, filtriert und eingeengt. Die aus Diethylether ausfallenden Kristalle werden abgesaugt, mit Diethylether/Petrolether 1:1 gewaschen und getrocknet. Es resultieren 94 mg (62% d. Th.) des Produkts als racemisches Gemisch.
LC-MS (Methode 8): Rₜ = 3.23 min.
MS (ESIpos): m/z = 538 (M+H)⁺
¹H-NMR (300 MHz, DMSO-d₆): δ = 7.15-6.94 (m, 7 H), 6.85-6.82 (m, 2 H), 6.29 (d, 1 H), 6.12 (d, 1 H), 5.16 (s, 1 H), 4.82-4.79 (m, 1 H), 4.56 (d, 1 H), 4.29 (d, 1 H), 4.08-3.99 (m, 4 H), 3.25 (s, 3 H), 2.76 (s, 1 H), 1.36-1.29 (m, 6 H).

Die präparative Trennung des Racemats in die Enantiomeren wird über HPLC an chiraler Phase nach Methode 26A durchgeführt.

### Analytische Daten (Methode 26B):

Enantiomer A: Rₜ = 3.96 min., Enantiomer B: Rₜ = 15.29 min.

### Beispiel 18

### (1R*,3S*,3aR*,8bS*)-6,8-Bis-(2-methoxyethoxy)-3a-(4-bromphenyl)-3-phenyl-2,3,3a,8b-tetrahydrocyclopenta[b]benzofuran-1,8b-(1H)-diol

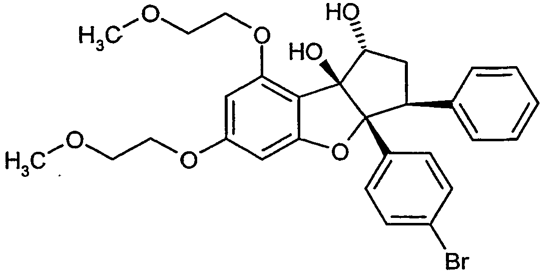

Die Titelverbindung wird in Analogie zur Synthese von Beispiel 6A ausgehend von Beispiel 22A hergestellt (anders als bei den anderen Reaktionen dieses Typs entsteht hier das trans-Diol).
Ausbeute: 72% d. Th.
LC-MS (Methode 2): Rₜ = 4.38 min.
MS (ESIpos): m/z = 572 (M+H)⁺
¹H-NMR (400 MHz, DMSO-d₆): δ = 7.27-7.22 (m, 2 H), 7.13-7.04 (m, 5 H), 7.00-6.95 (m, 2 H), 6.29 (d, 1 H), 6.18 (d, 1 H), 5.01 (s, 1 H), 4.56-4.51 (m, 1 H), 4.41 (d, 1 H), 4.22-4.09 (m, 4 H), 3.86 (dd, 1 H), 3.70-3.64 (m, 4 H), 3.34 (s, 6 H) 2.70 (ddd, 1 H), 1.96 (dd, 1 H).

### Beispiel 19

### (3S*,3aR*,8bR*)-6,8-Bis-(2-methoxyethoxy)-3a-(4-bromphenyl)-8b-hydroxy-3-phenyl-2,3,3a,8b-tetrahydrocyclopenta[b]benzofuran-1-on

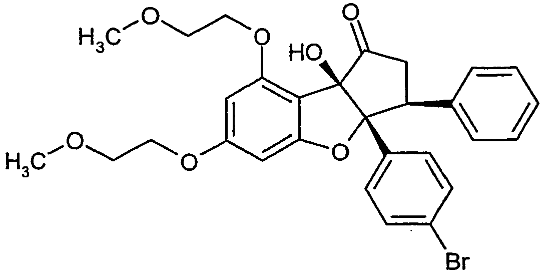

Die Titelverbindung wird in Analogie zur Synthese von Beispiel 7A ausgehend von Beispiel 18 hergestellt.
Ausbeute: 33% d. Th.
LC-MS (Methode 3): Rₜ = 4.30 min.
MS (ESIpos): m/z = 569 (M+H)⁺.

### Beispiel 20

### (1S*,3S*,3aR*,8bS*)-6,8-Bis-(2-dimethylaminoethoxy)-3a-(4-chlorphenyl)-3-phenyl-2,3,3a,8b-tetrahydrocyclopenta[b]benzofuran-1,8b-(1H)-diol

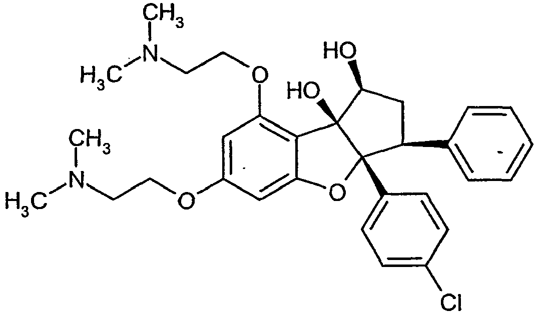

Die Titelverbindung wird in Analogie zur Synthese von Beispiel 32 ausgehend von Beispiel 28A hergestellt.
Ausbeute: 87% d. Th.
LC-MS (Methode 5): Rₜ = 2.12 min.
MS (ESIpos): m/z = 553 (M+H)⁺
¹H-NMR (300 MHz, DMSO-d₆): δ = 7.23-7.03 (m, 7 H), 6.93-6.88 (m, 2 H), 6.44 (d, 1 H), 6.30 (d, 1 H), 4.98 (s, 1 H), 4.67 (t, 1 H), 4.38-4.30 (m, 4 H), 3.48-3.23 (m, 5 H), 2.76 (s, 6 H), 2.70 (s, 6 H), 2.53-2.42 (m, 1 H), 2.21 (ddd, 1 H).

Die präparative Trennung des Racemats in die Enantiomeren wird über HPLC an chiraler Phase nach Methode 15A durchgeführt.

### Analytische Daten (Methode 15B):

Enantiomer A: Rₜ = 7.23 min., Enantiomer B: Rₜ = 9.41 min.

### Beispiel 21

### (1R*,3S*,3aR*,8bS*)-6,8-Bis-(2-dimethylaminoethoxy)-3a-(4-chlorphenyl)-3-phenyl-2,3,3a,8b-tetrahydrocydopenta[b]benzofuran-1,8b-(1H)-diol

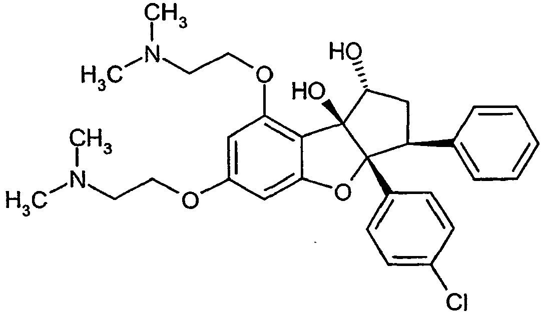

Die Titelverbindung wird in Analogie zur Synthese von Beispiel 32 ausgehend von Beispiel 30A hergestellt.
Ausbeute: 82% d. Th.
LC-MS (Methode 2): Rₜ = 2.03 min.
MS (ESIpos): m/z = 553 (M+H)⁺
¹H-NMR (200 MHz, DMSO-d₆): δ = 7.20-6.95 (m, 9 H), 6.27 (d, 1 H), 6.20 (d, 1 H), 5.16 (s, 1 H), 4.49 (d, 1 H), 4.26-4.11 (m, 1 H), 4.11-4.02 (m, 4 H), 3.84 (dd, 1 H), 2.86-2.55 (m, 5 H), 2.24 (s, 6 H), 2.19 (s, 6 H), 1.95 (dd, 1H).

Die präparative Trennung des Racemats in die Enantiomeren wird über HPLC an chiraler Phase nach Methode 15A durchgeführt.

### Analytische Daten (Methode 15B):

Enantiomer A: Rₜ = 6.78 min., Enantiomer B: Rₜ = 8.18 min.

### Beispiel 22

### (1S*,3S*,3aR*,8bS*)-6,8-Bis-(2-dimethylaminoethoxy)-3a-(4-bromphenyl)-3-phenyl-2,3,3a,8b-tetrahydrocyclopenta[b]benzofuran-1,8b-(1H)-diol

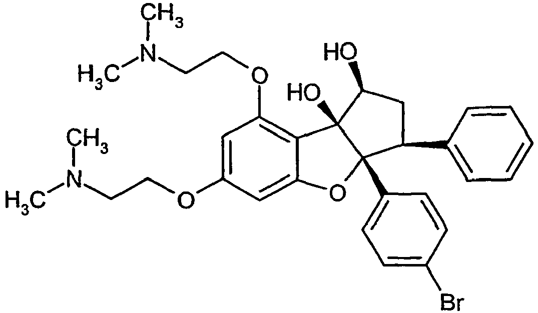

Die Titelverbindung wird in Analogie zur Synthese von Beispiel 32 ausgehend von Beispiel 35A hergestellt.
Ausbeute: 59% d. Th.
LC-MS (Methode 4): Rₜ = 2.19 min.
MS (ESIpos): m/z = 597 (M+H)⁺
¹H-NMR (400 MHz, DMSO-d₆): δ = 7.34-7.29 (m, 2 H), 7.18-7.12 (m, 2 H), 7.11-7.04 (m, 3 H), 6.94-6.88 (m, 2 H), 6.48 (d, 1 H), 6.32 (d, 1 H), 6.10 (s, 1 H), 5.01 (s, 1 H), 4.68 (t, 1 H), 4.43-4.37 (m, 4 H), 3.66-3.45 (m, 5 H), 2.87 (s, 6 H), 2.85 (s, 6 H), 2.55-2.43 (m, 1 H), 2.31-2.15 (m, 1 H).

### Beispiel 23

### (1S*,3S*,3aR*,8bS*)-3a-(4-Chlorphenyl)-6-ethoxy-3-phenyl-2,3,3a,8b-tetrahydrocyclopenta[b]-benzofuran-1,8b-(1H)-diol

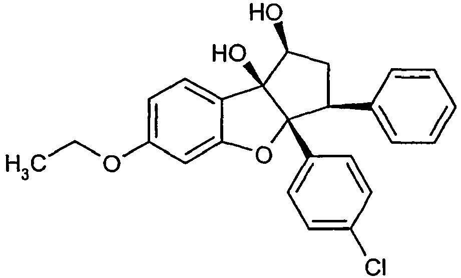

Die Titelverbindung wird in Analogie zur Synthese von Beispiel 6A ausgehend von Beispiel 39A hergestellt.
Ausbeute: 73% d. Th.
LC-MS (Methode 3): Rₜ = 3.57 min.
MS (ESIneg): m/z = 421 (M)⁻
¹H-NMR (200 MHz, DMSO-d₆): δ = 7.28-6.90 (m, 10 H), 6.66 (d, 1 H), 6.57 (dd, 1H), 5.84 (d, 1 H), 5.03 (s, 1 H), 4.49-4.39 (m, 1 H), 4.05 (q, 3 H), 3.31-3.25 (m, 1 H), 2.49-2.38 (m, 1 H), 2.24-2.15 (m, 1 H), 1.34 (t, 3 H).

Die präparative Trennung des Racemats in die Enantiomeren wird über HPLC an chiraler Phase nach Methode 30A durchgeführt.

### Analytische Daten (Methode 30B):

Enantiomer A: Rₜ = 3.90 min., Enantiomer B: Rₜ = 5.46 min.

### Beispiel 24

### (3S*,3aR*,8bR*)-3a-(4-Chlorphenyl)-6-ethoxy-8b-hydroxy-3-phenyl-2,3,3a,8b-tetrahydrocyclopenta[b]benzofuran-1-on

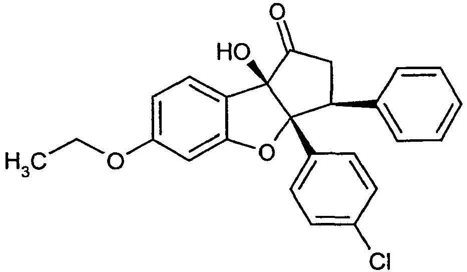

Die Titelverbindung wird in Analogie zur Synthese von Beispiel 7A ausgehend von Beispiel 23 hergestellt.
Ausbeute: 81 % d. Th.
LC-MS (Methode 9): Rₜ = 2.66 min.
MS (ESIpos): m/z = 403 [M+H-H₂O]⁺
¹H-NMR (200 MHz, DMSO-d₆): δ = 7.25-7.01 (m, 10 H), 6.82 (d, 1 H), 6.62 (dd, 1 H), 6.28 (s, 1 H), 4.08 (q, 2 H), 3.69-3.58 (m, 1 H), 3.42-3.22 (m, 1 H), 2.93-2.79 (m, 1 H), 1.35 (t, 3 H).

### Beispiel 25

### (3S*,3aR*,8bR*)-3a-(4-Chlorphenyl)-6-ethoxy-8b-hydroxy-3-phenyl-2,3,3a,8b-tetrahydrocyclopenta[b]benzofuran-1-oxim

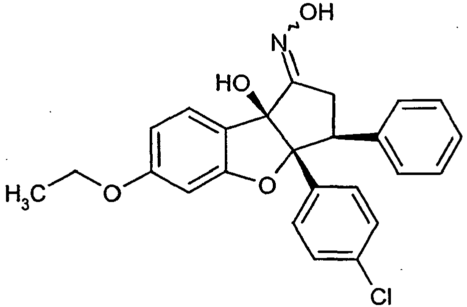

Die Titelverbindung wird in Analogie zur Synthese von Beispiel 14 ausgehend von Beispiel 24 hergestellt.
Ausbeute: 99% d. Th.
LC-MS (Methode 9): Rₜ = 2.57 min.
MS (ESIneg): m/z = 434 (M-H)⁻
¹H-NMR (300 MHz, DMSO-d₆): δ = 11.12 (s, 1 H), 7.32 (d, 1 H), 7.20 (d, 2 H), 7.11-7.09 (m, 5 H), 7.01-6.98 (m, 2 H), 6.73 (d, 1 H), 6.60 (dd, 1 H), 5.74 (s, 1 H), 4.07 (dq, 2 H), 3.40-3.38 (m, 1 H), 3.04-3.01 (m, 2 H), 1.34 (t, 3 H).

### Beispiel 26

### (1R*,3S*,3aR*,8bR*)-1-Amino-3a-(4-chlorphenyl)-6-ethoxy-8b-hydroxy-3-phenyl-2,3,3a,8b-tetrahydrocyclopenta[b]benzofuran

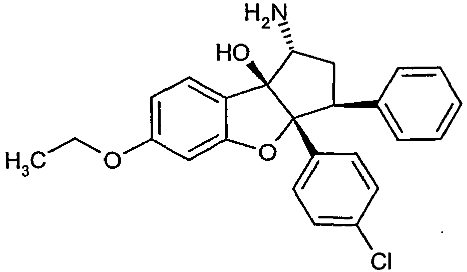

Die Titelverbindung wird in Analogie zur Synthese von Beispiel 15 ausgehend von Beispiel 25 hergestellt.
Ausbeute: 50% d. Th.
LC-MS (Methode 9): Rₜ =1.96 min.
MS (ESIpos): m/z = 422 (M-H)⁺
¹H-NMR (300 MHz, DMSO-d₆): δ = 7.19 (d, 1 H), 7.12-6.94 (m, 9 H), 6.62 (d, 1 H), 6.52 (dd, 1 H), 5.90 (br. s, 1 H), 4.04 (q, 2 H), 3.61 (dd, 1 H), 3.42 (dd, 1 H), 2.43-2.35 (m, 1 H), 2.19-2.07 (m, 1 H), 1.33 (t, 3 H).

Die präparative Trennung des Racemats in die Enantiomeren wird über HPLC an chiraler Phase nach Methode 27A durchgeführt.

### Analytische Daten (Methode 27B):

Enantiomer A: Rₜ = 4.22 min., Enantiomer B: Rₜ = 7.38 min.

### Beispiel 27

### (2R*,3S*,3aR*,8bR*)-3a-(4-Chlorphenyl)-6-ethoxy-2-dimethylcarbamid-8-hydroxy-3 phenyl-2,3,3a,8b-tetrahydrocyclopenta[b]benzofuran-1-on

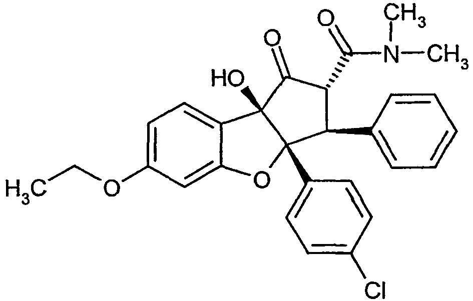

Die Titelverbindung wird in Analogie zur Synthese von Beispiel 16 ausgehend von Beispiel 40A hergestellt.
Ausbeute: 26% d. Th.
LC-MS (Methode 9): Rₜ = 2.49 min.
MS (ESIpos): m/z = 492 (M+H)⁺
¹H-NMR (400 MHz, DMSO-d₆): δ = 7.25-6.96 (m, 10 H), 6.82 (d, 1 H), 6.59 (dd, 1 H), 6.40 (s, 1 H), 4.77 (d, J = 13.41 Hz, 1 H), 4.19 (d, J = 13.41 Hz, 1 H), 4.14-4.05 (m, 2 H), 3.27 (s, 3 H), 2.77 (s, 3 H), 1.38-1.33 (m, 3 H).

### Beispiel 28

### (1R*,2R*,3S*,3aR*,8bS*)-3a-(4-Chlorphenyl)-6-ethoxy-2-dimethylcarbamid-3-phenyl-2,3,3a,8b-tetrahydrocyclopenta[b]benzofuran-1,8b-(1H)-diol

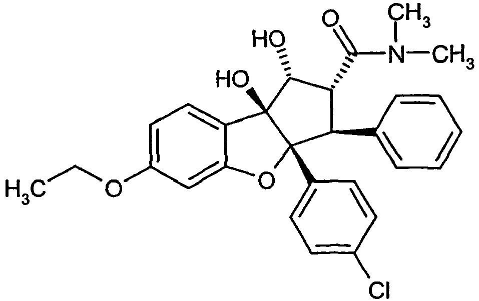

Die Titelverbindung wird in Analogie zur Synthese von Beispiel 17 ausgehend von Beispiel 27 hergestellt.
Ausbeute: 61 % d. Th.
LC-MS (Methode 9): Rₜ = 2.31 min.
MS (ESIpos): m/z = 494 (M+H)⁺
¹H-NMR (200 MHz, DMSO-d₆): δ = 7.34 (d, 1 H), 7.22-6.80 (m, 9 H), 6.64 (d, 1 H), 6.50 (dd, 1 H), 5.48 (d, 1 H), 5.29 (s, 1 H), 4.91-4.84 (m, 1 H), 4.24-3.95 (m, 4 H), 3.20 (s, 3 H), 2.72 (s, 3 H), 1.34 (t, 3H).

Die präparative Trennung des Racemats in die Enantiomeren wird über HPLC an chiraler Phase nach Methode 28A durchgeführt.

### Analytische Daten (Methode 28B):

Enantiomer A: Rₜ = 4.48 min., Enantiomer B: Rₜ = 10.97 min.

### Beispiel 29

### (1S*,3S*,3aR*,8bS*)-3a-(4-Bromphenyl)-6-ethoxy-3-phenyl-2,3,3a,8b-tetrahydrocyclopenta[b]-benzofuran-1,8b-(1H)-diol

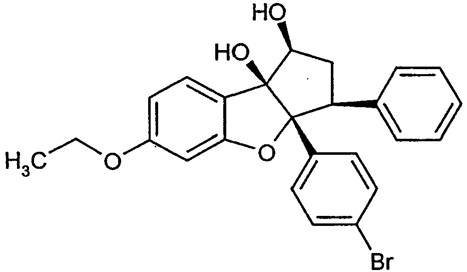

Die Titelverbindung wird in Analogie zur Synthese der Beispiele 2A - 6A ausgehend von 3-Ethoxyphenol hergestellt.
LC-MS (Methode 12): Rₜ = 4.39 min.
MS (ESIpos): m/z = 489 (M+Na)⁺
¹H-NMR (200 MHz, DMSO-d₆): δ = 7.32-7.23 (m, 3 H), 7.16-7.06 (m, 5 H), 6.95-6.91 (m, 2 H), 6.66-6.54 (m, 2 H), 5.83 (d, 1 H), 5.03 (s, 1 H), 4.49-4.39 (m, 1 H), 4.05 (q, 2 H), 3.36-3.26 (m, 1 H), 2.51-2.09 (m, 2 H), 1.34 (t, 3 H).

### Beispiel 30

### (3S*,3aR*,8bR*)-3a-(4-Bromphenyl)-6-ethoxy-8b-hydroxy-3-phenyl-2,3,3a,8b-tetrahydrocyclopenta[b]benzofuran-1-on

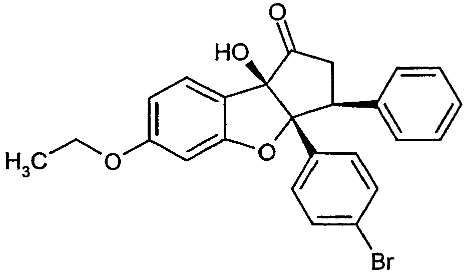

Die Titelverbindung wird in Analogie zur Synthese von Beispiel 7A ausgehend von Beispiel 29 hergestellt.
Ausbeute: 85% d. Th.
LC-MS (Methode 11): Rₜ = 4.5 min.
MS (ESIneg): m/z = 463 (M)⁻
¹H-NMR (300 MHz, DMSO-d₆): δ = 7.33-7.02 (m, 10 H), 6.81 (d, 1 H), 6.61 (dd, 1 H), 6.23 (s, 1 H), 4.12-4.05 (m, 2 H), 3.68-3.61 (m, 1 H), 3.40-3.23 (m, 1 H), 2.91-2.82 (m, 1 H), 1.35 (t, 3 H).

### Beispiel 31

### (1R*,3S*,3aR*,8bS*)-3a-(4-Bromphenyl)-6-ethoxy-3-phenyl-2,3,3a,8b-tetrahydrocyclopenta[b]-benzofuran-1,8b-(1H)-diol

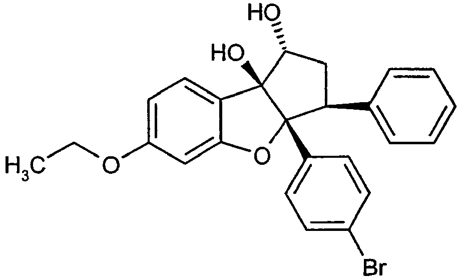

Die Titelverbindung wird in Analogie zur Synthese von Beispiel 8A ausgehend von Beispiel 30 hergestellt.
Ausbeute: 79% d. Th.
LC-MS (Methode 11): Rₜ = 4.2 min.
MS (ESIneg): m/z = 465 (M-H)⁻
¹H-NMR (200 MHz, DMSO-d₆): δ = 7.32-7.23 (m, 3 H), 7.11-6.94 (m, 7 H), 6.60 (d, 1 H), 6.50 (dd, 1 H), 5.22 (s, 1 H), 5.14 (d, 1 H), 4.56 (br. s, 1 H), 4.09-3.98 (m, 2 H), 3.89-3.79 (m, 1 H), 2.74-2.57 (m, 1 H), 1.96-1.84 (m, 1 H), 1.34 (t, 3 H).

### Beispiel 32

### (1S*,3S*,3aR*,8bS*)-3a-(4-Chlorphenyl)-6-(2-dimethylaminoethoxy)-3-phenyl-2,3,3a,8b-tetra-hydrocyclopenta[b]benzofuran-1,8b-(1H)-diol

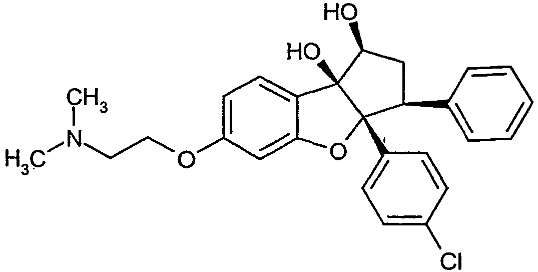

100 mg (0.22 mmol) (1S*,3S*,3aR*,8bS*)-6-(2-Chlorethoxy)-3a-(4-chlorphenyl)-3-phenyl-2,3,3a,8b-tetrahydrocylopenta[*b*]benzofuran-1,8b-(1*H*)-diol (Beispiel 48A) werden unter Argon in einer verschlossenen Apparatur in 10 ml 33%-iger ethanolischer Dimethylamin-Lösung auf 70°C über Nacht erhitzt. Nach dem Abkühlen engt man ein. Nach Säulenchromatographie an Kieselgel 60 (Laufmittel: Dichlormethan / Methanol / Triethylamin 95:5:1) erhält man 89 mg (87% d. Th.) des Produkts als racemisches Gemisch.
LC-MS (Methode 1): Rₜ = 2.90 min.
MS (ESIpos): m/z = 466 (M+H)⁺
¹H-NMR (400 MHz, DMSO-d₆): δ = 7.30-7.02 (m, 8 H), 6.96-6.88 (m, 2 H), 6.69 (d, 1 H), 6.59 (dd, 1 H), 5.85 (d, 1 H), 5.04 (s, 1 H), 4.51-4.38 (m, 1 H), 4.14-4.04 (m, 2 H), 3.38-3.23 (m, 1 H), 2.73-2.64 (m, 2 H), 2.50-2.37 (m, 1 H), 2.26 (s, 6 H), 2.24-2.10 (m, 1 H).

Die präparative Trennung des Racemats in die Enantiomeren wird über HPLC an chiraler Phase nach Methode 19A durchgeführt.

### Analytische Daten (Methode 19B):

Enantiomer A: Rₜ = 10.52 min., Enantiomer B: Rₜ = 12.53 min.

### Beispiel 33

### (1S*,3S*,3aR*,8bS*)-3a-(4-Chlorphenyl)-6-(2-pyrrolidin-1-ylethoxy)-3-phenyl-2,3,3a,8b-tetrahydrocyclopenta[b]benzofuran-1,8b-(1H)-diol

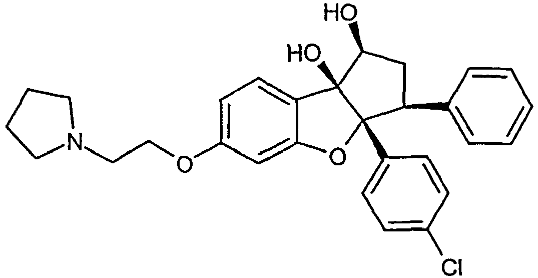

Die Titelverbindung wird in Analogie zur Synthese von Beispiel 32 mit Pyrrolidin als Amin-Komponente hergestellt.
Ausbeute: 99% d. Th.
LC-MS (Methode 2): Rₜ = 2.29 min.
MS (ESIpos): m/z = 492 (M+H)⁺
¹H-NMR (200 MHz, DMSO-d₆): δ = 7.30-7.02 (m, 8 H), 6.96-6.88 (m, 2 H), 6.68 (d, 1 H), 6.59 (dd, 1 H), 5.85 (d, 1 H), 5.04 (s, 1 H), 4.51-4.38 (m, 1 H), 4.09 (t, 2 H), 3.37-3.24 (m, 1 H), 2.79 (t, 2 H), 2.57-2.37 (m, 5 H), 2.20 (ddd, 1H), 1.73-1.65 (m, 4 H).

Die präparative Trennung des Racemats in die Enantiomeren wird über HPLC an chiraler Phase nach Methode 18A durchgeführt.

### Analytische Daten (Methode 18B):

Enantiomer A: Rₜ = 6.53 min., Enantiomer B: Rₜ = 8.48 min.

### Beispiel 34

### (1S*,3S*,3aR*,8bS*)-3a-(4-Chlorphenyl)-6-(2-methylaminoethoxy)-3-phenyl-2,3,3a,8b-tetra-hydrocyclopenta[b]benzofuran-1,8b-(1H)-diol

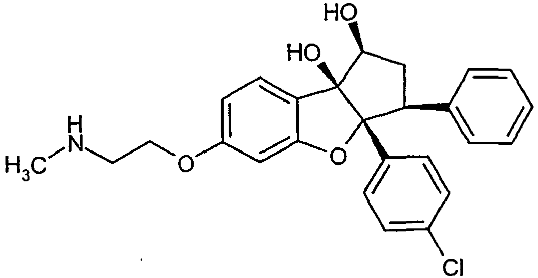

Die Titelverbindung wird in Analogie zur Synthese von Beispiel 32 mit Methylamin als Amin-Komponente hergestellt.
Ausbeute: 95% d. Th.
LC-MS (Methode 2): Rₜ = 2.19 min.
MS (ESIpos): m/z = 452 (M+H)⁺
¹H-NMR (300 MHz, DMSO-d₆): δ = 7.79 (s, 1 H), 7.31 (d, 1 H), 7.24-7.13 (m, 4 H), 7.12-7.01 (m, 3 H), 6.95-6.90 (m, 2 H), 6.73 (d, 1 H), 6.64 (dd, 1 H), 5.89 (d, 1 H), 5.03 (s, 1 H), 4.49-4.41 (m, 1 H), 4.22 (t, 2 H), 3.36-3.18 (m, 3 H), 2.57 (s, 3 H), 2.52-2.40 (m, 1 H), 2.22 (ddd, 1 H).

Die präparative Trennung des Racemats in die Enantiomeren wird über HPLC an chiraler Phase nach Methode 16A durchgeführt.

### Analytische Daten (Methode 16B):

Enantiomer A: Rₜ = 9.31 min., Enantiomer B: Rₜ = 13.90 min.

### Beispiel 35

### (1S*,3S*,3aR*,8bS*)-3a-(4-Chlorphenyl)-6-(2-diethylaminoethoxy)-3-phenyl-2,3,3a,8b-tetrahydrocyclopenta[b]benzofuran-1,8b-(1H)-diol

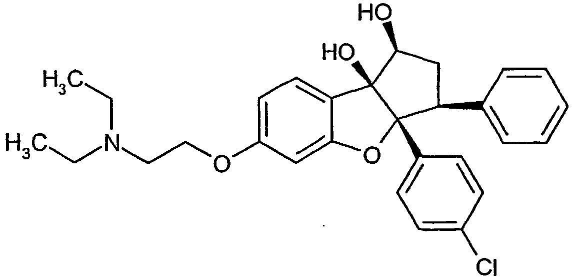

Die Titelverbindung wird in Analogie zur Synthese von Beispiel 32 mit Diethylamin als Amin-Komponente hergestellt.
Ausbeute: 56% d. Th.
LC-MS (Methode 4): Rₜ = 2.34 min.
MS (ESIpos): m/z = 494 (M+H)⁺
¹H-NMR (400 MHz, DMSO-d₆): δ = 7.26 (d, 1 H), 7.23-7.14 (m, 4 H), 7.11-7.01 (m, 3 H), 6.94-6.90 (m, 2 H), 6.67 (d, 1 H), 6.58 (dd, 1 H), 5.85 (d, 1 H), 5.02 (s, 1 H), 4.48-4.41 (m, 1 H), 4.06 (t, 2 H), 3.37-3.26 (m, 1 H), 2.86-2.73 (m, 2 H), 2.63-2.54 (m, 4 H), 2.49-2.40 (m, 1 H), 2.20 (ddd, 1 H), 1.00 (t, 6 H).

### Beispiel 36

### (1S*,3S*,3aR*,8bS*)-3a-(4-Chlorphenyl)-6-(2-ethylaminoethoxy)-3-phenyl-2,3,3a,8b-tetrahydrocyclopenta[b]benzofuran-1,8b-(1H)-diol

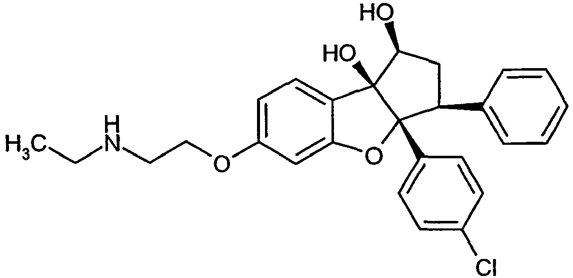

Die Titelverbindung wird in Analogie zur Synthese von Beispiel 32 mit Ethylamin als Amin-Komponente hergestellt.
Ausbeute: 31% d. Th.
LC-MS (Methode 13): Rₜ = 1.97 min.
MS (ESIpos): m/z = 466 (M+H)⁺
¹H-NMR (300 MHz, DMSO-d₆): δ = 7.27 (d, 1 H), 7.24-7.13 (m, 4 H), 7.12-7.03 (m, 3 H), 6.95-6.90 (m, 2 H), 6.69 (d, 1 H), 6.61 (dd, 1 H), 5.85 (s, 1 H), 5.02 (s, 1 H), 4.45 (t, 1 H), 4.10 (t, 2 H), 3.31 (dd, 1 H), 3.00 (t, 2 H), 2.72 (q, 2 H), 2.48-2.40 (m, 1 H), 2.28-2.14 (m, 1 H), 1.08 (t, 3 H).

### Beispiel 37

### (1S*,3S*,3aR*,8bS*)-3a-(4-Chlorphenyl)-6-(2-isopropylaminoethoxy)-3-phenyl-2,3,3a,8b-tetrahydrocyclopenta[b]benzofuran-1,8b-(1H)-diol

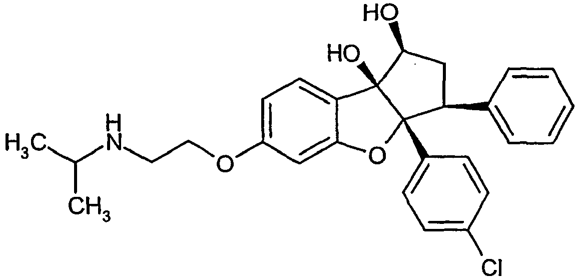

Die Titelverbindung wird in Analogie zur Synthese von Beispiel 32 mit Isopropylamin als Amin-Komponente hergestellt.
Ausbeute: 26% d. Th.
LC-MS (Methode 13): Rₜ = 1.97 min.
MS (ESIpos): m/z = 480 (M+H)⁺
¹H-NMR (300 MHz, DMSO-d₆): δ = 7.28 (d, 1 H), 7.24-7.13 (m, 4 H), 7.12-7.03 (m, 3 H), 6.95-6.90 (m, 2 H), 6.69 (d, 1 H), 6.61 (dd, 1 H), 5.85 (s, 1 H), 5.02 (s, 1 H), 4.45 (t, 1 H), 4.10 (t, 2 H), 3.36-3.27 (m, 1 H), 3.00 (t, 2 H), 2.95 (sept, 1 H), 2.48-2.40 (m, 1 H), 2.31-2.14 (m, 1 H), 1.08 (d, 6 H).

### Beispiel 38

### (1S*,3S*,3aR*,8bS*)-6-(2-Azetidin-1-ylethoxy)-3a-(4-chlorphenyl)-3-phenyl-2,3,3a,8b-tetrahydrocyclopenta[b]benzofuran-1,8b-(1H)-diol

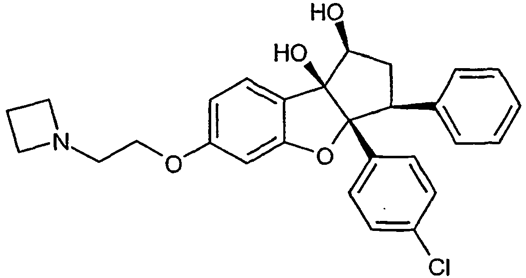

Die Titelverbindung wird in Analogie zur Synthese von Beispiel 32 mit Azetidin als Amin-Komponente hergestellt.
Ausbeute: 58% d. Th.
LC-MS (Methode 13): Rₜ = 1.96 min.
MS (ESIpos): m/z = 478 (M+H)⁺
¹H-NMR (300 MHz, DMSO-d₆): δ = 7.25 (d, 1 H), 7.23-7.13 (m, 4 H), 7.12-7.02 (m, 3 H), 6.95-6.90 (m, 2 H), 6.64 (d, 1 H), 6.56 (dd, 1 H), 5.82 (s, 1 H), 5.00 (s, 1 H), 4.44 (t, 1 H), 3.95 (t, 2 H), 3.36-3.27 (m, 1 H), 3.23 (t, 4 H), 2.74 (t, 2 H), 2.48-2.39 (m, 1 H), 2.28-2.14 (m, 1 H), 1.99 (quint, 2 H).

### Beispiel 39

### (1S*,3S*,3aR*,8bS*)-3a-(4-Chlorphenyl)-6-(2-morpholin-4-ylethoxy)-3-phenyl-2,3,3a,8b-tetrahydrocyclopenta[b]benzofuran-1,8b-(1H)-diol

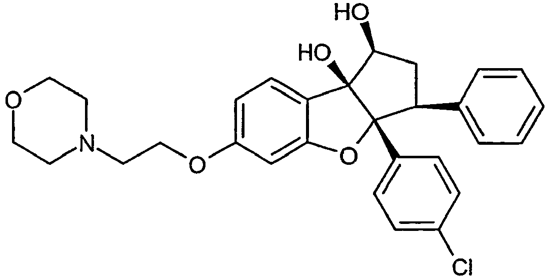

Die Titelverbindung wird in Analogie zur Synthese von Beispiel 32 mit Morpholin als Amin-Komponente hergestellt.
Ausbeute: 82% d. Th.
LC-MS (Methode 9): Rₜ = 1.76 min.
MS (ESIpos): m/z = 508 (M+H)⁺
¹H-NMR (200 MHz, DMSO-d₆): δ = 7.29-7.02 (m, 8 H), 6.96-6.87 (m, 2 H), 6.69 (d, 1 H), 6.59 (dd, 1 H), 5.85 (d, 1 H), 5.04 (s, 1 H), 4.50-4.38 (m, 1 H), 4.12 (t, 2 H), 3.63-3.54 (m, 4 H), 3.44-2.98 (m, 5 H), 2.70 (t, 2 H), 2.48-2.36 (m, 1 H), 2.32-2.07 (m, 1 H).

### Beispiel 40

### (1S*,3S*,3aR*,8bS*)-3a-(4-Chlorphenyl)-6-(2-cyclopropylaminoethoxy)-3-phenyl-2,3,3a,8b-tetrahydrocyclopenta[b]benzofuran-1,8b-(1H)-diol-Hydroformiat

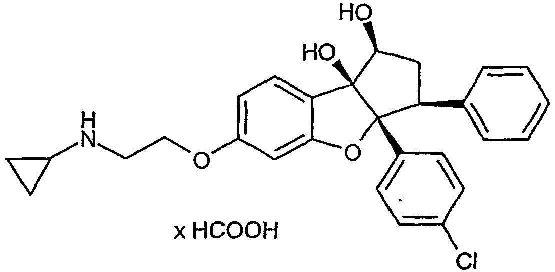

Die Titelverbindung wird in Analogie zur Synthese von Beispiel 32 mit Cyclopropylamin als Amin-Komponente hergestellt. Die Aufreinigung des Rohprodukts erfolgt mittels präparativer HPLC (RP18-Säule, Laufmittel: Acetonitril/Wasser-Gradient 5:95 → 95:5 mit 0.1% Ameisensäure).
Ausbeute: 48% d. Th.
LC-MS (Methode 13): Rₜ = 1.94 min.
MS (ESIpos): m/z = 478 (M+H)⁺
¹H-NMR (200 MHz, DMSO-d₆): δ = 8.17 (s, 1 H), 7.30-7.02 (m, 8 H), 6.96-6.88 (m, 2 H), 6.68 (d, 1 H), 6.59 (dd, 1 H), 5.76 (s, 1 H), 5.04 (s, 1 H), 4.50-4.38 (m, 1 H), 4.05 (t, 2 H), 3.48-3.25 (m, 1 H), 2.95 (t, 2 H), 2.47-2.35 (m, 1 H), 2.28-2.08 (m, 2 H), 0.45-0.32 (m, 2 H), 0.30-0.20 (m, 2 H).

### Beispiel 41

### (1S*,3S*,3aR*,8bS*)-3a-(4-Chlorphenyl)-6-[2-(2-dimethylamino-ethylamino)-ethoxy]-3-phenyl-2,3,3a,8b-tetrahydrocyclopenta[b]benzofuran-1,8b-(1H)-diol-Dihydroformiat

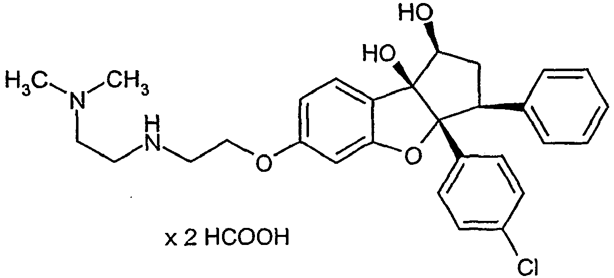

Die Titelverbindung wird in Analogie zur Synthese von Beispiel 32 mit 2-Dimethylaminoethylamin als Amin-Komponente hergestellt. Die Aufreinigung des Rohprodukts erfolgt mittels präparativer HPLC (RP18-Säule, Laufinittel: Acetonitril/ Wasser-Gradient 5:95 → 95:5 mit 0.1% Ameisensäure).
Ausbeute: 85% d. Th.
LC-MS (Methode 6): Rₜ = 1.37 min.
MS (ESIpos): m/z = 509 (M+H)⁺
¹H-NMR (300 MHz, DMSO-d₆): δ = 8.22 (s, 2 H), 7.30-7.02 (m, 8 H), 6.95-6.90 (m, 2 H), 6.70 (d, 1 H), 6.60 (dd, 1 H), 4.45 (t, 1 H), 4.12 (t, 2 H), 3.31 (dd, 1 H), 3.02 (t, 2 H), 2.81 (t, 2 H), 2.47-2.36 (m, 1 H), 2.25 (s, 6 H), 2.24-2.14 (m, 3 H).

### Beispiel 42

### (1S*,3S*,3aR*,8bS*)-6-(2-Aminoethoxy)-3a-(4-chlorphenyl)-3-phenyl-2,3,3a,8b-tetrahydrocylopenta[b]benzofuran-1,8b-(1H)-diol

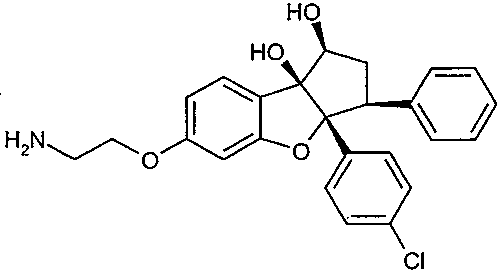

210 mg (0.45 mmol) (1S*,3S*,3aR*,8bS*)-6-(2-Azidoethoxy)-3a-(4-chlorphenyl)-3-phenyl-2,3,3a,8b-tetrahydrocyclopenta[b]benzofuran-1,8b-(1*H*)-diol (Beispiel 49A) werden in 34 ml Ethanol gelöst. Nach Zusatz von 56 mg 10%-igem Palladium auf Aktivkohle rührt man für 15 Minuten bei RT und Normaldruck unter einer Wasserstoff-Atmosphäre. Man filtriert, engt ein und filtriert den Rückstand über Kieselgel 60. Man wäscht mit Toluol und eluiert mit einem 1:1-Gemisch aus Dichlormethan / Ethanol. Nach dem Einengen erhält man 179 mg (90% d. Th.) des Produkts, das nach Methode 23A feingereinigt wird.
LC-MS (Methode 9): Rₜ = 1.74 min.
MS (ESIpos): m/z = 438 (M+H)⁺
¹H-NMR (200 MHz, DMSO-d₆): δ = 7.33-6.84 (m, 10 H), 6.70 (d, 1 H), 6.61 (dd, 1 H), 5.89 (s, 1 H), 5.76 (s, 2 H), 5.05 (s, 1 H), 4.52-4.38 (m, 1 H), 4.02 (t, 2 H), 3.39-3.20 (m, 1 H), 2.98 (t, 2 H), 2.50-2.36 (m, 1 H), 2.34-2.10 (m, 1 H).
HPLC (Methode 23B): Rₜ = 8.76 min.

Die präparative Trennung des Racemats in die Enantiomeren wird über HPLC an chiraler Phase nach Methode 24A durchgeführt (Enantiomer A: Rₜ = 11.0 min., Enantiomer B: Rₜ = 23.6 min.).

### Beispiel 43

### (1R*,3S*,3aR*,8bS*)-3a-(4-Chlorphenyl)-6-(2-dimethylaminoethoxy)-3-phenyl-2,3,3a,8b-tetrahydrocyclopenta[b]benzofuran-1,8b-(1H)-diol

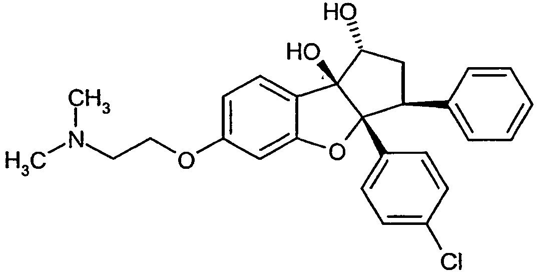

Die Titelverbindung wird in Analogie zur Synthese von Beispiel 32 ausgehend von Beispiel 51A hergestellt.
Ausbeute: 94% d. Th.
LC-MS (Methode 5): Rₜ = 2.41 min.
MS (ESIpos): m/z = 466 (M+H)⁺
¹H-NMR (200 MHz, DMSO-d₆): δ = 7.32 (d, 1 H), 7.19-7.02 (m, 7 H), 6.99-6.91 (m, 2 H), 6.60 (d, 1 H), 6.54 (dd, 1 H), 5.26 (s, 1 H), 5.18 (d, 1 H), 4.62-4.52 (m, 1 H), 4.16 (t, 2 H), 3.83 (dd, 1 H), 3.02-2.88 (m, 2 H), 2.75-2.57 (m, 1 H), 2.54 (s, 3 H), 2.46 (s, 3 H), 1.98-1.82 (m, 1 H).

### Beispiel 44

### (1R*,2R*,3S*,3aR*,8bS*)-3a-(4-Chlorphenyl)-6-(2-dimethylaminoethoxy)-2-dimethylcarbamid-3-phenyl-2,3,3a,8b-tetrahydrocyclopenta[b]benzofuran-1,8b-(1H)-diol

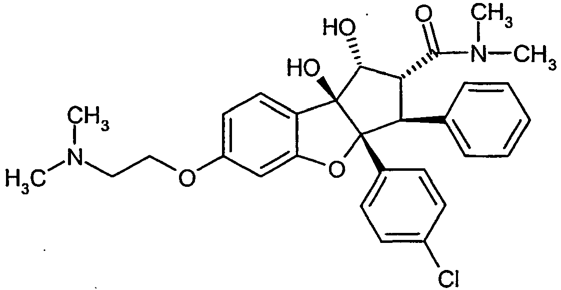

Die Titelverbindung wird in Analogie zur Synthese von Beispiel 32 ausgehend von Beispiel 54A hergestellt.
Ausbeute: 64% d. Th.
LC-MS (Methode 2): Rₜ = 2.10 min.
MS (ESIpos): m/z = 537 (M+H)⁺
¹H-NMR (200 MHz, DMSO-d₆): δ = 7.39 (d, 1 H), 7.25-6.98 (m, 7 H), 6.86-6.78 (m, 2 H), 6.75 (d, 1 H), 6.57 (dd, 1 H), 5.54 (d, 1 H), 5.33 (s, 1 H), 4.96-4.84 (m, 1 H), 4.34-4.12 (m, 3 H), 4.10-3.94 (m, 1 H), 3.48-3.28 (m, 2 H), 3.20 (s, 3 H), 2.72 (s, 3 H), 2.74-2.62 (m, 6 H).

### Beispiel 45

### (1R*,3S*,3aR*,8bS*)-3a-(4-Chlorphenyl)-8b-hydroxy-3-phenyl-6-(2-pyrrolidin-1-yl-ethoxy)-2,3,3 a,8b-tetrahydrocyclopenta[b]benzofuran-1-amin-Dihydroformiat

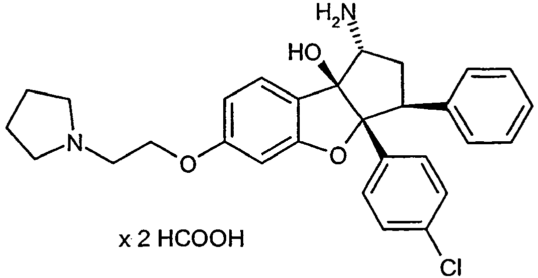

Die Titelverbindung wird in Analogie zur Synthese von Beispiel 32 ausgehend von Beispiel 56A hergestellt. Die Aufreinigung des Rohprodukts erfolgt mittels präparativer HPLC (RP18-Säule, Laufmittel: Acetonitril/Wasser-Gradient 5:95 → 95:5 mit 0.1% Ameisensäure).
Ausbeute: 67% d. Th.
LC-MS (Methode 6): Rₜ = 1.20 min.
MS (ESIpos): m/z = 491 (M+H)⁺
¹H-NMR (300 MHz, DMSO-d₆): δ = 8.21 (s, 2 H), 7.36 (d, 1 H), 7.21-7.04 (m, 7 H), 6.99-6.94 (m, 2 H), 6.67 (d, 1 H), 6.58 (dd, 1 H), 4.10 (t, 2 H), 3.80-3.70 (m, 1 H), 3.50 (dd, 1 H), 2.82 (t, 2 H), 2.59-2.53 (m, 4 H), 2.48-2.37 (m, 1 H), 2.35-2.18 (m, 1 H), 1.73-1.67 (m, 4 H).

### Beispiel 46

### (1R*,3S*,3aR*,8bS*)-3a-(4-Chlorphenyl)-6-(2-dimethylaminoethoxy)-8b-hydroxy-3-phenyl-2,3,3a,8b-tetrahydrocyclopenta[b]benzofuran-1-amin-Dihydroformiat

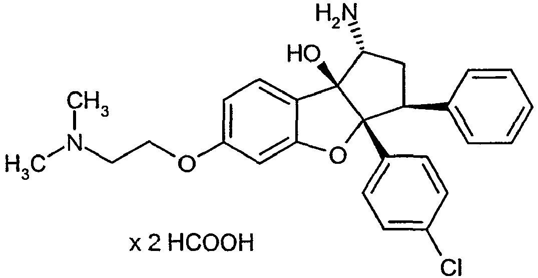

Die Titelverbindung wird in Analogie zur Synthese von Beispiel 32 ausgehend von Beispiel 56A hergestellt. Die Aufreinigung des Rohprodukts erfolgt mittels präparativer HPLC (RP18-Säule, Laufmittel: Acetonitril/Wasser-Gradient 5:95 → 95:5 mit 0.1% Ameisensäure).
Ausbeute: 36% d. Th.
LC-MS (Methode 6): Rₜ = 1.22 min.
MS (ESIpos): m/z = 465 (M+H)⁺
¹H-NMR (400 MHz, DMSO-d₆): δ = 8.23 (s, 2 H), 7.35 (d, 1 H), 7.21-7.02 (m, 7 H), 6.99-6.94 (m, 2 H), 6.68 (d, 1 H), 6.58 (dd, 1 H), 4.08 (t, 2 H), 3.75 (dd, 1 H), 3.50 (dd, 1 H), 2.64 (t, 2 H), 2.47-2.37 (m, 1 H), 2.34-2.23 (m, 1 H), 2.23 (s, 6 H).

### Beispiel 47

### (1S*,3S*,3aR*,8bS*)-6-Benzyloxy-3a-(4-chlorphenyl)-3-phenyl-2,3,3a,8b-tetrahydrocyclopenta-[b]benzofuran-1,8b-(1H)-diol

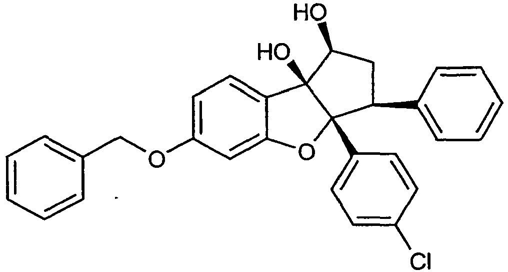

Die Titelverbindung wird in Analogie zur Synthese von Beispiel 6A ausgehend von Beispiel 61A hergestellt.
Ausbeute: 54% d. Th.
LC-MS (Methode 3): Rₜ = 4.85 min.
MS (ESIneg): m/z = 483 (M-H)⁻
¹H-NMR (300 MHz, DMSO-d₆): δ = 7.46-7.12 (m, 13 H), 6.88-6.85 (m, 2 H), 6.60-6.55 (m, 2 H), 5.64 (d, 1 H), 5.08-5.06 (m, 3 H), 4.16-4.15 (m, 1 H), 4.07-3.99 (m, 1 H), 1.94-1.91 (m, 2 H).

### Beispiel 48

### (1S*,3S*,3aR*,8bS*)-3a-(4-Chlorphenyl)-3-phenyl-8-(2-pyrrolidin-1-yl-ethoxy)-2,3,3a,8b-tetrahydrocyclopenta[b]benzofuran-1,8b-(1H)-diol

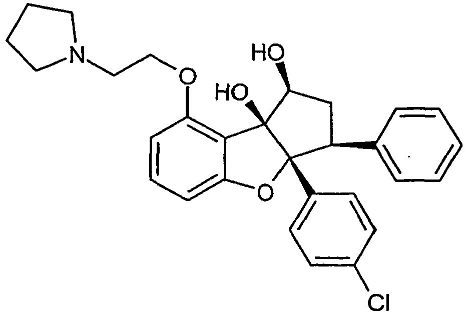

Die Titelverbindung wird in Analogie zur Synthese von Beispiel 32 ausgehend von Beispiel 69A hergestellt.
Ausbeute: 70% d. Th.
LC-MS (Methode 7): Rₜ = 2.10 min.
MS (ESIpos): m/z = 492 (M+H)⁺
¹H-NMR (300 MHz, DMSO-d₆): δ = 7.26 (t, 1 H), 7.21-7.17 (m, 2 H), 7.16-7.11 (m, 2 H), 7.10-7.02 (m, 3 H), 6.99-6.94 (m, 2 H), 6.68 (d, 1 H), 6.64 (d, 1 H), 6.49 (br. s, 1 H), 4.95 (br. s, 1 H), 4.54 (dd, 1 H), 4.28-4.19 (m, 1 H), 4.16-4.06 (m, 1 H), 3.41-3.25 (m, 1 H), 3.10-2.95 (m, 1 H), 2.75-2.51 (m, 5 H), 2.47-2.37 (m, 1 H), 2.32-2.18 (m, 1 H), 1.79-1.71 (m; 4 H).

Die präparative Trennung des Racemats in die Enantiomeren wird über HPLC an chiraler Phase nach Methode 17A durchgeführt.

### Analytische Daten (Methode 17B):

Enantiomer A: Rₜ = 8.60 min., Enantiomer B: Rₜ = 9.55 min.

### Beispiel 49

### (1S*,3S*,3aR*,8bS*)-3a-(4-Chlorphenyl)-8-(2-dimethylaminoethoxy)-3-phenyl-2,3,3a,8b-tetrahydrocyclopenta[b]benzofuran-1,8b-(1H)-diol

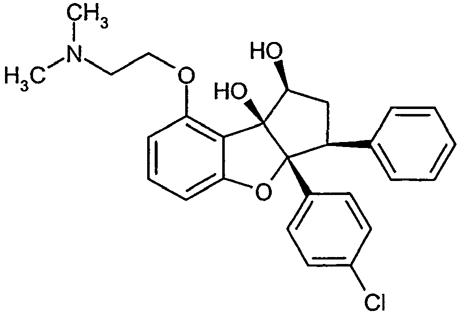

Die Titelverbindung wird in Analogie zur Synthese von Beispiel 32 ausgehend von Beispiel 69A hergestellt.
Ausbeute: 81% d. Th.
LC-MS (Methode 9): Rₜ = 1.88 min.
MS (ESIpos): m/z = 466 (M+H)⁺
¹H-NMR (300 MHz, DMSO-d₆): δ = 7.26 (t, 1 H), 7.22-7.17 (m, 2 H), 7.16-7.11 (m, 2 H), 7.10-7.02 (m, 3 H), 6.99-6.93 (m, 2 H), 6.68 (d, 1 H), 6.64 (d, 1 H), 6.52 (br. s, 1 H), 4.92 (br. s, 1 H), 4.55 (dd, 1 H), 4.23-4.15 (m, 1 H), 4.14-4.05 (m, 1 H), 3.38-3.26 (m, 1 H), 2.87-2.76 (m, 1 H), 2.60-2.38 (m, 2 H), 2.32-2.17 (m, 1 H), 2.25 (s, 6 H).

### Beispiel 50

### (1S*,3S*,3aR*,8bS*)-3a-(4-Chlorphenyl)-8-(2-methylaminoethoxy)-3-phenyl-2,3,3a,8b-tetrahydrocyclopenta[b]benzofuran-1,8b-(1H)-diol

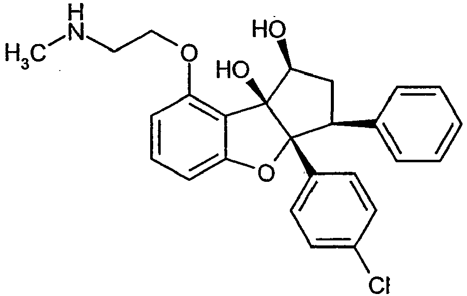

Die Titelverbindung wird in Analogie zur Synthese von Beispiel 32 ausgehend von Beispiel 69A hergestellt.
Ausbeute: 17% d. Th.
LC-MS (Methode 9): Rₜ = 1.88 min.
MS (ESIpos): m/z = 452 (M+H)⁺
¹H-NMR (300 MHz, DMSO-d₆): δ = 7.30 (t, 1 H), 7.26-7.14 (m, 4 H), 7.13-7.04 (m, 3 H), 6.95-6.89 (m, 2 H), 6.74 (d, 1 H), 6.66 (d, 1 H), 4.64 (dd, 1 H), 4.36-4.19 (m, 2 H), 3.41-3.25 (m, 1 H), 2.62 (s, 3 H), 2.48-2.39 (m, 1 H), 2.33-2.21 (m, 1 H).

### Beispiel 51

### (1R*,3S*,3aR*,8bS*)-3a-(4-Chlorphenyl)-8-(2-dimethylaminoethoxy)-3-phenyl-2,3,3a,8b-tetrahydrocyclopenta[b]benzofuran-1,8b-(1H)-diol

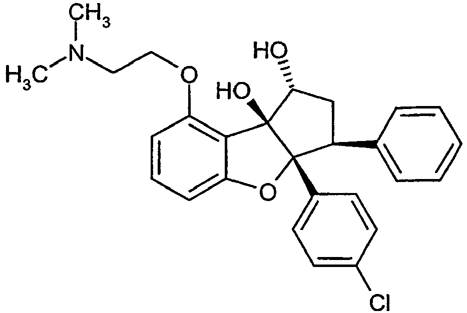

Die Titelverbindung wird in Analogie zur Synthese von Beispiel 32 ausgehend von Beispiel 71A hergestellt.
Ausbeute: 65% d. Th.
LC-MS (Methode 4): Rₜ = 2.24 min.
MS (ESIpos): m/z = 466 (M+H)⁺
¹H-NMR (300 MHz, DMSO-d₆): δ = 7.23 (t, 1 H), 7.19-7.14 (m, 2 H), 7.13-6.98 (m, 7 H), 6.63 (d, 1 H), 6.61 (d, 1 H), 5.30 (br. s, 1 H), 4.54 (d, 1 H), 4.29-4.12 (m, 3 H), 3.88 (dd, 1 H), 2.74 (dt, 1 H), 2.72-2.58 (m, 2 H), 2.23 (s, 6 H), 1.99 (dd, 1 H).

### Beispiel 52

### (1R*,3S*,3aR*,8bS*)-3a-(4-Chlorphenyl)-3-phenyl-8-(2-pyrrolidin-1-yl-ethoxy)-2,3,3a,8b-tetrahydrocyclopenta[b]benzofuran-1,8b-(1H)-diol

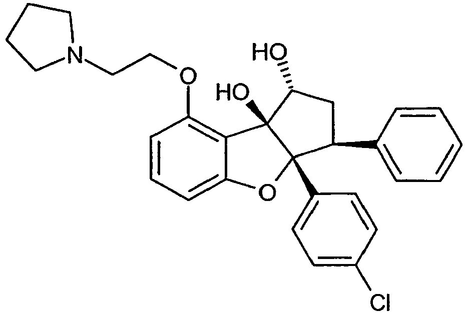

Die Titelverbindung wird in Analogie zur Synthese von Beispiel 32 ausgehend von Beispiel 71A hergestellt.
Ausbeute: 47% d. Th.
LC-MS (Methode 4): Rₜ = 2.29 min.
MS (ESIpos): m/z = 492 (M+H)⁺
¹H-NMR (300 MHz, DMSO-d₆): δ = 7.22 (t, 1 H), 7.18-7.12 (m, 2 H), 7.11-6.99 (m, 7 H), 6.63 (d, 1 H), 6.61 (d, 1 H), 5.42 (br. s, 1 H), 4.51 (d, 1 H), 4.31-4.13 (m, 3 H), 3.91 (dd, 1 H), 2.90-2.65 (m, 2 H), 2.75 (dt, 1 H), 2.63-2.45 (m, 4 H), 1.99 (dd, 1 H), 1.71-1.60 (m, 4 H).

### Beispiel 53

### (1R*,3S*,3aR*,8bS*)-3a-(4-Chlorphenyl)-8-(2-methoxyethoxy)-3-phenyl-2,3,3a,8b-tetrahydro-cyclopenta[b]benzofuran-1,8b-(1H)-diol

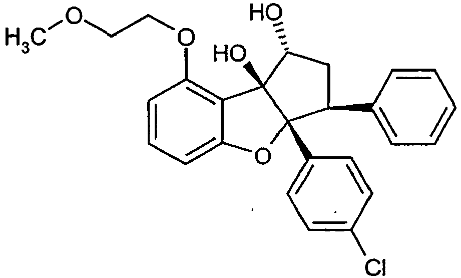

Die Titelverbindung wird in Analogie zur Synthese von Beispiel 6A ausgehend von Beispiel 78A hergestellt.
Ausbeute: 12% d. Th.
LC-MS (Methode 4): Rₜ = 3.50 min.
MS (ESIpos): m/z = 453 (M+H)⁺
¹H-NMR (200 MHz, DMSO-d₆): δ = 7.24 (t, 1 H), 7.15-6.94 (m, 9 H), 6.66 (d, 1 H), 6.59 (d, 1 H), 5.14 (s, 1 H), 4.62-4.51 (m, 2 H), 4.22-4.14 (m, 2 H), 3.89 (dd, 1 H), 3.73-3.66 (m, 2 H), 3.35 (s, 3 H) 2.74 (ddd, 1 H), 1.98 (dd, 1 H).

### Beispiel 54

### (1S*,3S*,3aR*,8bS*)-3a-(4-Chlorphenyl)-6-(3-methylaminopropoxy)-3-phenyl-2,3,3a,8b-tetrahydrocyclopenta[b]benzofuran-1,8b-(1H)-diol

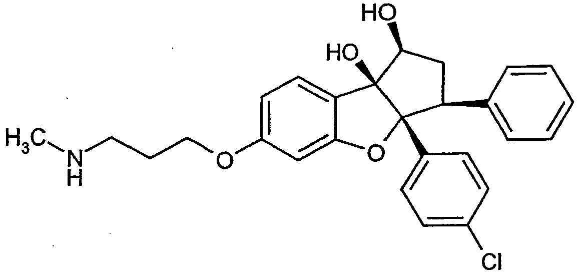

Die Titelverbindung wird in Analogie zur Synthese von Beispiel 32 ausgehend von Beispiel 79A hergestellt.
Ausbeute: 94% d. Th.
LC-MS (Methode 7): Rₜ = 1.83 min.
MS (ESIpos): m/z = 466 (M+H)⁺
¹H-NMR (200 MHz, DMSO-d₆): δ = 8.63 (br. s, 1 H), 7.32-6.90 (m, 10 H), 6.70 (d, 1 H), 6.60 (dd, 1 H), 5.92 (d, 1 H), 5.04 (s, 1 H), 4.49-4.40 (m, 1 H), 4.13-4.07 (m, 2 H), 3.35-3.20 (m, 1 H), 3.08-3.00 (m, 2 H), 2.58 (s, 3 H), 2.55-2.03 (m, 4 H).

### Beispiel 55

### (1 S*,3S*,3aR*,8bS*)-3a-(4-Chlorphenyl)-6-(3-dimethylaminopropoxy)-3-phenyl-2,3,3a,8b-tetrahydrocyclopenta[b]benzofuran-1,8b-(1H)-diol

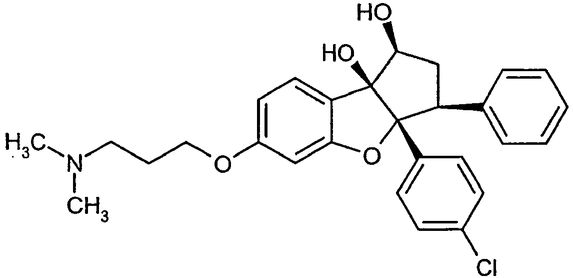

Die Titelverbindung wird in Analogie zur Synthese von Beispiel 32 ausgehend von Beispiel 79A hergestellt.
Ausbeute: 99% d. Th.
LC-MS (Methode 7): Rₜ = 1.86 min.
MS (ESIpos): m/z = 480 (M+H)⁺
¹H-NMR (200 MHz, DMSO-d₆): δ = 7.30-6.90 (m, 10 H), 6.68 (d, 1H), 6.58 (dd, 1 H), 5.89 (d, 1 H), 5.04 (s, 1 H), 4.50-4.44 (m, 1 H), 4.08-4.01 (m, 2 H), 3.35-3.25 (m, 1 H), 2.71-2.64 (m, 2 H), 2.39 (s, 6 H), 2.29-2.11 (m, 2 H), 2.02-1.91 (m, 2 H).

### Beispiel 56

### (1S*,3S*,3aR*,8bS*)-3a-(4-Chlorphenyl)-3-phenyl-6-(3-pyrrolidin-1-yl-propoxy)-2,3,3a,8b-tetrahydrocyclopenta[b]benzofuran-1,8b-(1H)-diol

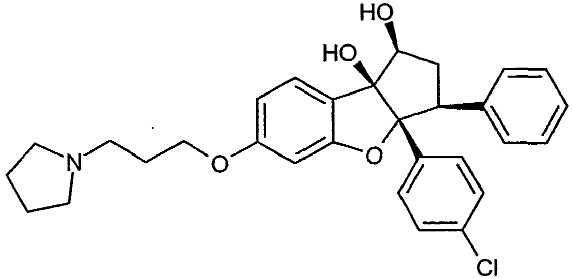

Die Titelverbindung wird in Analogie zur Synthese von Beispiel 32 ausgehend von Beispiel 79A hergestellt.
Ausbeute: 99% d. Th.
LC-MS (Methode 7): Rₜ = 1.91 min.
MS (ESIpos): m/z = 506 (M+H)⁺
¹H-NMR (200 MHz, DMSO-d₆): δ = 7.32-6.90 (m, 10 H), 6.70 (d, 1 H), 6.60 (dd, 1 H), 5.91 (d, 1 H), 5.05 (s, 1 H), 4.50-4.40 (m, 1 H), 4.13-4.07 (m, 2 H), 3.35-3.19 (m, 7 H), 2.50-1.92 (m, 8 H).

### Beispiel 57

### (1S*,3S*,3aR*,8bS*)-6-(3-Azetidin-1-yl-propoxy)-3a-(4-chlorphenyl)-3-phenyl-2,3,3a, 8b-tetrahydrocyclopenta[b]benzofuran-1,8b-(1H)-diol

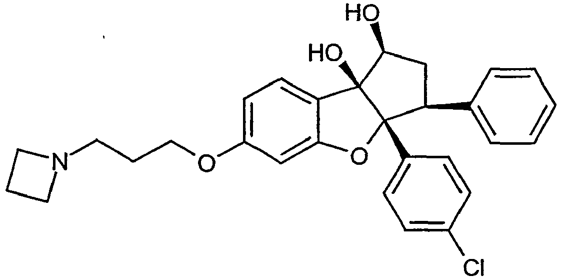

Die Titelverbindung wird in Analogie zur Synthese von Beispiel 32 ausgehend von Beispiel 79A hergestellt.
Ausbeute: 69% d. Th.
LC-MS (Methode 13): Rₜ = 1.97min.
MS (ESIpos): m/z = 492 (M+H)⁺
¹H-NMR (300 MHz, DMSO-d₆): δ = 7.27-7.03 (m, 8 H), 6.94-6.91 (m, 2 H), 6.64 (d, 1 H), 6.57 (dd, 1 H), 5.82 (d, 1 H), 4.99 (s, 1 H), 4.48-4.41 (m, 1 H), 4.02-3.98 (m, 2 H), 3.12 (m, 4 H), 2.49-2.40 (m, 2 H), 2.26-2.14 (m, 1 H), 1.96 (t, 2 H), 1.70 (t, 2 H), 0.86-0.81 (m, 2 H).

### Beispiel 58

### N-[(1S*,3S*,3aR*,8bS*)-3a-(4-Chlorphenyl)-1,8b-dihydroxy-3-phenyl-2,3,3a,8b-tetrahydro-1H-benzo[b]cyclopenta[d]furan-6-yl]-2-fluornicotinamid

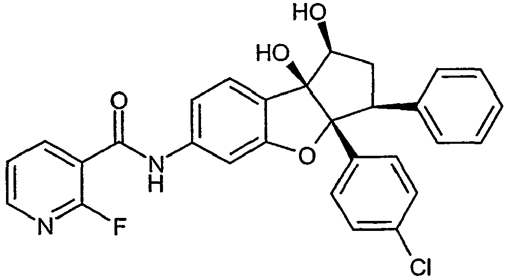

In 0.5 ml DMSO werden 39.4 mg (0.1 mmol) (1S*,3S*,3aR*,8bS*)-6-Amino-3a-(4-chlorphenyl)-3-phenyl-1,2,3,3a-tetrahydrocyclopenta[*b*]benzofuran-1,8b-(1*H*)-diol (Beispiel 84A), 14.1 mg (0.1 mmol) 2-Fluorpicolin, 41.7 mg (0.13 mmol) O-(Benzotriazol-1-yl)-*N,N,N*'*,N*'-tetramethyluronium-Tetrafluoroborat und 25.8 mg (0.2 mmol) Diisopropylethylamin zusammen gegeben und über Nacht bei Raumtemperatur gerührt. Es wird dann vom Feststoff abfiltriert und das Filtrat mittels präparativer HPLC gereinigt.
Ausbeute: 19.7 mg (38% d. Th.)
LC-MS (Methode 10): Rₜ = 2.21 min.
MS (ESIpos): m/z = 517 (M+H)⁺.

### Beispiel 59

### N-[(1S*,3S*,3aR*,8bS*)-3a-(4-Chlorphenyl)-1,8b-dihydroxy-3-phenyl-2,3,3a,8b-tetrahydro-1H-benzo[b]cyclopenta[d]furan-6-yl]-6-fluorpyridin-2-carboxamid

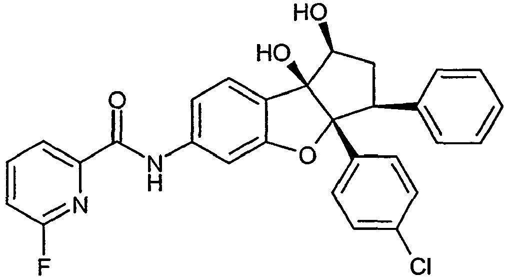

Die Titelverbindung wird in Analogie zur Synthese von Beispiel 58 ausgehend von Beispiel 84A hergestellt.
LC-MS (Methode 10): Rₜ = 2.35 min.
MS (ESIneg): m/z - 515 (M-H)⁻.

### Beispiel 60

### N-[(1S*,3S*,3aR*,8bS*)-3a-(4-Chlorphenyl)-1,8b-dihydroxy-3-phenyl-2,3,3a,8b-tetrahydro-1H-benzo[b]cyclopenta[d]furan-6-yl]-1-ethyl-1H-pyrazol-3-carboxamid

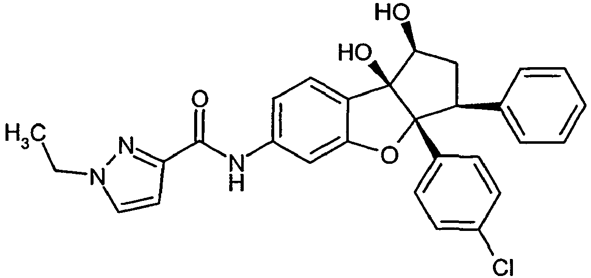

Die Titelverbindung wird in Analogie zur Synthese von Beispiel 58 ausgehend von Beispiel 84A hergestellt.
LC-MS (Methode 10): Rₜ = 2.25 min.
MS (ESIpos): m/z = 516 (M+H)⁺.

### Beispiel 61

### N-[(1S*,3S*,3aR*,8bS*)-3a-(4-Chlorphenyl)-1,8b-dihydroxy-3-phenyl-2,3,3a,8b-tetrahydro-1H-benzo[b]cyclopenta[d]furan-6-yl]-1,2,3-thiadiazol-4-carboxamid

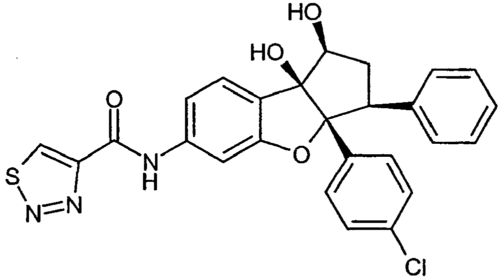

Die Titelverbindung wird in Analogie zur Synthese von Beispiel 58 ausgehend von Beispiel 84A hergestellt.
LC-MS (Methode 10): Rₜ = 2.23 min.
MS (ESIneg): m/z = 504 (M-H)⁻.

### Beispiel 62

### N-[(1S*,3S*,3aR*,8bS*)-3a-(4-Chlorphenyl)-1,8b-dihydroxy-3-phenyl-2,3,3a,8b-tetrahydro-1H-benzo[b]cyclopenta[d]furan-6-yl]-pyridin-2-carboxamid

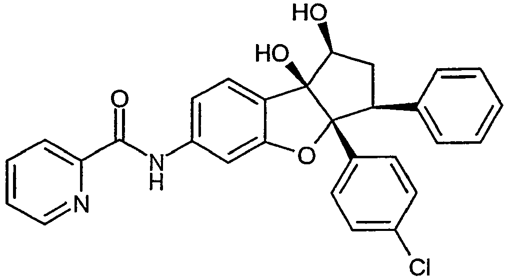

Die Titelverbindung wird in Analogie zur Synthese von Beispiel 58 ausgehend von Beispiel 84A hergestellt.
LC-MS (Methode 10): Rₜ = 2.38 min.
MS (ESIpos): m/z = 499 (M+H)⁺.

### Beispiel 63

### N-[(1S*,3S*,3aR*,8bS*)-3a-(4-Chlorphenyl)-1,8b-dihydroxy-3-phenyl-2,3,3a,8b-tetrahydro-1H-benzo[b]cyclopenta[d]furan-6-yl]-1H-1,2,4-triazol-5-carboxamid

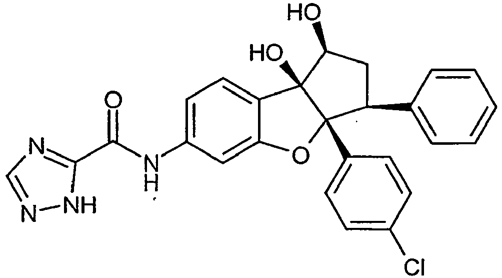

Die Titelverbindung wird in Analogie zur Synthese von Beispiel 58 ausgehend von Beispiel 84A hergestellt.
LC-MS (Methode 10): Rₜ = 2.00 min.
MS (ESIpos): m/z = 489 (M+H)⁺.

### B. Bewertung der pharmakoloigischen Wirksamkeit

Die pharmakologische Wirkung der erfindungsgemäßen Verbindungen kann in folgenden Assays gezeigt werden:

### 1. In vitro-Bestimmung der NF-κB- und AP-1-inhibitorischen Wirkung

### 1.a) Hemmung der Interleukin-8 (IL-8)-Freisetzung_aus humanen Endothelzellen

Die Expression des humanen IL-8-Gens wird durch eine Vielfalt von regulatorischen und Verstärker-Elementen kontrolliert, die oberhalb der Promoterregion des Gens lokalisiert sind. Diese Elemente enthalten Bindungsstellen für Transkriptionsfaktoren und erst deren Interaktion mit ihren DNA-Bindungsstellen ermöglicht die effiziente Transkription des IL-8-Gens. Die Aktivität der entsprechenden Transkriptionsfaktoren ist durch unterschiedliche Stimuli (z.B. Interleukin-1β, Tumor Nekrose Faktor-α) induzierbar und über verschiedene Signaltransduktionswege modulierbar [H. Holtmann et al., Mol. Cell. Biol. 19, 6742-6753 (1999)].

Für die maximale Expression von IL-8 ist die Aktivität zweier unterschiedlicher Transkriptionsfaktoren essentiell: NF-κB und AP-1 [Y.-H. Chang et al., Exp. Cell Res. 278, 166-174 (2000)]. Die Zytokin-induzierte IL-8-Synthese lässt sich deshalb als Testsystem zur Identifikation und Charakterisierung von Testsubstanzen einsetzen, die direkt oder indirekt die Bindung von NF-κB und AP-1 an ihre DNA-Bindungsstellen hemmen.

### Versuchsdurchführung:

Humane Nabelschnur-Endothelzellen (HUVEC) wurden von der Firma CellSystems (St. Katharinen, Deutschland) bezogen und entsprechend der Lieferantenempfehlung in EGM-2-Medium inklusive Wachstumszusätzen (CellSystems, St. Katharinen, Deutschland) in 165 cm²-Gewebekulturflaschen kultiviert. Nach Erreichen einer 60-80%-igen Konfluenz des Zellrasens werden die Zellen mittels Trypsin-Behandlung vom Flaschenboden abgelöst, mechanisch dissoziiert und in einer Zelldichte von 5000 Zellen/Vertiefung in 96-Loch-Mikrotiterplatten (Corning, Wiesbaden, Deutschland) ausgesät. Nach 3 Tagen wird das Kulturmedium komplett durch 170 µl frisches Medium pro Vertiefung ersetzt. Die Zellen werden am nächsten Tag für die Experimente eingesetzt.

Zur Bestimmung der hemmenden Wirkung der erfindungsgemäßen Substanzen auf die IL-8-Freisetzung werden diese zunächst in einer zehnfach höheren Konzentration, verglichen mit der gewünschten Endkonzentration im Test, in Medium, das 1% DMSO enthält, gelöst. Anschließend werden 20 µl der Substanzlösung pro Vertiefung zugesetzt. Die Bildung und Freisetzung von IL-8 wird danach durch Zugabe von Interleukin-1β (IL-1β, Endkonzentration 10 ng/ml; Biosource GmbH, Solingen, Deutschland) induziert und die Zellen für 6 h bei 37°C im CO₂-Inkubator inkubiert. Im Anschluss werden 150 µl des Zellüberstandes abgenommen und bis zur Messung des IL-8-Gehaltes mittels ELISA (Biosource GmbH, Solingen, Deutschland) bei -20°C eingefroren. Zur Durchführung des ELISA werden die Proben aufgetaut, 1:10 verdünnt und der Test entsprechend den Herstellerangaben durchgeführt. Die Hemmwirkung der erfindungsgemäßen Verbindungen auf die IL-8-Freisetzung wird durch den Vergleich zu Vehikel-behandelten Zellen ermittelt.

Repräsentative Wirkdaten zu den erfindungsgemäßen Verbindungen sind in der folgenden Tabelle 1 aufgeführt:

**Tabelle 1**

| **Beispiel Nr.** | **IC₅₀ [nM]** |
|---|---|
| 3 | 170 |
| 12 | 466 |
| 15 | 166 |
| 18 | 20 |
| 22 | 90 |
| 23 | 202 |
| 32 | 112 |
| 48 | 214 |

| | |
|---|---|
| [IC₅₀ = Wirkstoffkonzentration, die eine 50%-ige Hemmung der IL-8-Freisetzung bezogen auf deren Maximaleffekt bewirkt]. | |

### 1.b) Hemmung der AP-1-Aktivität in Astrozyten-Kulturen

AP-1 ist ein aus Homo- oder Heterodimeren der Jun-, Fos- und ATF-Familie gebildeter, im Zellkern lokalisierter Transkriptionsfaktor. Aktivierende Signale führen einerseits zu einer vermehrten Synthese der Einzelkomponenten, andererseits zu einer spezifischen Phosphorylierung der Jun- oder ATF-Untereinheiten. Beide Prozesse führen zu einer verstärkten Wechselwirkung des Proteinkomplexes mit dessen Zielgenen und ermöglicht so deren Expression. Die Stimulusinduzierte Phosphorylierung von z.B. c-Jun kann somit als Indikator für die AP-1-Aktivierung dienen. Mithilfe der nachfolgend beschriebenen immunozytochemischen Detektion der c-Jun-Phosphorylierung lässt sich der Einfluß der erfindungsgemäßen Verbindungen auf die AP-1-Aktivierung untersuchen.

### Versuchsdurchführung:

Gemischte Gliazellkulturen wurden aus Gehirnen von 1 Tag-alten Ratten (Wistar) hergestellt. Dazu werden die Tiere durch Enthauptung getötet, die Gehirne entnommen und in kalter Hank's Salzlösung (HBSS, Gibco, Karlsruhe, Deutschland) gesammelt. Hirnstamm und Kleinhirn werden entfernt, die zerebralen Hemisphären von Hirnhäuten befreit und die Gewebestücke mechanisch in Gegenwart von Papain (Papain dissociation kit, CellSystems, St. Katharinen, Deuschland) dissoziiert. Die Zellen werden durch Zentrifugation bei 450 x g gesammelt und in 175 cm²-Gewebe-kulturflaschen ausplattiert. Die Zellen werden für 12-14 Tage in DMEM/Ham's F12-Medium, 10% fötalem Kälberserum, 100 µg/ml Penicillin/Streptomycin (Sigma, Taufkirchen, Deutschland) kultiviert. Anschließend werden Zellbruchstücke und auf der Oberfläche des Zellrasens wachsende mikrogliale und oligodendrogliale Zellen für 2 h mithilfe eines Schüttlers abgeschüttelt und die verbleibenden Astrozyten durch Trypsinierung vom Flaschenboden abgelöst. Die Zellen werden mechanisch dissoziiert, durch Zentrifugation bei 450 x g gesammelt, erneut mechanisch dissoziiert und in einer Dichte von 100000 Zellen/Vertiefung auf Poly-D-Lysin-beschichtete 8-Kammer-Objektträger (NUNC, Dänemark) verteilt. Die so angereicherten Astrozyten werden in Phenolrotfreiem DMEM/Ham's F12-Medium, 10% fötalem Kälberserum, 100 µg/ml Penicillin/Streptomycin (Sigma, Taufkirchen, Deutschland) kultiviert. Einen Tag vor ihrer Verwendung im Versuch wird der Anteil von fötalem Kälberserum im Medium auf 1% reduziert.

Zur Untersuchung der Wirkung der erfindungsgemäßen Verbindungen auf die AP-1-Aktivierung werden diese in der gewünschten Konzentration dem Kulturmedium zugegeben (in der Regel finale Konzentration von 1 µM im Test). Anschließend wird der AP-1-Signalweg (c-Jun-Phosphorylierung) durch Zugabe von Lipopolysaccherid (LPS, Sigma, Taufkirchen, Deutschland) in einer Endkonzentration von 100 ng/ml oder durch Zugabe von Interleukin-1β (IL-1β, Biosource GmbH, Solingen, Deutschland) in einer Endkonzentration von 30 ng/ml stimuliert. Nach 90 min werden die Zellen kurz mit Phosphat-gepufferter Salzlösung (PBS) gewaschen und dann für 10 min in 4%-iger Paraformaldehyd-Lösung in PBS fixiert. Danach werden die Zellen für 5 min mit -20°C kaltem Methanol permeabilisiert, in PBS, 5% Sucrose, 0.3% TritonX-100 gewaschen und für 30 min bei Raumtemperatur in Blockierpuffer (PBS, 1% Ziegenserum, 2% BSA) inkubiert. Die Inkubation mit den gegen das Astrozyten-spezifische Protein GFAP (glial fibrillary acidic protein; monoklonaler Antikörper aus Maus, Sigma, Taufkirchen, Deutschland) und gegen die Serin-63-phosphorylierte Form von c-Jun (polyklonaler Antikörper aus Kaninchen, Calbiochem, Bad Soden, Deutschland) gerichteten, primären Antikörpern erfolgt im selben Ansatz bei 4°C über Nacht. Die Antikörper gegen GFAP werden 1:400 und die gegen phospho-c-Jun 1:50 in Blockierpuffer verdünnt. Überschüssiger Antikörper wird anschließend durch dreifaches Waschen in PBS, 5% Sucrose, 0.3% TritonX-100 entfernt und die Zellen bei Raumtemperatur für je 1 h nacheinander mit den jeweiligen sekundären, Spezies-spezifischen und in Blockierpuffer verdünnten Antikörpern inkubiert (anti-Maus Cy2-konjugierte Antikörper, 1:500, Amersham Biosciences, Freiburg, Deutschland; anti-Kaninchen Cy3-konjugierte Antikörper, 1:800, Sigma, Taufkirchen, Deutschland). Überschüssiger Antikörper wird anschließend durch dreifaches Waschen in PBS, 5% Sucrose, 0.3% Triton-100 entfernt und die Zellkerne durch 10 min Inkubation der Zellen mit Hoechst 33258 (4 µg/ml in PBS) angefärbt. Abschließend werden die Zellen erneut zweimal für 5 min mit PBS gewaschen, der Dichtring der 8-Kammer-Objektträger entfernt und die Zellen in Einbett-Medium (Sigma, Taufkirchen, Deutschland) unter Zuhilfenahme eines Deckgläschens eingebettet. Das Versuchsergebnis läßt sich mithilfe eines Fluoreszenz-Mikroskops (Objektiv-Vergrößerung 25-fach) beurteilen.

Phosphoryliertes c-Jun-Protein ist aufgrund der Bindung des Cy3-konjugierten sekundären Antikörpers an den detektierenden, gegen phospho-c-Jun gerichteten, primären Antikörper durch eine rote, im Zellkern lokalisierte Fluoreszenz erkennbar. Der Zellkern erscheint blau-fluoreszent. Astrozyten sind durch eine Cy-2-vermittelte, grün-fluoreszierende GFAP-Färbung identifizierbar.

Unter basalen Bedingungen enthalten nur wenige Zellkerne phosphoryliertes c-Jun. Durch Zugabe von LPS wird die AP-1-vermittelte Signaltransduktion induziert, so dass mehr als 80% der Zellkerne rötlich fluoreszieren, also phospho-c-Jun enthalten. Die Rotfärbung wird durch Zugabe der erfindungsgemäßen Verbindungen deutlich erniedrigt, was zum einen durch eine Reduktion der Farbintensität, zum anderen durch eine Verminderung der gefärbten Kerne zum Ausdruck kommt. Die Kolokalisation der Prozesse in Astrozyten wird durch eine Überlagerung der grünen, roten und blauen Fluoreszenzbilder ermöglicht.

### 2. Bestimmung der metabolischen Stabilität

### In vitro-Inkubation mit Lebermikrosomen:

Zur Bestimmung der hepatischen mikrosomalen Stabilität werden die erfindungsgemäßen Substanzen mit Lebermikrosomen unterschiedlicher Species inkubiert. Die Inkubationskonzentration der Substanzen wird möglichst niedrig gehalten (bevorzugt < 1 µM), ebenso die Konzentration der mikrosomalen Proteine im Testansatz (bevorzugt 0.2 mg mikrosomales Protein pro ml Inkubationsansatz). Diese Vorgehensweise ermöglicht für die meisten Verbindungen ein Arbeiten im linearen Bereich der Michaelis-Menten-Kinetik. Aus dem Inkubationsansatz wird zu verschiedenen Zeitpunkten (insgesamt 7 Zeitpunkte) eine Probe für die Bestimmung der verbliebenen Substanzkonzentration entnommen. Aus der Halbwertszeit der Substanz im Inkubationsansatz werden die Clearance (CL) und die maximale Bioverfügbarkeit (Fmax) der Testsubstanz für die jeweilige Species berechnet. Der Gehalt an organischen Lösungsvermittlern beträgt maximal 1% Acetonitril bzw. maximal 0.2% DMSO, um den Einfluss auf die mikrosomalen Enzyme zu minimieren.

### Versuchsdurchführung:

Die Versuche werden wie im Detail von J.B. Houston und D.J. Carlile [Drug Metab. Rev. 29, 891-922 (1997)] beschrieben durchgeführt.

Wie für die in der Tabelle 2 aufgeführten Verbindungen beispielhaft gezeigt, weisen die erfindungsgemäßen Substanzen in Lebennikrosomen-Präparationen eine erhöhte Stabilität im Vergleich zu den in WO 00/08007 beschriebenen Verbindungen auf:

**Tabelle 2**

| **Beispiel Nr.** | **Lebermikrosomen aus der Maus** | |
|---|---|---|
| | **CL [l/(kg x h)]** | **Fmax [%]** |
| 3 | <2.7 | >50 |
| 12 | 2.97 | 45.1 |
| 15 | <2.7 | >50 |
| 18 | <2.7 | >50 |
| 22 | <2.7 | >50 |
| 32 | <2.7 | >50 |
| 48 | <2.7 | >50 |
| Vergleich: I-41 aus WO 00/08007 | 5.0 | 6.8 |

### 3. In vitro-Proliferation von Tumorzellen

Verschiedene Formen von Krebs-Erkrankungen sind gekennzeichnet durch die unkontrollierte, übermäßige Proliferation unterschiedlicher Zelltypen, was zur Ausbildung von Metastasen und Tumoren führt. Die Verwendungsmöglichkeit der erfindungsgemäßen Substanzen zur Behandlung hyperproliferativer Erkrankungen lässt sich durch ihre Aktivität auf die Zellteilungsrate von Tumorzellen *in vitro* untersuchen. Die Verknüpfung zwischen anti-proliferativer Wirkung *in vitro* und klinischer anti-Tumor-Wirkung ist gut etabliert. Die therapeutische Verwendbarkeit von z.B. Taxol [Silvestrini et al., Stem Cells 11, 528-535 (1993)], Taxotere [Bissery et al., Anti Cancer Drugs 6, 339 (1995)] oder Topoisomerase-Inhibitoren [Edelman et al., Cancer Chemother. Pharmacol. 37, 385-393 (1996)] wurde durch deren Aktivität in *in vitro*-Tumorzellen-Proliferationstests belegt.

### Versuchsdurchführung:

Die Tumorzelllinien, wie z.B. MDA-MB-231-Zellen (humane Brust-Adenokarzinomzellen), H460-Zellen (humane Lungen-Karzinomzellen) oder HCT-15-Zellen (humane Darm-Adenokarzinomzellen), werden in entsprechenden, vom Lieferanten (z.B. LGC Promochem, Wesel, Deutschland) empfohlenen Wachstumsmedien (Sigma, Taufkirchen, Deutschland) expandiert. Einen Tag vor Zugabe der erfindungsgemäßen Verbindungen werden die Zellen in einer Dichte von 3000 Zellen/Vertiefung in 100 µl Wachstumsmedium auf schwarze 96-Loch-Mikrotiterplatten mit klarem Boden verteilt. Am Tag der Testsubstanzzugabe wird je eine Mikrotiterplatte pro Tumorzelllinie zur Bestimmung der in den Vertiefungen befindlichen Anfangszellzahl verwendet. Den Geschwisterplatten werden die in Wachstumsmedium und DMSO verdünnten erfindungsgemäßen Substanzen zugegeben. Die Testsubstanzen werden in der Regel in unterschiedlichen Konzentrationen, beginnend mit 10 µM Endkonzentration im Test, zugesetzt. Die DMSO-Konzentration im Test beträgt 0.1%. Vierundzwanzig Stunden nach Substanzzugabe werden die Zellzahlen pro Vertiefung mithilfe des CellTiter-Glo^{®} Luminescent Cell Viability-Tests (Promega GmbH, Mannheim, Deutschland) bestimmt. Der Test wird entsprechend den Herstellerangaben durchgeführt und mithilfe eines Luminometers ausgewertet. Zur Ermittlung der anti-proliferativen Aktivität der erfindungsgemäßen Verbindungen werden die vor Substanzzugabe in Geschwisterplatten bestimmten Zellzahlen abgezogen und der prozentuale Unterschied der Zellzahländerungen zwischen Substanz-behandelten und Vehikel-behandelten Zellen ermittelt. Die relative Wirksamkeit der unterschiedlichen Testsubstanzen wird über einen Vergleich der Konzentrationen, die 50% ihres Maximaleffektes bewirken (IC₅₀), bestimmt.

Repräsentative Wirkdaten zu den erfindungsgemäßen Verbindungen sind in der folgenden Tabelle 3 aufgeführt:

**Tabelle 3**

| **Beispiel Nr.** | **IC₅₀ [nM] (H460-Zellen)** |
|---|---|
| 21 | 1.5 |
| 23 | 99.7 |
| 32 | 0.5 |
| 48 | 47.9 |
| 49 | 29.7 |

| | |
|---|---|
| [IC₅₀ = Wirkstoffkonzentration, die eine 50%-ige Hemmung der Tumorzellproliferation bezogen auf deren Maximaleffekt bewirkt]. | |

### 4. Maus MPTP-Tiermodell der Parkinson'schen Krankheit

Die Parkinson'sche Krankheit ist histopathologisch durch den selektiven Verlust einer Gruppe von Nervenzellen der Substantia nigra, die den Neurotransmitter Dopamin synthetisieren, gekennzeichnet. 1-Methyl-4-phenyl-1,2,3,6-tetrahydropyridin (MPTP) ist eine zu Beginn der 80er Jahre identifizierte Verunreinigung synthetischer Drogen, die beim Menschen Symptome des Parkinsonismus und die charakteristische Degeneration dopaminerger Neurone induziert. Die Applikation von MPTP führt bei verschiedenen Tierarten, wie z.B. Maus oder Affe, zu Verhaltens- und histopathologischen Änderungen, die denen der menschlichen Erkrankung vergleichbar sind. Daher gilt das Maus MPTP-Tiermodell als valides Modell für die Parkinson'sche Krankheit und als geeignet, um neuroprotektive Wirkeigenschaften von Prüfsubstanzen zu untersuchen.

### Versuchsdurchführung:

Die Versuche werden im wesentlichen wie von E. Bezard et al. [Neurosci. Lett. 234, 47-50 (1997)] beschrieben durchgeführt. Männlichen 8-Wochen-alten C57/BL6-Mäusen wird an drei aufeinander folgenden Tagen je 4 mg/kg MPTP i.p. appliziert. Die erfindungsgemäßen Substanzen werden beginnend unmittelbar vor der ersten MPTP-Applikation täglich oder zweimal täglich oral verabreicht. An Tag 11 nach der ersten MPTP-Gabe werden die Tiere anästhesiert, intrakardial zunächst mit 25 ml 0.9%-iger Salzlösung, dann mit 75 ml 4%-iger Paraformaldehyd-Lösung perfundiert, und die Gehirne zur histologischen Bearbeitung entnommen. Wie von E. Bezard et al. [Neurosci. Lett. 234, 47-50 (1997)] beschrieben, werden dazu die dopaminergen Neurone immunohistochemisch sichtbar gemacht als Zellen, die das im Dopamin-Stoffwechsel essentielle Enzym Tyrosin-Hydroxylase enthalten. Die Anzahl der verbliebenen dopaminergen Neurone wird mithilfe eines Computerprogramms in drei unterschiedlichen Schnitten pro Tier, die repräsentativ für eine mittlere Schnittebene durch die Substantia nigra sind, quantitativ erfasst [Nelson et al., J. Comp. Neurol. 369, 361-371 (1996)]. Die neuroprotektive Wirksamkeit der Testsubstanzen wird durch den Vergleich zu Vehikel-behandelten MPTP-Tieren und komplett unbehandelten Tieren ermittelt.

### 5. Subdurales Hämatom an Ratten als Tiermodell für traumatische Schädel-Hirn-Verletzungen

Schwere traumatische Schädelverletzungen sind oft begleitet durch Einblutungen unterhalb der Hirnhäute. Die subdurale Blutansammlung führt zu einer lokalen Verminderung des zerebralen Blutflusses der angrenzenden kortikalen Gehirnregion bei gleichzeitigem Anstieg des Glukoseverbrauchs sowie der extrazellulären exzitatorischen Aminosäuren (z.B. Glutamat). Der dem Hämatom benachbarte Gehirnbereich wird ischämisch, was zur Schädigung und zum Absterben von Nervenzellen führt. Die subdurale Injektion von autologem Blut in kortikale Gehirnregionen der Ratte dient als Tiermodell zur Simulation traumatischer Schädel-Hirn-Verletzungen des Menschen. Das Tiermodell eignet sich, um die neuroprotektive Wirksamkeit der erfmdungsgemäßen Substanzen zu untersuchen.

### Versuchsdurchführung:

Der operative Eingriff an männlichen Wistar-Ratten und die unilaterale Applikation von autologem Blut wird wie im Detail von M. Eijkenboom et al. [Neuropharm. 39, 817-834 (2000)] beschrieben durchgeführt. Direkt nach Verschluss der Wunde und jeweils nach 2 h und 4 h werden die erfindungsgemäßen Substanzen intravenös in den gewünschten Dosen injiziert. Sieben Tage nach der Operation werden die Tiere getötet, die Gehirne entnommen und das Infarktvolumen wie von M. Eijkenboom et al. [Neuropharm. 39, 817-834 (2000)] beschrieben bestimmt. Die neuroprotektive Wirksamkeit der erfindungsgemäßen Substanzen wird durch den Vergleich zu Vehikel-behandelten Tieren ermittelt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfmdungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 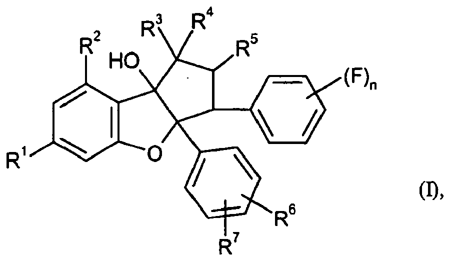 in welcher
R¹ Wasserstoff, Benzyloxy, Ethoxy, n-Propoxy oder eine Gruppe der Formel R⁸-C(=O)-NH- bedeutet, wobei Ethoxy in 2-Position und n-Propoxy in 2- oder 3-Position durch (C₁-C₆)-Alkoxy, Amino, Mono- oder Di-(C₁-C₆)-alkylamino, (C₃-C₈)-Cycloalkylamino, *N*-(C₃-C₈)-Cycloalkyl-*N*-(C₁-C₆)-alkylamino oder durch einen über ein N-Atom gebundenen 4- bis 7-gliedrigen Heterocyclus substituiert sein können,
wobei Mono- und Di-(C₁-C₆)-alkylamino ihrerseits durch Hydroxy, (C₁-C₄)-Alkoxy, Amino, Mono- oder Di-(C₁-C₄)-alkylamino substituiert sein können,
und worin
R⁸ für 5- oder 6-gliedriges Heteroaryl, das durch (C₁-C₄)-Alkyl oder Halogen substituiert sein kann, steht,
R² Wasserstoff, Ethoxy oder n-Propoxy bedeutet, wobei Ethoxy in 2-Position und n-Propoxy in 2- oder 3-Position durch (C₁-C₆)-Alkoxy, Amino, Mono- oder Di-(C₁-C₆)-alkylamino, (C₃-C₈)-Cycloalkylamino, *N*-(C₃-C₈)-Cycloalkyl-*N*-(C₁-C₆)-alkyl-amino oder durch einen über ein N-Atom gebundenen 4- bis 7-gliedrigen Heterocyclus substituiert sein können,
wobei Mono- und Di-(C₁-C₆)-alkylamino ihrerseits durch Hydroxy, (C₁-C₄)-Alkoxy, Amino, Mono- oder Di-(C₁-C₄)-alkylamino substituiert sein können,
wobei R¹ und R² jedoch nicht gleichzeitig für Wasserstoff stehen,
R³ Hydroxy oder Amino
und
R⁴ Wasserstoff bedeutet
oder
R³ und R⁴ gemeinsam mit dem Kohlenstoffatom, an das sie gebunden sind, eine Gruppe der Formel >C=O oder >C=N-OH bilden,
R⁵ Mono- oder Di-(C₁-C₆)-alkylaminocarbonyl bedeutet,
n für die Zahl 0, 1, 2 oder 3 steht,
R⁶ sich in meta- oder para-Position, relativ zur Verknüpfungsstelle des Phenylrings mit dem Tricyclus, befindet und Halogen, (C₁-C₆)-Alkyl, (C₁-C₆)-Alkoxy, (C₆-C₁₀)-Aryl, 5- bis 10-gliedriges Heteroaryl oder eine Gruppe der Formel -NR⁹R¹⁰) bedeutet,
wobei Aryl und Heteroaryl ihrerseits jeweils ein- bis zweifach, gleich oder verschieden, durch Halogen, Cyano, (C₁-C₄)-Alkylsulfonyl oder eine Gruppe der Formel -NR⁹R¹⁰ substituiert sein können,
und worin
R⁹ und R¹⁰ unabhängig voneinander für Wasserstoff, (C₁-C₆)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden,
und
R⁷ sich in meta- oder para-Position, relativ zur Verknüpfungsstelle des Phenylrings mit dem Tricyclus, und in ortho-Stellung zu R⁶ befindet und Wasserstoff, Halogen, (C₁-C₆)-Alkyl, (C₁-C₆)-Alkoxy oder eine Gruppe der Formel -NR¹¹R¹² bedeutet, worin
R¹¹ und R¹² unabhängig voneinander für Wasserstoff, (C₁-C₆)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden,
oder
R⁶ und R⁷ gemeinsam mit dem Phenylring, an den sie gebunden sind, eine Gruppe der Formel 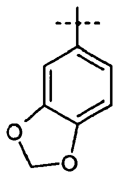
bilden,
sowie ihre Salze, Solvate und Solvate der Salze.

2. Verbindung der Formel (I) nach Anspruch 1, in welcher
R¹ Wasserstoff, Benzyloxy, Ethoxy, n-Propoxy oder eine Gruppe der Formel R⁸-C(=O)-NH- bedeutet, wobei Ethoxy in 2-Position und n-Propoxy in 2- oder 3-Position durch (C₁-C₄)-Alkoxy, Amino, Mono- oder Di-(C₁-C₄)-alkylamino, (C₃-C₆)-Cycloalkylamino, *N*-(C₁-C₆)-Cycloalkyl-*N*-(C₁-C₄)-alkylamino oder durch einen über ein N-Atom gebundenen 4- bis 7-gliedrigen Heterocyclus substituiert sein können,
wobei Mono- und Di-(C₁-C₄)-alkylamino ihrerseits durch Hydroxy, (C₁-C₄)-Alkoxy, Amino, Mono- oder Di-(C₁-C₄)-alkylamino substituiert sein können,
und worin
R⁸ für 5- oder 6-gliedriges Heteroaryl, das durch (C₁-C₄)-Alkyl oder Halogen substituiert sein kann, steht,
R² Wasserstoff, Ethoxy oder n-Propoxy bedeutet, wobei Ethoxy in 2-Position und n-Propoxy in 2- oder 3-Position durch (C₁-C₄)-Alkoxy, Amino, Mono- oder Di-(C₁-C₄)-alkylamino, (C₃-C₆)-Cycloalkylamino, *N*-(C₃-C₆)-Cycloalkyl-*N*-(C₁-C₄)-alkyl-amino oder durch einen über ein N-Atom gebundenen 4- bis 6-gliedrigen Heterocyclus substituiert sein können,
wobei Mono- und Di-(C₁-C₄)-alkylamino ihrerseits durch Hydroxy, (C₁-C₄)-Alkoxy, Amino, Mono- oder Di-(C₁-C₄)-alkylamino substituiert sein können,
wobei R¹ und R² jedoch nicht gleichzeitig für Wasserstoff stehen,
R³ Hydroxy oder Amino
und
R⁴ Wasserstoff bedeutet
oder
R³ und R⁴ gemeinsam mit dem Kohlenstoffatom, an das sie gebunden sind, eine Gruppe der Formel >C=O oder >C=N-OH bilden,
R⁵ Mono- oder Di-(C₁-C₄)-alkylaminocarbonyl bedeutet,
n für die Zahl 0, 1, 2 oder 3 steht,
R⁶ sich in meta- oder para-Position, relativ zur Verknüpfungsstelle des Phenylrings mit dem Tricyclus, befindet und Halogen, (C₁-C₄)-Alkyl, (C₁-C₄)-Alkoxy, (C₆-C₁₀)-Aryl, 5- bis 6-gliedriges Heteroaryl oder eine Gruppe der Formel -NR⁹R¹⁰ bedeutet,
wobei Aryl und Heteroaryl ihrerseits jeweils ein- bis zweifach, gleich oder verschieden, durch Halogen, Cyano, (C₁-C₄)-Alkylsulfonyl oder eine Gruppe der Formel -NR⁹R¹⁰ substituiert sein können,
und worin
R⁹ und R¹⁰ unabhängig voneinander für Wasserstoff, (C₁-C₄)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden,
und
R⁷ sich in meta- oder para-Position, relativ zur Verknüpfungsstelle des Phenylrings mit dem Tricyclus, und in ortho-Stellung zu R⁶ befindet und Wasserstoff, Halogen, (C₁-C₄)-Alkyl, (C₁-C₄)-Alkoxy oder eine Gruppe der Formel -NR¹¹R¹² bedeutet, worin
R¹¹ und R¹² unabhängig voneinander für Wasserstoff, (C₁-C₄)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden,
oder
R⁶ und R⁷ gemeinsam mit dem Phenylring, an den sie gebunden sind, eine Gruppe der Formel 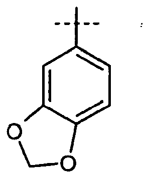
bilden,
sowie ihre Salze, Solvate und Solvate der Salze.

3. Verbindung der Formel (I) nach Ansprüchen 1 und 2, in welcher
R¹ und R² unabhängig voneinander Wasserstoff, Ethoxy oder n-Propoxy bedeuten, wobei Ethoxy in 2-Position und n-Propoxy in 3-Position durch Methoxy, Ethoxy, Amino, Methylamino, Ethylamino, Isopropylamino, Dimethylamino, Diethylamino, Cyclopropylamino, N-Cyclopropyl-N-methylamino, Azetidino oder Pyrrolidino substituiert sind,
wobei R¹ und R² jedoch nicht gleichzeitig für Wasserstoff stehen,
R³ Hydroxy oder Amino bedeutet,
R⁴ Wasserstoff bedeutet,
R⁵ Methylaminocarbonyl oder Dimethylaminocarbonyl bedeutet,
n für die Zahl 0 oder 1 steht,
R⁶ sich in para-Position, relativ zur Verknüpfungsstelle des Phenylrings mit dem Tricyclus, befindet und Fluor, Chlor, Brom, Methyl, Ethyl, Methoxy oder Ethoxy bedeutet,
und
R⁷ Wasserstoff bedeutet,
sowie ihre Salze, Solvate und Solvate der Salze.

4. Verbindung der Formel (I), in welcher
R¹ Wasserstoff, Benzyloxy, Ethoxy, n-Propoxy oder eine Gruppe der Formel R⁸-C(=O)-NH- bedeutet, wobei Ethoxy in 2-Position und n-Propoxy in 2- oder 3-Position durch (C₁-C₆)-Alkoxy, Amino, Mono- oder Di-(C₁-C₆)-alkylamino, (C₃-C₈)-Cycloalkylamino, *N*-(C₃-C₈)-Cycloalkyl-*N*-(C₁-C₆)-alkylamino oder durch einen über ein N-Atom gebundenen 4- bis 7-gliedrigen Heterocyclus substituiert sein können,
wobei Mono- und Di-(C₁-C₆)-alkylamino ihrerseits durch Hydroxy, (C₁-C₄)-Alkoxy, Amino, Mono- oder Di-(C₁-C₄)-alkylamino substituiert sein können,
und worin
R⁸ für 5- oder 6-gliedriges Heteroaryl, das durch (C₁-C₄)-Alkyl oder Halogen substituiert sein kann, steht,
R² Wasserstoff, Ethoxy oder n-Propoxy bedeutet, wobei Ethoxy in 2-Position und n-Propoxy in 2- oder 3-Position durch (C₁-C₆)-Alkoxy, Amino, Mono- oder Di-(C₁-C₆)-alkylamino, (C₃-C₈)-Cycloalkylamino, *N*-(C₃-C₈)-Cycloalkyl-*N*-(C₁-C₆)-alkyl-amino oder durch einen über ein N-Atom gebundenen 4- bis 7-gliedrigen Heterocyclus substituiert sein können,
wobei Mono- und Di-(C₁-C₆)-alkylamino ihrerseits durch Hydroxy, (C₁-C₄)-Alkoxy, Amino, Mono- oder Di-(C₁-C₄)-alkylamino substituiert sein können,
wobei R¹ und R² jedoch nicht gleichzeitig für Wasserstoff stehen,
R³ Hydroxy oder Amino
und
R⁴ Wasserstoff bedeutet
oder
R³ und R⁴ gemeinsam mit dem Kohlenstoffatom, an das sie gebunden sind, eine Gruppe der Formel >C=O oder >C=N-OH bilden,
R⁵ Wasserstoff bedeutet,
n für die Zahl 0, 1, 2 oder 3 steht,
R⁶ sich in meta- oder para-Position, relativ zur Verknüpfungsstelle des Phenylrings mit dem Tricyclus, befindet und (C₆-C₁₀)-Aryl, 5-bis 10-gliedriges Heteroaryl oder eine Gruppe der Formel -NR⁹R¹⁰ bedeutet,
wobei Aryl und Heteroaryl ihrerseits jeweils ein- bis zweifach, gleich oder verschieden, durch Halogen, Cyano, (C₁-C₄)-Alkylsulfonyl oder eine Gruppe der Formel -NR⁹R¹⁰ substituiert sein können,
und worin
R⁹ und R¹⁰ unabhängig voneinander für Wasserstoff, (C₁-C₆)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden,
und
R⁷ sich in meta- oder para-Position, relativ zur Verknüpfungsstelle des Phenylrings mit dem Tricyclus, und in ortho-Stellung zu R⁶ befindet und Wasserstoff, Halogen, (C₁-C₆)-Alkyl, (C₁-C₆)-Alkoxy oder eine Gruppe der Formel -NR¹¹R¹² bedeutet, worin
R¹¹ und R¹² unabhängig voneinander für Wasserstoff, (C₁-C₆)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden,
oder
R⁶ und R⁷ gemeinsam mit dem Phenylring, an den sie gebunden sind, eine Gruppe der Formel 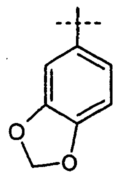
bilden,
sowie ihre Salze, Solvate und Solvate der Salze.

5. Verbindung der Formel (I) nach Anspruch 4, in welcher
R¹ Wasserstoff, Benzyloxy, Ethoxy, n-Propoxy oder eine Gruppe der Formel R⁸-C(=O)-NH- bedeutet, wobei Ethoxy in 2-Position und n-Propoxy in 2- oder 3-Position durch (C₁-C₄)-Alkoxy, Amino, Mono- oder Di-(C₁-C₄)-alkylamino, (C₃-C₆)-Cycloalkylamino, *N*-(C₁-C₆)-Cycloalkyl-*N*-(C₁-C₄)-alkylamino oder durch einen über ein N-Atom gebundenen 4- bis 7-gliedrigen Heterocyclus substituiert sein können,
wobei Mono- und Di-(C₁-C₄)-alkylamino ihrerseits durch Hydroxy, (C₁-C₄)-Alkoxy, Amino, Mono- oder Di-(C₁-C₄)-alkylamino substituiert sein können,
und worin
R⁸ für 5- oder 6-gliedriges Heteroaryl, das durch (C₁-C₄)-Alkyl oder Halogen substituiert sein kann, steht,
R² Wasserstoff, Ethoxy oder n-Propoxy bedeutet, wobei Ethoxy in 2-Position und n-Propoxy in 2- oder 3-Position durch (C₁-C₄)-Alkoxy, Amino, Mono- oder Di-(C₁-C₄)-alkylamino, (C₃-C₆)-Cycloalkylamino, *N*-(C₃-C₆)-Cycloalkyl-*N*-(C₁-C₄)-alkyl-amino oder durch einen über ein N-Atom gebundenen 4- bis 7-gliedrigen Heterocyclus substituiert sein können,
wobei Mono- und Di-(C₁-C₄)-alkylamino ihrerseits durch Hydroxy, (C₁-C₄)-Alkoxy, Amino, Mono- oder Di-(C₁-C₄)-alkylamino substituiert sein können,
wobei R¹ und R² jedoch nicht gleichzeitig für Wasserstoff stehen,
R³ Hydroxy oder Amino
und
R⁴ Wasserstoff bedeutet
oder
R³ und R⁴ gemeinsam mit dem Kohlenstoffatom, an das sie gebunden sind, eine Gruppe der Formel >C=O oder >C=N-OH bilden,
R⁵ Wasserstoff bedeutet,
n für die Zahl 0, 1, 2 oder 3 steht,
R⁶ sich in meta- oder para-Position, relativ zur Verknüpfungsstelle des.Phenylrings mit dem Tricyclus, befindet und (C₆-C₁₀)-Aryl, 5- bis 6-gliedriges Heteroaryl oder eine Gruppe der Formel -NR⁹R¹⁰ bedeutet,
wobei Aryl und Heteroaryl ihrerseits jeweils ein- bis zweifach, gleich oder verschieden, durch Halogen, Cyano, (C₁-C₄)-Alkylsulfonyl oder eine Gruppe der Formel -NR⁹R¹⁰ substituiert sein können,
und worin
R⁹ und R¹⁰ unabhängig voneinander für Wasserstoff, (C₁-C₄)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden,
und
R⁷ sich in meta- oder para-Position, relativ zur Verknüpfungsstelle des Phenylrings mit dem Tricyclus, und in ortho-Stellung zu R⁶ befindet und Wasserstoff, Halogen, (C₁-C₄)-Alkyl, (C₁-C₄)-Alkoxy oder eine Gruppe der Formel -NR¹¹R¹² bedeutet, worin
R¹¹ und R¹² unabhängig voneinander für Wasserstoff, (C₁-C₄)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden,
oder
R⁶ und R⁷ gemeinsam mit dem Phenylring, an den sie gebunden sind, eine Gruppe der Formel 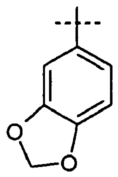
bilden,
sowie ihre Salze, Solvate und Solvate der Salze.

6. Verbindung der Formel (I) nach Ansprüchen 4 und 5, in welcher
R¹ Wasserstoff, Ethoxy, n-Propoxy oder eine Gruppe der Formel R⁸-C(=O)-NH-bedeutet, wobei Ethoxy in 2-Position und n-Propoxy in 3-Position durch Methoxy, Ethoxy, Amino, Methylamino, Ethylamino, Isopropylamino, Dimethylamino, Diethylamino, Cyclopropylamino, *N*-Cyclopropyl-*N*-methylamino, Azetidino oder Pyrrolidino substituiert sein können, worin
R⁸ für Pyridyl, Pyrrolyl, Imidazolyl, Pyrazolyl, Triazolyl oder Thiadiazolyl, die jeweils durch Methyl, Ethyl, Fluor oder Chlor substituiert sein können, steht,
R² Wasserstoff, Ethoxy oder n-Propoxy bedeutet, wobei Ethoxy in 2-Position und n-Propoxy in 3-Position durch Methoxy, Ethoxy, Amino, Methylamino, Ethylamino, Isopropylamino, Dimethylamino, Diethylamino, Cyclopropylamino, *N*-Cyclopropyl-N-methylamino, Azetidino oder Pyrrolidino substituiert sein können,
wobei R¹ und R² jedoch nicht gleichzeitig für Wasserstoff stehen,
R³ Hydroxy oder Amino bedeutet,
R⁴ Wasserstoff bedeutet,
R⁵ Wasserstoff bedeutet,
n für die Zahl 0 oder 1 steht,
R⁶ sich in meta- oder para-Position, relativ zur Verknüpfungsstelle des Phenylrings mit dem Tricyclus, befindet und Phenyl, Thienyl, Indolyl, Chinoxalinyl oder eine Gruppe der Formel -NR⁹R¹⁰ bedeutet,
wobei Phenyl, Thienyl und Indolyl ihrerseits jeweils ein- bis zweifach, gleich oder verschieden, durch Fluor, Chlor, Brom, Cyano oder Amino substituiert sein können,
und worin
R⁹ und R¹⁰ unabhängig voneinander für Wasserstoff, Methyl, Ethyl, Benzyl oder Pyridylmethyl stehen oder gemeinsam mit dem Stickstoffatom, an das sie gebunden sind, einen Pyrrolidino-Ring bilden,
und
R⁷ Wasserstoff bedeutet,
sowie ihre Salze, Solvate und Solvate der Salze.

7. Verbindung der Formel (I), in welcher
R¹ Wasserstoff, Benzyloxy, Ethoxy, n-Propoxy oder eine Gruppe der Formel R⁸-C(=O)-NH- bedeutet, wobei Ethoxy in 2-Position und n-Propoxy in 2- oder 3-Position durch (C₁-C₆)-Alkoxy, Amino, Mono- oder Di-(C₁-C₆)-alkylamino, (C₃-C₈)-Cycloalkylamino, *N*-(C₃-C₈)-Cycloalkyl-*N*-(C₁-C₆)-alkylamino oder durch einen über ein N-Atom gebundenen 4- bis 7-gliedrigen Heterocyclus substituiert sein können,
wobei Mono- und Di-(C₁-C₆)-alkylamino ihrerseits durch Hydroxy, (C₁-C₄)-Alkoxy, Amino, Mono- oder Di-(C₁-C₄)-alkylamino substituiert sein können,
und worin
R⁸ für 5- oder 6-gliedriges Heteroaryl, das durch (C₁-C₄)-Alkyl oder Halogen substituiert sein kann, steht,
R² Wasserstoff, Ethoxy oder n-Propoxy bedeutet, wobei Ethoxy in 2-Position und n-Propoxy in 2- oder 3-Position durch (C₁-C₆)-Alkoxy, Amino, Mono- oder Di-(C₁-C₆)-alkylamino, (C₃-C₈)-Cycloalkylamino, *N*-(C₃-C₈)-Cycloalkyl-*N*-(C₁-C₆)-alkyl-amino oder durch einen über ein N-Atom gebundenen 4- bis 7-gliedrigen Heterocyclus substituiert sein können,
wobei Mono- und Di-(C₁-C₆)-alkylamino ihrerseits durch Hydroxy, (C₁-C₄)-Alkoxy, Amino, Mono- oder Di-(C₁-C₄)-alkylamino substituiert sein können,
wobei R¹ oder R² jedoch nicht beide gleichzeitig für Wasserstoff stehen,
R³ Amino
und
R⁴ Wasserstoff bedeutet
oder
R³ und R⁴ gemeinsam mit dem Kohlenstoffatom, an das sie gebunden sind, eine Gruppe der Formel >C=N-OH bilden,
R⁵ Wasserstoff bedeutet,
n für die Zahl 0, 1, 2 oder 3 steht,
R⁶ sich in meta- oder para-Position, relativ zur Verknüpfungsstelle des Phenylrings mit dem Tricyclus, befindet und Halogen, (C₁-C₆)-Alkyl oder (C₁-C₆)-Alkoxy bedeutet,
und
R⁷ sich in meta- oder para-Position, relativ zur Verknüpfungsstelle des Phenylrings mit dem Tricyclus, und in ortho-Stellung zu R⁶ befindet und Wasserstoff, Halogen, (C₁-C₆)-Alkyl, (C₁-C₆)-Alkoxy oder eine Gruppe der Formel -NR¹¹R¹² bedeutet, worin
R¹¹ und R¹² unabhängig voneinander für Wasserstoff, (C₁-C₆)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden,
oder
R⁶ und R⁷ gemeinsam mit dem Phenylring, an den sie gebunden sind, eine Gruppe der Formel 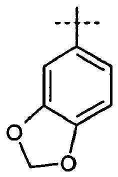
bilden,
sowie ihre Salze, Solvate und Solvate der Salze.

8. Verbindung der Formel (I) nach Anspruch 7, in welcher
R¹ Wasserstoff, Benzyloxy, Ethoxy, n-Propoxy oder eine Gruppe der Formel R⁸-C(=O)-NH- bedeutet, wobei Ethoxy in 2-Position und n-Propoxy in 2- oder 3-Position durch (C₁-C₄)-Alkoxy, Amino, Mono- oder Di-(C₁-C₄)-alkylamino, (C₃-C₆)-Cycloalkylamino, *N*-(C₃-C₆)-Cycloalkyl-*N*-(C₁-C₄)-alkylamino oder durch einen über ein N-Atom gebundenen 4- bis 7-gliedrigen Heterocyclus substituiert sein können,
wobei Mono- und Di-(C₁-C₄)-alkylamino ihrerseits durch Hydroxy, (C₁-C₄)-Alkoxy, Amino, Mono- oder Di-(C₁-C₄)-alkylamino substituiert sein können,
und worin
R⁸ für 5- oder 6-gliedriges Heteroaryl, das durch (C₁-C₄)-Alkyl oder Halogen substituiert sein kann, steht,
R² Wasserstoff, Ethoxy oder n-Propoxy bedeutet, wobei Ethoxy in 2-Position und n-Propoxy in 2- oder 3-Position durch (C₁-C₄)-Alkoxy, Amino, Mono- oder Di-(C₁-C₄)-alkylamino, (C₃-C₆)-Cycloalkylamino, *N*-(C₃-C₆)-Cycloalkyl-*N*-(C₁-C₄)-alkyl-amino oder durch einen über ein N-Atom gebundenen 4- bis 7-gliedrigen Heterocyclus substituiert sein können, wobei Mono- und Di-(C₁-C₄)-alkylamino ihrerseits durch Hydroxy, (C₁-C₄)-Alkoxy, Amino, Mono- oder Di-(C₁-C₄)-alkylamino substituiert sein können,
wobei R¹ oder R² jedoch nicht beide gleichzeitig für Wasserstoff stehen,
R³ Amino
und
R⁴ Wasserstoff bedeutet
oder
R³ und R⁴ gemeinsam mit dem Kohlenstoffatom, an das sie gebunden sind, eine Gruppe der Formel >C=N-OH bilden,
R⁵ Wasserstoff bedeutet,
n für die Zahl 0, 1, 2 oder 3 steht,
R⁶ sich in meta- oder para-Position, relativ zur Verknüpfungsstelle des Phenylrings mit dem Tricyclus, befindet und Halogen, (C₁-C₄)-Alkyl oder (C₁-C₄)-Alkoxy bedeutet,
und
R⁷ sich in meta- oder para-Position, relativ zur Verknüpfungsstelle des Phenylrings mit dem Tricyclus, und in ortho-Stellung zu R⁶ befindet und Wasserstoff, Halogen, (C₁-C₄)-Alkyl, (C₁-C₄)-Alkoxy oder eine Gruppe der Formel -NR¹¹R¹² bedeutet, worin
R¹¹ und R¹² unabhängig voneinander für Wasserstoff, (C₁-C₄)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden,
oder
R⁶ und R⁷ gemeinsam mit dem Phenylring, an den sie gebunden sind, eine Gruppe der Formel 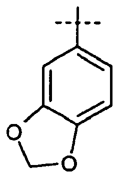
bilden,
sowie ihre Salze, Solvate und Solvate der Salze.

9. Verbindung der Formel (I) nach Ansprüchen 7 und 8, in welcher
R¹ Wasserstoff, Ethoxy, n-Propoxy oder eine Gruppe der Formel R⁸-C(=O)-NH-bedeutet, wobei Ethoxy in 2-Position und n-Propoxy in 3-Position durch Methoxy, Ethoxy, Amino, Methylamino, Ethylamino, Isopropylamino, Dimethylamino, Diethylamino, Cyclopropylamino, *N*-Cyclopropyl-*N*-methylamino, Azetidino oder Pyrrolidino substituiert sein können, worin
R⁸ für Pyridyl, Pyrrolyl, Imidazolyl, Pyrazolyl, Triazolyl oder Thiadiazolyl, die jeweils durch Methyl, Ethyl, Fluor oder Chlor substituiert sein können, steht,
R² Wasserstoff, Ethoxy oder n-Propoxy bedeutet, wobei Ethoxy in 2-Position und n-Propoxy in 3-Position durch Methoxy, Ethoxy, Amino, Methylamino, Ethylamino, Isopropylamino, Dimethylamino, Diethylamino, Cyclopropylamino, *N*-Cyclopropyl-*N*-methylamino, Azetidino oder Pyrrolidino substituiert sein können,
wobei R¹ und R² jedoch nicht gleichzeitig für Wasserstoff stehen,
R³ Amino bedeutet,
R⁴ Wasserstoff bedeutet,
R⁵ Wasserstoff bedeutet,
n für die Zahl 0 oder 1 steht,
R⁶ sich in meta- oder para-Position, relativ zur Verknüpfungsstelle des Phenylrings mit dem Tricyclus, befindet und Fluor, Chlor, Brom, Methyl, Ethyl, Methoxy oder Ethoxy bedeutet,
und
R⁷ sich in meta- oder para-Position, relativ zur Verknüpfungsstelle des Phenylrings mit dem Tricyclus, und in ortho-Stellung zu R⁶ befindet und Wasserstoff, Fluor, Chlor, Brom, Methyl, Ethyl, Methoxy, Ethoxy oder eine Gruppe der Formel -NR¹¹R¹² bedeutet, worin
R¹¹ und R¹² unabhängig voneinander für Wasserstoff, Methyl, Ethyl, Benzyl oder Pyridylmethyl stehen oder gemeinsam mit dem Stickstoffatom, an das sie gebunden sind, einen Pyrrolidino- oder Piperidino-Ring bilden,
oder
R⁶ und R⁷ gemeinsam mit dem Phenylring, an den sie gebunden sind, eine Gruppe der Formel 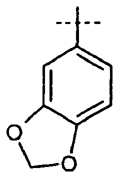
bilden,
sowie ihre Salze, Solvate und Solvate der Salze.

10. Verbindung der Formel (I), in welcher
R¹ Wasserstoff, Benzyloxy, Ethoxy, n-Propoxy oder eine Gruppe der Formel R⁸-C(=O)-NH- bedeutet, wobei Ethoxy in 2-Position und n-Propoxy in 2- oder 3-Position durch (C₁-C₆)-Alkoxy, Amino, Mono- oder Di-(C₁-C₆)-alkylamino, (C₃-C₈)-Cycloalkylamino, *N*-(C₃-C₈)-Cycloalkyl-*N*-(C₁-C₆)-alkylamino oder durch einen über ein N-Atom gebundenen 4- bis 7-gliedrigen Heterocyclus substituiert sein können,
wobei Mono- und Di-(C₁-C₆)-alkylamino ihrerseits durch Hydroxy, (C₁-C₄)-Alkoxy, Amino, Mono- oder Di-(C₁-C₄)-alkylamino substituiert sein können,
und worin
R⁸ für 5- oder 6-gliedriges Heteroaryl, das durch (C₁-C₄)-Alkyl oder Halogen substituiert sein kann, steht,
R² Wasserstoff, Ethoxy oder n-Propoxy bedeutet, wobei Ethoxy in 2-Position und n-Propoxy in 2- oder 3-Position durch (C₁-C₆)-Alkoxy, Amino, Mono- oder Di-(C₁-C₆)-alkylamino, (C₃-C₈)-Cycloalkylamino, *N*-(C₃-C₈)-Cycloalkyl-*N*-(C₁-C₆)-alkyl-amino oder durch einen über ein N-Atom gebundenen 4- bis 7-gliedrigen Heterocyclus substituiert sein können,
wobei Mono- und Di-(C₁-C₆)-alkylamino ihrerseits durch Hydroxy, (C₁-C₄)-Alkoxy, Amino, Mono- oder Di-(C₁-C₄)-alkylamino substituiert sein können,
wobei entweder R¹ oder R² für Wasserstoff steht, jedoch nicht beide gleichzeitig Wasserstoff bedeuten,
R³ Hydroxy
und
R⁴ Wasserstoff bedeutet
oder
R³ und R⁴ gemeinsam mit dem Kohlenstoffatom, an das sie gebunden sind, eine Gruppe der Formel >C=O bilden,
R⁵ Wasserstoff bedeutet,
n für die Zahl 0, 1, 2 oder 3 steht,
R⁶ sich in meta- oder para-Position, relativ zur Verknüpfungsstelle des Phenylrings mit dem Tricyclus, befindet und Halogen, (C₁-C₆)-Alkyl oder (C₁-C₆)-Alkoxy bedeutet,
und
R⁷ sich in meta- oder para-Position, relativ zur Verknüpfungsstelle des Phenylrings mit dem Tricyclus, und in ortho-Stellung zu R⁶ befindet und Wasserstoff, Halogen, (C₁-C₆)-Alkyl, (C₁-C₆)-Alkoxy oder eine Gruppe der Formel -NR¹¹R¹² bedeutet, worin
R¹¹ und R¹² unabhängig voneinander für Wasserstoff, (C₁-C₆)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden,
oder
R⁶ und R⁷ gemeinsam mit dem Phenylring, an den sie gebunden sind, eine Gruppe der Formel 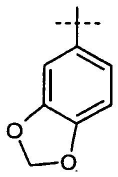
bilden,
sowie ihre Salze, Solvate und Solvate der Salze.

11. Verbindung der Formel (I) nach Anspruch 10, in welcher
R¹ Wasserstoff, Benzyloxy, Ethoxy, n-Propoxy oder eine Gruppe der Formel R⁸-C(=O)-NH- bedeutet, wobei Ethoxy in 2-Position und n-Propoxy in 2- oder 3-Position durch (C₁-C₄)-Alkoxy, Amino, Mono- oder Di-(C₁-C₄)-alkylamino, (C₃-C₆)-Cycloalkylamino, *N*-(C₃-C₆)-Cycloalkyl-*N*-(C₁-C₄)-alkylamino oder durch einen über ein N-Atom gebundenen 4- bis 7-gliedrigen Heterocyclus substituiert sein können,
wobei Mono- und Di-(C₁-C₄)-alkylamino ihrerseits durch Hydroxy, (C₁-C₄)-Alkoxy, Amino, Mono- oder Di-(C₁-C₄)-alkylamino substituiert sein können,
und worin
R⁸ für 5- oder 6-gliedriges Heteroaryl, das durch (C₁-C₄)-Alkyl oder Halogen substituiert sein kann, steht,
R² Wasserstoff, Ethoxy oder n-Propoxy bedeutet, wobei Ethoxy in 2-Position und n-Propoxy in 2- oder 3-Position durch (C₁-C₄)-Alkoxy, Amino, Mono- oder Di-(C₁-C₄)-alkylamino, (C₃-C₆)-Cycloalkylamino, *N*-(C₃-C₆)-Cycloalkyl-*N*-(C₁-C₄)-alkyl-amino oder durch einen über ein N-Atom gebundenen 4- bis 7-gliedrigen Heterocyclus substituiert sein können,
wobei Mono- und Di-(C₁-C₄)-alkylamino ihrerseits durch Hydroxy, (C₁-C₄)-Alkoxy, Amino, Mono- oder Di-(C₁-C₄)-alkylamino substituiert sein können,
wobei entweder R¹ oder R² für Wasserstoff steht, jedoch nicht beide gleichzeitig Wasserstoff bedeuten,
R³ Hydroxy
und
R⁴ Wasserstoff bedeutet
oder
R³ und R⁴ gemeinsam mit dem Kohlenstoffatom, an das sie gebunden sind, eine Gruppe der Formel >C=O bilden,
R⁵ Wasserstoff bedeutet,
n für die Zahl 0, 1, 2 oder 3 steht,
R⁶ sich in meta- oder para-Position, relativ zur Verknüpfungsstelle des Phenylrings mit dem Tricyclus, befindet und Halogen, (C₁-C₄)-Alkyl oder (C₁-C₄)-Alkoxy bedeutet,
und
R⁷ sich in meta- oder para-Position, relativ zur Verknüpfungsstelle des Phenylrings mit dem Tricyclus, und in ortho-Stellung zu R⁶ befindet und Wasserstoff, Halogen, (C₁-C₄)-Alkyl, (C₁-C₄)-Alkoxy oder eine Gruppe der Formel -NR¹¹R¹² bedeutet, worin
R¹¹ und R¹² unabhängig voneinander für Wasserstoff, (C₁-C₄)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden,
oder
R⁶ und R⁷ gemeinsam mit dem Phenylring, an den sie gebunden sind, eine Gruppe der Formel 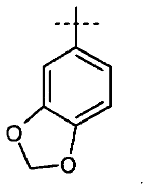
bilden,
sowie ihre Salze, Solvate und Solvate der Salze.

12. Verbindung der Formel (I) nach Ansprüchen 10 und 11, in welcher
R¹ Wasserstoff, Ethoxy, n-Propoxy oder eine Gruppe der Formel R⁸-C(=O)-NH-bedeutet, wobei Ethoxy in 2-Position und n-Propoxy in 3-Position durch Methoxy, Ethoxy, Amino, Methylamino, Ethylamino, Isopropylamino, Dimethylamino, Diethylamino, Cyclopropylamino, *N*-Cyclopropyl-*N*-methylamino, Azetidino oder Pyrrolidino substituiert sein können, worin
R⁸ für Pyridyl, Pyrrolyl, Imidazolyl, Pyrazolyl, Triazolyl oder Thiadiazolyl, die jeweils durch Methyl, Ethyl, Fluor oder Chlor substituiert sein können, steht,
R² Wasserstoff, Ethoxy oder n-Propoxy bedeutet, wobei Ethoxy in 2-Position und n-Propoxy in 3-Position durch Methoxy, Ethoxy, Amino, Methylamino, Ethylamino, Isopropylamino, Dimethylamino, Diethylamino, Cyclopropylamino, *N*-Cyclopropyl-*N*-methylamino, Azetidino oder Pyrrolidino substituiert sein können,
wobei entweder R¹ oder R² für Wasserstoff steht, jedoch nicht beide gleichzeitig Wasserstoff bedeuten,
R³ Hydroxy
und
R⁴ Wasserstoff bedeutet
oder
R³ und R⁴ gemeinsam mit dem Kohlenstoffatom, an das sie gebunden sind, eine Gruppe der Formel >C=O bilden,
R⁵ Wasserstoff bedeutet,
n für die Zahl 0 oder 1 steht,
R⁶ sich in meta- oder para-Position, relativ zur Verknüpfungsstelle des Phenylrings mit dem Tricyclus, befindet und Fluor, Chlor, Brom, Methyl, Ethyl, Methoxy oder Ethoxy bedeutet,
und
R⁷ sich in meta- oder para-Position, relativ zur Verknüpfungsstelle des Phenylrings mit dem Tricyclus, und in ortho-Stellung zu R⁶ befindet und Wasserstoff, Fluor, Chlor, Brom, Methyl, Ethyl, Methoxy, Ethoxy oder eine Gruppe der Formel -NR¹¹R¹² bedeutet, worin
R¹¹ und R¹² unabhängig voneinander für Wasserstoff, Methyl, Ethyl, Benzyl oder Pyridylmethyl stehen oder gemeinsam mit dem Stickstoffatom, an das sie gebunden sind, einen Pyrrolidino- oder Piperidino-Ring bilden,
oder
R⁶ und R⁷ gemeinsam mit dem Phenylring, an den sie gebunden sind, eine Gruppe der Formel 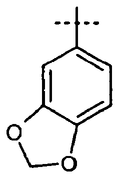
bilden,
sowie ihre Salze, Solvate und Solvate der Salze.

13. Verbindung der Formel (I), in welcher
R¹ Ethoxy, n-Propoxy oder eine Gruppe der Formel R⁸-C(=O)-NH- bedeutet, wobei Ethoxy in 2-Position und n-Propoxy in 2- oder 3-Position durch (C₁-C₆)-Alkoxy, Amino, Mono- oder Di-(C₁-C₆)-alkylamino, (C₃-C₈)-Cycloalkylamino, *N*-(C₃-C₈)-Cycloalkyl-*N*-(C₁-C₆)-alkylamino oder durch einen über ein N-Atom gebundenen 4- bis 7-gliedrigen Heterocyclus substituiert sind,
wobei Mono- und Di-(C₁-C₆)-alkylamino ihrerseits durch Hydroxy, (C₁-C₄)-Alkoxy, Amino, Mono- oder Di-(C₁-C₄)-alkylamino substituiert sein können,
und worin
R⁸ für 5- oder 6-gliedriges Heteroaryl, das durch (C₁-C₄)-Alkyl oder Halogen substituiert sein kann, steht,
R² Ethoxy oder n-Propoxy bedeutet, wobei Ethoxy in 2-Position und n-Propoxy in 2-oder 3-Position durch (C₁-C₆)-Alkoxy, Amino, Mono- oder Di-(C₁-C₆)-alkylamino, (C₃-C₈)-Cycloalkylamino, *N*-(C₃-C₈)-Cycloalkyl-*N*-(C₁-C₆)-alkylamino oder durch einen über ein N-Atom gebundenen 4- bis 7-gliedrigen Heterocyclus substituiert sind,
wobei Mono- und Di-(C₁-C₆)-alkylamino ihrerseits durch Hydroxy, (C₁-C₄)-Alkoxy, Amino, Mono- oder Di-(C₁-C₄)-alkylamino substituiert sein können,
R³ Hydroxy
und
R⁴ Wasserstoff bedeutet
oder
R³ und R⁴ gemeinsam mit dem Kohlenstoffatom, an das sie gebunden sind, eine Gruppe der Formel >C=O bilden,
R⁵ Wasserstoff bedeutet,
n für die Zahl 0, 1, 2 oder 3 steht,
R⁶ sich in meta- oder para-Position, relativ zur Verknüpfungsstelle des Phenylrings mit dem Tricyclus, befindet und Halogen, (C₁-C₆)-Alkyl oder (C₁-C₆)-Alkoxy bedeutet,
und
R⁷ sich in meta- oder para-Position, relativ zur Verknüpfungsstelle des Phenylrings mit dem Tricyclus, und in ortho-Stellung zu R⁶ befindet und Wasserstoff, Halogen, (C₁-C₆)-Alkyl, (C₁-C₆)-Alkoxy oder eine Gruppe der Formel -NR¹¹R¹² bedeutet, worin
R¹¹ und R¹² unabhängig voneinander für Wasserstoff, (C₁-C₆)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden,
oder
R⁶ und R⁷ gemeinsam mit dem Phenylring, an den sie gebunden sind, eine Gruppe der Formel 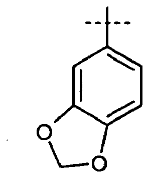
bilden,
sowie ihre Salze, Solvate und Solvate der Salze.

14. Verbindung der Formel (I) nach Anspruch 13, in welcher
R¹ Ethoxy, n-Propoxy oder eine Gruppe der Formel R⁸-C(=O)-NH- bedeutet, wobei Ethoxy in 2-Position und n-Propoxy in 2- oder 3-Position durch (C₁-C₄)-Alkoxy, Amino, Mono- oder Di-(C₁-C₄)-alkylamino, (C₃-C₆)-Cycloalkylamino, N-(C₃-C₆)-Cycloalkyl-N-(C₁-C₄)-alkylamino oder durch einen über ein N-Atom gebundenen 4- bis 7-gliedrigen Heterocyclus substituiert sind,
wobei Mono- und Di-(C₁-C₄)-alkylamino ihrerseits durch Hydroxy, (C₁-C₄)-Alkoxy, Amino, Mono- oder Di-(C₁-C₄)-alkylamino substituiert sein können,
und worin
R⁸ für 5- oder 6-gliedriges Heteroaryl, das durch (C₁-C₄)-Alkyl oder Halogen substituiert sein kann, steht,
R² Ethoxy oder n-Propoxy bedeutet, wobei Ethoxy in 2-Position und n-Propoxy in 2-oder 3-Position durch (C₁-C₄)-Alkoxy, Amino, Mono- oder Di-(C₁-C₄)-alkylamino, (C₃-C₆)-Cycloalkylamino, *N*-(C₃-C₆)-Cycloalkyl-*N*-(C₁-C₄)-alkylamino oder durch einen über ein N-Atom gebundenen 4- bis 7-gliedrigen Heterocyclus substituiert sind,
wobei Mono- und Di-(C₁-C₄)-alkylamino ihrerseits durch Hydroxy, (C₁-C₄)-Alkoxy, Amino, Mono- oder Di-(C₁-C₄)-alkylamino substituiert sein können,
R³ Hydroxy
und
R⁴ Wasserstoff bedeutet
oder
R³ und R⁴ gemeinsam mit dem Kohlenstoffatom, an das sie gebunden sind, eine Gruppe der Formel >C=O bilden,
R⁵ Wasserstoff bedeutet,
n für die Zahl 0, 1, 2 oder 3 steht,
R⁶ sich in meta- oder para-Position, relativ zur Verknüpfungsstelle des Phenylrings mit dem Tricyclus, befindet und Halogen, (C₁-C₄)-Alkyl oder (C₁-C₄)-Alkoxy bedeutet,
und
R⁷ sich in meta- oder para-Position, relativ zur Verknüpfungsstelle des Phenylrings mit dem Tricyclus, und in ortho-Stellung zu R⁶ befindet und Wasserstoff, Halogen, (C₁-C₄)-Alkyl, (C₁-C₄)-Alkoxy oder eine Gruppe der Formel -NR¹¹R¹² bedeutet, worin
R¹¹ und R¹² unabhängig voneinander für Wasserstoff, (C₁-C₄)-Alkyl, Phenyl, Benzyl oder Pyridylmethyl stehen oder gemeinsam mit dem Stickstoffatom, an das sie gebunden sind, einen 4- bis 7-gliedrigen Heterocyclus bilden,
oder
R⁶ und R⁷ gemeinsam mit dem Phenylring, an den sie gebunden sind, eine Gruppe der Formel 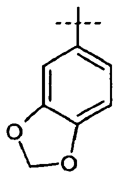
bilden,
sowie ihre Salze, Solvate und Solvate der Salze.

15. Verbindung der Formel (I) nach Ansprüchen 13 und 14, in welcher
R¹ Ethoxy, n-Propoxy oder eine Gruppe der Formel R⁸-C(=O)-NH- bedeutet, wobei Ethoxy in 2-Position und n-Propoxy in 3-Position durch Methoxy, Ethoxy, Amino, Methylamine, Ethylamino, Isopropylamino, Dimethylamino, Diethylamino, Cyclopropylamino, N-Cyclopropyl-N-methylamino, Azetidino oder Pyrrolidino substituiert sind, worin
R⁸ für Pyridyl, Pyrrolyl, Imidazolyl, Pyrazolyl, Triazolyl oder Thiadiazolyl, die jeweils durch Methyl, Ethyl, Fluor oder Chlor substituiert sein können, steht,
R² Ethoxy oder n-Propoxy bedeutet, wobei Ethoxy in 2-Position und n-Propoxy in 3-Position durch Methoxy, Ethoxy, Amino, Methylamino, Ethylamino, Isopropylamino, Dimethylamino, Diethylamino, Cyclopropylamino, N-Cyclopropyl-N methylamino, Azetidino oder Pyrrolidino substituiert sind,
R³ Hydroxy
und
R⁴ Wasserstoff bedeutet
oder
R³ und R⁴ gemeinsam mit dem Kohlenstoffatom, an das sie gebunden sind, eine Gruppe der Formel >C=O bilden,
R⁵ Wasserstoff bedeutet,
n für die Zahl 0 oder 1 steht,
R⁶ sich in meta- oder para-Position, relativ zur Verknüpfungsstelle des Phenylrings mit dem Tricyclus, befindet und Fluor, Chlor, Brom, Methyl, Ethyl, Methoxy oder Ethoxy bedeutet,
und
R⁷ sich in meta- oder para-Position, relativ zur Verknüpfüngsstelle des Phenylrings mit dem Tricyclus, und in ortho-Stellung zu R⁶ befindet und Wasserstoff, Fluor, Chlor, Brom, Methyl, Ethyl, Methoxy, Ethoxy oder eine Gruppe der Formel -NR¹¹R¹² bedeutet, worin
R¹¹ und R¹² unabhängig voneinander für Wasserstoff, Methyl, Ethyl, Benzyl oder Pyridylmethyl stehen oder gemeinsam mit dem Stickstoffatom, an das sie gebunden sind, einen Pyrrolidino- oder Piperidino-Ring bilden,
oder
R⁶ und R⁷ gemeinsam mit dem Phenylring, an den sie gebunden sind, eine Gruppe der Formel 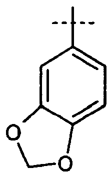
bilden,
sowie ihre Salze, Solvate und Solvate der Salze.

16. Verfahren zur Herstellung einer Verbindung der Formel (VI) 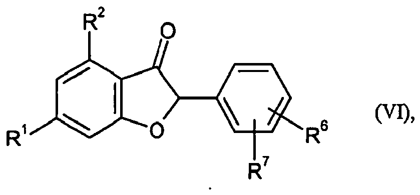 in welcher R¹, R², R⁶ und R⁷ jeweils die in den Ansprüchen 1 bis 15 angegebene Bedeutung haben, **dadurch gekennzeichnet, dass** man entweder
[A] Verbindungen der Formel (II) 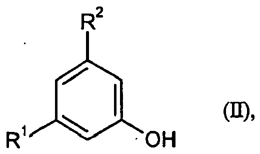 in welcher R¹ und R² jeweils die in den Ansprüchen 1 bis 15 angegebene Bedeutung haben,
in einem inerten Lösungsmittel in Gegenwart einer Base mit einer Verbindung der Formel (III) 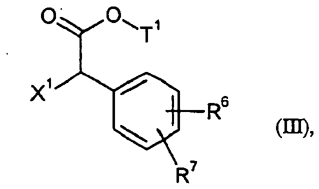 in welcher R⁶ und R⁷ jeweils die in den Ansprüchen 1 bis 15 angegebene Bedeutung haben,
X¹ für eine geeignete Fluchtgruppe wie beispielsweise Halogen, Mesylat, Tosylat oder Triflat
und
T¹ für (C₁-C₄)-Alkyl steht,
zu Verbindungen der Formel (IV) 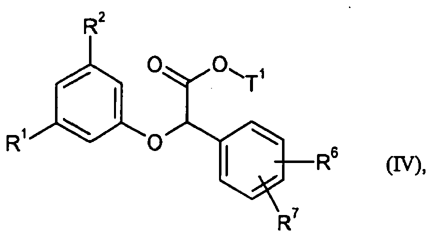 in welcher R¹, R², R⁶, R⁷ und T¹ jeweils die in den Ansprüchen 1 bis 15 angegebene Bedeutung haben,
umsetzt, anschließend durch basische oder saure Hydrolyse in Carbonsäuren der Formel (V) 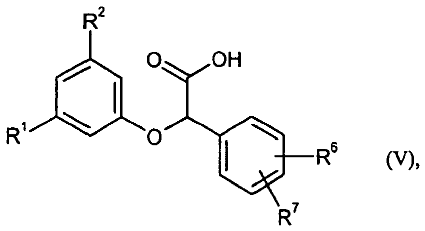 in welcher R¹, R², R⁶ und R⁷ jeweils die in den Ansprüchen 1 bis 15 angegebene Bedeutung haben,
überführt, diese dann nach Aktivierung mit Phosphorylchlorid in Gegenwart einer LewisSäure zu Verbindungen der Formel (VI) 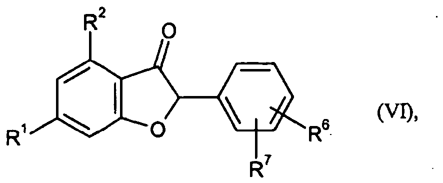 in welcher R¹, R², R⁶ und R⁷ jeweils die in den Ansprüchen 1 bis 15 angegebene Bedeutung haben,
cyclisiert,
oder
[B] Verbindungen der Formel (VII) 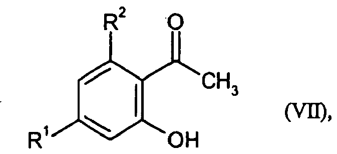
in welcher R¹ und R² jeweils die in den Ansprüchen 1 bis 15 angegebene Bedeutung haben,
zunächst nach üblichen Methoden in Phenacylbromide der Formel (VIII) 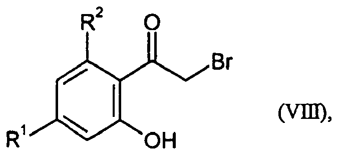 in welcher R¹ und R² jeweils die in den Ansprüchen 1 bis 15 angegebene Bedeutung haben,
überführt und diese dann in Gegenwart einer Base zu Verbindungen der Formel (IX) 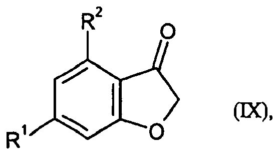 in welcher R¹ und R² jeweils die in den Ansprüchen 1 bis 15 angegebene Bedeutung haben,
cyclisiert, anschließend in einem inerten Lösungsmittel zu Verbindungen der Formel (X) 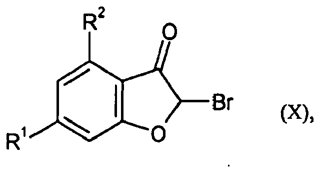 in welcher R¹ und R² jeweils die in den Ansprüchen 1 bis 15 angegebene Bedeutung haben,
bromiert und nach üblichen Methoden in Silylenolether der Formel (XI) 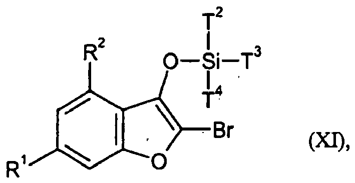 in welcher R¹ und R² jeweils die in den Ansprüchen 1 bis 15 angegebene Bedeutung haben und
T², T³ und T⁴ gleich oder verschieden sind und jeweils für (C₁-C₄)-Alkyl oder Phenyl stehen,
überführt, nachfolgend in einem inerten Lösungsmittel in Gegenwart eines geeigneten Palladium-Katalysators und einer Base mit einer Verbindung der Formel (XII) 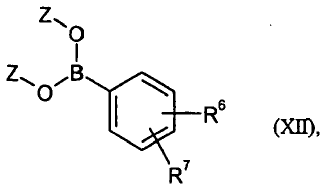 in welcher R⁶ und R⁷ jeweils die in den Ansprüchen 1 bis 15 angegebene Bedeutung haben und
Z für Wasserstoff oder Methyl steht oder beide Z-Gruppen zusammen eine CH₂CH₂-oder C(CH₃)₂-C(CH₃)₂-Brücke bilden,
zu Verbindungen der Formel (XIII) 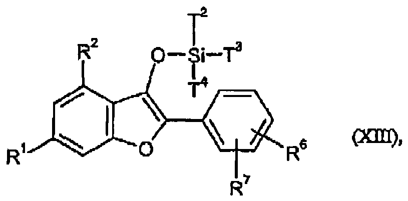 in welcher R¹, R², R⁶, R⁷, T², T³ und T⁴ jeweils die in den Ansprüchen 1 bis 15 angegebene Bedeutung haben,
umsetzt und die Silylgruppe anschließend nach üblichen Methoden wieder zu Verbindungen der Formel (VI) abspaltet.

17. Verbindung, wie in einem der Ansprüche 1 bis 15 definiert, zur Behandlung und/oder Prophylaxe von Krankheiten.

18. Verwendung einer Verbindung, wie in einem der Ansprüche 1 bis 15 definiert, zur Herstellung eines Arznelmittels zur Prophylaxe und/oder Behandlung von Entzündungs- und Autoimmunerkrankungen neurodegenerativen Erkrankungen und hyperproliferativen Erkrankungen

19. Arzneimittel enthaltend eine Verbindung, wie in einem der Ansprüche 1 bis 15 definiert, in Kombination mit einem inorten, nicht-toxischen, pharmazeutisch geeigneten Hilfsstoff.

20. Arzneimittel nach Anspruch 19 zur Prophylaxe und/oder Behandlung von Entzündungs-und Autoimmunerkrankungen, neurodegenerativen Erkrankungen und hyperproliferativen Erkrankungen.

## Claims

1. Compound of the formula (I) 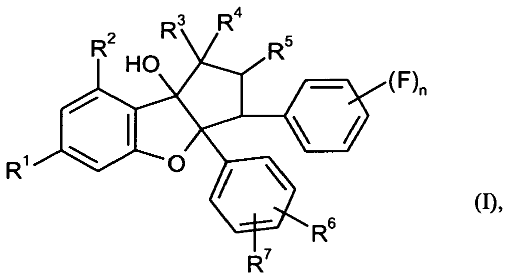 in which
R¹ is hydrogen, benzyloxy, ethoxy, n-propoxy or a group of the formula R⁸-C(=O)-NH-, where ethoxy may be substituted in position 2, and n-propoxy in position 2 or 3, by (C₁-C₆)-alkoxy, amino, mono- or di-(C₁-C₆)-alkylamino, (C₃-C₈)-cycloalkylamino, *N*-(C₃-C₈)-cycloalkyl-*N*-(C,-C₆)-alkylamino or by a 4- to 7-membered heterocycle which is bonded via an N atom,
where mono- and di-(C₁-C₆)-alkylamino in turn may be substituted by hydroxy, (C₁-C₄)-alkoxy, amino, mono- or di-(C₁-C₄)-alkylamino,
and in which
R⁸ is 5- or 6-membered heteroaryl which may be substituted by (C₁-C₄)-alkyl or halogen,
R² is hydrogen, ethoxy or n-propoxy, where ethoxy may be substituted in position 2, and n-propoxy in position 2 or 3, by (C₁-C₆)-alkoxy, amino, mono- or di-(C₁-C₆)-alkylamino, (C₃-C₈)-cycloalkylamino, *N*-(C₃-C₈)-cycloalkyl-*N*-(C₁-C₆)-alkylamino or by a 4- to 7-membered heterocycle which is bonded via an N atom,
where mono- and di-(C₁-C₆)-alkylamino in turn may be substituted by hydroxy, (C₁-C₄)-alkoxy, amino, mono- or di-(C₁-C₄)-alkylamino,
but where R¹ and R² are not simultaneously hydrogen,
R³ is hydroxy or amino
and
R⁴ is hydrogen,
or
R³ and R⁴ together with the carbon atom to which they are bonded form a group of the formula >C=O or >C=N-OH,
R⁵ is mono- or di-(C₁-C₆)-alkylaminocarbonyl,
n is the number 0, 1, 2 or 3,
R⁶ is located in the meta or para position relative to the point of linkage of the phenyl ring to the tricycle, and is halogen, (C₁-C₆)-alkyl, (C₁-C₆)-alkoxy, (C₆-C₁₀)-aryl, 5-to 10-membered heteroaryl or a group of the formula -NR⁹R¹⁰,
where aryl and heteroaryl in turn may each be substituted once to twice, identically or differently, by halogen, cyano, (C₁-C₄)-alkylsulphonyl or a group of the formula -NR⁹R¹⁰,
and in which
R⁹ and R¹⁰ are independently of one another hydrogen, (C₁-C₆)-alkyl, phenyl, benzyl or pyridylmethyl, or together with the nitrogen atom to which they are bonded form a 4- to 7-membered heterocycle,
and
R⁷ is located in the meta or para position relative to the point of linkage of the phenyl ring to the tricycle, and in the ortho position relative to R⁶, and is hydrogen, halogen, (C₁-C₆)-alkyl, (C₁-C₆)-alkoxy or a group of the formula -NR¹¹R¹², in which
R¹¹ and R¹² are independently of one another hydrogen, (C₁-C₆)-alkyl, phenyl, benzyl or pyridylmethyl, or together with the nitrogen atom to which they are bonded form a 4- to 7-membered heterocycle,
or
R⁶ and R⁷ together with the phenyl ring to which they are bonded form a group of the formula 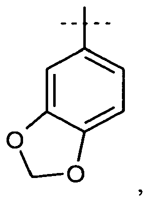
and the salts, solvates and solvates of the salts thereof.

2. Compound of the formula (I) according to Claim 1, in which
R¹ is hydrogen, benzyloxy, ethoxy, n-propoxy or a group of the formula R⁸-C(=O)-NH-, where ethoxy may be substituted in position 2, and n-propoxy in position 2 or 3, by (C₁-C₄)-alkoxy, amino, mono- or di-(C₁-C₄)-alkylamino, (C₃-C₆)-cycloalkylamino, *N*-(C₃-C₆)-cycloalkyl-*N*-(C₁-C₄)-alkylamino or by a 4- to 7-membered heterocycle which is bonded via an N atom,
where mono- and di-(C₁-C₄)-alkylamino in turn may be substituted by hydroxy, (C₁-C₄)-alkoxy, amino, mono- or di-(C₁-C₄)-alkylamino,
and in which
R⁸ is 5- or 6-membered heteroaryl which may be substituted by (C₁-C₄)-alkyl or halogen,
R² is hydrogen, ethoxy or n-propoxy, where ethoxy may be substituted in position 2, and n-propoxy in position 2 or 3, by (C₁-C₄)-alkoxy, amino, mono- or di-(C₁-C₄)-alkylamino, (C₃-C₆)-cycloalkylamino, *N*-(C₃-C₆)-cycloalkyl-*N*-(C₁-C₄)-alkylamino or by a 4- to 6-membered heterocycle which is bonded via an N atom, where mono- and di-(C₁-C₄)-alkylamino in turn may be substituted by hydroxy, (C₁-C₄)-alkoxy, amino, mono- or di-(C₁-C₄)-alkylamino,
but where R¹ and R² are not simultaneously hydrogen,
R³ is hydroxy or amino
and
R⁴ is hydrogen,
or
R³ and R⁴ together with the carbon atom to which they are bonded form a group of the formula >C=O or >C=N-OH,
R⁵ is mono- or di-(C₁-C₄)-alkylaminocarbonyl,
n is the number 0, 1, 2 or 3,
R⁶ is located in the meta or para position relative to the point of linkage of the phenyl ring to the tricycle, and is halogen, (C₁-C₄)-alkyl, (C₁-C₄)-alkoxy, (C₆-C₁₀)-aryl, 5-to 6-membered heteroaryl or a group of the formula -NR⁹R¹⁰,
where aryl and heteroaryl in turn may each be substituted once to twice, identically or differently, by halogen, cyano, (C₁-C₄)-alkylsulphonyl or a group of the formula -NR⁹R¹⁰,
and in which
R⁹ and R¹⁰ are independently of one another hydrogen, (C₁-C₄)-alkyl, phenyl, benzyl or pyridylmethyl, or together with the nitrogen atom to which they are bonded form a 4- to 7-membered heterocycle,
and
R⁷ is located in the meta or para position relative to the point of linkage of the phenyl ring to the tricycle, and in the ortho position relative to R⁶, and is hydrogen, halogen, (C₁-C₄)-alkyl, (C₁-C₄)-alkoxy or a group of the formula -NR¹¹R¹², in which
R¹¹ and R¹² are independently of one another hydrogen, (C₁-C₄)-alkyl, phenyl, benzyl or pyridylmethyl, or together with the nitrogen atom to which they are bonded form a 4- to 7-membered heterocycle,
or
R⁶ and R⁷ together with the phenyl ring to which they are bonded form a group of the formula 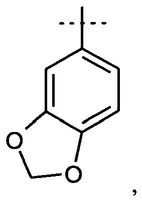 and the salts, solvates and solvates of the salts thereof.

3. Compound of the formula (I) according to Claims 1 and 2, in which
R¹ and R² are independently of one another hydrogen, ethoxy or n-propoxy, where ethoxy is substituted in position 2, and n-propoxy in position 3, by methoxy, ethoxy, amino, methylamino, ethylamino, isopropylamino, dimethylamino, diethylamino, cyclopropylamino, *N*-cyclopropyl-*N*-methylamino, azetidino or pyrrolidino,
but where R¹ and R² are not simultaneously hydrogen,
R³ is hydroxy or amino,
R⁴ is hydrogen,
R⁵ is methylaminocarbonyl or dimethylaminocarbonyl,
n is the number 0 or 1,
R⁶ is located in the para position relative to the point of linkage of the phenyl ring to the tricycle, and is fluorine, chlorine, bromine, methyl, ethyl, methoxy or ethoxy,
and
R⁷ is hydrogen,
and the salts, solvates and solvates of the salts thereof.

4. Compound of the formula (I) in which
R¹ is hydrogen, benzyloxy, ethoxy, n-propoxy or a group of the formula R⁸-C(=O)-NH-, where ethoxy may be substituted in position 2, and n-propoxy in position 2 or 3, by (C₁-C₆)-alkoxy, amino, mono- or di-(C₁-C₆)-alkylamino, (C₃-C₈)-cycloalkylamino, *N*-(C₃-C₈)-cycloalkyl-*N*-(C₁-C₆)-alkylamino or by a 4- to 7-membered heterocycle which is bonded via an N atom,
where mono- and di-(C₁-C₄)-alkylamino in turn may be substituted by hydroxy, (C₁-C₄)-alkoxy, amino, mono- or di-(C₁-C₄)-alkylamino,
and in which
R⁸ is 5- or 6-membered heteroaryl which may be substituted by (C₁-C₄)-alkyl or halogen,
R² is hydrogen, ethoxy or n-propoxy, where ethoxy may be substituted in position 2, and n-propoxy in position 2 or 3, by (C₁-C₆)-alkoxy, amino, mono- or di-(C₁-C₆)-alkylamino, (C₃-C₈)-cycloalkylamino, *N*-(C₃-C₈)-cycloalkyl-*N*-(C₁-C₆)-alkylamino or by a 4- to 7-membered heterocycle which is bonded via an N atom,
where mono- and di-(C₁-C₆)-alkylamino in turn may be substituted by hydroxy, (C₁-C₄)-alkoxy, amino, mono- or di-(C₁-C₄)-alkylamino,
but where R¹ and R² are not simultaneously hydrogen,
R³ is hydroxy or amino
and
R⁴ is hydrogen,
or
R³ and R⁴ together with the carbon atom to which they are bonded form a group of the formula >C=O or >C=N-OH,
R⁵ is hydrogen,
n is the number 0, 1, 2 or 3,
R⁶ is located in the meta or para position relative to the point of linkage of the phenyl ring to the tricycle, and is (C₆-C₁₀)-aryl, 5- to 10-membered heteroaryl or a group of the formula -NR⁹R¹⁰,
where aryl and heteroaryl in turn may each be substituted once to twice, identically or differently, by halogen, cyano, (C₁-C₄)-alkylsulphonyl or a group of the formula -NR⁹R¹⁰
and in which
R⁹ and R¹⁰ are independently of one another hydrogen, (C₁-C₆)-alkyl, phenyl, benzyl or pyridylmethyl, or together with the nitrogen atom to which they are bonded form a 4- to 7-membered heterocycle,
and
R⁷ is located in the meta or para position relative to the point of linkage of the phenyl ring to the tricycle, and in the ortho position relative to R⁶, and is hydrogen, halogen, (C₁-C₆)-alkyl, (C₁-C₆)-alkoxy or a group of the formula -NR¹¹R¹², in which
R¹¹ and R¹² are independently of one another hydrogen, (C₁-C₆)-alkyl, phenyl, benzyl or pyridylmethyl, or together with the nitrogen atom to which they are bonded form a 4- to 7-membered heterocycle,
or
R⁶ and R⁷ together with the phenyl ring to which they are bonded form a group of the formula 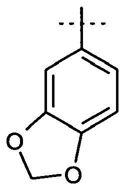 and the salts, solvates and solvates of the salts thereof.

5. Compound of the formula (I) according to Claim 4, in which
R¹ is hydrogen, benzyloxy, ethoxy, n-propoxy or a group of the formula R⁸-C(=O)-NH-, where ethoxy may be substituted in position 2, and n-propoxy in position 2 or 3, by (C₁-C₄)-alkoxy, amino, mono- or di-(C₁-C₄)-alkylamino, (C₃-C₆)-cycloalkylamino, *N*-(C₃-C₆)-cycloalkyl-*N*-(C₁-C₄)-alkylamino or by a 4- to 7-membered heterocycle which is bonded via an N atom,
where mono- and di-(C₁-C₄)-alkylamino in turn may be substituted by hydroxy, (C₁-C₄)-alkoxy, amino, mono- or di-(C₁-C₄)-alkylamino,
and in which
R⁸ is 5- or 6-membered heteroaryl which may be substituted by (C₁-C₄)-alkyl or halogen,
R² is hydrogen, ethoxy or n-propoxy, where ethoxy may be substituted in position 2, and n-propoxy in position 2 or 3, by (C₁-C₄)-alkoxy, amino, mono- or di-(C₁-C₄)-alkylamino, (C₃-C₆)-cycloalkylamino, *N*-(C₃-C₆)-cycloalkyl-*N*-(C₁-C₄)-alkylamino or by a 4- to 7-membered heterocycle which is bonded via an N atom,
where mono- and di-(C₁-C₄)-alkylamino in turn may be substituted by hydroxy, (C₁-C₄)-alkoxy, amino, mono- or di-(C₁-C₄)-alkylamino, but where R¹ and R² are not simultaneously hydrogen,
R³ is hydroxy or amino
and
R⁴ is hydrogen,
or
R³ and R⁴ together with the carbon atom to which they are bonded form a group of the formula >C=O or >C=N-OH,
R⁵ is hydrogen,
n is the number 0, 1, 2 or 3,
R⁶ is located in the meta or para position relative to the point of linkage of the phenyl ring to the tricycle, and is (C₆-C₁₀)-aryl, 5- to 6-membered heteroaryl or a group of the formula -NR⁹R¹⁰,
where aryl and heteroaryl in turn may each be substituted once to twice, identically or differently, by halogen, cyano, (C₁-C₄)-alkylsulphonyl or a group of the formula -NR⁹R¹⁰,
and in which
R⁹ and R¹⁰ are independently of one another hydrogen, (C₁-C₄)-alkyl, phenyl, benzyl or pyridylmethyl, or together with the nitrogen atom to which they are bonded form a 4- to 7-membered heterocycle,
and
R⁷ is located in the meta or para position relative to the point of linkage of the phenyl ring to the tricycle, and in the ortho position relative to R⁶, and is hydrogen, halogen, (C₁-C₄)-alkyl, (C₁-C₄)-alkoxy or a group of the formula -NR¹¹R¹² in which
R¹¹ and R¹² are independently of one another hydrogen, (C₁-C₄)-alkyl, phenyl, benzyl or pyridylmethyl, or together with the nitrogen atom to which they are bonded form a 4- to 7-membered heterocycle,
or
R⁶ and R⁷, together with the phenyl ring to which they are bonded form a group of the formula
and the salts, solvates and solvates of the salts thereof.

6. Compound of the formula (I) according to Claims 4 and 5, in which
R¹ is hydrogen, ethoxy, n-propoxy or a group of the formula R⁸-C(=O)-NH-, where ethoxy may be substituted in position 2, and n-propoxy in position 3, by methoxy, ethoxy, amino, methylamino, ethylamino, isopropylamino, dimethylamino, diethylamino, cyclopropylamino, *N*-cyclopropyl-*N*-methylamino, azetidino or pyrrolidino, in which
R⁸ is pyridyl, pyrrolyl, imidazolyl, pyrazolyl, triazolyl or thiadiazolyl, each of which may be substituted by methyl, ethyl, fluorine or chlorine,
R² is hydrogen, ethoxy or n-propoxy, where ethoxy may be substituted in position 2, and n-propoxy in position 3, by methoxy, ethoxy, amino, methylamino, ethylamino, isopropylamino, dimethylamino, diethylamino, cyclopropylamino, *N-*cyclopropyl-*N*-methylamino, azetidino or pyrrolidino, but where R¹ and R² are not simultaneously hydrogen,
R³ is hydroxy or amino,
R⁴ is hydrogen,
R⁵ is hydrogen,
n is the number 0 or 1,
R⁶ is located in the meta or para position relative to the point of linkage of the phenyl ring to the tricycle, and is phenyl, thienyl, indolyl, quinoxalinyl or a group of the formula -NR⁹R¹⁰,
where phenyl, thienyl and indolyl in turn may each be substituted once to twice, identically or differently, by fluorine, chlorine, bromine, cyano or amino,
and in which
R⁹ and R¹⁰ are independently of one another hydrogen, methyl, ethyl, benzyl or pyridylmethyl, or together with the nitrogen atom to which they are bonded form a pyrrolidino ring,
and
R⁷ is hydrogen,
and the salts, solvates and solvates of the salts thereof.

7. Compound of the formula (I) in which
R¹ is hydrogen, benzyloxy, ethoxy, n-propoxy or a group of the formula R⁸-C(=O)-NH-, where ethoxy may be substituted in position 2, and n-propoxy in position 2 or 3, by (C₁-C₆)-alkoxy, amino, mono- or di-(C₁-C₆)-alkylamino, (C₃-C₈)-cycloalkylamino, *N*-(C₃-C₈)-cycloalkyl-*N*-(C₁-C₆)-alkylamino or by a 4- to 7-membered heterocycle which is bonded via an N atom,
where mono- and di-(C₁-C₆)-alkylamino in turn may be substituted by hydroxy, (C₁-C₄)-alkoxy, amino, mono- or di-(C₁-C₄)-alkylamino,
and in which
R⁸ is 5- or 6-membered heteroaryl which may be substituted by (C₁-C₄)-alkyl or halogen,
R² is hydrogen, ethoxy or n-propoxy, where ethoxy may be substituted in position 2, and n-propoxy in position 2 or 3, by (C₁-C₆)-alkoxy, amino, mono- or di-(C₁-C₆)-alkylamino, (C₃-C₈)-cycloalkylamino, *N*-(C₃-C₈)-cycloalkyl-*N*-(C₁-C₆)-alkylamino or by a 4- to 7-membered heterocycle which is bonded via an N atom,
where mono- and di-(C₁-C₆)-alkylamino in turn may be substituted by hydroxy, (C₁-C₄)-alkoxy, amino, mono- or di-(C₁-C₄)-alkylamino,
but where R¹ and R² are not simultaneously hydrogen,
R³ is amino
and
R⁴ is hydrogen,
or
R³ and R⁴ together with the carbon atom to which they are bonded form a group of the formula >C=N-OH,
R⁵ is hydrogen,
n is the number 0, 1, 2 or 3,
R⁶ is located in the meta or para position relative to the point of linkage of the phenyl ring to the tricycle, and is halogen, (C₁-C₆)-alkyl or (C₁-C₆)-alkoxy,
and
R⁷ is located in the meta or para position relative to the point of linkage of the phenyl ring to the tricycle, and in the ortho position relative to R⁶, and is hydrogen, halogen, (C₁-C₆)-alkyl, (C₁-C₆)-alkoxy or a group of the formula -NR¹¹R¹² in which
R¹¹ and R¹² are independently of one another hydrogen, (C₁-C₆)-alkyl, phenyl, benzyl or pyridylmethyl, or together with the nitrogen atom to which they are bonded form a 4- to 7-membered heterocycle,
or
R⁶ and R⁷ together with the phenyl ring to which they are bonded form a group of the formula 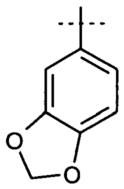 and the salts, solvates and solvates of the salts thereof.

8. Compound of the formula (I) according to Claim 7, in which
R¹ is hydrogen, benzyloxy, ethoxy, n-propoxy or a group of the formula R⁸-C(=O)-NH-, where ethoxy may be substituted in position 2, and n-propoxy in position 2 or 3, by (C₁ - C₄)-alkoxy, amino, mono- or di-(C₁-C₄)-alkylamino, (C₃-C₆)-cycloalkylamino, *N*-(C₃-C₆)-cycloalkyl-*N*-(C₁-C₄)-alkylamino or by a 4- to 7-membered heterocycle which is bonded via an N atom,
where mono- and di-(C₁-C₄)-alkylamino in turn may be substituted by hydroxy, (C₁-C₄)-alkoxy, amino, mono- or di-(C₁-C₄)-alkylamino,
and in which
R⁸ is 5- or 6-membered heteroaryl which may be substituted by (C₁-C₄)-alkyl or halogen,
R² is hydrogen, ethoxy or n-propoxy, where ethoxy may be substituted in position 2, and n-propoxy in position 2 or 3, by (C₁-C₄)-alkoxy, amino, mono- or di-(C₁-C₄)-alkylamino, (C₃-C₆)-cycloalkylamino, *N*-(C₃-C₆)-cydoalkyl-*N*-(C₁-C₄)-alkylamino or by a 4- to 7-membered heterocycle which is bonded via an N atom,
where mono- and di-(C₁-C₄)-alkylamino in turn may be substituted by hydroxy, (C₁-C₄)-alkoxy, amino, mono- or di-(C₁-C₄)-alkylamino,
but where R¹ and R² are not simultaneously hydrogen,
R³ is amino
and
R⁴ is hydrogen
or
R³ and R⁴ together with the carbon atom to which they are bonded form a group of the formula >C=N-OH,
R⁵ is hydrogen,
n is the number 0, 1, 2 or 3,
R⁶ is located in the meta or para position relative to the point of linkage of the phenyl ring to the tricycle, and is halogen, (C₁-C₄)-alkyl or (C₁-C₄)-alkoxy,
and
R⁷ is located in the meta or para position relative to the point of linkage of the phenyl ring to the tricycle, and in the ortho position relative to R⁶, and is hydrogen, halogen, (C₁-C₄)-alkyl, (C₁-C₄)-alkoxy or a group of the formula -NR¹¹R¹², in which
R¹¹ and R¹² are independently of one another hydrogen, (C₁-C₄)-alkyl, phenyl, benzyl or pyridylmethyl, or together with the nitrogen atom to which they are bonded form a 4- to 7-membered heterocycle,
or
R⁶ and R⁷ together with the phenyl ring to which they are bonded form a group of the formula 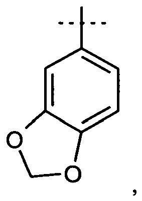 and the salts, solvates and solvates of the salts thereof.

9. Compound of the formula (I) according to Claims 7 and 8, in which
R¹ is hydrogen, ethoxy, n-propoxy or a group of the formula R⁸-C(=O)-NH-, where ethoxy may be substituted in position 2, and n-propoxy in position 3, by methoxy, ethoxy, amino, methylamino, ethylamino, isopropylamino, dimethylamino, diethylamino, cyclopropylamino, *N*-cyclopropyl-N-methylamino, azetidino or pyrrolidino, in which
R⁸ is pyridyl, pyrrolyl, imidazolyl, pyrazolyl, triazolyl or thiadiazolyl, each of which may be substituted by methyl, ethyl, fluorine or chlorine,
R² is hydrogen, ethoxy or n-propoxy, where ethoxy may be substituted in position 2, and n-propoxy in position 3, by methoxy, ethoxy, amino, methylamino, ethylamino, isopropylamino, dimethylamino, diethylamino, cyclopropylamino, *N-*cyclopropyl-*N*-methylamino, azetidino or pyrrolidino,
but where R¹ and R² are not simultaneously hydrogen,
R³ is amino,
R⁴ is hydrogen
R⁵ is hydrogen,
n is the number 0 or 1,
R⁶ is located in the meta or para position relative to the point of linkage of the phenyl ring to the tricycle, and is fluorine, chlorine, bromine, methyl, ethyl, methoxy or ethoxy,
and
R⁷ is located in the meta or para position relative to the point of linkage of the phenyl ring to the tricycle, and in the ortho position relative to R⁶, and is hydrogen, fluorine, chlorine, bromine, methyl, ethyl, methoxy, ethoxy or a group of the formula -NR¹¹R¹² in which
R¹¹ and R¹² are independently of one another hydrogen, methyl, ethyl, benzyl or pyridylmethyl, or together with the nitrogen atom to which they are bonded form a pyrrolidino or piperidino ring,
or
R⁶ and R⁷ together with the phenyl ring to which they are bonded form a group of the formula 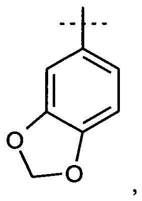 and the salts, solvates and solvates of the salts thereof.

10. Compound of the formula (I) in which
R¹ is hydrogen, benzyloxy, ethoxy, n-propoxy or a group of the formula R⁸-C(=O)-NH-, where ethoxy may be substituted in position 2, and n-propoxy in position 2 or 3, by (C₁-C₆)-alkoxy, amino, mono- or di-(C₁-C₆)-alkylamino, (C₃-C₈)-cycloalkylamino, N(C₃-C₈)-cycloalkyl-*N*(-C₁-C₆)-alkylamino or by a 4- to 7-membered heterocycle which is bonded via an N atom,
where mono- and di-(C₁-C₆)-alkylamino in turn may be substituted by hydroxy, (C₁-C₄)-alkoxy, amino, mono- or di-(C₁-C₄)-alkylamino, and in which
R⁸ is 5- or 6-membered heteroaryl which may be substituted by (C₁-C₄)-alkyl or halogen,
R² is hydrogen, ethoxy or n-propoxy, where ethoxy may be substituted in position 2, and n-propoxy in position 2 or 3, by (C₁-C₆)-alkoxy, amino, mono- or di-(C₁-C₆)-alkylamino, (C₃-C₈)-cycloalkylamino, *N*-(C₃-C₈)-cycloalkyl-*N*-(C₁-C₆)-alkylamino or by a 4- to 7-membered heterocycle which is bonded via an N atom, where mono- and di-(C₁-C₆)-alkylamino in turn may be substituted by hydroxy, (C₁-C₄)-alkoxy, amino, mono- or di-(C₁-C₄)-alkylamino,
where either R¹ or R² is hydrogen but both are not simultaneously hydrogen,
R³ is hydroxy
and
R⁴ is hydrogen,
or
R³ and R⁴ together with the carbon atom to which they are bonded form a group of the formula >C=O,
R⁵ is hydrogen,
n is the number 0, 1, 2 or 3,
R⁶ is located in the meta or para position relative to the point of linkage of the phenyl ring to the tricycle, and is halogen, (C₁-C₆)-alkyl or (C₁-C₆)-alkoxy,
and
R⁷ is located in the meta or para position relative to the point of linkage of the phenyl ring to the tricycle, and in the ortho position relative to R⁶, and is hydrogen, halogen, (C₁-C₆)-alkyl, (C₁-C₆)-alkoxy or a group of the formula -NR¹¹R¹², in which
R¹¹ and R¹² are independently of one another hydrogen, (C₁-C₆)-alkyl, phenyl, benzyl or pyridylmethyl, or together with the nitrogen atom to which they are bonded form a 4- to 7-membered heterocycle,
or
R⁶ and R⁷ together with the phenyl ring to which they are bonded form a group of the formula 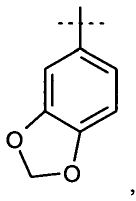 and the salts, solvates and solvates of the salts thereof.

11. Compound of the formula (I) according to Claim 10, in which
R¹ is hydrogen, benzyloxy, ethoxy, n-propoxy or a group of the formula R⁸-C(=O)-NH-, where ethoxy may be substituted in position 2, and n-propoxy in position 2 or 3, by (C₁-C₄)-alkoxy, amino, mono- or di-(C₁-C₄)-alkylamino, (C₃-C₆)-cycloalkylamino, *N*-(C₃-C₆)-cycloalkyl-*N*-(C₁-C₄)-alkylamino or by a 4- to 7-membered heterocycle which is bonded via an N atom,
where mono- and di-(C₁-C₄)-alkylamino in turn may be substituted by hydroxy, (C₁-C₄)-alkoxy, amino, mono- or di-(C₁-C₄)-alkylamino,
and in which
R⁸ is 5- or 6-membered heteroaryl which may be substituted by (C₁-C₄)-alkyl or halogen,
R² is hydrogen, ethoxy or n-propoxy, where ethoxy may be substituted in position 2, and n-propoxy in position 2 or 3, by (C₁-C₄)-alkoxy, amino, mono- or di-(C₁-C₄)-alkylamino, (C₃-C₆)-cycloalkylamino, *N*-(C₃-C₆)-cycloalkyl-*N*-(C₁-C₄)-alkylamino or by a 4- to 7-membered heterocycle which is bonded via an N atom, where mono- and di-(C₁-C₄)-alkylamino in turn may be substituted by hydroxy, (C₁-C₄)-alkoxy, amino, mono- or di-(C₁-C₄)-alkylamino,
where either R¹ or R² is hydrogen but both are not simultaneously hydrogen,
R³ is hydroxy
and
R⁴ is hydrogen,
or
R³ and R⁴ together with the carbon atom to which they are bonded form a group of the formula >C=O,
R⁵ is hydrogen,
n is the number 0, 1, 2 or 3,
R⁶ is located in the meta or para position relative to the point of linkage of the phenyl ring to the tricycle, and is halogen, (C₁-C₄)-alkyl or (C₁-C₄)-alkoxy,
and
R⁷ is located in the meta or para position relative to the point of linkage of the phenyl ring to the tricycle, and in the ortho position relative to R⁶, and is hydrogen, halogen, (C₁-C₄)-alkyl, (C₁-C₄)-alkoxy or a group of the formula -NR¹¹R¹² in which
R¹¹ and R¹² are independently of one another hydrogen, (C₁-C₄)-alkyl, phenyl, benzyl or pyridylmethyl or together with the nitrogen atom to which they are bonded form a 4- to 7-membered heterocycle,
or
R⁶ and R⁷ together with the phenyl ring to which they are bonded form a group of the formula 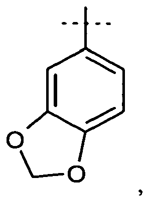 and the salts, solvates and solvates of the salts thereof.

12. Compound of the formula (I) according to Claims 10 and 11, in which
R¹ is hydrogen, ethoxy, n-propoxy or a grouop of the formula R⁸-C(=O)-NH-, where ethoxy may be substituted in position 2, and n-propoxy in position 3, by methoxy, ethoxy, amino, methylamino, ethylamino, isopropylamino, dimethylamino, diethylamino, cyclopropylamino, *N*-cyclopropyl-*N*-methylamino, azetidino or pyrrolidino, in which
R⁸ is pyridyl, pyrrolyl, imidazolyl, pyrazolyl, triazolyl or thiadiazolyl, each of which may be substituted by methyl, ethyl, fluorine or chlorine,
R² is hydrogen, ethoxy or n-propoxy, where ethoxy may be substituted in position 2, and n-propoxy in position 3, by methoxy, ethoxy, amino, methylamino, ethylamino, isopropylamino, dimethylamino, diethylamino, cyclopropylamino, *N-*cyclopropyl-*N*-methylamino, azetidino or pyrrolidino,
where either R¹ or R² is hydrogen, but both are not simultaneously hydrogen,
R³ is hydroxy
and
R⁴ is hydrogen,
or
R³ and R⁴ together with the carbon atom to which they are bonded form a group of the formula >C=O,
R⁵ is hydrogen,
n is the number 0 or 1,
R⁶ is located in the meta or para position relative to the point of linkage of the phenyl ring to the tricycle, and is fluorine, chlorine, bromine, methyl, ethyl, methoxy or ethoxy,
and
R⁷ is located in the meta or para position relative to the point of linkage of the phenyl ring to the tricycle, and in the ortho position relative to R⁶, and is hydrogen, fluorine, chlorine, bromine, methyl, ethyl, methoxy, ethoxy or a group of the formula -NR¹¹R¹² in which R¹¹ and R¹² are independently of one another hydrogen, methyl, ethyl, benzyl or pyridylmethyl, or together with the nitrogen atom to which they are bonded form a pyrrolidino or piperidino ring,
or
R⁶ and R⁷ together with the phenyl ring to which they are bonded form a group of the formula 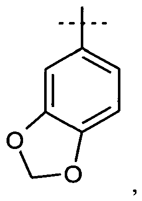 and the salts, solvates and solvates of the salts thereof.

13. Compound of the formula (I) in which
R¹ is ethoxy, n-propoxy or a group of the formula R⁸-C(=O)-NH-, where ethoxy is substituted in position 2, and n-propoxy in position 2 or 3, by (C₁-C₆)-alkoxy, amino, mono- or di-(C₁-C₆)-alkylamino, (C₃-C₈)-cycloalkylamino, *N*-(C₃-C₈)-cydoalkyl-*N*-(C₁-C₆)-alkylamino or by a 4- to 7-membered heterocycle which is bonded via an N atom,
where mono- and di-(C₁-C₆)-alkylamino in turn may be substituted by hydroxy, (C₁-C₄)-alkoxy, amino, mono- or di-(C₁-C₄)-alkylamino,
and in which
R⁸ is 5- or 6-membered heteroaryl which may be substituted by (C₁-C₄)-alkyl or halogen,
R² is ethoxy or n-propoxy, where ethoxy is substituted in position 2, and n-propoxy in position 2 or 3, by (C₁-C₆)-alkoxy, amino, mono- or di-(C₁-C₆)-alkylamino, (C₃-C₈)-cycloalkylamino, *N*-(C₃-C₈)-cycloalkyl-*N*-(C₁-C₆)-alkylamino or by a 4- to 7-membered heterocycle which is bonded via an N atom,
where mono- and di-(C₁-C₆)-alkylamino in turn may be substituted by hydroxy, (C₁-C₄)-alkoxy, amino, mono- or di-(C₁-C₄)-alkylamino,
R³ is hydroxy
and
R⁴ is hydrogen,
or
R³ and R⁴ together with the carbon atom to which they are bonded form a group of the formula >C=O,
R⁵ is hydrogen,
n is the number 0, 1, 2 or 3,
R⁶ is located in the meta or para position relative to the point of linkage of the phenyl ring to the tricycle, and is halogen, (C₁-C₆)-alkyl or (C₁-C₆)-alkoxy,
and
R⁷ is located in the meta or para position relative to the point of linkage of the phenyl ring to the tricycle, and in the ortho position relative to R⁶, and is hydrogen, halogen, (C₁-C₆)-alkyl, (C₁-C₆)-alkoxy or a group of the formula -NR¹¹R¹², in which
R¹¹ and R¹² are independently of one another hydrogen, (C₁-C₆)-alkyl, phenyl, benzyl or pyridylmethyl, or together with the nitrogen atom to which they are bonded form a 4- to 7-membered heterocycle,
or
R⁶ and R⁷ together with the phenyl ring to which they are bonded form a group of the formula 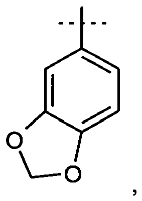 and the salts, solvates and solvates of the salts thereof.

14. Compound of the formula (I) according to Claim 13, in which
R¹ is ethoxy, n-propoxy or a group of the formula R⁸-C(=O)-NH-, where ethoxy is substituted in position 2, and n-propoxy in position 2 or 3, by (C₁-C₄)-alkoxy, amino, mono- or di-(C₁-C₄)-alkylamino, (C₃-C₆)-cycloalkylamino, *N*-(C₃-C₆)-cycloalkyl-*N*-(C₁-C₄)-alkylamino or by a 4- to 7-membered heterocycle which is bonded via an N atom,
where mono- and di-(C₁-C₄)-alkylamino in turn may be substituted by hydroxy, (C₁-C₄)-alkoxy, amino, mono- or di-(C₁-C₄)-alkylamino,
and in which
R⁸ is a 5- or 6-membered heteroaryl which may be substituted by (C₁-C₄)-alkyl or halogen,
R² is ethoxy or n-propoxy, where ethoxy is substituted in position 2, and n-propoxy in position 2 or 3, by (C₁-C₄)-alkoxy, amino, mono- or di-(C₁-C₄)-alkylamino, (C₃-C₆)-cycloalkylamino, *N*-(C₃-C₆)-cycloalkyl-*N*-(C₁-C₄)-alkylamino or by a 4- to 7-membered heterocycle which is bonded via an N atom, where mono- and di-(C₁-C₄)-alkylamino in turn may be substituted by hydroxy, (C₁-C₄)-alkoxy, amino, mono- or di-(C₁-C₄)-alkylamino,
R³ is hydroxy
and
R⁴ is hydrogen,
or
R³ and R⁴ together with the carbon atom to which they are bonded form a group of the formula >C=O,
R⁵ is hydrogen,
n is the number 0, 1, 2 or 3,
R⁶ is located in the meta or para position relative to the point of linkage of the phenyl ring to the tricycle, and is halogen, (C₁-C₄)-alkyl or (C₁-C₄)-alkoxy,
and
R⁷ is located in the meta or para position relative to the point of linkage of the phenyl ring to the tricycle, and in the ortho position relative to R⁶, and is hydrogen, halogen, (C₁-C₄)-alkyl, (C₁-C₄)-alkoxy or a group of the formula -NR¹¹R¹² in which
R¹¹ and R¹² are independently of one another hydrogen, (C₁-C₄)-alkyl, phenyl, benzyl or pyridylmethyl, or together with the nitrogen atom to which they are bonded form a 4- to 7-membered heterocycle,
or
R⁶ and R⁷ together with the phenyl ring to which they are bonded form a group of the formula 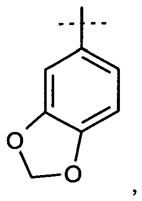 and the salts, solvates and solvates of the salts thereof.

15. Compound of the formula (I) according to Claims 13 and 14, in which
R¹ is ethoxy, n-propoxy or a group of the formula R⁸-C(=O)-NH-, where ethoxy is substituted in position 2, and n-propoxy in position 3, by methoxy, ethoxy, amino, methylamino, ethylamino, isopropylamino, dimethylamino, diethylamino, cyclopropylamino, *N*-cyclopropyl-*N*-methylamino, azetidino or pyrrolidino, in which
R⁸ is pyridyl, pyrrolyl, imidazolyl, pyrazolyl, triazolyl or thiadiazolyl, each of which may be substituted by methyl, ethyl, fluorine or chlorine,
R² is ethoxy or n-propoxy, where ethoxy is substituted in position 2, and n-propoxy in position 3, by methoxy, ethoxy, amino, methylamino, ethylamino, isopropylamino, dimethylamino, diethylamino, cyclopropylamino, *N*-cyclopropyl-*N*-methylamino, azetidino or pyrrolidino,
R³ is hydroxy
and
R⁴ is hydrogen,
or
R³ and R⁴ together with the carbon atom to which they are bonded form a group of the formula >C=O,
R⁵ is hydrogen,
n is the number 0 or 1,
R⁶ is located in the meta or para position relative to the point of linkage of the phenyl ring to the tricycle, and is fluorine, chlorine, bromine, methyl, ethyl, methoxy or ethoxy,
and
R⁷ is located in the meta or para position relative to the point of linkage of the phenyl ring to the tricycle, and in the ortho position relative to R⁶, and is hydrogen, fluorine, chlorine, bromine, methyl, ethyl, methoxy, ethoxy or a group of the formula -NR¹¹R¹² in which
R¹¹ and R¹² are independently of one another hydrogen, methyl, ethyl, benzyl or pyridylmethyl, or together with the nitrogen atom to which they are bonded form a pyrrolidino or piperidino ring,
or
R⁶ and R⁷ together with the phenyl ring to which they are bonded form a group of the formula 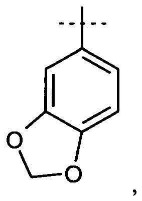 and the salts, solvates and solvates of the salts thereof.

16. Process for preparing a compound of the formula (VI) 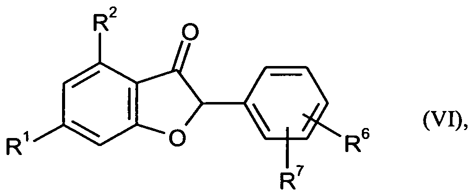 in which R¹, R², R⁶ and R⁷ each have the meaning indicated in claims 1 to 15, **characterized in that** either
[A] compounds of the formula (II) 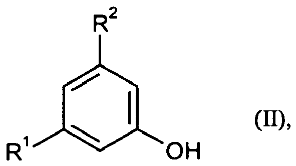 in which R¹ and R² each have the meaning indicated in Claims 1 to 15,
are reacted in an inert solvent in the presence of a base with a compound of the formula (III) 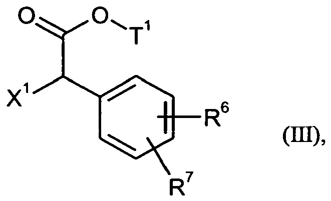 in which R⁶ and R⁷ each have the meaning indicated in Claims 1 to 15,
X¹ is a suitable leaving group such as, for example, halogen, mesylate, tosylate or triflate
and
T¹ is (C₁-C₄)-alkyl,
to give compounds of the formula (IV) 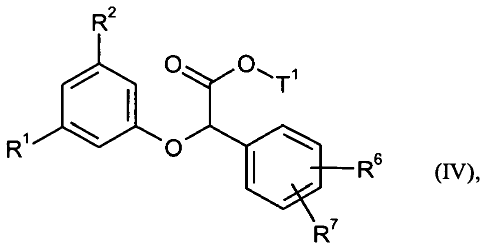 in which R¹, R², R⁶, R⁷ and T¹ each have the meanings indicated in Claims 1 to 15, subsequently converted by basic or acidic hydrolysis into carboxylic acids of the formula (V) 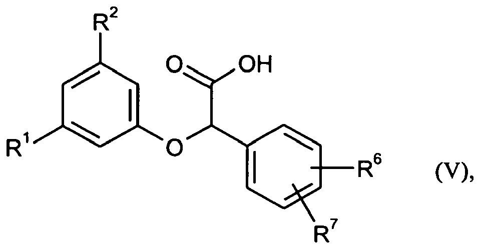 in which R¹, R², R⁶ and R⁷ each have the meaning indicated in Claims 1 to 15,
the latter are then cyclized after activation with phosphoryl chloride in the presence of a Lewis acid to give compounds of the formula (VI) 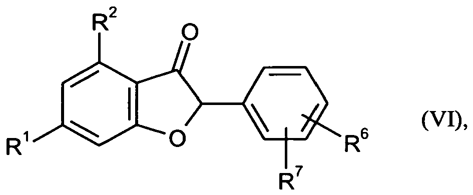 in which R¹, R², R⁶ and R⁷ each have the meaning indicated in Claims 1 to 15,
or
[B] compounds of the formula (VII) 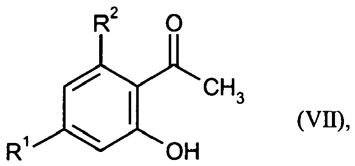
in which R¹ and R² each have the meaning indicated in Claims 1 to 15,
are initially converted by conventional methods into phenacyl bromides of the formula (VIII) 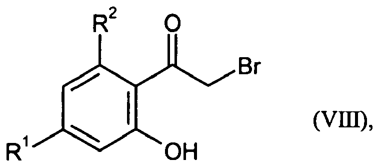 in which R¹ and R² each have the meaning indicated in Claims 1 to 15,
and the latter are then cyclized in the presence of a base to give compounds of the formula (IX) 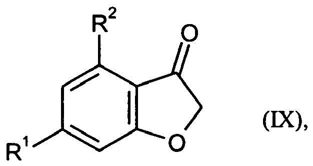 in which R¹ and R² each have the meaning indicated in Claims 1 to 15, subsequently brominated in an inert solvent to give compounds of the formula (X) 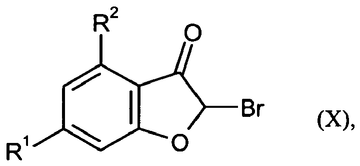 in which R¹ and R² each have the meaning indicated in Claims 1 to 15,
and converted by conventional methods into silyl enol ethers of the formula (XI) 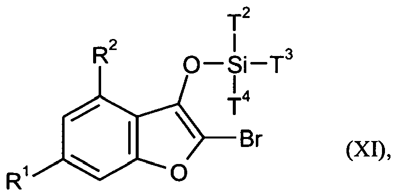 in which R¹ and R² each have the meaning indicated in Claims 1 to 15
and
T², T³ and T⁴ are identical or different and are each (C₁-C₄)-alkyl or phenyl,
subsequently reacted in an inert solvent in the presence of a suitable palladium catalyst and of a base with a compound of the formula (XII) 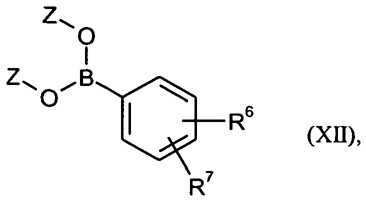 in which R⁶ and R⁷ each have the meaning indicated in Claims 1 to 15 and
Z is hydrogen or methyl, or the two Z groups together form a CH₂CH₂ or C(CH₃)₂-C(CH₃)₂ bridge,
to give compounds of the formula (XIII) 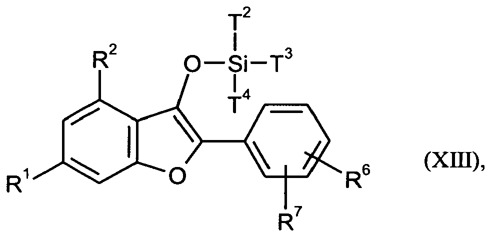 in which R¹, R², R⁶, R⁷, T², T³ and T⁴ each have the meaning indicated in Claims 1 to 15, the silyl group is subsequently eliminated again by conventional methods to give compounds of the formula (VI).

17. Compound as defined in any of Claims 1 to 15 for the treatment and/or prophylaxis of diseases.

18. Use of a compound as defined in any of Claims 1 to 15 for producing a medicament for the prophylaxis and/or treatment of inflammatory and autoimmune diseases, neurodegenerative disorders and hyperproliferative disorders.

19. Medicament comprising a compound as defined in any of Claims 1 to 15 in combination with an inert, non-toxic, pharmaceutically suitable excipient.

20. Medicament according to Claim 19 for the prophylaxis and/or treatment of inflammatory and autoimmune diseases, neurodegenerative disorders and hyperproliferative disorders.

## Revendications

1. Composé de formule (I) 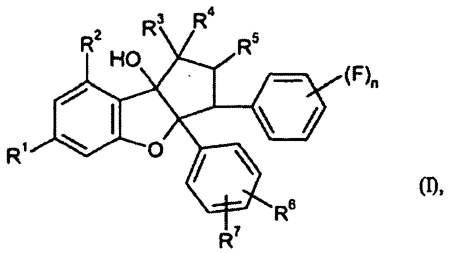 dans laquelle
R¹ signifie hydrogène, benzyloxy, éthoxy, n-propoxy ou un groupe de formule R⁸-C(=O)-NH-, éthoxy pouvant être substitué en position 2 et n-propoxy en position 2 ou 3 par alcoxy en C₁-C₆, amino, mono- ou dialkylamino en C₁-C₆, cycloalkylamino en C₃-C₈, *N*-cycloalkyle en C₃-C₈-*N*-alkylamino en C₁-C₆ ou par un hétérocycle de 4 à 7 éléments relié par un atome N, mono- et dialkylamino en C₁-C₆ pouvant de leur côté être substitués par hydroxy, alcoxy en C₁-C₄, amino, mono- ou dialkylamino en C₁-C₄,
et
R⁸ représentant un hétéroaryle à 5 ou 6 éléments, qui peut être substitué par alkyle en C₁-C₄ ou halogène,
R² signifie hydrogène, éthoxy ou n-propoxy, éthoxy pouvant être substitué en position 2 et n-propoxy en position 2 ou 3 par alcoxy en C₁-C₆, amino, mono- ou dialkylamino en C₁-C₆, cycloalkylamino en C₃-C₈, *N-*cycloalkyle en C₃₋₈-*N*-alkylamino en C₁-C₆ ou par un hétérocycle de 4 à 7 éléments relié par un atome N, mono- et dialkylamino en C₁-C₆ pouvant de leur côté être substitués par hydroxy, alcoxy en C₁-C₄, amino, mono- ou dialkylamino en C₁-C₄,
R¹ et R² ne représentant toutefois pas simultanément l'hydrogène,
R³ signifie hydroxy ou amino
et
R⁴ signifie hydrogène
ou
R³ et R⁴ forment ensemble avec l'atome de carbone auquel ils sont reliés un groupe de formule >C=O ou >C=N-OH,
R⁵ signifie mono- ou dialkylaminocarbonyle en C₁-C₆,
n représente le nombre 0, 1, 2 ou 3,
R⁶ se trouve en position méta ou para, par rapport à l'emplacement de liaison du cycle phényle avec le tricycle, et signifie halogène, alkyle en C₁-C₆, alcoxy en C₁-C₆, aryle en C₆-C₁₀, hétéroaryle de 5 à 10 éléments ou un groupe de formule -NR⁹R¹⁰,
aryle et hétéroaryle pouvant de leur côté être substitués chacun une à deux fois, de manière identique ou différente, par halogène, cyano, alkylsulfonyle en C₁-C₄ ou un groupe de formule -NR⁹R¹⁰,
et
R⁹ et R¹⁰ représentant indépendamment l'un de l'autre hydrogène, alkyle en C₁-C₆, phényle, benzyle ou pyridylméthyle, ou formant ensemble avec l'atome d'azote auquel ils sont reliés un hétérocycle de 4 à 7 éléments,
et
R⁷ se trouve en position méta ou para par rapport à l'emplacement de liaison du cycle phényle avec le tricycle, et en position ortho par rapport à R⁶, et signifie hydrogène, halogène, alkyle en C₁-C₆, alcoxy en C₁-C₆ ou un groupe de formule -NR¹¹R¹²,
R¹¹ et R¹² représentant indépendamment l'un de l'autre hydrogène, alkyle en C₁-C₆, phényle, benzyle ou pyridylméthyle, ou formant ensemble avec l'atome d'azote auquel ils sont reliés un hétérocycle de 4 à 7 éléments,
ou
R⁶ et R⁷ forment ensemble avec le cycle phényle auquel ils sont reliés un groupe de formule 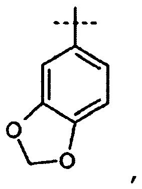
ainsi que leurs sels, solvates et solvates des sels.

2. Composé de formule (I) selon la revendication 1, dans lequel
R¹ signifie hydrogène, benzyloxy, éthoxy, n-propoxy ou un groupe de formule R⁸-C(=O)-NH-, éthoxy pouvant être substitué en position 2 et n-propoxy en position 2 ou 3 par alcoxy en C₁-C₄, amino, mono- ou dialkylamino en C₁-C₄, cycloalkylamino en C₃-C₆, N-cycloalkyle en C₃-C₆-N-alkylamino en C₁-C₄ ou par un hétérocycle de 4 à 7 éléments relié par un atome N,
mono- et dialkylamino en C₁-C₄ pouvant de leur côté être substitués par hydroxy, alcoxy en C₁-C₄, amino, mono- ou dialkylamino en C₁-C₄,
et
R⁸ représentant un hétéroaryle à 5 ou 6 éléments, qui peut être substitué par alkyle en C₁-C₄ ou halogène,
R² signifie hydrogène, éthoxy ou n-propoxy, éthoxy pouvant être substitué en position 2 et n-propoxy en position 2 ou 3 par alcoxy en C₁-C₄, amino, mono- ou dialkylamino en C₁-C₄, cycloalkylamino en C₃-C₆, N-cycloalkyle en C₃-C₆-*N*-alkylamino en C₁-C₄ ou par un hétérocycle de 4 à 6 éléments relié par un atome N, mono- et dialkylamino en C₁-C₄ pouvant de leur côté être substitués par hydroxy, alcoxy en C₁-C₄, amino, mono- ou dialkylamino en C₁-C₄,
R¹ et R² ne représentant toutefois pas simultanément l'hydrogène,
R³ signifie hydroxy ou amino
et
R⁴ signifie hydrogène
ou
R³ et R⁴ forment ensemble avec l'atome de carbone auquel ils sont reliés un groupe de formule >C=O ou >C=N-OH,
R⁵ signifie mono- ou dialkylaminocarbonyle en C₁-C₄,
n représente le nombre 0, 1, 2 ou 3,
R⁶ se trouve en position méta ou para, par rapport à l'emplacement de liaison du cycle phényle avec le tricycle, et signifie halogène, alkyle en C₁-C₄, alcoxy en C₁-C₄, aryle en C₆-C₁₀, hétéroaryle de 5 à 6 éléments ou un groupe de formule -NR⁹R¹⁰,
aryle et hétéroaryle pouvant de leur côté être substitués chacun une à deux fois, de manière identique ou différente, par halogène, cyano, alkylsulfonyle en C₁-C₄ ou un groupe de formule -NR⁹R¹⁰,
et
R⁹ et R¹⁰ représentant indépendamment l'un de l'autre hydrogène, alkyle en C₁-C₄, phényle, benzyle ou pyridylméthyle, ou formant ensemble avec l'atome d'azote auquel ils sont reliés un hétérocycle de 4 à 7 éléments,
et
R⁷ se trouve en position méta ou para par rapport à l'emplacement de liaison du cycle phényle avec le tricycle, et en position ortho par rapport à R⁶, et signifie hydrogène, halogène, alkyle en C₁-C₄, alcoxy en C₁-C₄ ou un groupe de formule -NR¹¹R¹²,
R¹¹ et R¹² représentant indépendamment l'un de l'autre hydrogène, alkyle en C₁-C₄, phényle, benzyle ou pyridylméthyle, ou forment ensemble avec l'atome d'azote auquel ils sont reliés un hétérocycle de 4 à 7 éléments,
ou
R⁶ et R⁷ forment ensemble avec le cycle phényle auquel ils sont reliés un groupe de formule 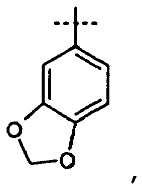 ainsi que leurs sels, solvates et solvates des sels.

3. Composé de formule (I) selon les revendications 1 et 2, dans lequel
R¹ et R² signifient indépendamment l'un de l'autre hydrogène, éthoxy ou n-propoxy, éthoxy pouvant être substitué en position 2 et n-propoxy en position 3 par méthoxy, éthoxy, amino, méthylamino, éthylamino, isopropylamino, diméthylamino, diéthylamino, cyclopropylamino, *N*-cyclopropyl-*N*-méthylamino, azétidino ou pyrrolidino,
R¹ et R² ne représentant toutefois pas simultanément l'hydrogène,
R³ signifie hydroxy ou amino,
R⁴ signifie hydrogène,
R⁵ signifie méthylaminocarbonyle ou diméthylaminocarbonyle,
n représente le nombre 0 ou 1,
R⁶ se trouve en position para par rapport à l'emplacement de liaison du cycle phényle avec le tricycle, et signifie fluor, chlore, brome, méthyle, éthyle, méthoxy ou éthoxy,
et
R⁷ signifie hydrogène,
ainsi que leurs sels, solvates et solvates des sels.

4. Composé de formule (I), dans lequel R¹ signifie hydrogène, benzyloxy, éthoxy, n-propoxy ou un groupe de formule R⁸-C (=P) -NH-, éthoxy pouvant être substitué en position 2 et n-propoxy en position 2 ou 3 par alcoxy en C₁-C₆, amino, mono- ou dialkylamino en C₁-C₆, cycloalkylamino en C₃-C₈, *N*-cycloalkyle en C₃-C₈-*N*-alkylamino en C₁-C₆ ou par un hétérocycle de 4 à 7 éléments relié par un atome N,
mono- et dialkylamino en C₁-C₆ pouvant de leur côté être substitués par hydroxy, alcoxy en C₁-C₄, amino, mono- ou dialkylamino en C₁-C₄,
et
R⁸ représentant un hétéroaryle à 5 ou 6 éléments, qui peut être substitué par alkyle en C₁-C₄ ou halogène,
R² signifie hydrogène, éthoxy ou n-propoxy, éthoxy pouvant être substitué en position 2 et n-propoxy en position 2 ou 3 par alcoxy en C₁-C₆, amino, mono- ou dialkylamino en C₁-C₆, cycloalkylamino en C₃-C₈, *N-*cycloalkyle en C₃-C₈-*N*-alkylamino en C₁-C₆ ou par un hétérocycle de 4 à 7 éléments relié par un atome N, mono- et dialkylamino en C₁-C₆ pouvant de leur côté être substitués par hydroxy, alcoxy en C₁-C₄, amino, mono- ou dialkylamino en C₁-C₄,
R¹ et R² ne représentant toutefois pas simultanément l'hydrogène,
R³ signifie hydroxy ou amino
et
R⁴ signifie hydrogène
ou
R³ et R⁴ forment ensemble avec l'atome de carbone auquel ils sont reliés un groupe de formule >C=O ou >C=N-OH,
R⁵ signifie hydrogène,
n représente le nombre 0, 1, 2 ou 3,
R⁶ se trouve en position méta ou para, par rapport à l'emplacement de liaison du cycle phényle avec le tricycle, et signifie aryle en C₆-C₁₀, hétéroaryle de 5 à 10 éléments ou un groupe de formule -NR⁹R¹⁰,
aryle et hétéroaryle pouvant de leur côté être substitués chacun une à deux fois, de manière identique ou différente, par halogène, cyano, alkylsulfonyle en C₁-C₄ ou un groupe de formule -NR⁹R¹⁰,
et
R⁹ et R¹⁰ représentant indépendamment l'un de l'autre hydrogène, alkyle en C₁-C₆, phényle, benzyle ou pyridylméthyle, ou formant ensemble avec l'atome d'azote auquel ils sont reliés un hétérocycle de 4 à 7 éléments,
et
R⁷ se trouve en position méta ou para par rapport à l'emplacement de liaison du cycle phényle avec le tricycle, et en position ortho par rapport à R⁶, et signifie hydrogène, halogène, alkyle en C₁-C₆, alcoxy en C₁-C₆ ou un groupe de formule -NR¹¹R¹²,
R¹¹ et R¹² représentant indépendamment l'un de l'autre hydrogène, alkyle en C₁-C₆, phényle, benzyle ou pyridylméthyle, ou formant ensemble avec l'atome d'azote auquel ils sont reliés un hétérocycle de 4 à 7 éléments,
ou
R⁶ et R⁷ forment ensemble avec le cycle phényle auquel ils sont reliés un groupe de formule 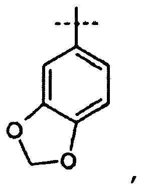 ainsi que leurs sels, solvates et solvates des sels.

5. Composé de formule (I) selon la revendication 4, dans lequel
R¹ signifie hydrogène, benzyloxy, éthoxy, n-propoxy ou un groupe de formule R⁸-C (=O) -NH-, éthoxy pouvant être substitué en position 2 et n-propoxy en position 2 ou 3 par alcoxy en C₁-C₄, amino, mono- ou dialkylamino en C₁-C₄, cycloalkylamino en C₃-C₆, *N*-cycloalkyle en C₃-C₆-*N*-alkylamino en C₁-C₄ ou par un hétérocycle de 4 à 7 éléments relié par un atome N,
mono- et dialkylamino en C₁-C₄ pouvant de leur côté être substitués par hydroxy, alcoxy en C₁-C₄, amino, mono- ou dialkylamino en C₁-C₄,
et
R⁸ représentant un hétéroaryle à 5 ou 6 éléments, qui peut être substitué par alkyle en C₁-C₄ ou halogène,
R² signifie hydrogène, éthoxy ou n-propoxy, éthoxy pouvant être substitué en position 2 et n-propoxy en position 2 ou 3 par alcoxy en C₁-C₄, amino, mono- ou dialkylamino en C₁-C₄, cycloalkylamino en C₃-C₆, N-cycloalkyle en C₃-C₆-*N*-alkylamino en C₁-C₄ ou par un hétérocycle de 4 à 7 éléments relié par un atome N, mono- et dialkylamino en C₁-C₄ pouvant de leur côté être substitués par hydroxy, alcoxy en C₁-C₄, amino, mono- ou dialkylamino en C₁-C₄,
R¹ et R² ne représentant toutefois pas simultanément l'hydrogène,
R³ signifie hydroxy ou amino
et
R⁴ signifie hydrogène
ou
R³ et R⁴ forment ensemble avec l'atome de carbone auquel ils sont reliés un groupe de formule >C=O ou >C=N-OH,
R⁵ signifie hydrogène,
n représente le nombre 0, 1, 2 ou 3,
R⁶ se trouve en position méta ou para, par rapport à l'emplacement de liaison du cycle phényle avec le tricycle, et signifie aryle en C₆-C₁₀, hétéroaryle de 5 à 6 éléments ou un groupe de formule -NR⁹R¹⁰,
aryle et hétéroaryle pouvant de leur côté être substitués chacun une à deux fois, de manière identique ou différente, par halogène, cyano, alkylsulfonyle en C₁-C₄ ou un groupe de formule -NR⁹R¹⁰,
et
R⁹ et R¹⁰ représentant indépendamment l'un de l'autre hydrogène, alkyle en C₁-C₄, phényle, benzyle ou pyridylméthyle, ou formant ensemble avec l'atome d'azote auquel ils sont reliés un hétérocycle de 4 à 7 éléments,
et
R⁷ se trouve en position méta ou para par rapport à l'emplacement de liaison du cycle phényle avec le tricycle, et en position ortho par rapport à R⁶, et signifie hydrogène, halogène, alkyle en C₁-C₄, alcoxy en C₁-C₄ ou un groupe de formule -NR¹¹R¹²,
R¹¹ et R¹² représentant indépendamment l'un de l'autre hydrogène, alkyle en C₁-C₄, phényle, benzyle ou pyridylméthyle, ou formant ensemble avec l'atome d'azote auquel ils sont reliés un hétérocycle de 4 à 7 éléments,
ou
R⁶ et R⁷ forment ensemble avec le cycle phényle auquel ils sont reliés un groupe de formule 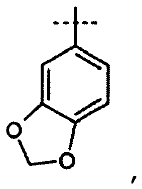 ainsi que leurs sels, solvates et solvates des sels.

6. Composé de formule (I) selon les revendications 4 et 5, dans lequel
R¹ signifie hydrogène, éthoxy, n-propoxy ou un groupe de formule R⁸-C(=O)-NH-, éthoxy pouvant être substitué en position 2 et n-propoxy en position 3 par méthoxy, éthoxy, amino, méthylamino, éthylamino, isopropylamino, diméthylamino, diéthylamino, cyclopropylamino, *N-*cyclopropyl-*N*-méthylamino, azétidino ou pyrrolidino,
R⁸ représentant pyridyle, pyrrolyle, imidazolyle, pyrazolyle, triazolyle ou thiadiazolyle, qui peuvent chacun être substitués par méthyle, éthyle, fluor ou chlore,
R² signifie hydrogène, éthoxy ou n-propoxy, éthoxy pouvant être substitué en position 2 et n-propoxy en position 3 par méthoxy, éthoxy, amino, méthylamino, éthylamino, isopropylamino, diméthylamino, diéthylamino, cyclopropylamino, *N*-cyclopropyl-*N-*méthylamino, azétidino ou pyrrolidino,
R¹ et R² ne représentant toutefois pas simultanément l'hydrogène,
R³ signifie hydroxy ou amino,
R⁴ signifie hydrogène,
R⁵ signifie hydrogène,
n représente le nombre 0 ou 1,
R⁶ se trouve en position méta ou para, par rapport à l'emplacement de liaison du cycle phényle avec le tricycle, et signifie phényle, thiényle, indolyle, quinoxalinyle ou un groupe de formule -NR⁹R¹⁰, phényle, thiényle et indolyle pouvant de leur côté être substitués chacun une à deux fois, de manière identique ou différente, par fluor, chlore, brome, cyano ou amino,
et
R⁹ et R¹⁰ représentant indépendamment l'un de l'autre hydrogène, méthyle, éthyle, benzyle ou pyridylméthyle, ou formant ensemble avec l'atome d'azote auquel ils sont reliés un cycle pyrrolidino,
et
R⁷ signifie hydrogène,
ainsi que leurs sels, solvates et solvates des sels.

7. Composé de formule (I), dans lequel R¹ signifie hydrogène, benzyloxy, éthoxy, n-propoxy ou un groupe de formule R⁸-C(=O)-NH-, éthoxy pouvant être substitué en position 2 et n-propoxy en position 2 ou 3 par alcoxy en C₁-C₆, amino, mono- ou dialkylamino en C₁-C₆, cycloalkylamino en C₃-C₈, *N*-cycloalkyle en C₃-C₈-*N*-alkylamino en C₁-C₆ ou par un hétérocycle de 4 à 7 éléments relié par un atome N,
mono- et dialkylamino en C₁-C₆ pouvant de leur côté être substitués par hydroxy, alcoxy en C₁-C₄, amino, mono- ou dialkylamino en C₁-C₄,
et
R⁸ représentant un hétéroaryle à 5 ou 6 éléments, qui peut être substitué par alkyle en C₁-C₄ ou halogène,
R² signifie hydrogène, éthoxy ou n-propoxy, éthoxy pouvant être substitué en position 2 et n-propoxy en position 2 ou 3 par alcoxy en C₁-C₆, amino, mono- ou dialkylamino en C₁-C₆, cycloalkylamino en C₃-C₈, N-cycloalkyle en C₃-C₈-*N*-alkylamino en C₁-C₆ ou par un hétérocycle de 4 à 7 éléments relié par un atome N, mono- et dialkylamino en C₁-C₆ pouvant de leur côté être substitués par hydroxy, alcoxy en C₁-C₄, amino, mono- ou dialkylamino en C₁-C₄,
R¹ et R² ne représentant toutefois pas simultanément l'hydrogène,
R³ signifie amino
et
R⁴ signifie hydrogène
ou
R³ et R⁴ forment ensemble avec l'atome de carbone auquel ils sont reliés un groupe de formule >C=N-OH,
R⁵ signifie hydrogène,
n représente le nombre 0, 1, 2 ou 3,
R⁶ se trouve en position méta ou para, par rapport à l'emplacement de liaison du cycle phényle avec le tricycle, et signifie halogène, alkyle en C₁-C₆ ou alcoxy en C₁-C₆,
et
R⁷ se trouve en position méta ou para par rapport à l'emplacement de liaison du cycle phényle avec le tricycle, et en position ortho par rapport à R⁶, et signifie hydrogène, halogène, alkyle en C₁-C₆, alcoxy en C₁-C₆ ou un groupe de formule -NR¹¹R¹²,
R¹¹ et R¹² représentant indépendamment l'un de l'autre hydrogène, alkyle en C₁-C₆, phényle, benzyle ou pyridylméthyle, ou formant ensemble avec l'atome d'azote auquel ils sont reliés un hétérocycle de 4 à 7 éléments,
ou
R⁶ et R⁷ forment ensemble avec le cycle phényle auquel ils sont reliés un groupe de formule 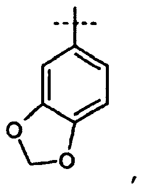 ainsi que leurs sels, solvates et solvates des sels.

8. Composé de formule (I) selon la revendication 7, dans lequel
R¹ signifie hydrogène, benzyloxy, éthoxy, n-propoxy ou un groupe de formule R⁸-C (=O) -NH-, éthoxy pouvant être substitué en position 2 et n-propoxy en position 2 ou 3 par alcoxy en C₁-C₄, amino, mono- ou dialkylamino en C₁-C₄, cycloalkylamino en C₃-C₆, *N*-cycloalkyle en C₃-C₆-*N*-alkylamino en C₁-C₄ ou par un hétérocycle de 4 à 7 éléments relié par un atome N,
mono- et dialkylamino en C₁-C₄ pouvant de leur côté être substitués par hydroxy, alcoxy en C₁-C₄, amino, mono- ou dialkylamino en C₁-C₄,
et
R⁸ représentant un hétéroaryle à 5 ou 6 éléments, qui peut être substitué par alkyle en C₁-C₄ ou halogène,
R² signifie hydrogène, éthoxy ou n-propoxy, éthoxy pouvant être substitué en position 2 et n-propoxy en position 2 ou 3 par alcoxy en C₁-C₄, amino, mono- ou dialkylamino en C₁-C₄, cycloalkylamino en C₃-C₆, *N-*cycloalkyle en C₃-C₆-*N*-alkylamino en C₁-C₄ ou par un hétérocycle de 4 à 7 éléments relié par un atome N, mono- et dialkylamino en C₁-C₄ pouvant de leur côté être substitués par hydroxy, alcoxy en C₁-C₄, amino, mono- ou dialkylamino en C₁-C₄,
R¹ et R² ne représentant toutefois pas simultanément l'hydrogène,
R³ signifie amino
et
R⁴ signifie hydrogène
ou
R³ et R⁴ forment ensemble avec l'atome de carbone auquel ils sont reliés un groupe de formule >C=N-OH,
R⁵ signifie hydrogène,
n représente le nombre 0, 1, 2 ou 3,
R⁶ se trouve en position méta ou para, par rapport à l'emplacement de liaison du cycle phényle avec le tricycle, et signifie halogène, alkyle en C₁-C₄ ou alcoxy en C₁-C₄,
et
R⁷ se trouve en position méta ou para par rapport à l'emplacement de liaison du cycle phényle avec le tricycle, et en position ortho par rapport à R⁶, et signifie hydrogène, halogène, alkyle en C₁-C₄, alcoxy en C₁-C₄ ou un groupe de formule -NR¹¹R¹²,
R¹¹ et R¹² représentant indépendamment l'un de l'autre hydrogène, alkyle en C₁-C₄, phényle, benzyle ou pyridylméthyle, ou formant ensemble avec l'atome d'azote auquel ils sont reliés un hétérocycle de 4 à 7 éléments,
ou
R⁶ et R⁷ forment ensemble avec le cycle phényle auquel ils sont reliés un groupe de formule 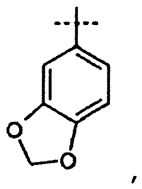 ainsi que leurs sels, solvates et solvates des sels.

9. Composé de formule (I) selon les revendications 7 et 8, dans lequel
R¹ signifie hydrogène, éthoxy, n-propoxy ou un groupe de formule R⁸-C (=O) -NH-, éthoxy pouvant être substitué en position 2 et n-propoxy en position 3 par méthoxy, éthoxy, amino, méthylamino, éthylamino, isopropylamino, diméthylamino, diéthylamino, cyclopropylamino, *N-*cyclopropyl-*N*-méthylamino, azétidino ou pyrrolidino,
R⁸ représentant pyridyle, pyrrolyle, imidazolyle, pyrazolyle, triazolyle ou thiadiazolyle, qui peuvent chacun être substitués par méthyle, éthyle, fluor ou chlore,
R² signifie hydrogène, éthoxy ou n-propoxy, éthoxy pouvant être substitué en position 2 et n-propoxy en position 3 par méthoxy, éthoxy, amino, méthylamino, éthylamino, isopropylamino, diméthylamino, diéthylamino, cyclopropylamino, *N*-cyclopropyl-*N-*méthylamino, azétidino ou pyrrolidino,
R¹ et R² ne représentant toutefois pas simultanément l'hydrogène,
R³ signifie amino,
R⁴ signifie hydrogène,
R⁵ signifie hydrogène,
n représente le nombre 0 ou 1,
R⁶ se trouve en position méta ou para, par rapport à l'emplacement de liaison du cycle phényle avec le tricycle, et signifie fluor, chlore, brome, méthyle, éthyle, méthoxy ou éthoxy,
et
R⁷ se trouve en position méta ou para, par rapport à l'emplacement de liaison du cycle phényle avec le tricycle, et en position ortho par rapport à R⁶, et signifie hydrogène, fluor, chlore, brome, méthyle, éthyle, méthoxy, éthoxy ou un groupe de formule -NR¹¹R¹², R¹¹ et R¹² représentant indépendamment l'un de l'autre hydrogène, méthyle, éthyle, benzyle ou pyridylméthyle, ou formant ensemble avec l'atome d'azote auquel ils sont reliés un cycle pyrrolidino ou pipéridino,
ou
R⁶ et R⁷ forment ensemble avec le cycle phényle auquel ils sont reliés un groupe de formule 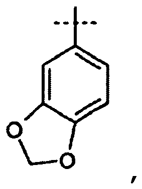 ainsi que leurs sels, solvates et solvates des sels.

10. Composé de formule (I), dans lequel R¹ signifie hydrogène, benzyloxy, éthoxy, n-propoxy ou un groupe de formule R⁸-C(=O)-NH-, éthoxy pouvant être substitué en position 2 et n-propoxy en position 2 ou 3 par alcoxy en C₁-C₆, amino, mono- ou dialkylamino en C₁-C₆, cycloalkylamino en C₃-C₈, *N*-cycloalkyle en C₃-C₈-*N*-alkylamino en C₁-C₆ ou par un hétérocycle de 4 à 7 éléments relié par un atome N,
mono- et dialkylamino en C₁-C₆ pouvant de leur côté être substitués par hydroxy, alcoxy en C₁-C₄, amino, mono- ou dialkylamino en C₁-C₄,
et
R⁸ représentant un hétéroaryle à 5 ou 6 éléments, qui peut être substitué par alkyle en C₁-C₄ ou halogène,
R² signifie hydrogène, éthoxy ou n-propoxy, éthoxy pouvant être substitué en position 2 et n-propoxy en position 2 ou 3 par alcoxy en C₁-C₆, amino, mono- ou dialkylamino en C₁-C₆, cycloalkylamino en C₃-C₈, *N-*cycloalkyle en C₃-C₈-*N*-alkylamino en C₁-C₆ ou par un hétérocycle de 4 à 7 éléments relié par un atome N, mono- et dialkylamino en C₁-C₆ pouvant de leur côté être substitués par hydroxy, alcoxy en C₁-C₄, amino, mono- ou dialkylamino en C₁-C₄,
R¹ ou R² représentant l'hydrogène, mais les deux ne représentant toutefois pas simultanément l'hydrogène,
R³ signifie hydroxy
et
R⁴ signifie hydrogène
ou
R³ et R⁹ forment ensemble avec l'atome de carbone auquel ils sont reliés un groupe de formule >C=O,
R⁵ signifie hydrogène,
n représente le nombre 0, 1, 2 ou 3,
R⁶ se trouve en position méta ou para, par rapport à l'emplacement de liaison du cycle phényle avec le tricycle, et signifie halogène, alkyle en C₁-C₆ ou alcoxy en C₁-C₆,
et
R⁷ se trouve en position méta ou para par rapport à l'emplacement de liaison du cycle phényle avec le tricycle, et en position ortho par rapport à R⁶, et signifie hydrogène, halogène, alkyle en C₁-C₆, alcoxy en C₁-C₆ ou un groupe de formule -NR¹¹R¹²,
R¹¹ et R¹² représentant indépendamment l'un de l'autre hydrogène, alkyle en C₁-C₆, phényle, benzyle ou pyridylméthyle, ou formant ensemble avec l'atome d'azote auquel ils sont reliés un hétérocycle de 4 à 7 éléments,
ou
R⁶ et R⁷ forment ensemble avec le cycle phényle auquel ils sont reliés un groupe de formule 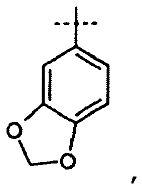 ainsi que leurs sels, solvates et solvates des sels.

11. Composé de formule (I) selon la revendication 10, dans lequel
R¹ signifie hydrogène, benzyloxy, éthoxy, n-propoxy ou un groupe de formule R⁸-C(=O)-NH-, éthoxy pouvant être substitué en position 2 et n-propoxy en position 2 ou 3 par alcoxy en C₁-C₄, amino, mono- ou dialkylamino en C₁-C₄, cycloalkylamino en C₃-C₆, *N*-cycloalkyle en C₃-C₆-*N*-alkylamino en C₁-C₄ ou par un hétérocycle de 4 à 7 éléments relié par un atome N,
mono- et dialkylamino en C₁-C₄ pouvant de leur côté être substitués par hydroxy, alcoxy en C₁-C₄, amino, mono- ou dialkylamino en C₁-C₄,
et
R⁸ représentant un hétéroaryle à 5 ou 6 éléments, qui peut être substitué par alkyle en C₁-C₄ ou halogène,
R² signifie hydrogène, éthoxy ou n-propoxy, éthoxy pouvant être substitué en position 2 et n-propoxy en position 2 ou 3 par alcoxy en C₁-C₄, amino, mono- ou dialkylamino en C₁-C₄, cycloalkylamino en C₃-C₆, N-cycloalkyle en C₃-C₆-*N*-alkylamino en C₁-C₄ ou par un hétérocycle de 4 à 7 éléments relié par un atome N, mono- et dialkylamino en C₁-C₄ pouvant de leur côté être substitués par hydroxy, alcoxy en C₁-C₄, amino, mono- ou dialkylamino en C₁-C₄,
R¹ ou R² représentant l'hydrogène, mais les deux ne représentant toutefois pas simultanément l'hydrogène,
R³ signifie hydroxy
et
R⁴ signifie hydrogène
ou
R³ et R⁴ forment ensemble avec l'atome de carbone auquel ils sont reliés un groupe de formule >C=O,
R⁵ signifie hydrogène,
n représente le nombre 0, 1, 2 ou 3,
R⁶ se trouve en position méta ou para, par rapport à l'emplacement de liaison du cycle phényle avec le tricycle, et signifie halogène, alkyle en C₁-C₄ ou alcoxy en C₁-C₄,
et
R⁷ se trouve en position méta ou para par rapport à l'emplacement de liaison du cycle phényle avec le tricycle, et en position ortho par rapport à R⁶, et signifie hydrogène, halogène, alkyle en C₁-C₄, alcoxy en C₁-C₄ ou un groupe de formule -NR¹¹R¹²,
R¹¹ et R¹² représentant indépendamment l'un de l'autre hydrogène, alkyle en C₁-C₄, phényle, benzyle ou pyridylméthyle, ou formant ensemble avec l'atome d'azote auquel ils sont reliés un hétérocycle de 4 à 7 éléments,
ou
R⁶ et R⁷ forment ensemble avec le cycle phényle auquel ils sont reliés un groupe de formule 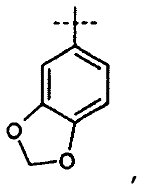 ainsi que leurs sels, solvates et solvates des sels.

12. Composé de formule (I) selon les revendications 10 et 11, dans lequel
R¹ signifie hydrogène, éthoxy, n-propoxy ou un groupe de formule R^{B}-C (=O) -NH-, éthoxy pouvant être substitué en position 2 et n-propoxy en position 3 par méthoxy, éthoxy, amino, méthylamino, éthylamino, isopropylamino, diméthylamino, diéthylamino, cyclopropylamino, *N-*cyclopropyl-*N*-méthylamino, azétidino ou pyrrolidino,
R⁸ représentant pyridyle, pyrrolyle, imidazolyle, pyrazolyle, triazolyle ou thiadiazolyle, qui peuvent chacun être substitués par méthyle, éthyle, fluor ou chlore,
R² signifie hydrogène, éthoxy ou n-propoxy, éthoxy pouvant être substitué en position 2 et n-propoxy en position 3 par méthoxy, éthoxy, amino, méthylamino, éthylamino, isopropylamino, diméthylamino, diéthylamino, cyclopropylamino, *N*-cyclopropyl-*N-*méthylamino, azétidino ou pyrrolidino,
R¹ ou R² représentant l'hydrogène, mais les deux ne représentant toutefois pas simultanément l'hydrogène,
R³ signifie hydroxy
et
R⁴ signifie hydrogène
ou
R³ et R⁴ forment ensemble avec l'atome de carbone auquel ils sont reliés un groupe de formule >C=O,
R⁵ signifie hydrogène,
n représente le nombre 0 ou 1,
R⁶ se trouve en position méta ou para, par rapport à l'emplacement de liaison du cycle phényle avec le tricycle, et signifie fluor, chlore, brome, méthyle, éthyle, méthoxy ou éthoxy,
et
R⁷ se trouve en position méta ou para par rapport à l'emplacement de liaison du cycle phényle avec le tricycle, et en position ortho par rapport à R⁶, et signifie hydrogène, fluor, chlore, brome, méthyle, éthyle, méthoxy, éthoxy ou un groupe de formule -NR¹¹R¹², R¹¹ et R¹² représentant indépendamment l'un de l'autre hydrogène, méthyle, éthyle, benzyle ou pyridylméthyle, ou formant ensemble avec l'atome d'azote auquel ils sont reliés un cycle pyrrolidino ou pipéridino,
ou
R⁶ et R⁷ forment ensemble avec le cycle phényle auquel ils sont reliés un groupe de formule 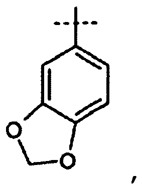 ainsi que leurs sels, solvates et solvates des sels.

13. Composé de formule (I), dans lequel R¹ signifie éthoxy, n-propoxy ou un groupe de formule R⁸-C(=O)-NH-, éthoxy pouvant être substitué en position 2 et n-propoxy en position 2 ou 3 par alcoxy en C₁-C₆, amino, mono- ou dialkylamino en C₁-C₆, cycloalkylamino en C₃-C₈, *N*-cycloalkyle en C₃-C₈-*N-*alkylamino en C₁-C₆ ou par un hétérocycle de 4 à 7 éléments relié par un atome N,
mono- et dialkylamino en C₁-C₆ pouvant de leur côté être substitués par hydroxy, alcoxy en C₁-C₄, amino, mono- ou dialkylamino en C₁-C₄,
et
R⁸ représentant un hétéroaryle à 5 ou 6 éléments, qui peut être substitué par alkyle en C₁-C₄ ou halogène,
R² signifie éthoxy ou n-propoxy, éthoxy pouvant être substitué en position 2 et n-propoxy en position 2 ou 3 par alcoxy en C₁-C₆, amino, mono- ou dialkylamino en C₁-C₆, cycloalkylamino en C₃-C₈, *N*-cycloalkyle en C₃-C₈-*N-*alkylamino en C₁-C₆ ou par un hétérocycle de 4 à 7 éléments relié par un atome N,
mono- et dialkylamino en C₁-C₆ pouvant de leur côté être substitués par hydroxy, alcoxy en C₁-C₄, amino, mono- ou dialkylamino en C₁-C₄,
R³ signifie hydroxy
et
R⁴ signifie hydrogène
ou
R³ et R⁴ forment ensemble avec l'atome de carbone auquel ils sont reliés un groupe de formule >C=O,
R⁵ signifie hydrogène,
n représente le nombre 0, 1, 2 ou 3,
R⁶ se trouve en position méta ou para, par rapport à l'emplacement de liaison du cycle phényle avec le tricycle, et signifie halogène, alkyle en C₁-C₆ ou alcoxy en C₁-C₆,
et
R⁷ se trouve en position méta ou para par rapport à l'emplacement de liaison du cycle phényle avec le tricycle, et en position ortho par rapport à R⁶, et signifie hydrogène, halogène, alkyle en C₁-C₆, alcoxy en C₁-C₆ ou un groupe de formule -NR¹¹R¹²,
R¹¹ et R¹² représentant indépendamment l'un de l'autre hydrogène, alkyle en C₁-C₆, phényle, benzyle ou pyridylméthyle, ou formant ensemble avec l'atome d'azote auquel ils sont reliés un hétérocycle de 4 à 7 éléments,
ou
R⁶ et R⁷ forment ensemble avec le cycle phényle auquel ils sont reliés un groupe de formule 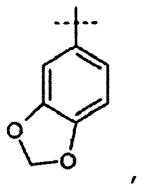 ainsi que leurs sels, solvates et solvates des sels.

14. Composé de formule (I) selon la revendication 13, dans lequel
R¹ signifie éthoxy, n-propoxy ou un groupe de formule R⁸-C(=O)-NH-, éthoxy pouvant être substitué en position 2 et n-propoxy en position 2 ou 3 par alcoxy en C₁-C₄, amino, mono- ou dialkylamino en C₁-C₄, cycloalkylamino en C₃-C₆, *N*-cycloalkyle en C₃-C₆-*N-*alkylamino en C₁-C₄ ou par un hétérocycle de 4 à 7 éléments relié par un atome N,
mono- et dialkylamino en C₁-C₄ pouvant de leur côté être substitués par hydroxy, alcoxy en C₁-C₄, amino, mono- ou dialkylamino en C₁-C₄,
et
R⁸ représentant un hétéroaryle à 5 ou 6 éléments, qui peut être substitué par alkyle en C₁-C₄ ou halogène,
R² signifie éthoxy ou n-propoxy, éthoxy pouvant être substitué en position 2 et n-propoxy en position 2 ou 3 par alcoxy en C₁-C₄, amino, mono- ou dialkylamino en C₁-C₄, cycloalkylamino en C₃-C₆, N-cycloalkyle en C₃-C₆-*N-*alkylamino en C₁-C₄ ou par un hétérocycle de 4 à 7 éléments relié par un atome N,
mono- et dialkylamino en C₁-C₄ pouvant de leur côté être substitués par hydroxy, alcoxy en C₁-C₄, amino, mono- ou dialkylamino en C₁-C₄,
R³ signifie hydroxy
et
R⁴ signifie hydrogène
ou
R³ et R⁴ forment ensemble avec l'atome de carbone auquel ils sont reliés un groupe de formule >C=O,
R⁵ signifie hydrogène,
n représente le nombre 0, 1, 2 ou 3,
R⁶ se trouve en position méta ou para, par rapport à l'emplacement de liaison du cycle phényle avec le tricycle, et signifie halogène, alkyle en C₁-C₄ ou alcoxy en C₁-C₄,
et
R⁷ se trouve en position méta ou para par rapport à l'emplacement de liaison du cycle phényle avec le tricycle, et en position ortho par rapport à R⁶, et signifie hydrogène, halogène, alkyle en C₁-C₄, alcoxy en C₁-C₄ ou un groupe de formule -NR¹¹R¹²,
R¹¹ et R¹² représentant indépendamment l'un de l'autre hydrogène, alkyle en C₁-C₄, phényle, benzyle ou pyridylméthyle, ou formant ensemble avec l'atome d'azote auquel ils sont reliés un hétérocycle de 4 à 7 éléments,
ou
R⁶ et R⁷ forment ensemble avec le cycle phényle auquel ils sont reliés un groupe de formule 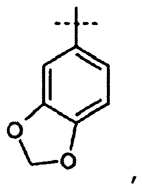 ainsi que leurs sels, solvates et solvates des sels.

15. Composé de formule (I) selon les revendications 13 et 14, dans lequel
R¹ signifie éthoxy, n-propoxy ou un groupe de formule R⁸-C(=O)-NH-, éthoxy pouvant être substitué en position 2 et n-propoxy en position 3 par méthoxy, éthoxy, amino, méthylamino, éthylamino, isopropylamino, diméthylamino, diéthylamino, cyclopropylamino, *N-*cyclopropyl-*N*-méthylamino, azétidino ou pyrrolidino,
R⁸ représentant pyridyle, pyrrolyle, imidazolyle, pyrazolyle, triazolyle ou thiadiazolyle, qui peuvent chacun être substitués par méthyle, éthyle, fluor ou chlore,
R² signifie éthoxy ou n-propoxy, éthoxy pouvant être substitué en position 2 et n-propoxy en position 3 par méthoxy, éthoxy, amino, méthylamino, éthylamino, isopropylamino, diméthylamino, diéthylamino, cyclopropylamino, *N*-cyclopropyl-*N*-méthylamino, azétidino ou pyrrolidino,
R³ signifie hydroxy
et
R⁴ signifie hydrogène
ou
R³ et R⁴ forment ensemble avec l'atome de carbone auquel ils sont reliés un groupe de formule >C=O,
R⁵ signifie hydrogène,
n représente le nombre 0 ou 1,
R⁶ se trouve en position méta ou para, par rapport à l'emplacement de liaison du cycle phényle avec le tricycle, et signifie fluor, chlore, brome, méthyle, éthyle, méthoxy ou éthoxy,
et
R⁷ se trouve en position méta ou para par rapport à l'emplacement de liaison du cycle phényle avec le tricycle, et en position ortho par rapport à R⁶, et signifie hydrogène, fluor, chlore, brome, méthyle, éthyle, méthoxy, éthoxy ou un groupe de formule -NR¹¹R¹², R¹¹ et R¹² représentant indépendamment l'un de l'autre hydrogène, méthyle, éthyle, benzyle ou pyridylméthyle, ou formant ensemble avec l'atome d'azote auquel ils sont reliés un cycle pyrrolidino ou pipéridino,
ou
R⁶ et R⁷ forment ensemble avec le cycle phényle auquel ils sont reliés un groupe de formule 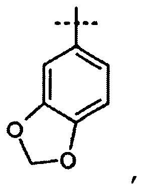 ainsi que leurs sels, solvates et solvates des sels.

16. Procédé de fabrication d'un composé de formule (VI) 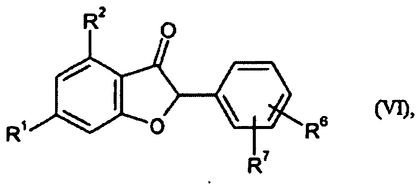 dans laquelle R¹, R², R⁶ et R⁷ ont chacun la signification donnée dans les revendications 1 à 15, **caractérisé en ce que**
[A] des composés de formule (II)
dans laquelle R¹ et R² ont chacun la signification donnée dans les revendications 1 à 15,
sont mis en réaction dans un solvant inerte en présence d'une base avec un composé de formule (III) 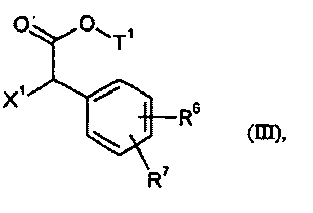 dans laquelle R⁶ et R⁷ ont chacun la signification donnée dans les revendications 1 à 15,
X¹ représente un groupe partant approprié tel que par exempla halogène, mésylate, tosylate ou triflate, et
T¹ représente un alkyle en C₁-C₄, pour former des composés de formule (IV) 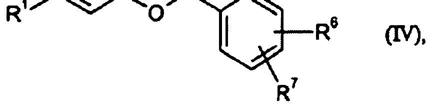 R¹, R², R⁶, R⁷ et T¹ ont chacun la signification donnée dans les revendications 1 à 15,
puis, transformés par hydrolyse basique ou acide en acides carboxyliques de formule (V) 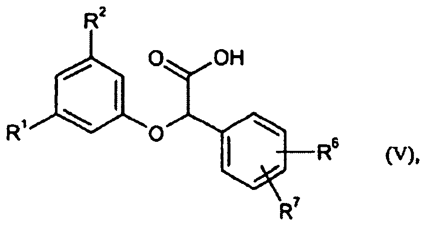 dans laquelle R¹, R², R⁶ et R⁷ ont chacun la signification donnée dans les revendications 1 à 15, puis ceux-ci sont cyclisés après activation avec du chlorure de phosphoryle en présence d'un acide de Lewis pour former des composés de formule (VI) 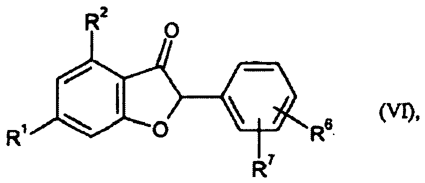 dans laquelle R¹, R², R⁶ et R⁷ ont chacun la signification donnée dans les revendications 1 à 15,
ou
[B] des composés de formule (VII) 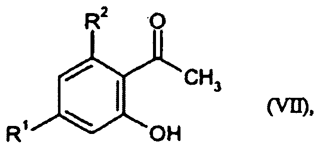
dans laquelle R¹ et R² ont chacun la signification donnée dans les revendications 1 à 15,
sont tout d'abord transformés par des procédés usuels en bromures de phénacyle de formule (VIII) 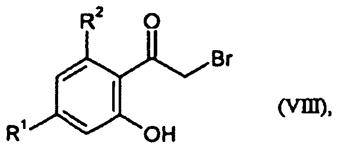 dans laquelle R¹ et R² ont chacun la signification donnée dans les revendications 1 à 15,
puis ceux-ci sont cyclisés en présence d'une base pour former des composés de formule (IX) 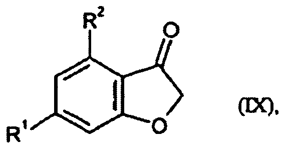 dans laquelle R¹ et R² ont chacun la signification donnée dans les revendications 1 à 15,
puis bromés dans un solvant inerte pour former des composés de formule (X) 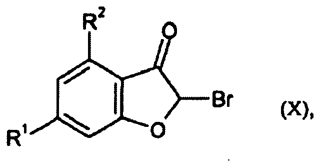 dans laquelle R¹ et R² ont chacun la signification donnée dans les revendications 1 à 15,
et transformés par des procédés usuels en éthers de silylénol de formule (XI) 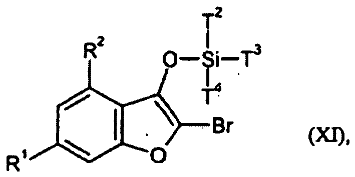 dans laquelle R¹ et R² ont chacun la signification donnée dans les revendications 1 à 15,
et
T², T³ et T⁴ sont identiques ou différents et représentent chacun alkyle en C₁-C₄ ou phényle,
puis mis en réaction dans un solvant inerte en présence d'un catalyseur de palladium approprié et d'une base avec un composé de formule (XII) 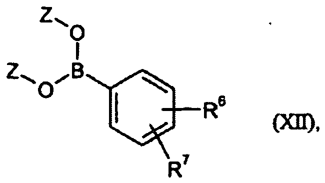 dans laquelle R⁶ et R⁷ ont chacun la signification donnée dans les revendications 1 à 15,
et
Z représente hydrogène ou méthyle, ou les deux groupes Z forment ensemble un pont CH₂CH₂ ou C(CH₃)₂-C (CH₃) ₂,
pour former des composés de formule (XIII) 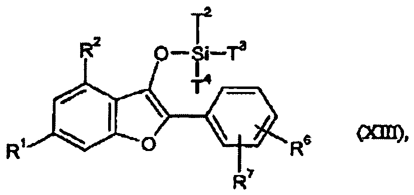 dans laquelle R¹, R², R⁶, R⁷, T², T³ et T⁴ ont chacun la signification donnée dans les revendications 1 à 15, puis le groupe silyle est clivé par des procédés usuels pour former des composés de formule (VI).

17. Composé, tel que défini dans l'une quelconque des revendications 1 à 15, pour le traitement et/ou la prophylaxie de maladies.

18. Utilisation d'un composé, tel que défini dans l'une quelconque des revendications 1 à 15, pour la fabrication d'un médicament pour la prophylaxie et/ou le traitement de maladies inflammatoires et auto-immunes, de maladies neurodégénératives et de maladies hyperprolifératives.

19. Médicament contenant un composé tel que défini dans l'une quelconque des revendications 1 à 15, en combinaison avec un adjuvant inerte, non toxique, pharmaceutiquement approprié.

20. Médicament selon la revendication 19, pour la prophylaxie et/ou le traitement de maladies inflammatoires et auto-immunes, de maladies neurodégénératives et de maladies hyperprolifératives.
